# EUROPEAN PATENT APPLICATION

(11) **EP 4 606 886 A1**
(43) Date of publication of application: **27.08.2025**
(21) Application number: 23879012.5
(22) Date of filing: 12.10.2023
(51) Int. Cl.: C12N 5/00, C07K 16/00, A61K 39/395

(54) **ANTIBODY COMBINATION SPECIFICALLY BINDING TO TRAIL OR FASL, AND BISPECIFIC ANTIBODY**

(30) Priority: 20.10.2022 WO PCT/CN2022/126336
(71) Applicant: Beijing SoloBio Genetechnology Company Ltd., Beijing 100176 (CN)
(72) Inventor: WANG, Guangfei, Beijing 100176 (CN); LI, Zhong, Beijing 100176 (CN); LI, Ping, Beijing 100176 (CN)
(74) Representative: Schmitz, Joseph
(86) International application number: PCT/CN2023/124152
(87) International publication number: WO 2024/083021

(57) **Abstract**

An antibody specifically binding to TRAIL, a bispecific antibody specifically binding to TRAIL and FasL, and a pharmaceutical composition comprising the antibody specifically binding to TRAIL and an antibody and/or bispecific antibody specifically binding to FasL, and a preparation method therefor and a use thereof.

## Description

### CROSS REFERENCE TO RELATED APPLICATIONS

This application claims priority to PCT Application No.PCT/CN2022/126336, filed on October 20, 2022 and entitled "COMBINATION OF ANTIBODY AND BISPECIFIC ANTIBODY SPECIFICALLY BINDING TO TRAIL OR FASL", the contents of which are incorporated herein by reference in their entirety.

### REFERENCE TO AN ELECTRONIC SEQUENCE LISTING

The contents of the electronic sequence listing (file name: CN_20220714_SEQLIST.xml, date recorded: 2023.09.14, size: 219 KB) is herein incorporated by reference in its entirety.

### FIELD OF THE APPLICATION

This application pertains to antibody specifically binding to TRAIL, bispecific antibody specifically binding to TRAIL and FasL, and pharmaceutical compositions comprising an antibody specifically binding TRAIL and an antibody specifically binding FasL and/or bispecific antibody, as well as methods of manufacture and uses thereof.

### BACKGROUND OF THE APPLICATION

Tumor necrosis factor (TNF)-related apoptosis-inducing ligand (TRAIL) is a type II transmembrane protein of the TNF superfamily, and it can be cleaved from the cell surface by cysteine proteases and matrix-metalloproteinase-2 (MMP-2) to produce a soluble form that retains the proapoptotic activity (Wiley, S R et al. Immunity vol. 3,6 (1995): 673-82.; Mariani, S M, and P H Krammer. European journal of immunology vol. 28,3 (1998): 973-82.). TRAIL is constitutively present in a variety of tissues, but it is mainly expressed in nature killer (NK) cells and macrophages (Wang, S. Current medicinal chemistry vol. 17,29 (2010): 3309-17.).

TRAIL has five different receptors: TRAIL-R1, TRAIL-R2, DcR1, DcR2 and osteoprotegerin. Wherein, TRAIL-R1 (also known as DR4) and TRAIL-R2 (also known as DR5) contain a cytoplasmic region known as death domain that enables the receptors to initiate cytotoxic signal when engaged by cognate ligands. DcR1 do not contain death domain, DcR2 do not contain an intact death domain, and osteoprotegerin is described as a TRAIL soluble receptor. These three receptors can function as decoy receptors to prevent apoptosis by sequestering TRAIL from binding to its proapoptotic receptors or interrupting the death receptor-mediated death signal (Walczak, H, and P H Krammer. Experimental cell research vol. 256,1 (2000): 58-66. ). DR4 is generally expressed in most of the human tissues including spleen, thymus, liver, peripheral blood leukocytes, activated T cells and small intestine. DR5 expression exerts a ubiquitous distribution in both normal and tumor tissues, but is particularly abundant in spleen, peripheral blood leukocytes, and activated lymphocytes (Wang, S. Current medicinal chemistry vol. 17,29 (2010): 3309-17.).

TRAIL binding to death receptors DR4 or DR5 induces their trimerisation and recruitment of Fas-associated protein(FADD) and membrane-proximal caspases (caspase 8 or 10) to form a receptor complex named the death-inducing signaling complex (DISC). Caspases are synthesised as inactive pro-enzymes and recruitment to the DISC induces their activation and then activated caspases directly cleave the downstream effector caspases (caspase 3, 6, and 7) leading to their activation. The activated caspase 3 in turn cleaves numerous cellular proteins leading to apoptosis (Ashkenazi, A, and V M Dixit. Science (New York, N.Y.) vol. 281,5381 (1998): 1305-8.). In type I cells, relatively large amount of DISC are rapidly assembled and internalized, which generate enough caspase-8 (or 10) activation and signaling to promote cell death. In contrast, the DISC formation is delayed and less effective in type II cells. Therefore, activation of caspase 8 in type II cells is not enough to activate apoptosis. The execution of apoptosis needs the engagement of mitochondria through caspase 8-mediated BH3 interacting-domain death agonist (Bid) cleavage to generate a truncated form of Bid(tBid), which in turn induces the activation of Bax and Bak in mitochondria. The activated Bax and Bak promote the loss of mitochondrial membrane potential and release of cytochrome c, leading to apoptosis (Barnhart, Bryan C et al. Seminars in immunology vol. 15,3 (2003): 185-93.; Kohlhaas, Susan L et al. The Journal of biological chemistry vol. 282,17 (2007): 12831-41.).

Apart from inducing cell death, TRAIL signaling also promotes cell development, survival, and proliferation via activation of nuclear factor-κB (NF-κB), MAP kinases(MAPK), and Akt(Lin, Y et al. Molecular and cellular biology vol. 20,18 (2000): 6638-45.; Lee, Tae-Jin et al. Biochemical and biophysical research communications vol. 351,4 (2006): 1024-30.). For example, TRAIL can trigger the formation of a cytosolic complex retaining FAS-associated death domain (FADD), TNF receptor associated factor 2 (TRAF2) and NF-κB essential modulator (NEMO), which may result in the activation of other signaling pathways, including NF-κB and MAPK activation, and the production of pro-inflammatory cytokines and chemokines (von Karstedt, Silvia et al. Nature reviews. Cancer vol. 17,6 (2017): 352-366.).

Fas Ligand (FasL) is also a type II transmembrane protein of the TNF superfamily and shares 28% homology with TRAIL (Rossin, Aurélie et al. Cancers vol. 11,5 639. 8 May. 2019, doi:10.3390/cancers11050639). Binding of FasL to its death receptor and initiates a similar cascade as TRAIL does, recruiting FADD and caspase-8 or caspase-10 to form a receptor complex and signaling to promote cell death. In type II cells, mitochondria-dependent pathway involving Bid cleavage and cytochrome c release also can be activated to induce apoptosis (Yin, Xiao-Ming, and Wen-Xing Ding. Current molecular medicine vol. 3,6 (2003): 491-508.).

Studies have shown that FASL and TRAIL, as ligands of death receptors, are associated with the occurrence and development of a variety of diseases. Yin et al. show that Death receptor-induced hepatocyte apoptosis contributes to the development of a number of liver diseases, including viral hepatitis, inflammatory hepatitis, Wilson's disease, alcoholic liver disease, endotoxiemia-induced liver failure and ischemia/reperfusion-induced liver damage(Yin, Xiao-Ming, and Wen-Xing Ding. Current molecular medicine vol. 3,6 (2003): 491-508.). Ochi et al. show that hepatocyte toxicity of liver NK cells was inhibited partially by an anti-TRAIL monoclonal antibody alone and completely by the combination with anti-FasL mAb and a perforin inhibitor (Ochi, Makoto et al. Hepatology (Baltimore, Md.) vol. 39,5 (2004): 1321-31.). Zhang et al. show that TRAIL and FasL can participate in cell mediated cytotoxicity via their death domain-mediated apoptotic signaling in the host-versus-graft disease occurred after renal transplantation (Zhang, Yun et al. Immunology letters vol. 152,1 (2013): 1-7.). Tasew et al. show that FasL and TRAIL expressing cells are present in dermis in ulcerative cutaneous leishmaniasis(CL) and inhibition of FasL and TRAIL reduce Leishmaniasis induced skin ulceration (Tasew, Geremew et al. PLoS neglected tropical diseases vol. 4,10 e844. 12 Oct. 2010). Therefore, the development of new antibody that bind to TRAIL or FasL, or bispecific antibody that bind to both TRAIL and FasL, as well as pharmaceutical compositions that include antibody that bind TRAIL and/or FasL, is of critical importance in the field of medicine.

At present, there have been reports the inhibitors targeting FasL or TRAIL, such as the anti-FASL antibody 119-4A (used as a control antibody in the embodiments of this application) and the uses thereof has been disclosed in Chinese patent CN108290950B; The fusion protein sDR5-Fc, which has the function of blocking TRAIL-DR5 signaling pathway, has been disclosed in the Chinese patent CN108997503B (as a contrast in the embodiments of this application). However, there are not yet monoclonal antibody drug targeting TRAIL and bispecific antibody drugs targeting TRAIL and FasL on the market, so it is an urgent to develop monoclonal antibody targeting TRAIL and bispecific antibody targeting TRAIL and FasL.

The disclosures of all publications, patents, patent applications and published patent applications referred to herein are hereby incorporated herein by reference in their entirety.

### BRIEF SUMMARY OF THE APPLICATION

In one aspect, the present application provides an isolated antibody or antigen-binding fragment specifically binding to TRAIL. In some embodiments, the present application provides an isolated antibody or antigen-binding fragment specifically binding to TRAIL comprising: a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22.

In some embodiments, there is provided an isolated anti-TRAIL antibody or antigen-binding fragment comprising: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs.

In some embodiments, there is provided an isolated anti-TRAIL antibody or antigen-binding fragment comprising: a V_{H} comprising the amino acid sequence of any one of SEQ ID NOs: 13-14, or a variant thereof having at least about 80% sequence identity to the amino acid sequence of any one of SEQ ID NOs: 13-14; and a V_{L} comprising the amino acid sequence of any one of SEQ ID NOs: 22-27, or a variant thereof having at least about 80% sequence identity to the amino acid sequence of any one of SEQ ID NOs: 22-27.

In some embodiments, there is provided an isolated anti-TRAIL antibody or antigen-binding fragment comprising: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27.

In some embodiments, the present application provides an isolated antibody or antigen-binding fragment specifically binding to TRAIL comprising: a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, there is provided an isolated anti-TRAIL antibody or antigen-binding fragment comprising: (i) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

In some embodiments, there is provided an isolated anti-TRAIL antibody or antigen-binding fragment comprising: a V_{H} comprising the amino acid sequence of any one of SEQ ID NOs: 15-21, or a variant thereof having at least about 80% sequence identity to the amino acid sequence of any one of SEQ ID NOs: 15-21; and a V_{L} comprising the amino acid sequence of any one of SEQ ID NOs: 28-32, or a variant thereof having at least about 80% sequence identity to the amino acid sequence of any one of SEQ ID NOs: 28-32.

In some embodiments, there is provided an isolated anti-TRAIL antibody or antigen-binding fragment comprising: (i) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; (ii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29; (iii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29; (iv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29; (v) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29; (vi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29; (vii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30; (viii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30; (ix) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30; (x) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30; (xi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; (xii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; (xiii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31; (xiv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31; (xv) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31; (xvi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or (xvii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

In some embodiments, there is provided an isolated an antibody or antigen-binding fragment specifically binding to TRAIL, which competitively with any one of the isolated antibodies described above for specifically binding to TRAIL. In some embodiments, there is provided an isolated antibody or antigen-binding fragment specifically binding to TRAIL, which specifically binds to the same epitope as any one of isolated antibodies described above.

In some embodiments, according to any of the isolated antibodies described above specifically binding to TRAIL, the isolated antibody comprises an Fc region. In some embodiments, the isolated antibody specifically binding to TRAIL is a full-length IgG antibody. In some embodiments, the isolated antibody specifically binding to TRAIL is a full-length IgG1, IgG2, IgG3, or IgG4 antibody. In some embodiments, the antibody specifically binding to TRAIL is a chimeric, human, or humanized antibody. In some embodiments, the antibody specifically binding to TRAIL is an antigen-binding fragment selected from the group consisting of a Fab, a Fab', a F(ab)'₂, a Fab'-SH, a single-chain Fv (scFv), an Fv fragment, a dAb, an Fd, a nanobody, a diabody, and a linear antibody.

In some embodiments, there is provided isolated nucleic acid molecule(s) that encodes any one of the antibodies or antigen-binding fragments described above specifically binding to TRAIL. In some embodiments, there is provided a vector comprising any one of the nucleic acid molecules described above. In some embodiments, there is provided a host cell comprising any one of the antibodies or antigen-binding fragments described above specifically binding to TRAIL, any one of the nucleic acid molecules described above, or any one of the vectors described above. In some embodiments, there is provided a method of producing an antibody or antigen-binding fragment specifically binding to TRAIL, comprising: a) culturing any one of the host cells described above under conditions effective to express the antibody or antigen-binding fragment specifically binding to TRAIL; and b) obtaining the expressed antibody from the host cell.

In some embodiments, there is provided pharmaceutical compositions, kits and articles of manufacture comprising any one of the antibodies or antigen-binding fragments described above specifically binding to TRAIL.

In some embodiments, there is provided a method of treating a disease or condition in an individual in need thereof, comprising administering to the individual an effective amount of any one of the antibodies or antigen-binding fragments described above specifically binding to TRAIL, or a pharmaceutical composition containing it. In some embodiments, there is provided the use of any one of the anti-TRAIL antibodies or antigen-binding fragments described above in the preparation of pharmaceutical compositions for treating a disease or condition in an individual in need. In some embodiments, provided is the use of any one of the anti-TRAIL antibodies or antigen-binding fragments described above, or a pharmaceutical composition comprising any one of anti-TRAIL antibodies or antigen-binding fragments described above in the manufacture of a medicament for treating a disease or condition. In some embodiments, the disease or condition is associated with TRAIL signaling pathway, comprising inflammatory diseases, autoimmune diseases, transplant-related diseases, liver diseases, neurodegenerative disorders or cancer. In some embodiments, the disease or condition is selected from the group consisting of transplant rejection, graft-versus-host disorders, liver injury, pulmonary arterial hypertension, Alzheimer's disease, systemic inflammatory response syndrome, sepsis, multiple organ dysfunction syndrome, trauma, multiple sclerosis, idiopathic pulmonary fibrosis, osteoarthritis, inflammatory bowel disease, Crohn's disease, ulcerative colitis, acute lung injury, acute respiratory distress syndrome, myocardial infarction, cardiomyopathy, ischemic reperfusion injury, diabetes, brain injury, spinal cord injury, acute viral hepatitis B, acute viral hepatitis C, chronic hepatitis C, chronic Hepatitis B, alcoholic hepatitis, non-alcoholic steatohepatitis, cirrhosis, drug-induced liver injury/liver failure, autoimmune hepatitis, chronic kidney disease, acute kidney disease, diabetic kidney disease, and cancer.

On the other hand, this application provides bispecific antibodies specifically binding TRAIL and FasL, and pharmaceutical compositions that contain bispecific antibodies specifically binding TRAIL and FasL. In some embodiments, the application also provides methods for the prevention and/or treatment of diseases associated with TRAIL and/or FasL signaling pathways using the said bispecific antibodies or their pharmaceutical compositions. In other embodiments, the application also provides for the use of said bispecific antibodies or their pharmaceutical compositions in the manufacture of a medicament for preventing and/or treating diseases associated with TRAIL and/or FasL signaling pathways.

In some embodiments, this application provides a bispecific antibody, comprising a first antigen-binding domain specifically binding to TRAIL, and a second antigen-binding domain specifically binding to FasL, wherein the first antigen-binding domain comprises: (i) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; or (ii) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; or (iii) a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12. In some embodiments, the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, this application provides a bispecific antibody, comprising a first antigen-binding domain specifically binding to TRAIL, and a second antigen-binding domain specifically binding to FasL, wherein the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, according to any one of bispecific antibodies described in this application, wherein the first antigen-binding domain comprises: (i) a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (ii) a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28; or (iii) a V_{H} comprising an HC-CDR1, an HC-CDR2, and an HC-CDR3 of a V_{H} comprising the amino acid sequence of SEQ ID NO: 164; and a V_{L} comprising an LC-CDR1, an LC-CDR2, and an LC-CDR3 of a V_{L} comprising the amino acid sequence of SEQ ID NO: 168; or (iii) a V_{H} comprising an HC-CDR1, an HC-CDR2, and an HC-CDR3 of a V_{H} comprising the amino acid sequence of SEQ ID NO: 166; and a V_{L} comprising an LC-CDR1, an LC-CDR2, and an LC-CDR3 of a V_{L} comprising the amino acid sequence of SEQ ID NO: 170.

In some embodiments, according to any one of bispecific antibodies described in this application, wherein the first antigen-binding domain comprises: a V_{H} comprising the amino acid sequence of any one of SEQ ID NOs: 13-20, 33-40, 164 and 166, or a variant thereof having at least about 80% sequence identity to the amino acid sequence of any one of SEQ ID NOs: 13-20, 33-40, 164 and 166; and a V_{L} comprising the amino acid sequence of any one of SEQ ID NOs: 22-31, 41-50, 168 and 170, or a variant thereof having at least about 80% sequence identity to the amino acid sequence of any one of SEQ ID NOs: 22-31, 41-50, 168 and 170.

In some embodiments, according to any one of bispecific antibodies described in this application, wherein the first antigen-binding domain comprises: (i)a V_{H} comprising the amino acid sequence of any one of SEQ ID NOs: 13 and 33, or a variant thereof having at least about 80% sequence identity to the amino acid sequence of any one of SEQ ID NOs: 13 and 33; and a V_{L} comprising the amino acid sequence of any one of SEQ ID NOs: 22 and 41, or a variant thereof having at least about 80% sequence identity to the amino acid sequence of any one of SEQ ID NOs: 22 and 41; (ii)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42; (iii)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43; (iv)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 25 and 44, or a variant thereof having at least about 80% sequence identity to the amino acid sequence of any one of SEQ ID NOs: 25 and 44; (v)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 26 and 45, or a variant thereof having at least about 80% sequence identity to the amino acid sequence of any one of SEQ ID NOs: 26 and 45; (vi)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 27 and 46, or a variant thereof having at least about 80% sequence identity to the amino acid sequence of any one of SEQ ID NOs: 27 and 46; (vii)a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47; (viii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (ix)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (x)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xiii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xiv)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xv)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xvi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xvii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xviii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xix)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xx)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxiii) a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; or (xxiv) a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170.

In some embodiments, according to any one of bispecific antibodies described in this application, wherein the second antigen-binding domain comprises: (i)a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73; or (ii)a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76; or (iii)a V_{H} comprising an HC-CDR1, an HC-CDR2, and an HC-CDR3 of a V_{H} comprising the amino acid sequence of SEQ ID NO: 163; and a V_{L} comprising an LC-CDR1, an LC-CDR2, and an LC-CDR3 of a V_{L} comprising the amino acid sequence of SEQ ID NO: 167; or (iv)a V_{H} comprising an HC-CDR1, an HC-CDR2, and an HC-CDR3 of a V_{H} comprising the amino acid sequence of SEQ ID NO: 165; and a V_{L} comprising an LC-CDR1, an LC-CDR2, and an LC-CDR3 of a V_{L} comprising the amino acid sequence of SEQ ID NO: 169.

In some embodiments, according to any one of bispecific antibodies described in this application, wherein the second antigen-binding domain comprises: a V_{H} comprising the amino acid sequence of any one of SEQ ID NOs: 63-72, 80-88, 163 and 165, or a variant thereof having at least about 80% sequence identity to the amino acid sequence of any one of SEQ ID NOs: 63-72, 80-88, 163 and 165; and a V_{L} comprising the amino acid sequence of any one of SEQ ID NOs: 73-79, 89-95, 167 and 169, or a variant thereof having at least about 80% sequence identity to the amino acid sequence of any one of SEQ ID NOs: 73-79, 89-95, 167 and 169.

In some embodiments, according to any one of bispecific antibodies described in this application, wherein the second antigen-binding domain comprises: (i)a V_{H} comprising the amino acid sequence of any one of SEQ ID NOs: 63 and 80, or a variant thereof having at least about 80% sequence identity to the amino acid sequence of any one of SEQ ID NOs: 63 and 80; and a V_{L} comprising the amino acid sequence of any one of SEQ ID NOs: 73 and 89, or a variant thereof having at least about 80% sequence identity to the amino acid sequence of any one of SEQ ID NOs: 73 and 89; (ii)a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90; (iii)a V_{H} comprising the amino acid sequence of any one of SEQ ID NOs: 65 and 81, or a variant thereof having at least about 80% sequence identity to the amino acid sequence of any one of SEQ ID NOs: 65 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90; (iv)a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 75 and 91, or a variant thereof having at least about 80% sequence identity to the amino acid sequence of any one of SEQ ID NOs: 75 and 91; (v)a V_{H} comprising the amino acid sequence of any one of SEQ ID NOs: 65 and 81, or a variant thereof having at least about 80% sequence identity to the amino acid sequence of any one of SEQ ID NOs: 65 and 81; and a V_{L} comprising the amino acid sequence of any one of SEQ ID NOs: 75 and 91, or a variant thereof having at least about 80% sequence identity to the amino acid sequence of any one of SEQ ID NOs: 75 and 91; (vi)a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92; (vii)a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (viii)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (ix)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (x)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xi)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xii)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xiii) a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xiv)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xv)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xvi)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xvii)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xviii)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xix) a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xx)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxi)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxii)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxiii)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxiv)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxv) a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167; or (xxvi) a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

In some embodiments, according to any one of bispecific antibodies described in this application, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

In some embodiments, according to any one of bispecific antibodies described in this application, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, according to any one of bispecific antibodies described in this application, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

In some embodiments, according to any one of bispecific antibodies described in this application, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, according to any one of bispecific antibodies described in this application, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, according to any one of bispecific antibodies described in this application, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, according to any one of bispecific antibodies described in this application, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, according to any one of bispecific antibodies described in this application, wherein the first antigen-binding domain comprises: a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, according to any one of bispecific antibodies described in this application, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, according to any one of bispecific antibodies described in this application, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95.

In some embodiments, according to any one of bispecific antibodies described in this application, wherein the first antigen-binding domain comprises: a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; and wherein the second antigen-binding domain comprises: a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167.

In some embodiments, according to any one of bispecific antibodies described in this application, wherein the first antigen-binding domain comprises: a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170; and wherein the second antigen-binding domain comprises: a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

In some embodiments, the bispecific antibody described in this application comprises an Fc region. In other embodiments, the Fc region described in this application is selected from the group consisting of an Fc region from an IgGl, IgG2, IgG3, IgG4, IgA, IgM, IgE, and IgD. In some embodiments, the Fc region comprises a variant Fc region. In some embodiments, the Fc region is glycosylated. In some embodiments, the Fc region is deglycosylated. In some embodiments, the Fc region has reduced fucosylation or is afucosylated. In some embodiments, the variant Fc region comprises a substitution at one or more of positions 239, 282, 289, 297, 312, 324, 330, 335, 337, 339, 356, 359, 361, 383, 384, 398, 400, 440, 422, and 442, as numbered by the EU numbering system. In some embodiments, the variant Fc region comprises a substitution at position 297. In some embodiments, the substitution at position 297 is 297Q.

In some embodiments, the bispecific antibody has a DVD-Ig format. In some embodiments, the bispecific antibody comprises four polypeptide chains:
Two polypeptide chains contain V_{H}1-L-V_{H}2-C_{H}1 structure from the N-terminal to the C-terminal, in which V_{H}1 is the heavy chain variable domain specifically binding to TRAIL, V_{H}2 is the heavy chain variable domain specifically binding to FasL; L is a peptide linker; C_{H}1 is the heavy chain constant domain 1; wherein the polypeptide chain further comprises Fc, which comprises the C_{H}2 and C_{H}3 domains;
The other two polypeptide chains contain the structure of V_{L}1-L-V_{L}2-C_{L}, in which V_{L}1 is the light chain variable domain specifically binding to TRAIL; V_{L}2 is a light chain variable domain specifically binding to FasL; L is a peptide linker; C_{L} is the light chain constant domain;
Wherein V_{H}1 and V_{L}1 formed the antigen-binding domain (Fv) specifically binding to TRAIL, and V_{H} 2-C_{H}1 and V_{L}2-C_{L} formed the antigen-binding domain (Fab) specifically binding to FasL.

In other embodiments, the bispecific antibody comprises four polypeptide chains:
Two polypeptide chains contain the structure of V_{H}1-L-V_{H}2-C_{H}1, in which V_{H}1 is the heavy chain variable domain specifically binding to FasL; V_{H}2 is the heavy chain variable domain specifically binding to TRAIL; L is a peptide linker; C_{H}1 is the heavy chain constant domain 1; wherein the polypeptide chain further comprises Fc, which comprises the C_{H}2 and C_{H}3 domains;
The other two polypeptide chains contain the structure of V_{L}1-L-V_{L}2-C_{L}, in which V_{L}1 is the light chain variable domain specifically binding to FasL; V_{L}2 is a light chain variable domain specifically binding to TRAIL; L is a peptide linker; C_{L} is the light chain constant domain;
Wherein V_{H}1 and V_{L}1 formed the antigen-binding domain (Fv) specifically binding to FasL, and V_{H} 2-C_{H}1 and V_{L}2-C_{L} formed the antigen-binding domain (Fab) specifically binding to TRAIL.

In some embodiments, the bispecific antibody described in this application comprises: (a) the amino acid sequence of SEQ ID NO: 104, or a variant thereof having at least about 80% sequence identity to the amino acid sequence of SEQ ID NO: 104; and/or the amino acid sequence of SEQ ID NO: 105, or a variant thereof having at least about 80% sequence identity to the amino acid sequence of SEQ ID NO: 105; (b) the amino acid sequence of SEQ ID NO: 104, or a variant thereof having at least about 80% sequence identity to the amino acid sequence of SEQ ID NO: 104; and/or the amino acid sequence of SEQ ID NO: 106, or a variant thereof having at least about 80% sequence identity to the amino acid sequence of SEQ ID NO: 106; (c) the amino acid sequence of SEQ ID NO: 107, or a variant thereof having at least about 80% sequence identity to the amino acid sequence of SEQ ID NO: 107; and/or the amino acid sequence of SEQ ID NO: 108, or a variant thereof having at least about 80% sequence identity to the amino acid sequence of SEQ ID NO: 108; (d) the amino acid sequence of SEQ ID NO: 107, or a variant thereof having at least about 80% sequence identity to the amino acid sequence of SEQ ID NO: 107; and/or the amino acid sequence of SEQ ID NO: 109, or a variant thereof having at least about 80% sequence identity to the amino acid sequence of SEQ ID NO: 109; (e) the amino acid sequence of SEQ ID NO: 110, or a variant thereof having at least about 80% sequence identity to the amino acid sequence of SEQ ID NO: 110; and/or the amino acid sequence of SEQ ID NO: 111, or a variant thereof having at least about 80% sequence identity to the amino acid sequence of SEQ ID NO: 111; or (f) the amino acid sequence of SEQ ID NO: 172, or a variant thereof having at least about 80% sequence identity to the amino acid sequence of SEQ ID NO: 172; and/or the amino acid sequence of SEQ ID NO: 174, or a variant thereof having at least about 80% sequence identity to the amino acid sequence of SEQ ID NO: 174.

In some embodiments, the bispecific antibody described in this application comprises: (a) the amino acid sequence of SEQ ID NO: 131, or a variant thereof having at least about 80% sequence identity to the amino acid sequence of SEQ ID NO: 131; and/or the amino acid sequence of SEQ ID NO: 105, or a variant thereof having at least about 80% sequence identity to the amino acid sequence of SEQ ID NO: 105; (b) the amino acid sequence of SEQ ID NO: 131, or a variant thereof having at least about 80% sequence identity to the amino acid sequence of SEQ ID NO: 131; and/or the amino acid sequence of SEQ ID NO: 106, or a variant thereof having at least about 80% sequence identity to the amino acid sequence of SEQ ID NO: 106; (c) the amino acid sequence of SEQ ID NO: 132, or a variant thereof having at least about 80% sequence identity to the amino acid sequence of SEQ ID NO: 132; and/or the amino acid sequence of SEQ ID NO: 108, or a variant thereof having at least about 80% sequence identity to the amino acid sequence of SEQ ID NO: 108; (d) the amino acid sequence of SEQ ID NO: 132, or a variant thereof having at least about 80% sequence identity to the amino acid sequence of SEQ ID NO: 132; and/or the amino acid sequence of SEQ ID NO: 109, or a variant thereof having at least about 80% sequence identity to the amino acid sequence of SEQ ID NO: 109; (e) the amino acid sequence of SEQ ID NO: 133, or a variant thereof having at least about 80% sequence identity to the amino acid sequence of SEQ ID NO: 133; and/or the amino acid sequence of SEQ ID NO: 111, or a variant thereof having at least about 80% sequence identity to the amino acid sequence of SEQ ID NO: 111; or (f) the amino acid sequence of SEQ ID NO: 176, or a variant thereof having at least about 80% sequence identity to the amino acid sequence of SEQ ID NO: 176; and/or the amino acid sequence of SEQ ID NO: 174, or a variant thereof having at least about 80% sequence identity to the amino acid sequence of SEQ ID NO: 174.

In some embodiments, the bispecific antibody has a Bs4Ab format. In some embodiments, the bispecific antibody comprises four polypeptide chains:
Two polypeptide chains contain V_{H}1-C_{H}1-L1-V_{H}2-L3-V_{L}2 or V_{H}1-C_{H}1-L1-V_{L}2-L3-V_{H}2 structure from the N-terminal to the C-terminal, in which V_{H}1 is the heavy chain variable domain specifically binding to TRAIL; V_{H}2 is the heavy chain variable domain specifically binding to FasL, V_{L}2 is the light chain variable domain specifically binding to FasL; L1 and L3 are peptide linkers; C_{H}1 is the heavy chain constant domain 1; wherein the polypeptide chain further comprises Fc, which comprises the C_{H}2 and C_{H}3 domains; and
The other two polypeptide chains contain V_{L}1-C_{L} structure from the N-terminal to the C-terminal, in which V_{L}1 is the light chain variable domain specifically binding to TRAIL, C_{L} is the light chain constant domain.

Wherein V_{H}1 and V_{L}1 formed the antigen-binding domain (Fab) specifically binding to TRAIL, and V_{H}2-L3-V_{L}2 or V_{L}2-L3-V_{H}2 formed the antigen-binding domain (scFv) specifically binding to FasL.

In some embodiments, the bispecific antibody comprises four polypeptide chains:
Two polypeptide chains contain the structure of V_{H}1-C_{H}1-L1-V_{H}2-L3-V_{L}2 or V_{H}1-C_{H}1-L1-V_{L}2-L3-V_{H}2, in which V_{H}1 is the heavy chain variable domain specifically binding to FasL; V_{H}2 is the heavy chain variable domain specifically binding to TRAIL; V_{L}2 is the light chain variable domain specifically binding to TRAIL; L1 and L3 are peptide linkers; C_{H}1 is the heavy chain constant domain 1; wherein the polypeptide chain further comprises Fc, which comprises the C_{H}2 and C_{H}3 domains; and
The other two polypeptide chains contain V_{L}1-C_{L} structure from the N-terminal to the C-terminal, in which V_{L}1 is the light chain variable domain specifically binding to FasL, C_{L} is the light chain constant domain.

Wherein V_{H}1 and V_{L}1 formed the antigen-binding domain (Fab) specifically binding to FasL, and V_{H}2-L3-V_{L}2 or V_{L}2-L3-V_{H}2 formed the antigen-binding domain (scFv) specifically binding to TRAIL.

In some embodiments, the bispecific antibody comprises: (a) the amino acid sequence of SEQ ID NO: 112, or a variant thereof having at least about 80% sequence identity to the amino acid sequence of SEQ ID NO: 112; and/or the amino acid sequence of SEQ ID NO: 113, or a variant thereof having at least about 80% sequence identity to the amino acid sequence of SEQ ID NO: 113; (b) the amino acid sequence of SEQ ID NO: 114, or a variant thereof having at least about 80% sequence identity to the amino acid sequence of SEQ ID NO: 114; and/or the amino acid sequence of SEQ ID NO: 115, or a variant thereof having at least about 80% sequence identity to the amino acid sequence of SEQ ID NO: 115; (c) the amino acid sequence of SEQ ID NO: 116, or a variant thereof having at least about 80% sequence identity to the amino acid sequence of SEQ ID NO: 116; and/or the amino acid sequence of SEQ ID NO: 119, or a variant thereof having at least about 80% sequence identity to the amino acid sequence of SEQ ID NO: 119; (d) the amino acid sequence of SEQ ID NO: 117, or a variant thereof having at least about 80% sequence identity to the amino acid sequence of SEQ ID NO: 117; and/or the amino acid sequence of SEQ ID NO: 119, or a variant thereof having at least about 80% sequence identity to the amino acid sequence of SEQ ID NO: 119; (e) the amino acid sequence of SEQ ID NO: 118, or a variant thereof having at least about 80% sequence identity to the amino acid sequence of SEQ ID NO: 118; and/or the amino acid sequence of SEQ ID NO: 119, or a variant thereof having at least about 80% sequence identity to the amino acid sequence of SEQ ID NO: 119; (f) the amino acid sequence of SEQ ID NO: 120, or a variant thereof having at least about 80% sequence identity to the amino acid sequence of SEQ ID NO: 120; and/or the amino acid sequence of SEQ ID NO: 121, or a variant thereof having at least about 80% sequence identity to the amino acid sequence of SEQ ID NO: 121; (g) the amino acid sequence of SEQ ID NO: 120, or a variant thereof having at least about 80% sequence identity to the amino acid sequence of SEQ ID NO: 120; and/or the amino acid sequence of SEQ ID NO: 122, or a variant thereof having at least about 80% sequence identity to the amino acid sequence of SEQ ID NO: 122; (h) the amino acid sequence of SEQ ID NO: 123, or a variant thereof having at least about 80% sequence identity to the amino acid sequence of SEQ ID NO: 123; and/or the amino acid sequence of SEQ ID NO: 124, or a variant thereof having at least about 80% sequence identity to the amino acid sequence of SEQ ID NO: 124; or (i) the amino acid sequence of SEQ ID NO: 171, or a variant thereof having at least about 80% sequence identity to the amino acid sequence of SEQ ID NO: 171; and/or the amino acid sequence of SEQ ID NO: 173, or a variant thereof having at least about 80% sequence identity to the amino acid sequence of SEQ ID NO: 173.

In some embodiments, the bispecific antibody comprises: (a) the amino acid sequence of SEQ ID NO: 134, or a variant thereof having at least about 80% sequence identity to the amino acid sequence of SEQ ID NO: 134; and/or the amino acid sequence of SEQ ID NO: 113, or a variant thereof having at least about 80% sequence identity to the amino acid sequence of SEQ ID NO: 113; (b) the amino acid sequence of SEQ ID NO: 135, or a variant thereof having at least about 80% sequence identity to the amino acid sequence of SEQ ID NO: 135; and/or the amino acid sequence of SEQ ID NO: 113, or a variant thereof having at least about 80% sequence identity to the amino acid sequence of SEQ ID NO: 113; (c) the amino acid sequence of SEQ ID NO: 136, or a variant thereof having at least about 80% sequence identity to the amino acid sequence of SEQ ID NO: 136; and/or the amino acid sequence of SEQ ID NO: 115, or a variant thereof having at least about 80% sequence identity to the amino acid sequence of SEQ ID NO: 115; (d) the amino acid sequence of SEQ ID NO: 137, or a variant thereof having at least about 80% sequence identity to the amino acid sequence of SEQ ID NO: 137; and/or the amino acid sequence of SEQ ID NO: 115, or a variant thereof having at least about 80% sequence identity to the amino acid sequence of SEQ ID NO: 115; (e) the amino acid sequence of SEQ ID NO: 138, or a variant thereof having at least about 80% sequence identity to the amino acid sequence of SEQ ID NO: 138; and/or the amino acid sequence of SEQ ID NO: 119, or a variant thereof having at least about 80% sequence identity to the amino acid sequence of SEQ ID NO: 119; (f) the amino acid sequence of SEQ ID NO: 139, or a variant thereof having at least about 80% sequence identity to the amino acid sequence of SEQ ID NO: 139; and/or the amino acid sequence of SEQ ID NO: 119, or a variant thereof having at least about 80% sequence identity to the amino acid sequence of SEQ ID NO: 119; (g) the amino acid sequence of SEQ ID NO: 140, or a variant thereof having at least about 80% sequence identity to the amino acid sequence of SEQ ID NO: 140; and/or the amino acid sequence of SEQ ID NO: 119, or a variant thereof having at least about 80% sequence identity to the amino acid sequence of SEQ ID NO: 119; (h) the amino acid sequence of SEQ ID NO: 141, or a variant thereof having at least about 80% sequence identity to the amino acid sequence of SEQ ID NO: 141; and/or the amino acid sequence of SEQ ID NO: 121, or a variant thereof having at least about 80% sequence identity to the amino acid sequence of SEQ ID NO: 121; (i) the amino acid sequence of SEQ ID NO: 141, or a variant thereof having at least about 80% sequence identity to the amino acid sequence of SEQ ID NO: 141; and/or the amino acid sequence of SEQ ID NO: 122, or a variant thereof having at least about 80% sequence identity to the amino acid sequence of SEQ ID NO: 122; (j) the amino acid sequence of SEQ ID NO: 142, or a variant thereof having at least about 80% sequence identity to the amino acid sequence of SEQ ID NO: 142; and/or the amino acid sequence of SEQ ID NO: 124, or a variant thereof having at least about 80% sequence identity to the amino acid sequence of SEQ ID NO: 124; or (k) the amino acid sequence of SEQ ID NO: 175, or a variant thereof having at least about 80% sequence identity to the amino acid sequence of SEQ ID NO: 175; and/or the amino acid sequence of SEQ ID NO: 173, or a variant thereof having at least about 80% sequence identity to the amino acid sequence of SEQ ID NO: 173.

In some embodiments, the bispecific antibody described in this application has a Hetero H, CrossMab format. In some embodiments, the bispecific antibody comprises four polypeptide chains:
One polypeptide chain contains V_{H}1-C_{H}1 structure from the N-terminal to the C-terminal, in which V_{H}1 is the heavy chain variable domain specifically binding to TRAIL, C_{H}1 is the heavy chain constant domain 1; wherein the polypeptide chain further comprises Fc, which comprises the C_{H}2 and C_{H}3 domains; and
One polypeptide chain contains V_{L}1-C_{L} structure from the N-terminal to the C-terminal, in which V_{L}1 is the light chain variable domain specifically binding to TRAIL, C_{L} is the light chain constant domain; and
One polypeptide chain contains V_{H}2-C_{L} structure from the N-terminal to the C-terminal, in which V_{H}2 is the heavy chain variable domain specifically binding to FasL, C_{L} is the light chain constant domain; and
One polypeptide chain contains V_{L}2-C_{H}1 structure from the N-terminal to the C-terminal, in which V_{L}2 is the light chain variable domain specifically binding to FasL, C_{H}1 is the heavy chain constant domain 1.

Wherein V_{H}1-C_{H}1 and V_{L}1-C_{L} formed the antigen-binding domain (Fab) specifically binding to TRAIL, and V_{H}2-C_{L} and V_{L}2-C_{H}1 formed the antigen-binding domain (Fab) specifically binding to FasL.

In some embodiments, the bispecific antibody comprises four polypeptide chains:
One polypeptide chain contains V_{H}1-C_{H}1 structure from the N-terminal to the C-terminal, in which V_{H}1 is the heavy chain variable domain specifically binding to FasL, C_{H}1 is the heavy chain constant domain; wherein the polypeptide chain further comprises Fc, which comprises the C_{H}2 and C_{H}3 domains; and
One polypeptide chain contains V_{L}1-C_{L} structure from the N-terminal to the C-terminal, in which V_{L}1 is the light chain variable domain specifically binding to FasL, C_{L} is the light chain constant domain; and
One polypeptide chain contains V_{H}2-C_{L} structure from the N-terminal to the C-terminal, in which V_{H}2 is the heavy chain variable domain specifically binding to TRAIL, C_{L} is the light chain constant domain; and
One polypeptide chain contains V_{L}2-C_{H}1 structure from the N-terminal to the C-terminal, in which V_{L}2 is the light chain variable domain specifically binding to TRAIL, C_{H}1 is the heavy chain constant domain 1.

Wherein V_{H}1-C_{H}1 and V_{L}1-C_{L} formed the antigen-binding domain (Fab) specifically binding to FasL, and V_{H}2-C_{L} and V_{L}2-C_{H}1 formed the antigen-binding domain (Fab) specifically binding to TRAIL.

In some embodiments, the bispecific antibody comprises: (a) the amino acid sequence of SEQ ID NO: 125, or a variant thereof having at least about 80% sequence identity to the amino acid sequence of SEQ ID NO: 125; and/or the amino acid sequence of SEQ ID NO: 126, or a variant thereof having at least about 80% sequence identity to the amino acid sequence of SEQ ID NO: 126; and/or the amino acid sequence of SEQ ID NO: 127, or a variant thereof having at least about 80% sequence identity to the amino acid sequence of SEQ ID NO: 127; and/or the amino acid sequence of SEQ ID NO: 129 or a variant thereof having at least about 80% sequence identity to the amino acid sequence of SEQ ID NO: 129; or (b) the amino acid sequence of SEQ ID NO: 125, or a variant thereof having at least about 80% sequence identity to the amino acid sequence of SEQ ID NO: 125; and/or the amino acid sequence of SEQ ID NO: 130, or a variant thereof having at least about 80% sequence identity to the amino acid sequence of SEQ ID NO: 130; and/or the amino acid sequence of SEQ ID NO: 128, or a variant thereof having at least about 80% sequence identity to the amino acid sequence of SEQ ID NO: 128; and/or the amino acid sequence of SEQ ID NO: 129 or a variant thereof having at least about 80% sequence identity to the amino acid sequence of SEQ ID NO: 129.

In some embodiments, the bispecific antibody comprises: (a) the amino acid sequence of SEQ ID NO: 143, or a variant thereof having at least about 80% sequence identity to the amino acid sequence of SEQ ID NO: 143; and/or the amino acid sequence of SEQ ID NO: 126, or a variant thereof having at least about 80% sequence identity to the amino acid sequence of SEQ ID NO: 126; and/or the amino acid sequence of SEQ ID NO: 144, or a variant thereof having at least about 80% sequence identity to the amino acid sequence of SEQ ID NO: 144; and/or the amino acid sequence of SEQ ID NO: 129 or a variant thereof having at least about 80% sequence identity to the amino acid sequence of SEQ ID NO: 129; or (b) the amino acid sequence of SEQ ID NO: 145, or a variant thereof having at least about 80% sequence identity to the amino acid sequence of SEQ ID NO: 145; and/or the amino acid sequence of SEQ ID NO: 130, or a variant thereof having at least about 80% sequence identity to the amino acid sequence of SEQ ID NO: 130; and/or the amino acid sequence of SEQ ID NO: 146, or a variant thereof having at least about 80% sequence identity to the amino acid sequence of SEQ ID NO: 146; and/or the amino acid sequence of SEQ ID NO: 129 or a variant thereof having at least about 80% sequence identity to the amino acid sequence of SEQ ID NO: 129.

In some embodiments, there is provided a method of treating a disease or condition in an individual in need thereof, comprising administering to the individual an effective amount of any one of the bispecific antibodies described above or a pharmaceutical composition containing it. In some embodiments, there is provided the use of any one of the bispecific antibodies described above in the preparation of pharmaceutical compositions for treating a disease or condition in an individual in need. In some embodiments, provided is the use of any one of the bispecific antibodies described above or a pharmaceutical composition containing it in the manufacture of a medicament for treating a disease or condition. In some embodiments, the disease or condition is associated with TRAIL and/or FasL signaling pathway, comprising inflammatory diseases, autoimmune diseases, transplant-related diseases, liver diseases, neurodegenerative disorders or cancer. In some embodiments, the disease or condition is selected from the group consisting of transplant rejection, graft-versus-host disorders, liver injury, systemic inflammatory response syndrome, sepsis, multiple organ dysfunction syndrome, trauma, multiple sclerosis, idiopathic pulmonary fibrosis, osteoarthritis, inflammatory bowel disease, Crohn's disease, ulcerative colitis, acute lung injury, acute respiratory distress syndrome, myocardial infarction, cardiomyopathy, ischemic reperfusion injury, diabetes, brain injury, spinal cord injury, acute viral hepatitis B, acute viral hepatitis C, chronic hepatitis C, chronic Hepatitis B, alcoholic hepatitis, non-alcoholic steatohepatitis, cirrhosis, drug-induced liver injury/liver failure, autoimmune hepatitis, chronic kidney disease, acute kidney disease, diabetic kidney disease, and cancer.

In some embodiments, there is provided isolated nucleic acid molecule(s) that encodes any one of the bispecific antibodies described above. In some embodiments, there is provided a vector comprising any one of the nucleic acid molecules described above. In some embodiments, there is provided a host cell comprising any one of bispecific antibodies described above, any one of the nucleic acid molecules described above, or any one of the vectors described above. In some embodiments, there is provided a method of producing a bispecific antibody specifically binding TRAIL and FasL comprising: a) culturing any one of the host cells described above under conditions effective to express the bispecific antibody specifically binding TRAIL and FasL; and b) obtaining the expressed bispecific antibody from the host cell.

In some embodiments, there is provided pharmaceutical compositions, kits and articles of manufacture comprising any one of the bispecific antibodies, nucleic acid molecules, vectors, or host cells described above.

In one aspect, this application provides a pharmaceutical composition comprising: (i) an antibody or antigen-binding fragment specifically binding to TRAIL, and (ii) an antibody or antigen-binding fragment specifically binding to FasL.

In one aspect, there is provided a method of treating a disease or condition in an individual in need thereof, comprising administering to the individual an effective amount of (i) an antibody or antigen-binding fragment specifically binding to TRAIL, and (ii) an antibody or antigen-binding fragment specifically binding to FasL, or a pharmaceutical composition comprising the antibody or antigen-binding fragment specifically binding to TRAIL, and the antibody or antigen-binding fragment specifically binding to FasL.

On the other hand, there is provided the use of the antibody or antigen-binding fragment specifically binding to TRAIL and the antibody or antigen-binding fragment specifically binding to FasL in the preparation of pharmaceutical compositions for treating a disease or condition in an individual in need. In some embodiments, provided is the use of the antibody or antigen-binding fragment specifically binding to TRAIL, and the antibody or antigen-binding fragment specifically binding to FasL or a pharmaceutical composition containing the antibody or antigen-binding fragment specifically binding to TRAIL, and the antibody or antigen-binding fragment specifically binding to FasL in the manufacture of a medicament for treating a disease or condition.

In some embodiments, in the pharmaceutical compositions, methods or uses described herein, the antibody or antigen-binding fragment specifically binding to TRAIL comprises: (a) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or (b) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

In some embodiments, in the pharmaceutical compositions, methods or uses described herein, the antibody or antigen-binding fragment specifically binding to FasL comprises: (a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or (b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the pharmaceutical compositions, methods or uses described herein, the antibody or antigen-binding fragment specifically binding to TRAIL comprises: (a) a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (b) a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, in the pharmaceutical compositions, methods or uses described herein, the antibody or antigen-binding fragment specifically binding to TRAIL comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27. (vii) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; (viii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29; (ix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29; (x) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29; (xi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29; (xii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29; (xiii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30; (xiv) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30; (xv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30; (xvi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30; (xvii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; (xviii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; (xix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31; (xx) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31; (xxi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31; (xxii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or (xxiii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

In some embodiments, in the pharmaceutical compositions, methods or uses described herein, the antibody or antigen-binding fragment specifically binding to FasL comprises: (a) a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73; or (b) a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76.

In some embodiments, in the pharmaceutical compositions, methods or uses described herein, the antibody or antigen-binding fragment specifically binding to FasL comprises: (i) a V_{H} comprising the amino acid sequence of SEQ ID NO: 63, or a variant thereof having at least about 80% sequence identity to the amino acid sequence of SEQ ID NO:63; and a V_{L} comprising the amino acid sequence of SEQ ID NO: 73, or a variant thereof having at least about 80% sequence identity to the amino acid sequence of SEQ ID NO: 73; (ii) a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74; (iii) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 74, or a variant thereof having at least about 80% sequence identity to the amino acid sequence of SEQ ID NO: 74; (iv) a V_{H} comprising the amino acid sequence of SEQ ID NO: 64, or a variant thereof having at least about 80% sequence identity to the amino acid sequence of SEQ ID NO: 64; and a V_{L} comprising the amino acid sequence of SEQ ID NO: 75, or a variant thereof having at least about 80% sequence identity to the amino acid sequence of SEQ ID NO: 75; (v) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 75, or a variant thereof having at least about 80% sequence identity to the amino acid sequence of SEQ ID NO: 75; (vi) a V_{H} comprising the amino acid sequence of SEQ ID NO:66, or a variant thereof having at least about 80% sequence identity to the amino acid sequence of SEQ ID NO: 66; and a V_{L} comprising the amino acid sequence of SEQ ID NO: 76, or a variant thereof having at least about 80% sequence identity to the amino acid sequence of SEQ ID NO: 76. (vii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 77, or a variant thereof having at least about 80% sequence identity to the amino acid sequence of SEQ ID NO: 77; (viii) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 77, or a variant thereof having at least about 80% sequence identity to the amino acid sequence of SEQ ID NO: 77; (ix) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 77, or a variant thereof having at least about 80% sequence identity to the amino acid sequence of SEQ ID NO: 77; (x) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 77, or a variant thereof having at least about 80% sequence identity to the amino acid sequence of SEQ ID NO: 77; (xi) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 77, or a variant thereof having at least about 80% sequence identity to the amino acid sequence of SEQ ID NO: 77; (xii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 77, or a variant thereof having at least about 80% sequence identity to the amino acid sequence of SEQ ID NO: 77; (xiii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 78, or a variant thereof having at least about 80% sequence identity to the amino acid sequence of SEQ ID NO: 78; (xiv) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 78, or a variant thereof having at least about 80% sequence identity to the amino acid sequence of SEQ ID NO: 78; (xv) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 78, or a variant thereof having at least about 80% sequence identity to the amino acid sequence of SEQ ID NO: 78; (xvi) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78; (xvii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 78, or a variant thereof having at least about 80% sequence identity to the amino acid sequence of SEQ ID NO: 78; (xviii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 78, or a variant thereof having at least about 80% sequence identity to the amino acid sequence of SEQ ID NO: 78; (xix) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 79, or a variant thereof having at least about 80% sequence identity to the amino acid sequence of SEQ ID NO: 79; (xx) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 79, or a variant thereof having at least about 80% sequence identity to the amino acid sequence of SEQ ID NO: 79; (xxi) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 79, or a variant thereof having at least about 80% sequence identity to the amino acid sequence of SEQ ID NO: 79; (xxii) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 79, or a variant thereof having at least about 80% sequence identity to the amino acid sequence of SEQ ID NO: 79; (xxiii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 79, or a variant thereof having at least about 80% sequence identity to the amino acid sequence of SEQ ID NO: 79; or (xxiv) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 79, or a variant thereof having at least about 80% sequence identity to the amino acid sequence of SEQ ID NO: 79.

In some embodiments, in the pharmaceutical compositions, methods or uses described herein, the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, in the pharmaceutical compositions, methods or uses described herein, the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, in the pharmaceutical compositions, methods or uses described herein, the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the pharmaceutical compositions, methods or uses described herein, the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the pharmaceutical compositions, methods or uses described herein, the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, in the pharmaceutical compositions, methods or uses described herein, the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, in the pharmaceutical compositions, methods or uses described herein, the antibody or antigen-binding fragment specifically binding to TRAIL comprises: a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, in the pharmaceutical compositions, methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, in the pharmaceutical compositions, methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78.

In some embodiments, in the pharmaceutical compositions, methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 69, or a variant thereof having at least about 80% sequence identity to the amino acid sequence of SEQ ID NO:69; and a V_{L} comprising the amino acid sequence of SEQ ID NO: 79, or a variant thereof having at least about 80% sequence identity to the amino acid sequence of SEQ ID NO: 79.

In some embodiments, in the methods or uses described herein, the antibody or antigen-binding fragment specifically binding to TRAIL and the antibody or antigen-binding fragment specifically binding to FasL are administered concurrently. In some embodiments, the antibody or antigen-binding fragment specifically binding to TRAIL and the antibody or antigen-binding fragment specifically binding to FasL are administered consecutively.

In some embodiments, in the pharmaceutical compositions, methods or uses described herein, the ratio by molar mass of the antibody or antigen-binding fragment specifically binding to TRAIL and the antibody or antigen-binding fragment specifically binding to FasL is about 5:1, 4:1, 3:1, 2:1, 1:1, 1:2, 1:3, 1:4 or 1:5. In other embodiments, the ratio by molar mass of the antibody or antigen-binding fragment specifically binding to TRAIL and the antibody or antigen-binding fragment specifically binding to FasL is about 2:1 or 1:1.

In some embodiments, there are provided methods of treating and/or preventing a disease or condition in an individual in need thereof, comprising administering to the individual an effective amount of any one of the antibodies or antigen-binding fragments and/or bispecific antibodies and/or any one of pharmaceutical compositions described herein.

In some embodiments, according to any of the methods or uses described herein, the disease or condition is associated with TRAIL and/or FasL signaling pathway, comprising inflammatory diseases, autoimmune diseases, transplant-related diseases, liver diseases, neurodegenerative disorders or cancer. In some embodiments, the disease or condition is selected from the group consisting of transplant rejection, graft-versus-host disorders, liver injury, pulmonary arterial hypertension, Alzheimer's disease, systemic inflammatory response syndrome, sepsis, multiple organ dysfunction syndrome, trauma, multiple sclerosis, idiopathic pulmonary fibrosis, osteoarthritis, inflammatory bowel disease, Crohn's disease, ulcerative colitis, acute lung injury, acute respiratory distress syndrome, myocardial infarction, cardiomyopathy, ischemic reperfusion injury, diabetes, brain injury, spinal cord injury, acute viral hepatitis B, acute viral hepatitis C, chronic hepatitis C, chronic Hepatitis B, alcoholic hepatitis, non-alcoholic steatohepatitis, cirrhosis, drug-induced liver injury/liver failure, autoimmune hepatitis, chronic kidney disease, acute kidney disease, diabetic kidney disease, and cancer.

### BRIEF DESCRIPTION OF THE DRAWINGS

FIG.1 shows the results of humanized antibodies G10-1, G10-2, G72-10 and G72-11 inhibiting PBMC killing of HepG2 cells.
FIG.2 shows the results of humanized antibodies G10-1, G10-2, G72-10 and G72-11 inhibiting granulocyte killing of HepG2 cells.
FIG.3 shows the effect of anti-TRAIL antibody G72 on the elevation of ALT induced by APAP.
FIG.4A-4B show the combination of an antibody specifically binding to TRAIL and an antibody specifically binding to FasL in inhibiting PBMC killing of HepG2 cells (Figure 4A) as well as Jurkat cells (Figure 4B).
FIG.5A shows the schematic diagram of DVD-Ig (Dual-variable domain-Ig) format bispecific antibody. FIG. 5B shows the schematic diagram of Bs4Ab format bispecific antibody. FIG. 5C shows the schematic diagram of Hetero H CrossMab format bispecific antibody. FIG. 5D shows the schematic diagram of IgG-(scFv)2 format bispecific antibody. FIG. 5E shows the schematic diagram of scFv-Fab IgG format bispecific antibody.
FIG. 6A-6C shows the results of inhibition of PBMC killing of HepG2 cells by bispecific antibodies specifically binding to TRAIL and FasL.
FIG. 7A-7B shows the result of inhibition of PBMC killing of Jurkat cells by bispecific antibodies specifically binding to TRAIL and FasL.
FIG. 8A-8B show the result of inhibition of APAP-induced ALT elevation in mice by either bispecific antibody specifically binding to TRAIL and FasL, or antibody specifically binding to TRAIL in combination with antibody specifically binding to FasL.
FIG. 9A-9B show the result of inhibition of APAP-induced ALT (FIG. 9A) and AST (FIG. 9B) elevation in cynomolgus monkeys by bispecific antibody specifically binding to TRAIL and FasL.

### DETAILED DESCRIPTION OF THE APPLICATION

The present application in one aspect provides antibodies or antigen-binding fragments specifically binding to TRAIL. The present application in other aspect provides bispecific antibodies specifically binding to TRAIL and FasL. The present application in other aspect provides pharmaceutical compositions containing antibodies or antigen-binding fragments specifically binding to TRAIL and/or antibodies or antigen-binding fragments specifically binding to FasL, or pharmaceutical compositions containing bispecific antibodies specifically binding to TRAIL and FasL. The present application in other aspect also provides a method of treating a disease or condition in an individual in need thereof, comprising administering to the individual an effective amount of an antibody or antigen-binding fragment specifically binding to TRAIL or a pharmaceutical composition containing it, a bispecific antibody specifically binding TRAIL and FasL or a pharmaceutical composition containing it, or an antibody or antigen-binding fragment specifically binding to TRAIL and an antibody or antigen-binding fragment specifically binding to FasL or a pharmaceutical composition containing them. The present application in other aspect also provides the use of an antibody or antigen-binding fragment specifically binding to TRAIL or a pharmaceutical composition containing it, a bispecific antibody specifically binding TRAIL and FasL or a pharmaceutical composition containing it, or an antibody or antigen-binding fragment specifically binding to TRAIL and an antibody or antigen-binding fragment specifically binding to FasL or a pharmaceutical composition containing them in the manufacture of a medicament for treating a disease or condition.

By using a combination of selections on scFv phage libraries, affinity maturation and appropriately designed biochemical and biological assays, we have identified antibodies or antigen-binding fragments specifically binding to TRAIL and antibodies or antigen-binding fragments specifically binding to FasL. At the same time, bispecific antibodies specifically binding TRAIL and FasL were also produced. Additive or synergistic effects can be achieved when disease is treated in the form of (i) pharmaceutical compositions, (ii) bispecific antibody, or (iii) combinations.

The present application also provides nucleic acids encoding antibodies or antigen-binding fragments specifically binding to TRAIL or bispecific antibodies specifically binding TRAIL and FasL, a composition comprising an antibody or antigen-binding fragment specifically binding to TRAIL and an antibody or antigen-binding fragment specifically binding to FasL, and methods for preparing and using antibodies or antigen-binding fragments specifically binding to TRAIL, antibodies or antigen-binding fragments specifically binding to FasL, or bispecific antibodies specifically binding to TRAIL and FasL, and a pharmaceutical composition that contain any one of the antibodies or antigen-binding fragments described above.

### Definitions

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread of the disease, preventing or delaying the recurrence of the disease, delaying or slowing the progression of the disease, ameliorating the disease state, providing a remission (partial or total) of the disease, decreasing the dose of one or more of other medications required to treat the disease, delaying the progression of the disease, increasing or improving the quality of life, increasing weight gain, and/or prolonging survival. Also encompassed by "treatment" is a reduction of pathological consequence of the disease (such as, for example, lysis or necrosis of the host cells). The methods of the application contemplate any one or more of these aspects of treatment.

The term "prevent," and similar words such as "prevented," "preventing," "prevention" or "prophylactic" etc., indicate an approach for preventing, inhibiting, or reducing the likelihood of the occurrence or recurrence of, a disease or condition, *e.g.,* a pathogenic infection. It also refers to delaying the occurrence or recurrence of a disease or condition, or delaying the occurrence or recurrence of the symptoms of a disease or condition. As used herein, "prevention" and similar words also includes reducing the intensity, effect, symptoms and/or burden of a disease or condition prior to occurrence or recurrence of the disease or condition. As used herein, "prevention" and similar words also includes reducing the risk and susceptibility to occurrence or recurrence of the disease or condition, *e.g.,* a pathogenic infection.

Antibody or antigen-binding fragment as used herein, the term "antibody" herein is used in the broadest sense and encompasses a variety of antibody structures, including, but not limited to, monoclonal antibodies, polyclonal antibodies, monospecific, multi-specific antibodies (e.g., bispecific antibodies), full-length antibodies and antigen-binding fragments thereof, so long as they exhibit the desired antigen binding activity. A full-length antibody comprises two heavy chains and two light chains. The variable regions of the light and heavy chains are responsible for antigen binding. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain).

The term "antigen binding protein" refers in its broadest sense to a protein comprising a moiety specifically binding to an antigen or target. Examples of antigen binding proteins are antibodies and antibody fragments.

As used herein, the term "antigen-binding fragment" as used herein includes an antibody fragment including, for example, a diabody, a Fab, a Fab', a F(ab')2, an Fv fragment, a disulfide stabilized Fv fragment (dsFv), a (dsFv)2, a bispecific dsFv (dsFv-dsFv'), a disulfide stabilized diabody (ds diabody), a single-chain Fv (scFv), an scFv dimer (bivalent diabody), a multi-specific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. Fab (fragment antigen-binding), as described herein, is a monovalent fragment that includes a V_{L}, V_{H}, C_{L} and C_{H}1 domain of an antibody. An antigen-binding fragment also includes a fusion protein comprising the antibody fragment described above. An antigen-binding fragment is capable of binding to the same antigen to which the parent antibody or a parent antibody fragment (*e.g.,* a parent scFv) binds. In some embodiments, an antigen-binding fragment may comprise one or more CDRs from a particular human antibody grafted to a framework region from one or more different human antibodies.

The term "bispecific antibody" as used herein refers to an antibody having binding specificity to two different antigen or epitopes in one molecule. Bispecific antibody is produced through a process that involves design of the intact molecule, synthesis and cloning of the nucleotide sequences for each domain, expression in mammalian cells and purification of the final product. Exemplary bispecific antibody structures include structures known in the art, e.g., DVD-Ig format, Bs4Ab format, Hetero H, CrossMab format, IgG-(scFv)2 format or scFv-Fab IgG format, etc. (see, e.g., l Labrijn AF, et al. Nat Rev Drug Discov. 2019 Aug;18(8):585-608).

DVD-Ig (Dual-variable domain-Ig) format bispecific antibodies, in which the V_{L} and V_{H} structural domains of another antibody are attached to the N-terminal of the light and heavy chains of a full-length IgG antibody, respectively, and the bispecificity is achieved by the interaction of V_{H} and V_{L} to form an antigen-binding domain (Fv), which simultaneously binds to the corresponding antigen. Exemplary DVD-Ig bispecific antibodies are described in the literature Wu C, et al. Molecular construction and optimization of anti-human IL-1alpha/beta dual variable domain immunoglobulin (DVD-Ig) molecules. MAbs. 2009 Jul-Aug;1(4):339-47. Bs4Ab format bispecific antibody is a bispecific tetravalent antibody comprising a full-length IgG1 structure, and the bispecificity is achieved by insertion of another binding unit, scFv, into its hinge region. The Bs4Ab format bispecific antibodies are described in the literature Bezabeh B, et al. Insertion of scFv into the hinge domain of full-length IgG1 monoclonal antibody results in tetravalent bispecific molecule with robust properties. MAbs. 2017 Feb/Mar;9(2):240-256. Hetero H, a crossmab format bispecific antibody, has designed knobs-in-holes (KIH) in the Fc region and introduces mutations in two Cys residues that form stable disulfide Bridges (S354C on the "knob" side and Y349C on the "hole" side). CrossMab technology is also used to ensure the correct pairing between the light and heavy chains of antibodies. CrossMab technology is based on the exchange of antibody domains within a Fab arm of bispecific IgG antibodies, which can be the exchange of complete Fab domains (CrossMAb Fab), only variable region exchange (CrossMAb V_{H}-V_{L}) or only constant region exchange (CrossMAb C_{H}1-C_{L}) in the Fab domain. The Hetero H CrossMab format bispecific antibodies are described in the literature Klein C, et al. The use of CrossMAb technology for the generation of bi- and multispecific antibodies. MAbs. 2016 Aug-Sep;8(6):1010-20. IgG-(scFv) ₂ format bispecific antibodies are made by connecting the scFv fragment of another antibody to the Fc ends of two heavy chains of IgG antibody. The IgG-(scFv)2 format bispecific antibodies are described in Coloma MJ, Morrison SL. Design and production of novel tetravalent bispecific antibodies. Nat Biotechnol. 1997 Feb;15(2):159-63. Scfv-Fab IgG format bispecific antibody, which is a heterodimer antibody, IgG antibody structure, where one Fab arm is replaced by an scFv structure, where the first monomer contains scFv and IgG Fc, and the scFv is connected to the N-terminal of the IgG Fc C_{H}2 domain by a peptide linker, and the second monomer contains Fab and IgG Fc.

The term "antigen-binding domain" as used herein refers to the portion of an antigen binding molecule specifically binding to an antigen. More specifically, the term "antigen-binding domain" refers to a portion of an antibody that comprises a region specifically binding to and is complementary to a portion or all of an antigen. In the case of large antigens, the antigen binding molecule may bind only a specific part of the antigen, which part is called an epitope. The antigen-binding domain may be provided by, for example, one or more variable domains (also referred to as variable regions). Preferably, the antigen-binding domain comprises an antibody light chain variable domain (V_{L}) and an antibody heavy chain variable domain (V_{H}). In one aspect, the antigen-binding domain is capable of binding its antigen and blocking or partially blocking the function of said antigen. Antigen-binding domains specifically binding TRAIL or FasL include antibodies and fragments thereof as further defined herein.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target TRAIL with a second antibody when the first antibody inhibits target TRAIL binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa*. A high throughput process for "binning" antibodies based upon their cross-competition is described in PCT Publication No. WO 03/48731.

As used herein, the term "specifically binds," "specifically recognizing," or "is specific for" refers to measurable and reproducible interactions, such as binding between a target and an antibody, which is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its binding to other targets. In some embodiments, an antibody that specifically recognizes an antigen reacts with one or more antigenic determinants of the antigen with a binding affinity that is at least about 10 times its binding affinity for other targets.

An "isolated" antibody as used herein refers to an antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**Table 1: CDR DEFINITIONS**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | |

The term "chimeric antibodies" refer to antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and -binding site. This fragment consists of a dimer of one heavy- and one light-chain variable domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a peptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e.,* fragment having two antigen-binding sites. Bispecific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g.,* rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

The term "Fc (fragment crystallizable)" or "Fc region" refers to a polypeptide comprising the intact constant region of antibody, excluding C_{H}1 domain, and in some cases comprising part of a hinge, are absent whether in monomeric or multimeric form. The original immunoglobulin source of the native Fc is preferably of human origin and may be any immunoglobulin, e.g., IgG1, IgG2, IgG3 or IgG4. Natural Fc consists of monomeric polypeptides that can be linked into dimeric or multimeric forms by covalent (i.e., disulfide bonds) and non-covalent associations. The Fc region of an immunoglobulin generally comprises the C_{H}2 domain and the C_{H}3 domain of the heavy chain constant region, and optionally comprising the C_{H}4 domain.

In some embodiments, each of the two Fc monomers in an Fc dimer contains an amino acid substitution that promotes heterodimerization of the two monomers. In some embodiments, heterodimerization of Fc monomers can be facilitated by introducing different but compatible substitutions such as "knob-into-hole" residue pairs in the two Fc monomers. The knob-into-hole technology is also published in U.S. Patent Publication No. 8,216,805. In some embodiments, one Fc monomer contains the knob mutation T366W, and another Fc monomer contains the hole mutations T366S, L358A, and Y407V. In some embodiments, two Cys residues are introduced to form a stabilized disulphide bridge (S354C on the "knob" side, and Y349C on the "hole" side).

The terms "Fc receptor" or "FcR" are used to describe a receptor that binds to the Fc region of an antibody. In some embodiments, an FcR of this application is one that binds an IgG antibody (a γ receptor) and includes receptors of the FcγRI, FcγRII, and FcγRIII subclasses, including allelic variants and alternatively spliced forms of these receptors. FcyRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review M. in Daëron, Annu. Rev. Immunol. 15:203-234 (1997)). The term includes allotypes, such as FcγRIIIA allotypes: FcγRIIIA-Phe158, FcγRIIIA-Val158, FcγRIIA-R131 and/or FcγRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "C_{H}1 domain" of a human IgG heavy chain constant region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions, the hinge regions of other IgG subtypes can be aligned with IgG1 sequences.

The "C_{H}2 domain" of a human IgG Fc region usually extends from about amino acid 231 to about amino acid 340. The C_{H}2 domain is unique in that it is not closely paired with another domain. Rather, two N-linked branched carbohydrate chains are interposed between the two C_{H}2 domains of an intact native IgG molecule. It has been speculated that the carbohydrate may provide a substitute for the domain-domain pairing and help stabilize the C_{H}2 domain. Burton, Molec Immunol. 22:161-206 (1985).

The "C_{H}3 domain" comprises the stretch of residues C-terminal to a C_{H}2 domain in an Fc region (*i.e.* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g.* B cell receptor; BCR), etc. Such effector functions generally require the Fc region to be combined with a binding domain (e.g. an antibody variable domain) and can be assessed using various assays known in the art.

An antibody with a variant IgG Fc with "altered" FcR binding affinity or ADCC activity is one which has either enhanced or diminished FcR binding activity (*e.g.,* FcγR or FcRn) and/or ADCC activity compared to a parent polypeptide or to a polypeptide comprising a native sequence Fc region. The variant Fc which "exhibits increased binding" to an FcR binds at least one FcR with higher affinity (*e.g.,* lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g.,* higher apparent Kd or higher IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells (*e.g.,* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS (USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g.,* in an animal model *etc.* In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, *e.g.*, if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an antibody (comprising bispecific antibody) or composition as disclosed herein, is an amount sufficient to carry out a specifically stated purpose. An "effective amount" can be determined empirically and by known methods relating to the stated purpose.

The term "therapeutically effective amount" refers to an amount of an antibody (comprising bispecific antibody) or composition as disclosed herein, effective to "treat" a disease or disorder in an individual. As used herein, the term "effective amount" refers to a sufficient degree of severity and/or duration of treatment to reduce or ameliorate a disorder or one or more symptoms thereof; preventing the progression of the disorder; causing regression of the condition; preventing the recurrence, development, onset, or progression of one or more symptoms associated with the disorder; detecting a condition; or an amount that enhances or improves the prophylactic or therapeutic effect of another therapy (e.g., prophylactic or therapeutic agent).

As used herein, by "pharmaceutically acceptable" or "pharmacologically compatible" is meant a material that is not biologically or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of" and/or "consisting essentially of" embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat infection of type X means the method is used to treat infection of types other than X.

As used herein and in the appended claims, the singular forms "a," "or," and "the" include plural referents unless the context clearly dictates otherwise.

### Antibodies that specifically bind to TRAIL

In one aspect, the present application provides antibodies or antigen-binding fragments specifically binding to TRAIL, which include, but are not limited to, humanized antibodies, chimeric antibodies, mouse antibodies, human antibodies, and antibodies comprising the heavy chain and/or light chain CDRs discussed herein. In one aspect, antibodies or antigen-binding fragments are isolated antibodies that bind to TRAIL. Contemplated antibodies or antigen-binding fragments specifically binding to TRAIL include, for example, full-length antibodies specifically binding to TRAIL (*e.g.,* full-length IgG1, IgG2 or IgG4), single-chain antibodies specifically binding to TRAIL, multi-specific (such as bispecific) antibodies that bind to TRAIL, immunoconjugates specifically binding to TRAI, and the like. In some embodiments, the antibody or antigen-binding fragment specifically binding to TRAIL is a Fab, a Fab', a F(ab)'₂, a Fab'-SH, a single-chain Fv (scFv), an Fv fragment, a dAb, an Fd, a nanobody, a diabody, or a linear antibody. In some embodiments, reference to an antibody or antigen-binding fragment specifically binding to TRAIL means that the antibody or antigen-binding fragment binds to TRAIL with an affinity that is at least about 10 times (including for example at least about any one of 10, 10², 10³, 10⁴, 10⁵, 10⁶, or 10⁷ times) more tightly than its binding affinity for a non-target. In some embodiments, the non-target is an antigen that is not TRAIL.

Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although antibodies or antigen-binding fragments specifically binding to TRAIL containing human sequences (*e.g.,* human heavy and light chain variable domain sequences comprising human CDR sequences) are extensively discussed herein, non-human antibodies are also contemplated. In some embodiments, non-human antibodies comprise human CDR sequences from antibodies or antigen-binding fragments specifically binding to TRAIL as described herein and non-human framework sequences. Non-human framework sequences include, in some embodiments, any sequence that can be used for generating synthetic heavy and/or light chain variable domains using one or more human CDR sequences as described herein, including, *e.g*., mammals, *e.g.,* mouse, rat, rabbit, pig, bovine (*e.g.,* cow, bull, buffalo), deer, sheep, goat, chicken, cat, dog, ferret, primate (*e.g.,* marmoset, rhesus monkey), *etc.* In some embodiments, a non-human antibody or antigen-binding fragment specifically binding to TRAIL includes an antibody or antigen-binding fragment specifically binding to TRAIL generated by grafting one or more human CDR sequences as described herein onto a non-human framework sequence (*e.g.,* a mouse or chicken framework sequence).

In some embodiments, the antibody or antigen-binding fragment specifically binding TRAIL described herein recognizes an epitope within human TRAIL. In some embodiments, the antibody or antigen-binding fragment specifically binding TRAIL described herein cross-reacts with TRAIL antigens from species other than humans. In some embodiments, the TRAIL specific antibody or antigen-binding fragment is completely specific to human TRAIL and does not cross-react with other non-human species.

In some embodiments, the antibody or antigen-binding fragment specifically binding to TRAIL described herein comprises: a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22.

In some embodiments, the antibody or antigen-binding fragment specifically binding to TRAIL described herein comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs.

In some embodiments, the antibody or antigen-binding fragment specifically binding to TRAIL described herein comprises: a V_{H} comprising the amino acid sequence of any one of SEQ ID NOs: 13-14, or a variant thereof having at least about 80% sequence identity to the amino acid sequence of any one of SEQ ID NOs: 13-14; and a V_{L} comprising the amino acid sequence of any one of SEQ ID NOs: 22-27, or a variant thereof having at least about 80% sequence identity to the amino acid sequence of any one of SEQ ID NOs: 22-27.

In some embodiments, the antibody or antigen-binding fragment specifically binding to TRAIL described herein comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27.

In some embodiments, the antibody or antigen-binding fragment specifically binding to TRAIL described herein comprises: a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, the antibody or antigen-binding fragment specifically binding to TRAIL described herein comprises: (i) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs. In some embodiments, the antibody or antigen-binding fragment specifically binding to TRAIL described herein comprises: a V_{H} comprising the amino acid sequence of any one of SEQ ID NOs: 15-21, or a variant thereof having at least about 80% (such as at least about any of 80%, 85%, 90%, 95%, 96%, 97%, 98% or 99%) sequence identity to the amino acid sequence of any one of SEQ ID NOs: 15-21; and a V_{L} comprising the amino acid sequence of any one of SEQ ID NOs: 28-32, or a variant thereof having at least about 80% (such as at least about any of 80%, 85%, 90%, 95%, 96%, 97%, 98% or 99%) sequence identity to the amino acid sequence of any one of SEQ ID NOs: 28-32.

In some embodiments, the antibody or antigen-binding fragment specifically binding to TRAIL described herein comprises: (i) a V_{H} comprising the amino acid sequence of SEQ ID NO: 15, or a variant thereof having at least about 80% (such as at least about any of 80%, 85%, 90%, 95%, 96%, 97%, 98% or 99%) sequence identity to the amino acid sequence of SEQ ID NO: 15; and a V_{L} comprising the amino acid sequence of SEQ ID NO: 28, or a variant thereof having at least about 80% (such as at least about any of 80%, 85%, 90%, 95%, 96%, 97%, 98% or 99%) sequence identity to the amino acid sequence of SEQ ID NO: 28; (ii) a V_{H} comprising the amino acid sequence of SEQ ID NO: 16, or a variant thereof having at least about 80% (such as at least about any of 80%, 85%, 90%, 95%, 96%, 97%, 98% or 99%) sequence identity to the amino acid sequence of SEQ ID NO: 16; and a V_{L} comprising the amino acid sequence of SEQ ID NO: 29, or a variant thereof having at least about 80% (such as at least about any of 80%, 85%, 90%, 95%, 96%, 97%, 98% or 99%) sequence identity to the amino acid sequence of SEQ ID NO: 29; (iii) a V_{H} comprising the amino acid sequence of SEQ ID NO: 17, or a variant thereof having at least about 80% (such as at least about any of 80%, 85%, 90%, 95%, 96%, 97%, 98% or 99%) sequence identity to the amino acid sequence of SEQ ID NO: 17; and a V_{L} comprising the amino acid sequence of SEQ ID NO: 29, or a variant thereof having at least about 80% (such as at least about any of 80%, 85%, 90%, 95%, 96%, 97%, 98% or 99%) sequence identity to the amino acid sequence of SEQ ID NO: 29; (iv) a V_{H} comprising the amino acid sequence of SEQ ID NO: 18, or a variant thereof having at least about 80% (such as at least about any of 80%, 85%, 90%, 95%, 96%, 97%, 98% or 99%) sequence identity to the amino acid sequence of SEQ ID NO: 18; and a V_{L} comprising the amino acid sequence of SEQ ID NO: 29, or a variant thereof having at least about 80% (such as at least about any of 80%, 85%, 90%, 95%, 96%, 97%, 98% or 99%) sequence identity to the amino acid sequence of SEQ ID NO: 29; (v) a V_{H} comprising the amino acid sequence of SEQ ID NO: 19, or a variant thereof having at least about 80% (such as at least about any of 80%, 85%, 90%, 95%, 96%, 97%, 98% or 99%) sequence identity to the amino acid sequence of SEQ ID NO: 19; and a V_{L} comprising the amino acid sequence of SEQ ID NO: 29, or a variant thereof having at least about 80% (such as at least about any of 80%, 85%, 90%, 95%, 96%, 97%, 98% or 99%) sequence identity to the amino acid sequence of SEQ ID NO: 29; (vi) a V_{H} comprising the amino acid sequence of SEQ ID NO: 20, or a variant thereof having at least about 80% (such as at least about any of 80%, 85%, 90%, 95%, 96%, 97%, 98% or 99%) sequence identity to the amino acid sequence of SEQ ID NO: 20; and a V_{L} comprising the amino acid sequence of SEQ ID NO: 29, or a variant thereof having at least about 80% (such as at least about any of 80%, 85%, 90%, 95%, 96%, 97%, 98% or 99%) sequence identity to the amino acid sequence of SEQ ID NO: 29; (vii) a V_{H} comprising the amino acid sequence of SEQ ID NO: 16, or a variant thereof having at least about 80% (such as at least about any of 80%, 85%, 90%, 95%, 96%, 97%, 98% or 99%) sequence identity to the amino acid sequence of SEQ ID NO: 16; and a V_{L} comprising the amino acid sequence of SEQ ID NO: 30, or a variant thereof having at least about 80% (such as at least about any of 80%, 85%, 90%, 95%, 96%, 97%, 98% or 99%) sequence identity to the amino acid sequence of SEQ ID NO: 30; (viii) a V_{H} comprising the amino acid sequence of SEQ ID NO: 17, or a variant thereof having at least about 80% (such as at least about any of 80%, 85%, 90%, 95%, 96%, 97%, 98% or 99%) sequence identity to the amino acid sequence of SEQ ID NO: 17; and a V_{L} comprising the amino acid sequence of SEQ ID NO: 30, or a variant thereof having at least about 80% (such as at least about any of 80%, 85%, 90%, 95%, 96%, 97%, 98% or 99%) sequence identity to the amino acid sequence of SEQ ID NO: 30; (ix) a V_{H} comprising the amino acid sequence of SEQ ID NO: 18, or a variant thereof having at least about 80% (such as at least about any of 80%, 85%, 90%, 95%, 96%, 97%, 98% or 99%) sequence identity to the amino acid sequence of SEQ ID NO: 18; and a V_{L} comprising the amino acid sequence of SEQ ID NO: 30, or a variant thereof having at least about 80% (such as at least about any of 80%, 85%, 90%, 95%, 96%, 97%, 98% or 99%) sequence identity to the amino acid sequence of SEQ ID NO: 30; (x) a V_{H} comprising the amino acid sequence of SEQ ID NO: 19, or a variant thereof having at least about 80% (such as at least about any of 80%, 85%, 90%, 95%, 96%, 97%, 98% or 99%) sequence identity to the amino acid sequence of SEQ ID NO: 19; and a V_{L} comprising the amino acid sequence of SEQ ID NO: 30, or a variant thereof having at least about 80% (such as at least about any of 80%, 85%, 90%, 95%, 96%, 97%, 98% or 99%) sequence identity to the amino acid sequence of SEQ ID NO: 30; (xi) a V_{H} comprising the amino acid sequence of SEQ ID NO: 20, or a variant thereof having at least about 80% (such as at least about any of 80%, 85%, 90%, 95%, 96%, 97%, 98% or 99%) sequence identity to the amino acid sequence of SEQ ID NO: 20; and a V_{L} comprising the amino acid sequence of SEQ ID NO: 30, or a variant thereof having at least about 80% (such as at least about any of 80%, 85%, 90%, 95%, 96%, 97%, 98% or 99%) sequence identity to the amino acid sequence of SEQ ID NO: 30; (xii) a V_{H} comprising the amino acid sequence of SEQ ID NO: 16, or a variant thereof having at least about 80% (such as at least about any of 80%, 85%, 90%, 95%, 96%, 97%, 98% or 99%) sequence identity to the amino acid sequence of SEQ ID NO: 16; and a V_{L} comprising the amino acid sequence of SEQ ID NO: 31, or a variant thereof having at least about 80% (such as at least about any of 80%, 85%, 90%, 95%, 96%, 97%, 98% or 99%) sequence identity to the amino acid sequence of SEQ ID NO: 31; (xiii) a V_{H} comprising the amino acid sequence of SEQ ID NO: 17, or a variant thereof having at least about 80% (such as at least about any of 80%, 85%, 90%, 95%, 96%, 97%, 98% or 99%) sequence identity to the amino acid sequence of SEQ ID NO: 17; and a V_{L} comprising the amino acid sequence of SEQ ID NO: 31, or a variant thereof having at least about 80% (such as at least about any of 80%, 85%, 90%, 95%, 96%, 97%, 98% or 99%) sequence identity to the amino acid sequence of SEQ ID NO: 31; (xiv) a V_{H} comprising the amino acid sequence of SEQ ID NO: 18, or a variant thereof having at least about 80% (such as at least about any of 80%, 85%, 90%, 95%, 96%, 97%, 98% or 99%) sequence identity to the amino acid sequence of SEQ ID NO: 18; and a V_{L} comprising the amino acid sequence of SEQ ID NO: 31, or a variant thereof having at least about 80% (such as at least about any of 80%, 85%, 90%, 95%, 96%, 97%, 98% or 99%) sequence identity to the amino acid sequence of SEQ ID NO: 31; (xv) a V_{H} comprising the amino acid sequence of SEQ ID NO: 19, or a variant thereof having at least about 80% (such as at least about any of 80%, 85%, 90%, 95%, 96%, 97%, 98% or 99%) sequence identity to the amino acid sequence of SEQ ID NO: 19; and a V_{L} comprising the amino acid sequence of SEQ ID NO: 31, or a variant thereof having at least about 80% (such as at least about any of 80%, 85%, 90%, 95%, 96%, 97%, 98% or 99%) sequence identity to the amino acid sequence of SEQ ID NO: 31; (xvi) a V_{H} comprising the amino acid sequence of SEQ ID NO: 20, or a variant thereof having at least about 80% (such as at least about any of 80%, 85%, 90%, 95%, 96%, 97%, 98% or 99%) sequence identity to the amino acid sequence of SEQ ID NO: 20; and a V_{L} comprising the amino acid sequence of SEQ ID NO: 31, or a variant thereof having at least about 80% (such as at least about any of 80%, 85%, 90%, 95%, 96%, 97%, 98% or 99%) sequence identity to the amino acid sequence of SEQ ID NO: 31; or (xvii) a V_{H} comprising the amino acid sequence of SEQ ID NO: 21, or a variant thereof having at least about 80% (such as at least about any of 80%, 85%, 90%, 95%, 96%, 97%, 98% or 99%) sequence identity to the amino acid sequence of SEQ ID NO: 21; and a V_{L} comprising the amino acid sequence of SEQ ID NO: 32, or a variant thereof having at least about 80% (such as at least about any of 80%, 85%, 90%, 95%, 96%, 97%, 98% or 99%) sequence identity to the amino acid sequence of SEQ ID NO: 32.

In some embodiments, the amino acid substitutions described above are limited to "exemplary substitutions" shown in Table 10 of this application. In some embodiments, the amino acid substitutions are limited to "preferred substitutions" shown in Table 10 of this application.

In some embodiments, functional epitopes can be mapped by combinatorial alanine scanning. In this process, a combinatorial alanine-scanning strategy can be used to identify amino acids in the TRAIL protein that are necessary for interaction with antibodies or antigen-binding fragments specifically binding to TRAIL. In some embodiments, the epitope is conformational and crystal structure of anti-TRAIL antibodies bound to TRAIL may be employed to identify the epitopes.

In some embodiments, the present application provides antibodies or antigen-binding fragments which compete with any one of antibodies or antigen-binding fragments specifically binding to TRAIL described herein for binding to TRAIL. In some embodiments, the present application provides antibodies or antigen-binding fragments which compete with any one of the antibodies or antigen-binding fragments specifically binding to TRAIL provided herein for binding to an epitope on the TRAIL. In some embodiments, the present application provides antibody or antigen-binding fragment specifically binding to TRAIL that binds to the same epitope as an antibody or antigen-binding fragment specifically binding to TRAIL comprising a V_{H} comprising the amino acid sequence of any one of SEQ ID NOs: 13-21, and a V_{L} comprising the amino acid sequence of any one of SEQ ID NOs: 22-32. In some embodiments, an antibody or antigen-binding fragment specifically binding to TRAIL is provided that competitively binds to TRAIL with an anti-TRAIL antibody comprising a V_{H} comprising the amino acid sequence of any one of SEQ ID NOs: 13-21 and a V_{L} comprising the amino acid sequence of any one of SEQ ID NOs: 22-32.

In some embodiments, competition assays may be used to identify a monoclonal antibody that competes with an antibody or antigen-binding fragment specifically binding to TRAIL described herein for binding to TRAIL. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch.14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibody that competes with an antibody or antigen-binding fragment specifically binding to TRAIL described herein is a chimeric, humanized or human antibody.

Exemplary sequences of antibodies or antigen-binding fragments specifically binding to TRAIL are shown in Tables 2, 3-1 and 3-2, wherein the CDR numbering is according to the EU numbering system of Chothia. Those skilled in the art will recognize that many algorithms are known for prediction of CDR positions and for delimitation of antibody heavy chain and light chain variable domains. Antibodies comprising CDRs, V_{H} and/or V_{L} sequences from antibodies described herein, but based on prediction algorithms other than those exemplified in the tables below, are within the scope of this invention.

### The full-length antibodies that specifically bind to TRAIL

In some embodiments, the antibody specifically binding to TRAIL described herein is a full-length antibody. In some embodiments, the full-length antibody specifically binding to TRAIL is an IgA, IgD, IgE, IgG, or IgM. In some embodiments, the full-length antibody specifically binding to TRAIL comprises an antibody heavy chain constant region and an antibody light chain constant region. In some embodiments, the full-length antibody specifically binding to TRAIL comprises IgG constant domains, such as constant domains of any one of IgG1, IgG2, IgG3, and IgG4 including variants thereof. In some embodiments, the full-length antibody specifically binding to TRAIL comprises IgG1 heavy chain constant region. In some embodiments, the full-length antibody specifically binding to TRAIL comprises IgG2 heavy chain constant region. In some embodiments, the full-length antibody specifically binding to TRAIL comprises IgG3 heavy chain constant region. In some embodiments, the full-length antibody specifically binding to TRAIL comprises IgG4 heavy chain constant region. In some embodiments, the IgG is human IgG. In some embodiments, the heavy chain constant region comprises or consists of the amino acid sequence of SEQ ID NO: 96. In some embodiments, the light chain constant region comprises or consists of the amino acid sequence of SEQ ID NO: 97. In some embodiments, the full-length antibody specifically binding to TRAIL comprises a kappa light chain constant region. In some embodiments, the light chain constant region comprises or consists of the amino acid sequence of SEQ ID NO: 98. In some embodiments, the full-length antibody specifically binding to TRAIL comprises a lambda light chain constant region. In some embodiments, the light chain constant region comprises or consists of the amino acid sequence of SEQ ID NO: 99. In some embodiments, the full-length antibody specifically binding to TRAIL comprises an antibody heavy chain variable domain and an antibody light chain variable domain. In some embodiments, the full-length antibody specifically binding to TRAIL comprises an Fc sequence that has been altered or otherwise changed so that it has enhanced antibody dependent cellular cytotoxicity (ADCC) or complement dependent cytotoxicity (CDC) effector function.

In some embodiments, there is provided a full-length antibody specifically binding to TRAIL comprising IgG1, IgG2, IgG3 or IgG4 constant region, wherein the antibody specifically binding to TRAIL comprises a) a heavy chain variable domain comprising an HC-CDR1 comprising the amino acid sequence of any one of SEQ ID NOs: 1-2, or a variant thereof comprising up to about 3 (such as about any of 1, 2, or 3) amino acid substitutions, an HC-CDR2 comprising the amino acid sequence of any one of SEQ ID NOs: 3-4, or a variant thereof comprising up to about 3 (such as about any of 1, 2, or 3) amino acid substitutions, and an HC-CDR3 comprising the amino acid sequence of any one of SEQ ID NOs: 5-6, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 7-8, or a variant thereof comprising up to about 3 (such as about any of 1, 2, or 3) amino acid substitutions, an LC-CDR2 comprising the amino acid sequence of any one of SEQ ID NOs: 9-10, or a variant thereof comprising up to about 3 (such as about any of 1, 2, or 3) amino acid substitutions, and an LC-CDR3 comprising the amino acid sequence of any one of SEQ ID NOs: 11-12, or a variant thereof comprising up to about 3 (such as about any of 1, 2, or 3) amino acid substitutions. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG2 is human IgG2. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the IgG3 is human IgG3. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1, IgG2, IgG3 or IgG4 constant region, wherein the antibody specifically binding to TRAIL comprises a) a heavy chain variable domain comprising an HC-CDR1 comprising the amino acid sequence of any one of SEQ ID NOs: 1-2, an HC-CDR2 comprising the amino acid sequence of any one of SEQ ID NOs: 3-4, and an HC-CDR3 comprising the amino acid sequence of any one of SEQ ID NOs:5-6, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 7-8, an LC-CDR2 comprising the amino acid sequence of any one of SEQ ID NOs: 9-10, and an LC-CDR3 comprising the amino acid sequence of any one of SEQ ID NOs: 11-12, or a variant thereof comprising up to about 5 (such as about any of 1, 2, 3, 4, or 5) amino acid substitutions in the LC-CDR sequences. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG2 is human IgG2. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the IgG3 is human IgG3. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a) a heavy chain variable domain comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and b) a light chain variable domain comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a) a heavy chain variable domain comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and b) a light chain variable domain comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a) a heavy chain variable domain comprising the amino acid sequence of any one of SEQ ID NOs: 13-21, or a variant thereof having at least about 80% (such as at least about any of 80%, 85%, 90%, 95%, 96%, 97%, 98%, or 99%) sequence identity to the amino acid sequence of any one of SEQ ID NOs: 13-21; and b) a light chain variable domain comprising the amino acid sequence of any one of SEQ ID NOs: 22-32, or a variant thereof having at least about 80% (such as at least about any of 80%, 85%, 90%, 95%, 96%, 97%, 98%, or 99%) sequence identity to the amino acid sequence of any one of SEQ ID NOs: 22-32. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

In some embodiments, there is provided a full-length antibody specifically binding to TRAIL comprising IgG1 or IgG4 constant region, wherein the antibody specifically binding to TRAIL comprises a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32. In some embodiments, the IgG1 is human IgG1. In some embodiments, the heavy chain constant region comprises or consists of the amino acid sequence of SEQ ID NO: 96. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 96 and the light chain constant region comprises or consists of the amino acid sequence of SEQ ID NO: 98. In some embodiments, the heavy chain constant region comprises or consists of the amino acid sequence of SEQ ID NO: 96 and the light chain constant region comprises or consists of the amino acid sequence of SEQ ID NO: 99. In some embodiments, the IgG4 is human IgG4. In some embodiments, the heavy chain constant region comprises or consists of the amino acid sequence of SEQ ID NO: 97. In some embodiments, the light chain constant region comprises or consists of the amino acid sequence of SEQ ID NO: 98. In some embodiments, the light chain constant region comprises or consists of the amino acid sequence of SEQ ID NO: 99. In some embodiments, the heavy chain constant region comprises or consists of the amino acid sequence of SEQ ID NO: 97 and the light chain constant region comprises or consists of the amino acid sequence of SEQ ID NO: 98. In some embodiments, the heavy chain constant region comprises or consists of the amino acid sequence of SEQ ID NO: 97 and the light chain constant region comprises or consists of the amino acid sequence of SEQ ID NO: 99.

### Antibodies that specifically bind to FasL

In one aspect, the present application provides antibodies or antigen-binding fragments specifically binding to FasL, which include, but are not limited to, humanized antibodies, chimeric antibodies, mouse antibodies, human antibodies, and antibodies comprising the heavy chain and/or light chain CDRs discussed herein. In one aspect, antibodies or antigen-binding fragments are isolated antibodies that bind to FasL. Contemplated antibodies or antigen-binding fragments specifically binding to FasL include, for example, full-length antibodies specifically binding to FasL (*e.g.,* full-length IgG1, IgG2 or IgG4), single-chain antibodies specifically binding to FasL, multi-specific (such as bispecific) antibodies that bind to FasL, immunoconjugates specifically binding to FasL, and the like. In some embodiments, the antibody or antigen-binding fragment specifically binding to FasL is a Fab, a Fab', a F(ab)'₂, a Fab'-SH, a single-chain Fv (scFv), an Fv fragment, a dAb, an Fd, a nanobody, a diabody, or a linear antibody. In some embodiments, an antibody or antigen-binding fragment specifically binding to FasL means that the antibody or antigen-binding fragment binds to FasL with an affinity that is at least about 10 times (including for example at least about any one of 10, 10², 10³, 10⁴, 10⁵, 10⁶, or 10⁷ times) more tightly than its binding affinity for a non-target. In some embodiments, the non-target is an antigen that is not FasL.

Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although antibodies or antigen-binding fragments specifically binding to FasL containing human sequences (*e.g.,* human heavy and light chain variable domain sequences comprising human CDR sequences) are extensively discussed herein, non-human antibodies or antigen-binding fragments are also contemplated. In some embodiments, non-human antibodies or antigen-binding fragments comprise human CDR sequences from antibodies or antigen-binding fragments specifically binding to FasL as described herein and non-human framework sequences. Non-human framework sequences include, in some embodiments, any sequence that can be used for generating synthetic heavy and/or light chain variable domains using one or more human CDR sequences as described herein, including, *e.g.,* mammals, *e.g.,* mouse, rat, rabbit, pig, bovine (*e.g.,* cow, bull, buffalo), deer, sheep, goat, chicken, cat, dog, ferret, primate (*e.g*., marmoset, rhesus monkey), *etc.* In some embodiments, a non-human antibody or antigen-binding fragment specifically binding to FasL includes an antibody or antigen-binding fragment specifically binding to FasL generated by grafting one or more human CDR sequences as described herein onto a non-human framework sequence (*e.g.,* a mouse or chicken framework sequence).

In some embodiments, the antibody or antigen-binding fragment specifically binding FasL described herein recognizes an epitope within human FasL. In some embodiments, the FasL specific antibody or antigen-binding fragment is completely specific to human FasL and does not cross-react with other species or non-FasL. In some embodiments, the antibody or antigen-binding fragment specifically binding FasL described herein cross-reacts with FasL antigens from species other than humans.

In some embodiments, the antibody or antigen-binding fragment specifically binding to FasL described herein comprises an antibody heavy chain constant region and an antibody light chain constant region. In some embodiments, the antibody or antigen-binding fragment specifically binding to FasL comprises IgG1 heavy chain constant region. In some embodiments, the full-length antibody or antigen-binding fragment specifically binding to FasL comprises IgG2 heavy chain constant region. In some embodiments, the full-length antibody or antigen-binding fragment specifically binding to FasL comprises IgG3 heavy chain constant region. In some embodiments, the full-length antibody or antigen-binding fragment specifically binding to FasL comprises IgG4 heavy chain constant region. In some embodiments, the IgG is human IgG. In some embodiments, the heavy chain constant region comprises or consists of the amino acid sequence of SEQ ID NO: 96. In some embodiments, the light chain constant region comprises or consists of the amino acid sequence of SEQ ID NO: 97. In some embodiments, the full-length antibody or antigen-binding fragment specifically binding to FasL comprises a kappa light chain constant region. In some embodiments, the light chain constant region comprises or consists of the amino acid sequence of SEQ ID NO: 98. In some embodiments, the full-length antibody or antigen-binding fragment specifically binding to FasL comprises a lambda light chain constant region. In some embodiments, the light chain constant region comprises or consists of the amino acid sequence of SEQ ID NO: 99. In some embodiments, the antibody or antigen-binding fragment specifically binding to FasL comprises an antibody heavy chain variable domain and an antibody light chain variable domain. In some embodiments, the full-length antibody specifically binding to FasL comprises an Fc sequence that has been altered or otherwise changed so that it has enhanced antibody dependent cellular cytotoxicity (ADCC) or complement dependent cytotoxicity (CDC) effector function.

In some embodiments, the antibody or antigen-binding fragment specifically binding to FasL described herein may be selected from the FasL-specific antibody described in the PCT application PCT/CN2023/072293.

### Bispecific antibodies that specifically bind to TRAIL and FasL

In one aspect, this application provides a bispecific antibody comprising a first antigen-binding domain specifically binding to TRAIL, and a second antigen-binding domain specifically binding to FasL. In some embodiments, the bispecific antibody can binds to TRAIL or FasL. In other preferred embodiments, the bispecific antibody can binds to both TRAIL and FasL. In other more preferred embodiments, the bispecific antibody can binds to both TRAIL and FasL simultaneously.

In some embodiments, this application provides a bispecific antibody comprising a first antigen-binding domain specifically binding to TRAIL, and a second antigen-binding domain specifically binding to FasL, wherein the first antigen-binding domain comprises: (i) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; or (ii) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; or (iii) a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12. In some embodiments, the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, this application provides a bispecific antibody comprising a first antigen-binding domain specifically binding to TRAIL, and a second antigen-binding domain specifically binding to FasL, wherein the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, the first antigen-binding domain specifically binding to TRAIL comprises: a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22.

In some embodiments, the first antigen-binding domain specifically binding to TRAIL comprises: a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, the first antigen-binding domain specifically binding to TRAIL comprises: a V_{H} comprising an HC-CDR1, an HC-CDR2, and an HC-CDR3 of a V_{H} comprising the amino acid sequence of SEQ ID NO: 164; and a V_{L} comprising an LC-CDR1, an LC-CDR2, and an LC-CDR3 of a V_{L} comprising the amino acid sequence of SEQ ID NO: 168.

In some embodiments, the first antigen-binding domain specifically binding to TRAIL comprises: a V_{H} comprising an HC-CDR1, an HC-CDR2, and an HC-CDR3 of a V_{H} comprising the amino acid sequence of SEQ ID NO: 166; and a V_{L} comprising an LC-CDR1, an LC-CDR2, and an LC-CDR3 of a V_{L} comprising the amino acid sequence of SEQ ID NO: 170.

In some embodiments, the second antigen-binding domain specifically binding to FasL comprises: a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73.

In some embodiments, the second antigen-binding domain specifically binding to FasL comprises: a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76.

In some embodiments, the second antigen-binding domain specifically binding to FasL comprises: a V_{H} comprising an HC-CDR1, an HC-CDR2, and an HC-CDR3 of a V_{H} comprising the amino acid sequence of SEQ ID NO: 163; and a V_{L} comprising an LC-CDR1, an LC-CDR2, and an LC-CDR3 of a V_{L} comprising the amino acid sequence of SEQ ID NO: 167.

In some embodiments, the second antigen-binding domain specifically binding to FasL comprises: a V_{H} comprising an HC-CDR1, an HC-CDR2, and an HC-CDR3 of a V_{H} comprising the amino acid sequence of SEQ ID NO: 165; and a V_{L} comprising an LC-CDR1, an LC-CDR2, and an LC-CDR3 of a V_{L} comprising the amino acid sequence of SEQ ID NO: 169.

In some embodiments, the first antigen-binding domain specifically binding to TRAIL comprises: a V_{H} comprising the amino acid sequence of any one of SEQ ID NOs: 13-20, 33-40, 164 and 166, or a variant thereof having at least about 80% (such as at least about any of 80%, 85%, 90%, 95%, 96%, 97%, 98%, or 99%) sequence identity to the amino acid sequence of any one of SEQ ID NOs: 13-20, 33-40, 164 and 166; and a V_{L} comprising the amino acid sequence of any one of SEQ ID NOs: 22-31, 41-50, 168 and 170, or a variant thereof having at least about 80% (such as at least about any of 80%, 85%, 90%, 95%, 96%, 97%, 98%, or 99%) sequence identity to the amino acid sequence of any one of SEQ ID NOs: 22-31, 41-50, 168 and 170.

In some embodiments, the first antigen-binding domain specifically binding to TRAIL comprises: a V_{H} comprising the amino acid sequence of any one of SEQ ID NOs: 13 and 33, or a variant thereof having at least about 80% (such as at least about any of 80%, 85%, 90%, 95%, 96%, 97%, 98%, or 99%) sequence identity to the amino acid sequence of any one of SEQ ID NOs: 13 and 33; and a V_{L} comprising the amino acid sequence of any one of SEQ ID NOs: 22 and 41, or a variant thereof having at least about 80% (such as at least about any of 80%, 85%, 90%, 95%, 96%, 97%, 98%, or 99%) sequence identity to the amino acid sequence of any one of SEQ ID NOs: 22 and 41.

In some embodiments, the first antigen-binding domain specifically binding to TRAIL comprises: a V_{H} comprising the amino acid sequence of any one of SEQ ID NOs: 14 and 34, or a variant thereof having at least about 80% (such as at least about any of 80%, 85%, 90%, 95%, 96%, 97%, 98%, or 99%) sequence identity to the amino acid sequence of any one of SEQ ID NOs: 14 and 34; and a V_{L} comprising the amino acid sequence of any one of SEQ ID NOs: 23 and 42, or a variant thereof having at least about 80% (such as at least about any of 80%, 85%, 90%, 95%, 96%, 97%, 98%, or 99%) sequence identity to the amino acid sequence of any one of SEQ ID NOs: 23 and 42.

In some embodiments, the first antigen-binding domain specifically binding to TRAIL comprises: a V_{H} comprising the amino acid sequence of any one of SEQ ID NOs: 14 and 34, or a variant thereof having at least about 80% (such as at least about any of 80%, 85%, 90%, 95%, 96%, 97%, 98%, or 99%) sequence identity to the amino acid sequence of any one of SEQ ID NOs: 14 and 34; and a V_{L} comprising the amino acid sequence of any one of SEQ ID NOs: 24 and 43, or a variant thereof having at least about 80% (such as at least about any of 80%, 85%, 90%, 95%, 96%, 97%, 98%, or 99%) sequence identity to the amino acid sequence of any one of SEQ ID NOs: 24 and 43.

In some embodiments, the first antigen-binding domain specifically binding to TRAIL comprises: a V_{H} comprising the amino acid sequence of any one of SEQ ID NOs: 14 and 34, or a variant thereof having at least about 80% (such as at least about any of 80%, 85%, 90%, 95%, 96%, 97%, 98%, or 99%) sequence identity to the amino acid sequence of any one of SEQ ID NOs: 14 and 34; and a V_{L} comprising the amino acid sequence of any one of SEQ ID NOs: 25 and 44, or a variant thereof having at least about 80% (such as at least about any of 80%, 85%, 90%, 95%, 96%, 97%, 98%, or 99%) sequence identity to the amino acid sequence of any one of SEQ ID NOs: 25 and 44.

In some embodiments, the first antigen-binding domain specifically binding to TRAIL comprises: a V_{H} comprising the amino acid sequence of any one of SEQ ID NOs: 14 and 34, or a variant thereof having at least about 80% (such as at least about any of 80%, 85%, 90%, 95%, 96%, 97%, 98%, or 99%) sequence identity to the amino acid sequence of any one of SEQ ID NOs: 14 and 34; and a V_{L} comprising the amino acid sequence of any one of SEQ ID NOs: 26 and 45, or a variant thereof having at least about 80% (such as at least about any of 80%, 85%, 90%, 95%, 96%, 97%, 98%, or 99%) sequence identity to the amino acid sequence of any one of SEQ ID NOs: 26 and 45.

In some embodiments, the first antigen-binding domain specifically binding to TRAIL comprises: a V_{H} comprising the amino acid sequence of any one of SEQ ID NOs: 14 and 34, or a variant thereof having at least about 80% (such as at least about any of 80%, 85%, 90%, 95%, 96%, 97%, 98%, or 99%) sequence identity to the amino acid sequence of any one of SEQ ID NOs: 14 and 34; and a V_{L} comprising the amino acid sequence of any one of SEQ ID NOs: 27 and 46, or a variant thereof having at least about 80% (such as at least about any of 80%, 85%, 90%, 95%, 96%, 97%, 98%, or 99%) sequence identity to the amino acid sequence of any one of SEQ ID NOs: 27 and 46.

In some embodiments, the first antigen-binding domain specifically binding to TRAIL comprises: a V_{H} comprising the amino acid sequence of any one of SEQ ID NOs: 15 and 35, or a variant thereof having at least about 80% (such as at least about any of 80%, 85%, 90%, 95%, 96%, 97%, 98%, or 99%) sequence identity to the amino acid sequence of any one of SEQ ID NOs: 15 and 35; and a V_{L} comprising the amino acid sequence of any one of SEQ ID NOs: 28 and 47, or a variant thereof having at least about 80% (such as at least about any of 80%, 85%, 90%, 95%, 96%, 97%, 98%, or 99%) sequence identity to the amino acid sequence of any one of SEQ ID NOs: 28 and 47.

In some embodiments, the first antigen-binding domain specifically binding to TRAIL comprises: a V_{H} comprising the amino acid sequence of any one of SEQ ID NOs: 16 and 36, or a variant thereof having at least about 80% (such as at least about any of 80%, 85%, 90%, 95%, 96%, 97%, 98%, or 99%) sequence identity to the amino acid sequence of any one of SEQ ID NOs: 16 and 36; and a V_{L} comprising the amino acid sequence of any one of SEQ ID NOs: 29 and 48, or a variant thereof having at least about 80% (such as at least about any of 80%, 85%, 90%, 95%, 96%, 97%, 98%, or 99%) sequence identity to the amino acid sequence of any one of SEQ ID NOs: 29 and 48.

In some embodiments, the first antigen-binding domain specifically binding to TRAIL comprises: a V_{H} comprising the amino acid sequence of any one of SEQ ID NOs: 17 and 37, or a variant thereof having at least about 80% (such as at least about any of 80%, 85%, 90%, 95%, 96%, 97%, 98%, or 99%) sequence identity to the amino acid sequence of any one of SEQ ID NOs: 17 and 37; and a V_{L} comprising the amino acid sequence of any one of SEQ ID NOs: 29 and 48, or a variant thereof having at least about 80% (such as at least about any of 80%, 85%, 90%, 95%, 96%, 97%, 98%, or 99%) sequence identity to the amino acid sequence of any one of SEQ ID NOs: 29 and 48.

In some embodiments, the first antigen-binding domain specifically binding to TRAIL comprises: a V_{H} comprising the amino acid sequence of any one of SEQ ID NOs: 18 and 38, or a variant thereof having at least about 80% (such as at least about any of 80%, 85%, 90%, 95%, 96%, 97%, 98%, or 99%) sequence identity to the amino acid sequence of any one of SEQ ID NOs: 18 and 38; and a V_{L} comprising the amino acid sequence of any one of SEQ ID NOs: 29 and 48, or a variant thereof having at least about 80% (such as at least about any of 80%, 85%, 90%, 95%, 96%, 97%, 98%, or 99%) sequence identity to the amino acid sequence of any one of SEQ ID NOs: 29 and 48.

In some embodiments, the first antigen-binding domain specifically binding to TRAIL comprises: a V_{H} comprising the amino acid sequence of any one of SEQ ID NOs: 19 and 39, or a variant thereof having at least about 80% (such as at least about any of 80%, 85%, 90%, 95%, 96%, 97%, 98%, or 99%) sequence identity to the amino acid sequence of any one of SEQ ID NOs: 19 and 39; and a V_{L} comprising the amino acid sequence of any one of SEQ ID NOs: 29 and 48, or a variant thereof having at least about 80% (such as at least about any of 80%, 85%, 90%, 95%, 96%, 97%, 98%, or 99%) sequence identity to the amino acid sequence of any one of SEQ ID NOs: 29 and 48.

In some embodiments, the first antigen-binding domain specifically binding to TRAIL comprises: a V_{H} comprising the amino acid sequence of any one of SEQ ID NOs: 20 and 40, or a variant thereof having at least about 80% (such as at least about any of 80%, 85%, 90%, 95%, 96%, 97%, 98%, or 99%) sequence identity to the amino acid sequence of any one of SEQ ID NOs: 20 and 40; and a V_{L} comprising the amino acid sequence of any one of SEQ ID NOs: 29 and 48, or a variant thereof having at least about 80% (such as at least about any of 80%, 85%, 90%, 95%, 96%, 97%, 98%, or 99%) sequence identity to the amino acid sequence of any one of SEQ ID NOs: 29 and 48.

In some embodiments, the first antigen-binding domain specifically binding to TRAIL comprises: a V_{H} comprising the amino acid sequence of any one of SEQ ID NOs: 16 and 36, or a variant thereof having at least about 80% (such as at least about any of 80%, 85%, 90%, 95%, 96%, 97%, 98%, or 99%) sequence identity to the amino acid sequence of any one of SEQ ID NOs: 16 and 36; and a V_{L} comprising the amino acid sequence of any one of SEQ ID NOs: 30 and 49, or a variant thereof having at least about 80% (such as at least about any of 80%, 85%, 90%, 95%, 96%, 97%, 98%, or 99%) sequence identity to the amino acid sequence of any one of SEQ ID NOs: 30 and 49.

In some embodiments, the first antigen-binding domain specifically binding to TRAIL comprises: a V_{H} comprising the amino acid sequence of any one of SEQ ID NOs: 17 and 37, or a variant thereof having at least about 80% (such as at least about any of 80%, 85%, 90%, 95%, 96%, 97%, 98%, or 99%) sequence identity to the amino acid sequence of any one of SEQ ID NOs: 17 and 37; and a V_{L} comprising the amino acid sequence of any one of SEQ ID NOs: 30 and 49, or a variant thereof having at least about 80% (such as at least about any of 80%, 85%, 90%, 95%, 96%, 97%, 98%, or 99%) sequence identity to the amino acid sequence of any one of SEQ ID NOs: 30 and 49.

In some embodiments, the first antigen-binding domain specifically binding to TRAIL comprises: a V_{H} comprising the amino acid sequence of any one of SEQ ID NOs: 18 and 38, or a variant thereof having at least about 80% (such as at least about any of 80%, 85%, 90%, 95%, 96%, 97%, 98%, or 99%) sequence identity to the amino acid sequence of any one of SEQ ID NOs: 18 and 38; and a V_{L} comprising the amino acid sequence of any one of SEQ ID NOs: 30 and 49, or a variant thereof having at least about 80% (such as at least about any of 80%, 85%, 90%, 95%, 96%, 97%, 98%, or 99%) sequence identity to the amino acid sequence of any one of SEQ ID NOs: 30 and 49.

In some embodiments, the first antigen-binding domain specifically binding to TRAIL comprises: a V_{H} comprising the amino acid sequence of any one of SEQ ID NOs: 19 and 39, or a variant thereof having at least about 80% (such as at least about any of 80%, 85%, 90%, 95%, 96%, 97%, 98%, or 99%) sequence identity to the amino acid sequence of any one of SEQ ID NOs: 19 and 39; and a V_{L} comprising the amino acid sequence of any one of SEQ ID NOs: 30 and 49, or a variant thereof having at least about 80% (such as at least about any of 80%, 85%, 90%, 95%, 96%, 97%, 98%, or 99%) sequence identity to the amino acid sequence of any one of SEQ ID NOs: 30 and 49.

In some embodiments, the first antigen-binding domain specifically binding to TRAIL comprises: a V_{H} comprising the amino acid sequence of any one of SEQ ID NOs: 20 and 40, or a variant thereof having at least about 80% (such as at least about any of 80%, 85%, 90%, 95%, 96%, 97%, 98%, or 99%) sequence identity to the amino acid sequence of any one of SEQ ID NOs: 20 and 40; and a V_{L} comprising the amino acid sequence of any one of SEQ ID NOs: 30 and 49, or a variant thereof having at least about 80% (such as at least about any of 80%, 85%, 90%, 95%, 96%, 97%, 98%, or 99%) sequence identity to the amino acid sequence of any one of SEQ ID NOs: 30 and 49.

In some embodiments, the first antigen-binding domain specifically binding to TRAIL comprises: a V_{H} comprising the amino acid sequence of any one of SEQ ID NOs: 16 and 36, or a variant thereof having at least about 80% (such as at least about any of 80%, 85%, 90%, 95%, 96%, 97%, 98%, or 99%) sequence identity to the amino acid sequence of any one of SEQ ID NOs: 16 and 36; and a V_{L} comprising the amino acid sequence of any one of SEQ ID NOs: 31 and 50, or a variant thereof having at least about 80% (such as at least about any of 80%, 85%, 90%, 95%, 96%, 97%, 98%, or 99%) sequence identity to the amino acid sequence of any one of SEQ ID NOs: 31 and 50.

In some embodiments, the first antigen-binding domain specifically binding to TRAIL comprises: a V_{H} comprising the amino acid sequence of any one of SEQ ID NOs: 17 and 37, or a variant thereof having at least about 80% (such as at least about any of 80%, 85%, 90%, 95%, 96%, 97%, 98%, or 99%) sequence identity to the amino acid sequence of any one of SEQ ID NOs: 17 and 37; and a V_{L} comprising the amino acid sequence of any one of SEQ ID NOs: 31 and 50, or a variant thereof having at least about 80% (such as at least about any of 80%, 85%, 90%, 95%, 96%, 97%, 98%, or 99%) sequence identity to the amino acid sequence of any one of SEQ ID NOs: 31 and 50.

In some embodiments, the first antigen-binding domain specifically binding to TRAIL comprises: a V_{H} comprising the amino acid sequence of any one of SEQ ID NOs: 18 and 38, or a variant thereof having at least about 80% (such as at least about any of 80%, 85%, 90%, 95%, 96%, 97%, 98%, or 99%) sequence identity to the amino acid sequence of any one of SEQ ID NOs: 18 and 38; and a V_{L} comprising the amino acid sequence of any one of SEQ ID NOs: 31 and 50, or a variant thereof having at least about 80% (such as at least about any of 80%, 85%, 90%, 95%, 96%, 97%, 98%, or 99%) sequence identity to the amino acid sequence of any one of SEQ ID NOs: 31 and 50.

In some embodiments, the first antigen-binding domain specifically binding to TRAIL comprises: a V_{H} comprising the amino acid sequence of any one of SEQ ID NOs: 19 and 39, or a variant thereof having at least about 80% (such as at least about any of 80%, 85%, 90%, 95%, 96%, 97%, 98%, or 99%) sequence identity to the amino acid sequence of any one of SEQ ID NOs: 19 and 39; and a V_{L} comprising the amino acid sequence of any one of SEQ ID NOs: 31 and 50, or a variant thereof having at least about 80% (such as at least about any of 80%, 85%, 90%, 95%, 96%, 97%, 98%, or 99%) sequence identity to the amino acid sequence of any one of SEQ ID NOs: 31 and 50.

In some embodiments, the first antigen-binding domain specifically binding to TRAIL comprises: a V_{H} comprising the amino acid sequence of any one of SEQ ID NOs: 16 and 36, or a variant thereof having at least about 80% (such as at least about any of 80%, 85%, 90%, 95%, 96%, 97%, 98%, or 99%) sequence identity to the amino acid sequence of any one of SEQ ID NOs: 16 and 36; and a V_{L} comprising the amino acid sequence of any one of SEQ ID NOs: 31 and 50, or a variant thereof having at least about 80% (such as at least about any of 80%, 85%, 90%, 95%, 96%, 97%, 98%, or 99%) sequence identity to the amino acid sequence of any one of SEQ ID NOs: 31 and 50.

In some embodiments, the first antigen-binding domain specifically binding to TRAIL comprises: a V_{H} comprising the amino acid sequence of SEQ ID NO: 164, or a variant thereof having at least about 80% (such as at least about any of 80%, 85%, 90%, 95%, 96%, 97%, 98%, or 99%) sequence identity to the amino acid sequence of SEQ ID NO: 164; and a V_{L} comprising the amino acid sequence of SEQ ID NO: 168, or a variant thereof having at least about 80% (such as at least about any of 80%, 85%, 90%, 95%, 96%, 97%, 98%, or 99%) sequence identity to the amino acid sequence of SEQ ID NO: 168.

In some embodiments, the first antigen-binding domain specifically binding to TRAIL comprises: a V_{H} comprising the amino acid sequence of SEQ ID NO: 166, or a variant thereof having at least about 80% (such as at least about any of 80%, 85%, 90%, 95%, 96%, 97%, 98%, or 99%) sequence identity to the amino acid sequence of SEQ ID NO: 166; and a V_{L} comprising the amino acid sequence of SEQ ID NO: 170, or a variant thereof having at least about 80% (such as at least about any of 80%, 85%, 90%, 95%, 96%, 97%, 98%, or 99%) sequence identity to the amino acid sequence of SEQ ID NO: 170.

In some embodiments, the second antigen-binding domain specifically binding to FasL comprises: a V_{H} comprising the amino acid sequence of any one of SEQ ID NOs: 63-72, 80-88, 163 and 165, or a variant thereof having at least about 80% sequence identity to the amino acid sequence of any one of SEQ ID NOs: 63-72, 80-88, 163 and 165; and a V_{L} comprising the amino acid sequence of any one of SEQ ID NOs: 73-79, 89-95, 167 and 169, or a variant thereof having at least about 80% sequence identity to the amino acid sequence of any one of SEQ ID NOs: 73-79, 89-95, 167 and 169.

In some embodiments, the second antigen-binding domain specifically binding to FasL comprises: a V_{H} comprising the amino acid sequence of any one of SEQ ID NOs: 63 and 80, or a variant thereof having at least about 80% (such as at least about any of 80%, 85%, 90%, 95%, 96%, 97%, 98%, or 99%) sequence identity to the amino acid sequence of any one of SEQ ID NOs: 63 and 80; and a V_{L} comprising the amino acid sequence of any one of SEQ ID NOs: 73 and 89, or a variant thereof having at least about 80% (such as at least about any of 80%, 85%, 90%, 95%, 96%, 97%, 98%, or 99%) sequence identity to the amino acid sequence of any one of SEQ ID NOs: 73 and 89.

In some embodiments, the second antigen-binding domain specifically binding to FasL comprises: a V_{H} comprising the amino acid sequence of any one of SEQ ID NOs: 64 and 81, or a variant thereof having at least about 80% (such as at least about any of 80%, 85%, 90%, 95%, 96%, 97%, 98%, or 99%) sequence identity to the amino acid sequence of any one of SEQ ID NOs: 64 and 81; and a V_{L} comprising the amino acid sequence of any one of SEQ ID NOs: 74 and 90, or a variant thereof having at least about 80% (such as at least about any of 80%, 85%, 90%, 95%, 96%, 97%, 98%, or 99%) sequence identity to the amino acid sequence of any one of SEQ ID NOs: 74 and 90.

In some embodiments, the second antigen-binding domain specifically binding to FasL comprises: a V_{H} comprising the amino acid sequence of any one of SEQ ID NOs: 65 and 81, or a variant thereof having at least about 80% (such as at least about any of 80%, 85%, 90%, 95%, 96%, 97%, 98%, or 99%) sequence identity to the amino acid sequence of any one of SEQ ID NOs: 65 and 81; and a V_{L} comprising the amino acid sequence of any one of SEQ ID NOs: 74 and 90, or a variant thereof having at least about 80% (such as at least about any of 80%, 85%, 90%, 95%, 96%, 97%, 98%, or 99%) sequence identity to the amino acid sequence of any one of SEQ ID NOs: 74 and 90.

In some embodiments, the second antigen-binding domain specifically binding to FasL comprises: a V_{H} comprising the amino acid sequence of any one of SEQ ID NOs: 64 and 81, or a variant thereof having at least about 80% (such as at least about any of 80%, 85%, 90%, 95%, 96%, 97%, 98%, or 99%) sequence identity to the amino acid sequence of any one of SEQ ID NOs: 64 and 81; and a V_{L} comprising the amino acid sequence of any one of SEQ ID NOs: 75 and 91, or a variant thereof having at least about 80% (such as at least about any of 80%, 85%, 90%, 95%, 96%, 97%, 98%, or 99%) sequence identity to the amino acid sequence of any one of SEQ ID NOs: 75 and 91.

In some embodiments, the second antigen-binding domain specifically binding to FasL comprises: a V_{H} comprising the amino acid sequence of any one of SEQ ID NOs: 65 and 81, or a variant thereof having at least about 80% (such as at least about any of 80%, 85%, 90%, 95%, 96%, 97%, 98%, or 99%) sequence identity to the amino acid sequence of any one of SEQ ID NOs: 65 and 81; and a V_{L} comprising the amino acid sequence of any one of SEQ ID NOs: 75 and 91, or a variant thereof having at least about 80% (such as at least about any of 80%, 85%, 90%, 95%, 96%, 97%, 98%, or 99%) sequence identity to the amino acid sequence of any one of SEQ ID NOs: 75 and 91.

In some embodiments, the second antigen-binding domain specifically binding to FasL comprises: a V_{H} comprising the amino acid sequence of any one of SEQ ID NOs: 66 and 82, or a variant thereof having at least about 80% (such as at least about any of 80%, 85%, 90%, 95%, 96%, 97%, 98%, or 99%) sequence identity to the amino acid sequence of any one of SEQ ID NOs: 66 and 82; and a V_{L} comprising the amino acid sequence of any one of SEQ ID NOs: 76 and 92, or a variant thereof having at least about 80% (such as at least about any of 80%, 85%, 90%, 95%, 96%, 97%, 98%, or 99%) sequence identity to the amino acid sequence of any one of SEQ ID NOs: 76 and 92.

In some embodiments, the second antigen-binding domain specifically binding to FasL comprises: a V_{H} comprising the amino acid sequence of any one of SEQ ID NOs: 67 and 83, or a variant thereof having at least about 80% (such as at least about any of 80%, 85%, 90%, 95%, 96%, 97%, 98%, or 99%) sequence identity to the amino acid sequence of any one of SEQ ID NOs: 67 and 83; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 68 and 84, or a variant thereof having at least about 80% (such as at least about any of 80%, 85%, 90%, 95%, 96%, 97%, 98%, or 99%) sequence identity to the amino acid sequence of any one of SEQ ID NOs: 68 and 84,; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 69 and 85, or a variant thereof having at least about 80% (such as at least about any of 80%, 85%, 90%, 95%, 96%, 97%, 98%, or 99%) sequence identity to the amino acid sequence of any one of SEQ ID NOs: 69 and 85; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 70 and 86, or a variant thereof having at least about 80% (such as at least about any of 80%, 85%, 90%, 95%, 96%, 97%, 98%, or 99%) sequence identity to the amino acid sequence of any one of SEQ ID NOs: 70 and 86; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 71 and 87, or a variant thereof having at least about 80% (such as at least about any of 80%, 85%, 90%, 95%, 96%, 97%, 98%, or 99%) sequence identity to the amino acid sequence of any one of SEQ ID NOs: 71 and 87; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 72 and 88, or a variant thereof having at least about 80% (such as at least about any of 80%, 85%, 90%, 95%, 96%, 97%, 98%, or 99%) sequence identity to the amino acid sequence of any one of SEQ ID NOs: 72 and 88; and a V_{L} comprising the amino acid sequence of any one of SEQ ID NOs: 77 and 93, or a variant thereof having at least about 80% (such as at least about any of 80%, 85%, 90%, 95%, 96%, 97%, 98%, or 99%) sequence identity to the amino acid sequence of any one of SEQ ID NOs: 77 and 93.

In some embodiments, the second antigen-binding domain specifically binding to FasL comprises: a V_{H} comprising the amino acid sequence of any one of SEQ ID NOs: 67 and 83, or a variant thereof having at least about 80% (such as at least about any of 80%, 85%, 90%, 95%, 96%, 97%, 98%, or 99%) sequence identity to the amino acid sequence of any one of SEQ ID NOs: 67 and 83; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 68 and 84, or a variant thereof having at least about 80% (such as at least about any of 80%, 85%, 90%, 95%, 96%, 97%, 98%, or 99%) sequence identity to the amino acid sequence of any one of SEQ ID NOs: 68 and 84,; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 69 and 85, or a variant thereof having at least about 80% (such as at least about any of 80%, 85%, 90%, 95%, 96%, 97%, 98%, or 99%) sequence identity to the amino acid sequence of any one of SEQ ID NOs: 69 and 85; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 70 and 86, or a variant thereof having at least about 80% (such as at least about any of 80%, 85%, 90%, 95%, 96%, 97%, 98%, or 99%) sequence identity to the amino acid sequence of any one of SEQ ID NOs: 70 and 86; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 71 and 87, or a variant thereof having at least about 80% (such as at least about any of 80%, 85%, 90%, 95%, 96%, 97%, 98%, or 99%) sequence identity to the amino acid sequence of any one of SEQ ID NOs: 71 and 87; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 72 and 88, or a variant thereof having at least about 80% (such as at least about any of 80%, 85%, 90%, 95%, 96%, 97%, 98%, or 99%) sequence identity to the amino acid sequence of any one of SEQ ID NOs: 72 and 88; and a V_{L} comprising the amino acid sequence of any one of SEQ ID NOs: 78 and 94, or a variant thereof having at least about 80% (such as at least about any of 80%, 85%, 90%, 95%, 96%, 97%, 98%, or 99%) sequence identity to the amino acid sequence of any one of SEQ ID NOs: 78 and 94.

In some embodiments, the second antigen-binding domain specifically binding to FasL comprises: a V_{H} comprising the amino acid sequence of any one of SEQ ID NOs: 67 and 83, or a variant thereof having at least about 80% (such as at least about any of 80%, 85%, 90%, 95%, 96%, 97%, 98%, or 99%) sequence identity to the amino acid sequence of any one of SEQ ID NOs: 67 and 83; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of any one of SEQ ID NOs: 68 and 84, or a variant thereof having at least about 80% (such as at least about any of 80%, 85%, 90%, 95%, 96%, 97%, 98%, or 99%) sequence identity to the amino acid sequence of any one of SEQ ID NOs: 68 and 84,; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of any one of SEQ ID NOs: 69 and 85, or a variant thereof having at least about 80% (such as at least about any of 80%, 85%, 90%, 95%, 96%, 97%, 98%, or 99%) sequence identity to the amino acid sequence of any one of SEQ ID NOs: 69 and 85; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of any one of SEQ ID NOs: 70 and 86, or a variant thereof having at least about 80% (such as at least about any of 80%, 85%, 90%, 95%, 96%, 97%, 98%, or 99%) sequence identity to the amino acid sequence of any one of SEQ ID NOs: 70 and 86; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of any one of SEQ ID NOs: 71 and 87, or a variant thereof having at least about 80% (such as at least about any of 80%, 85%, 90%, 95%, 96%, 97%, 98%, or 99%) sequence identity to the amino acid sequence of any one of SEQ ID NOs: 71 and 87; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of any one of SEQ ID NOs: 72 and 88, or a variant thereof having at least about 80% (such as at least about any of 80%, 85%, 90%, 95%, 96%, 97%, 98%, or 99%) sequence identity to the amino acid sequence of any one of SEQ ID NOs: 72 and 88; and a V_{L} comprising the amino acid sequence of any one of SEQ ID NOs: 79 and 95, or a variant thereof having at least about 80% (such as at least about any of 80%, 85%, 90%, 95%, 96%, 97%, 98%, or 99%) sequence identity to the amino acid sequence of any one of SEQ ID NOs: 79 and 95.

In some embodiments, the second antigen-binding domain specifically binding to FasL comprises: a V_{H} comprising the amino acid sequence of SEQ ID NO: 163, or a variant thereof having at least about 80% (such as at least about any of 80%, 85%, 90%, 95%, 96%, 97%, 98%, or 99%) sequence identity to the amino acid sequence of SEQ ID NO: 163; and a V_{L} comprising the amino acid sequence of SEQ ID NO: 167, or a variant thereof having at least about 80% (such as at least about any of 80%, 85%, 90%, 95%, 96%, 97%, 98%, or 99%) sequence identity to the amino acid sequence of SEQ ID NO: 167.

In some embodiments, the second antigen-binding domain specifically binding to FasL comprises: a V_{H} comprising the amino acid sequence of SEQ ID NO: 165, or a variant thereof having at least about 80% (such as at least about any of 80%, 85%, 90%, 95%, 96%, 97%, 98%, or 99%) sequence identity to the amino acid sequence of SEQ ID NO: 165; and a V_{L} comprising the amino acid sequence of SEQ ID NO: 169, or a variant thereof having at least about 80% (such as at least about any of 80%, 85%, 90%, 95%, 96%, 97%, 98%, or 99%) sequence identity to the amino acid sequence of SEQ ID NO: 169.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

I In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; and wherein the second antigen-binding domain comprises: a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167.

In some embodiments, this application provides a bispecific antibody comprising a first antigen-binding domain specifically binding to TRAIL, and a second antigen-binding domain specifically binding to FasL, wherein the first antigen-binding domain comprises: a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170; and wherein the second antigen-binding domain comprises: a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

### DVD-Ig format bispecific antibody

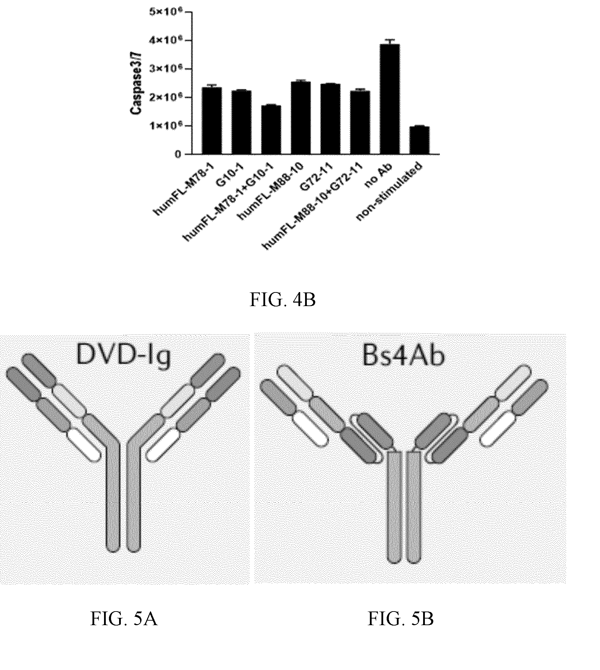

In some embodiments, any one of the bispecific antibody described herein has a dual variable region immunoglobulin molecule (DVD-Ig) format, which connects the V_{L} and V_{H} domains of another antibody respectively to the N-terminus of the V_{L} and V_{H} of a normal IgG antibody, forms an antigen-binding domain by the interaction between V_{H} and V_{L} of two antibodies, which can simultaneously bind to the corresponding antigen to achieve bispecific binding. In some embodiments, the DVD-IgG format is a homodimeric format, consisting of two identical monomers, each monomer comprising two antigen-binding domains, one of which is Fv and the other of which is Fab. The two domains described above are linked in series by a peptide linker (L). In some embodiments, the DVD-Ig format further comprises an Fc. An exemplary DVD-Ig format is shown in FIG. 5A.

In one embodiment of the application, one of the antigen-binding domains specifically binds to FasL and the other antigen-binding domain specifically binds to TRAIL. In some embodiments, the bispecific antibody can bind to TRAIL and FasL simultaneously.

In some embodiments, the bispecific antibody consists of two identical monomers, each of the monomer comprises two polypeptide chains, a heavy chain and a light chain, for a total of four polypeptide chains. Wherein, the heavy chain comprises V_{H}1-L-V_{H}2-C_{H}1 from N- terminus to C-terminus, the light chain comprises V_{L}1-L-V_{L}2-C_{L} from N- terminus to C-terminus. In some embodiments, the heavy chain *further* comprises an Fc comprising C_{H}2 and C_{H}3 domains. In other embodiments, the heavy chain comprises V_{H}1-L-V_{H}2-C_{H}1-C_{H}2-C_{H}3 from N- terminus to C-terminus, the light chain comprises V_{L}1-L-V_{L}2-C_{L} from N- terminus to C-terminus. Wherein the V_{H}1 and V_{L}1 are the heavy chain variable domain and light chain variable domain specifically binding to one of the antigens, respectively; the V_{H}2 and V_{L}2 are the heavy chain variable domain and light chain variable domain specifically binding to another antigen, respectively; L is a peptide linker; C_{H}1 is a heavy chain constant domain 1; C_{L} is a light chain constant domain. Wherein, the V_{H}1 and V_{L}1 form one of the antigen-binding domains (Fv) of the bispecific antibody; the V_{H}2-C_{H}1 and V_{L}2-C_{L} form the other antigen-binding domain (Fab) of the bispecific antibody.

In some embodiments, the V_{H}1 and V_{L}1 are the heavy chain variable domain and light chain variable domain specifically binding to FasL, respectively, *i.e.,* the V_{H}1 and V_{L}1 form the antigen-binding domain (Fv) specifically binding to FasL; the V_{H}2 and V_{L}2 are the heavy chain variable domain and light chain variable domain specifically binding to TRAIL, respectively, i.e., the V_{H}2 and V_{L}2 form the antigen-binding domain (Fab) specifically binding to TRAIL. In some embodiments, the V_{H}1 and V_{L}1 are the heavy chain variable domain and light chain variable domain specifically binding to TRAIL, respectively, *i.e.,* the V_{H}1 and V_{L}1 form the antigen-binding domain (Fv) specifically binding to TRAIL; the V_{H}2 and V_{L}2 are the heavy chain variable domain and light chain variable domain specifically binding to FasL, respectively, i.e., the V_{H}2 and V_{L}2 form the antigen-binding domain (Fab) specifically binding to FasL.

Peptide linker (or linker) sequence can be single amino acid or polypeptide sequence. In some embodiments, the peptide linker (or linker) comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, *i.e.,* GGGGSGGGGS (SEQ ID NO: 147), and (Gly₄Ser)₄, *i.e.,* GGGGSGGGGSGGGGSGGGGS(SEQ ID NO: 148). Another preferred Gly-Ser linker is (Gly₄Ser)₃, *i.e.,* GGGGSGGGGSGGGGS (SEQ ID NO: 149). In other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ). In some embodiments, the peptide linker can also be selected to comprise amino acid sequence ASTKGP (SEQ ID NO: 150) or *amino acid sequence* TVAAP (SEQ ID NO: 151).

In some embodiments, the bispecific antibody described in this application comprises: the amino acid sequence of SEQ ID NO: 104, or a variant thereof having at least about 80% (such as at least about any of 80%, 85%, 90%, 95%, 96%, 97%, 98%, or 99%) sequence identity to the amino acid sequence of SEQ ID NO: 104.

In some embodiments, the bispecific antibody described in this application comprises: the amino acid sequence of SEQ ID NO: 105, or a variant thereof having at least about 80% (such as at least about any of 80%, 85%, 90%, 95%, 96%, 97%, 98%, or 99%) sequence identity to the amino acid sequence of SEQ ID NO: 105.

In some embodiments, the bispecific antibody described in this application comprises: the amino acid sequence of SEQ ID NO: 106, or a variant thereof having at least about 80% (such as at least about any of 80%, 85%, 90%, 95%, 96%, 97%, 98%, or 99%) sequence identity to the amino acid sequence of SEQ ID NO: 106.

In some embodiments, the bispecific antibody described in this application comprises: the amino acid sequence of SEQ ID NO: 107, or a variant thereof having at least about 80% (such as at least about any of 80%, 85%, 90%, 95%, 96%, 97%, 98%, or 99%) sequence identity to the amino acid sequence of SEQ ID NO: 107.

In some embodiments, the bispecific antibody described in this application comprises: the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108.

In some embodiments, the bispecific antibody described in this application comprises: the amino acid sequence of SEQ ID NO: 109, or a variant thereof having at least about 80% (such as at least about any of 80%, 85%, 90%, 95%, 96%, 97%, 98%, or 99%) sequence identity to the amino acid sequence of SEQ ID NO: 109.

In some embodiments, the bispecific antibody described in this application comprises: the amino acid sequence of SEQ ID NO: 110, or a variant thereof having at least about 80% (such as at least about any of 80%, 85%, 90%, 95%, 96%, 97%, 98%, or 99%) sequence identity to the amino acid sequence of SEQ ID NO: 110.

In some embodiments, the bispecific antibody described in this application comprises: the amino acid sequence of SEQ ID NO: 111, or a variant thereof having at least about 80% (such as at least about any of 80%, 85%, 90%, 95%, 96%, 97%, 98%, or 99%) sequence identity to the amino acid sequence of SEQ ID NO: 111.

In some embodiments, the bispecific antibody described in this application comprises: the amino acid sequence of SEQ ID NO: 172, or a variant thereof having at least about 80% (such as at least about any of 80%, 85%, 90%, 95%, 96%, 97%, 98%, or 99%) sequence identity to the amino acid sequence of SEQ ID NO: 172.

In some embodiments, the bispecific antibody described in this application comprises: the amino acid sequence of SEQ ID NO: 174, or a variant thereof having at least about 80% (such as at least about any of 80%, 85%, 90%, 95%, 96%, 97%, 98%, or 99%) sequence identity to the amino acid sequence of SEQ ID NO: 174.

In some embodiments, the bispecific antibody described in this application comprises: the amino acid sequence of SEQ ID NO: 104, or a variant thereof having at least about 80% (such as at least about any of 80%, 85%, 90%, 95%, 96%, 97%, 98%, or 99%) sequence identity to the amino acid sequence of SEQ ID NO: 104; and/or the amino acid sequence of SEQ ID NO: 105, or a variant thereof having at least about 80% (such as at least about any of 80%, 85%, 90%, 95%, 96%, 97%, 98%, or 99%) sequence identity to the amino acid sequence of SEQ ID NO: 105.

In some embodiments, the bispecific antibody described in this application comprises: the amino acid sequence of SEQ ID NO: 104, or a variant thereof having at least about 80% (such as at least about any of 80%, 85%, 90%, 95%, 96%, 97%, 98%, or 99%) sequence identity to the amino acid sequence of SEQ ID NO: 104; and/or the amino acid sequence of SEQ ID NO: 106, or a variant thereof having at least about 80% (such as at least about any of 80%, 85%, 90%, 95%, 96%, 97%, 98%, or 99%) sequence identity to the amino acid sequence of SEQ ID NO: 106.

In some embodiments, the bispecific antibody described in this application comprises: the amino acid sequence of SEQ ID NO: 107, or a variant thereof having at least about 80% (such as at least about any of 80%, 85%, 90%, 95%, 96%, 97%, 98%, or 99%) sequence identity to the amino acid sequence of SEQ ID NO: 107; and/or the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108.

In some embodiments, the bispecific antibody described in this application comprises: the amino acid sequence of SEQ ID NO: 107, or a variant thereof having at least about 80% (such as at least about any of 80%, 85%, 90%, 95%, 96%, 97%, 98%, or 99%) sequence identity to the amino acid sequence of SEQ ID NO: 107; and/or the amino acid sequence of SEQ ID NO: 109, or a variant thereof having at least about 80% (such as at least about any of 80%, 85%, 90%, 95%, 96%, 97%, 98%, or 99%) sequence identity to the amino acid sequence of SEQ ID NO: 109.

In some embodiments, the bispecific antibody described in this application comprises: the amino acid sequence of SEQ ID NO: 110, or a variant thereof having at least about 80% (such as at least about any of 80%, 85%, 90%, 95%, 96%, 97%, 98%, or 99%) sequence identity to the amino acid sequence of SEQ ID NO: 110; and/or the amino acid sequence of SEQ ID NO: 111, or a variant thereof having at least about 80% (such as at least about any of 80%, 85%, 90%, 95%, 96%, 97%, 98%, or 99%) sequence identity to the amino acid sequence of SEQ ID NO: 111.

In some embodiments, the bispecific antibody described in this application comprises: the amino acid sequence of SEQ ID NO: 172, or a variant thereof having at least about 80% (such as at least about any of 80%, 85%, 90%, 95%, 96%, 97%, 98%, or 99%) sequence identity to the amino acid sequence of SEQ ID NO: 172; and/or the amino acid sequence of SEQ ID NO: 174, or a variant thereof having at least about 80% (such as at least about any of 80%, 85%, 90%, 95%, 96%, 97%, 98%, or 99%) sequence identity to the amino acid sequence of SEQ ID NO: 174.

In some embodiments, the bispecific antibody described in this application comprises: (a) the amino acid sequence of SEQ ID NO: 131, or a variant thereof having at least about 80% (such as at least about any of 80%, 85%, 90%, 95%, 96%, 97%, 98%, or 99%) sequence identity to the amino acid sequence of SEQ ID NO: 131; and/or the amino acid sequence of SEQ ID NO: 105, or a variant thereof having at least about 80% (such as at least about any of 80%, 85%, 90%, 95%, 96%, 97%, 98%, or 99%) sequence identity to the amino acid sequence of SEQ ID NO: 105.

In some embodiments, the bispecific antibody described in this application comprises: the amino acid sequence of SEQ ID NO: 131, or a variant thereof having at least about 80% (such as at least about any of 80%, 85%, 90%, 95%, 96%, 97%, 98%, or 99%) sequence identity to the amino acid sequence of SEQ ID NO: 131; and/or the amino acid sequence of SEQ ID NO: 106, or a variant thereof having at least about 80% (such as at least about any of 80%, 85%, 90%, 95%, 96%, 97%, 98%, or 99%) sequence identity to the amino acid sequence of SEQ ID NO: 106.

In some embodiments, the bispecific antibody described in this application comprises: the amino acid sequence of SEQ ID NO: 132, or a variant thereof having at least about 80% (such as at least about any of 80%, 85%, 90%, 95%, 96%, 97%, 98%, or 99%) sequence identity to the amino acid sequence of SEQ ID NO: 132; and/or the amino acid sequence of SEQ ID NO: 108, or a variant thereof having at least about 80% (such as at least about any of 80%, 85%, 90%, 95%, 96%, 97%, 98%, or 99%) sequence identity to the amino acid sequence of SEQ ID NO: 108.

In some embodiments, the bispecific antibody described in this application comprises: the amino acid sequence of SEQ ID NO: 132, or a variant thereof having at least about 80% (such as at least about any of 80%, 85%, 90%, 95%, 96%, 97%, 98%, or 99%) sequence identity to the amino acid sequence of SEQ ID NO: 132; and/or the amino acid sequence of SEQ ID NO: 109, or a variant thereof having at least about 80% (such as at least about any of 80%, 85%, 90%, 95%, 96%, 97%, 98%, or 99%) sequence identity to the amino acid sequence of SEQ ID NO: 109.

In some embodiments, the bispecific antibody described in this application comprises: the amino acid sequence of SEQ ID NO: 133, or a variant thereof having at least about 80% (such as at least about any of 80%, 85%, 90%, 95%, 96%, 97%, 98%, or 99%) sequence identity to the amino acid sequence of SEQ ID NO: 133; and/or the amino acid sequence of SEQ ID NO: 111, or a variant thereof having at least about 80% (such as at least about any of 80%, 85%, 90%, 95%, 96%, 97%, 98%, or 99%) sequence identity to the amino acid sequence of SEQ ID NO: 111.

In some embodiments, the bispecific antibody described in this application comprises: the amino acid sequence of SEQ ID NO: 176, or a variant thereof having at least about 80% (such as at least about any of 80%, 85%, 90%, 95%, 96%, 97%, 98%, or 99%) sequence identity to the amino acid sequence of SEQ ID NO: 176; and/or the amino acid sequence of SEQ ID NO: 174, or a variant thereof having at least about 80% (such as at least about any of 80%, 85%, 90%, 95%, 96%, 97%, 98%, or 99%) sequence identity to the amino acid sequence of SEQ ID NO: 174.

### Bs4Ab format bispecific antibody

In some embodiments, the bispecific antibody described herein has Bs4Ab format, which consists of two identical monomers, each monomer comprising two antigen-binding domains, one of which is Fab and the other of which is scFv. In some embodiments, the bispecific antibody further comprises an Fc comprising C_{H}2 and C_{H}3 domains. The scFv connects to the Fab through the first peptide linker (L1) and to the Fc through the second peptide linker (L2). An exemplary Bs4Ab format is shown in FIG. 5B.

In some embodiments of the application, one of the antigen-binding domains (Fab or scFv) specifically binds to FasL and the other antigen-binding domain (scFv or Fab) specifically binds to TRAIL. In some embodiments, the bispecific antibody can bind to TRAIL and FasL simultaneously.

In some embodiments, the bispecific antibody described herein consists of two identical monomers, each of which comprises two polypeptide chains, a heavy chain and a light chain, for a total of four polypeptide chains. In some embodiments, the heavy chain of the bispecific antibody comprises V_{H}1-C_{H}1-L1-V_{H}2-L3-V_{L}2 structure from N- terminus to C-terminus. In other embodiments, the heavy chain of the bispecific antibody comprises V_{H}1-C_{H}1-L1-V_{L}2-L3-V_{H}2 structure from N- terminus to C-terminus. In some embodiments, the heavy chain further comprises an Fc comprising C_{H}2 and C_{H}3 domains. In some embodiments, the heavy chain of the bispecific antibody comprises V_{H}1-C_{H}1-L1-V_{H}2-L3-V_{L}2-L2-C_{H}2-C_{H}3 structure from N-terminus to C-terminus. In other embodiments, the heavy chain of the bispecific antibody comprises V_{H}1-C_{H}1-L1-V_{L}2-L3-V_{H}2-L2-C_{H}2-C_{H}3 structure from N- terminus to C-terminus. In some embodiments, the light chain of the bispecific antibody comprises V_{L}1-C_{L} structure from N- terminus to C-terminus. Wherein, the V_{H}1 and V_{L}1 are the heavy chain variable domain and light chain variable domain specifically binding to one of the antigens, respectively; the V_{H}2 and V_{L}2 are the heavy chain variable domain and light chain variable domain specifically binding to the other antigen, respectively; C_{H}1 is a heavy chain constant domain 1; C_{L} is a light chain constant domain; L1, L2 and L3 are peptide linkers. The V_{H}1-C_{H}1 and V_{L}l-C_{L} form one of the antigen-binding domains (Fab) of the bispecific antibody; the V_{H}2-L3-V_{L}2 or V_{L}2-L3-V_{H}2 form the other antigen-binding domain (scFv) of the bispecific antibody.

In some embodiments, the V_{H}1 and V_{L}1 are the heavy chain variable domain and light chain variable domain specifically binding to FasL, respectively, *i.e.,* the V_{H}1-C_{H}1 and V_{L}1-C_{L} form the antigen-binding domain (Fab) specifically binding to FasL; the V_{H}2 and V_{L}2 are the heavy chain variable domain and light chain variable domain specifically binding to TRAIL, respectively, *i.e.,* the V_{H}2-L3-V_{L}2 or V_{L}2-L3-V_{H}2 form the antigen-binding domain (scFv) specifically binding to TRAIL. In other embodiments, the V_{H}1 and V_{L}1 are the heavy chain variable domain and light chain variable domain specifically binding to TRAIL, respectively, *i.e.,* the V_{H}1-C_{H}1 and V_{L}1-C_{L} form the antigen-binding domain (Fab) specifically binding to TRAIL; the V_{H}2 and V_{L}2 are the heavy chain variable domain and light chain variable domain specifically binding to FasL, respectively, *i.e.,* the V_{H}2-L3-V_{L}2 or V_{L}2-L3-V_{H}2 form the antigen-binding domain (scFv) specifically binding to FasL. In some embodiments, the bispecific antibody comprises C_{H}1 comprising amino acid sequence SEQ ID No: 102 or 103. In some embodiments, the bispecific antibody comprises C_{H}2-C_{H}3 comprising amino acid sequence SEQ ID No: 100 or 101.

In some embodiments, the antigen-binding domain scFv specifically binding to TRAIL or FasL comprises genetic engineering cysteine mutations. Bispecific antibodies with disulfide bond stability are obtained through introducing two cysteine mutations at the V_{H} and V_{L} interfaces.

Peptide linker (or linker) may be used to join domains and/or regions of the chimeric heavy chain of the bispecific antibody into a contiguous molecule. In some embodiments, the bispecific antibody includes at least two peptide linkers. In some embodiments, the bispecific antibody may include additional linkers, such as a flexible linker interconnecting the variable heavy and light chains of an scFv. In some embodiments, the bispecific antibody may include additional linkers, such as a flexible linker interconnecting the variable heavy and light chains of an scFv and other linkers that connect other binding units to the core structure of the bispecific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (e.g., the same or different length and/or amino acid sequence).

In some aspects, the peptide linker comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, *i.e.,* GGGGSGGGGS(SEQ ID NO: 147), and (Gly₄Ser)₄, *i.e.,* GGGGSGGGGSGGGGSGGGGS(SEQ ID NO: 148). Another preferred Gly-Ser linker is (Gly₄Ser)₃, *i.e.,,* GGGGSGGGGSGGGGS (SEQ ID NO: 149). In other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ).

In some embodiments, L1 and/or L2 include both a hinge portion and a linker portion, such as a linker portion comprising a Gly-Ser linker. In other aspects, L1 and/or L2 include only a hinge portion or only a linker portion, such as a Gly-Ser linker. In some embodiments, L1 and L2 include a Gly-Ser linker. In certain aspects, the Gly-Ser linker portion of L1 and L2 is the same length, whereas in other aspects, the Gly-Ser linker portion of LI and L2 are different lengths. When a bispecific molecule comprises an scFv, the heavy and light chains of the scFv may be connected by a flexible linker. In some embodiments, this flexible linker generally does not include a hinge portion, but rather, is a Gly-Ser linker or other flexible linker. The length and amino acid sequence of a flexible linker interconnecting the scFv domain may be selected and optimized.

In some embodiments, the peptide linker (for example L1 and/or L2) comprises a Gly-Ser or all Gly linker and a portion or modified portion of a hinge domain. In some aspects, the peptide linker (L1) connecting one of the antigen-binding domains (e.g. the Fab or scFv) to the other antigen-binding domain (e.g. scFv or Fab) of the bispecific antibody comprises the amino acid sequence EPKSDKTGGGGSGGGGS (SEQ ID NO: 152) or EPKSCGKTGGGGSGGGGS (SEQ ID NO: 153) or EPKSCGGGGSGGGGS (SEQ ID NO: 154). In some aspects the peptide linker (L2) connecting the antigen-binding domain scFv to the Fc domain of the bispecific antibody comprises the amino acid sequence GGGGSGGGGSEPKSDKTHTCPPCP (SEQ ID NO: 155) or GGGGSGGGGSCPPCP (SEQ ID NO: 156) or GGGGSGGGGSDKTHTCPPCP (SEQ ID NO: 157) or GGGGSGGGGSESKYGPPCPPCP (SEQ ID NO: 160).

In some embodiments, regardless of the peptide linker used to interconnect one antigen-binding domain to the other antigen-binding domain or one of the antigen-binding domains to Fc (e.g., L1 and L2), the bispecific antibody may optionally comprise additional peptide linkers. The lengths and sequence of such additional peptide linkers are independently selected. For example, the bispecific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 148).

In some embodiments, the bispecific antibody described in this application comprises: the amino acid sequence of SEQ ID NO: 112, or a variant thereof having at least about 80% (such as at least about any of 80%, 85%, 90%, 95%, 96%, 97%, 98%, or 99%) sequence identity to the amino acid sequence of SEQ ID NO: 112.

In some embodiments, the bispecific antibody described in this application comprises: he amino acid sequence of SEQ ID NO: 113, or a variant thereof having at least about 80% (such as at least about any of 80%, 85%, 90%, 95%, 96%, 97%, 98%, or 99%) sequence identity to the amino acid sequence of SEQ ID NO: 113.

In some embodiments, the bispecific antibody described in this application comprises: the amino acid sequence of SEQ ID NO: 114, or a variant thereof having at least about 80% (such as at least about any of 80%, 85%, 90%, 95%, 96%, 97%, 98%, or 99%) sequence identity to the amino acid sequence of SEQ ID NO: 114.

In some embodiments, the bispecific antibody described in this application comprises: the amino acid sequence of SEQ ID NO: 115, or a variant thereof having at least about 80% (such as at least about any of 80%, 85%, 90%, 95%, 96%, 97%, 98%, or 99%) sequence identity to the amino acid sequence of SEQ ID NO: 115.

In some embodiments, the bispecific antibody described in this application comprises: the amino acid sequence of SEQ ID NO: 116, or a variant thereof having at least about 80% (such as at least about any of 80%, 85%, 90%, 95%, 96%, 97%, 98%, or 99%) sequence identity to the amino acid sequence of SEQ ID NO: 116.

In some embodiments, the bispecific antibody described in this application comprises: the amino acid sequence of SEQ ID NO: 117, or a variant thereof having at least about 80% (such as at least about any of 80%, 85%, 90%, 95%, 96%, 97%, 98%, or 99%) sequence identity to the amino acid sequence of SEQ ID NO: 117.

In some embodiments, the bispecific antibody described in this application comprises: the amino acid sequence of SEQ ID NO: 118, or a variant thereof having at least about 80% (such as at least about any of 80%, 85%, 90%, 95%, 96%, 97%, 98%, or 99%) sequence identity to the amino acid sequence of SEQ ID NO: 118.

In some embodiments, the bispecific antibody described in this application comprises: the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 120, or a variant thereof having at least about 80% (such as at least about any of 80%, 85%, 90%, 95%, 96%, 97%, 98%, or 99%) sequence identity to the amino acid sequence of SEQ ID NO: 120.

In some embodiments, the bispecific antibody described in this application comprises: the amino acid sequence of SEQ ID NO: 121, or a variant thereof having at least about 80% (such as at least about any of 80%, 85%, 90%, 95%, 96%, 97%, 98%, or 99%) sequence identity to the amino acid sequence of SEQ ID NO: 121.

In some embodiments, the bispecific antibody described in this application comprises: the amino acid sequence of SEQ ID NO: 122, or a variant thereof having at least about 80% (such as at least about any of 80%, 85%, 90%, 95%, 96%, 97%, 98%, or 99%) sequence identity to the amino acid sequence of SEQ ID NO: 122.

In some embodiments, the bispecific antibody described in this application comprises: the amino acid sequence of SEQ ID NO: 123, or a variant thereof having at least about 80% (such as at least about any of 80%, 85%, 90%, 95%, 96%, 97%, 98%, or 99%) sequence identity to the amino acid sequence of SEQ ID NO: 123.

In some embodiments, the bispecific antibody described in this application comprises: the amino acid sequence of SEQ ID NO: 124, or a variant thereof having at least about 80% (such as at least about any of 80%, 85%, 90%, 95%, 96%, 97%, 98%, or 99%) sequence identity to the amino acid sequence of SEQ ID NO: 124.

In some embodiments, the bispecific antibody described in this application comprises: the amino acid sequence of SEQ ID NO: 171, or a variant thereof having at least about 80% (such as at least about any of 80%, 85%, 90%, 95%, 96%, 97%, 98%, or 99%) sequence identity to the amino acid sequence of SEQ ID NO: 171.

In some embodiments, the bispecific antibody described in this application comprises: the amino acid sequence of SEQ ID NO: 173, or a variant thereof having at least about 80% (such as at least about any of 80%, 85%, 90%, 95%, 96%, 97%, 98%, or 99%) sequence identity to the amino acid sequence of SEQ ID NO: 173.

In some embodiments, the bispecific antibody described in this application comprises: the amino acid sequence of SEQ ID NO: 112, or a variant thereof having at least about 80% (such as at least about any of 80%, 85%, 90%, 95%, 96%, 97%, 98%, or 99%) sequence identity to the amino acid sequence of SEQ ID NO: 112; and/or the amino acid sequence of SEQ ID NO: 113, or a variant thereof having at least about 80% (such as at least about any of 80%, 85%, 90%, 95%, 96%, 97%, 98%, or 99%) sequence identity to the amino acid sequence of SEQ ID NO: 113.

In some embodiments, the bispecific antibody described in this application comprises: the amino acid sequence of SEQ ID NO: 114, or a variant thereof having at least about 80% (such as at least about any of 80%, 85%, 90%, 95%, 96%, 97%, 98%, or 99%) sequence identity to the amino acid sequence of SEQ ID NO: 114; and/or the amino acid sequence of SEQ ID NO: 115, or a variant thereof having at least about 80% (such as at least about any of 80%, 85%, 90%, 95%, 96%, 97%, 98%, or 99%) sequence identity to the amino acid sequence of SEQ ID NO: 115.

In some embodiments, the bispecific antibody described in this application comprises: the amino acid sequence of SEQ ID NO: 116, or a variant thereof having at least about 80% (such as at least about any of 80%, 85%, 90%, 95%, 96%, 97%, 98%, or 99%) sequence identity to the amino acid sequence of SEQ ID NO: 116; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 117, or a variant thereof having at least about 80% (such as at least about any of 80%, 85%, 90%, 95%, 96%, 97%, 98%, or 99%) sequence identity to the amino acid sequence of SEQ ID NO: 117; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 118, or a variant thereof having at least about 80% (such as at least about any of 80%, 85%, 90%, 95%, 96%, 97%, 98%, or 99%) sequence identity to the amino acid sequence of SEQ ID NO: 118; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 120, or a variant thereof having at least about 80% (such as at least about any of 80%, 85%, 90%, 95%, 96%, 97%, 98%, or 99%) sequence identity to the amino acid sequence of SEQ ID NO: 120; and/or the amino acid sequence of SEQ ID NO: 121, or a variant thereof having at least about 80% (such as at least about any of 80%, 85%, 90%, 95%, 96%, 97%, 98%, or 99%) sequence identity to the amino acid sequence of SEQ ID NO: 121.

In some embodiments, the bispecific antibody described in this application comprises: the amino acid sequence of SEQ ID NO: 120, or a variant thereof having at least about 80% (such as at least about any of 80%, 85%, 90%, 95%, 96%, 97%, 98%, or 99%) sequence identity to the amino acid sequence of SEQ ID NO: 120; and/or the amino acid sequence of SEQ ID NO: 122, or a variant thereof having at least about 80% (such as at least about any of 80%, 85%, 90%, 95%, 96%, 97%, 98%, or 99%) sequence identity to the amino acid sequence of SEQ ID NO: 122.

In some embodiments, the bispecific antibody described in this application comprises: the amino acid sequence of SEQ ID NO: 123, or a variant thereof having at least about 80% sequence identity to the amino acid sequence of SEQ ID NO: 123; and/or the amino acid sequence of SEQ ID NO: 124, or a variant thereof having at least about 80% (such as at least about any of 80%, 85%, 90%, 95%, 96%, 97%, 98%, or 99%) sequence identity to the amino acid sequence of SEQ ID NO: 124.

In some embodiments, the bispecific antibody described in this application comprises: the amino acid sequence of SEQ ID NO: 171, or a variant thereof having at least about 80% (such as at least about any of 80%, 85%, 90%, 95%, 96%, 97%, 98%, or 99%) sequence identity to the amino acid sequence of SEQ ID NO: 171; and/or the amino acid sequence of SEQ ID NO: 173, or a variant thereof having at least about 80% sequence identity to the amino acid sequence of SEQ ID NO: 173.

In some embodiments, the bispecific antibody described in this application comprises: the amino acid sequence of SEQ ID NO: 134, or a variant thereof having at least about 80% (such as at least about any of 80%, 85%, 90%, 95%, 96%, 97%, 98%, or 99%) sequence identity to the amino acid sequence of SEQ ID NO: 134; and/or the amino acid sequence of SEQ ID NO: 113, or a variant thereof having at least about 80% (such as at least about any of 80%, 85%, 90%, 95%, 96%, 97%, 98%, or 99%) sequence identity to the amino acid sequence of SEQ ID NO: 113.

In some embodiments, the bispecific antibody described in this application comprises: the amino acid sequence of SEQ ID NO: 135, or a variant thereof having at least about 80% sequence identity to the amino acid sequence of SEQ ID NO: 135; and/or the amino acid sequence of SEQ ID NO: 113, or a variant thereof having at least about 80% (such as at least about any of 80%, 85%, 90%, 95%, 96%, 97%, 98%, or 99%) sequence identity to the amino acid sequence of SEQ ID NO: 113.

In some embodiments, the bispecific antibody described in this application comprises: the amino acid sequence of SEQ ID NO: 136, or a variant thereof having at least about 80% (such as at least about any of 80%, 85%, 90%, 95%, 96%, 97%, 98%, or 99%) sequence identity to the amino acid sequence of SEQ ID NO: 136; and/or the amino acid sequence of SEQ ID NO: 115, or a variant thereof having at least about 80% sequence identity to the amino acid sequence of SEQ ID NO: 115.

In some embodiments, the bispecific antibody described in this application comprises: the amino acid sequence of SEQ ID NO: 137, or a variant thereof having at least about 80% (such as at least about any of 80%, 85%, 90%, 95%, 96%, 97%, 98%, or 99%) sequence identity to the amino acid sequence of SEQ ID NO: 137; and/or the amino acid sequence of SEQ ID NO: 115, or a variant thereof having at least about 80% (such as at least about any of 80%, 85%, 90%, 95%, 96%, 97%, 98%, or 99%) sequence identity to the amino acid sequence of SEQ ID NO: 115.

In some embodiments, the bispecific antibody described in this application comprises: the amino acid sequence of SEQ ID NO: 138, or a variant thereof having at least about 80% (such as at least about any of 80%, 85%, 90%, 95%, 96%, 97%, 98%, or 99%) sequence identity to the amino acid sequence of SEQ ID NO: 138; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 139, or a variant thereof having at least about 80% (such as at least about any of 80%, 85%, 90%, 95%, 96%, 97%, 98%, or 99%) sequence identity to the amino acid sequence of SEQ ID NO: 139; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 140, or a variant thereof having at least about 80% (such as at least about any of 80%, 85%, 90%, 95%, 96%, 97%, 98%, or 99%) sequence identity to the amino acid sequence of SEQ ID NO: 140; and/or the amino acid sequence of SEQ ID NO: 119, or a variant thereof having at least about 80% (such as at least about any of 80%, 85%, 90%, 95%, 96%, 97%, 98%, or 99%) sequence identity to the amino acid sequence of SEQ ID NO: 119.

In some embodiments, the bispecific antibody described in this application comprises: the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141; and/or the amino acid sequence of SEQ ID NO: 121, or a variant thereof having at least about 80% (such as at least about any of 80%, 85%, 90%, 95%, 96%, 97%, 98%, or 99%) sequence identity to the amino acid sequence of SEQ ID NO: 121.

In some embodiments, the bispecific antibody described in this application comprises: the amino acid sequence of SEQ ID NO: 141, or a variant thereof having at least about 80% (such as at least about any of 80%, 85%, 90%, 95%, 96%, 97%, 98%, or 99%) sequence identity to the amino acid sequence of SEQ ID NO: 141; and/or the amino acid sequence of SEQ ID NO: 122, or a variant thereof having at least about 80% (such as at least about any of 80%, 85%, 90%, 95%, 96%, 97%, 98%, or 99%) sequence identity to the amino acid sequence of SEQ ID NO: 122.

In some embodiments, the bispecific antibody described in this application comprises: the amino acid sequence of SEQ ID NO: 142, or a variant thereof having at least about 80% sequence identity to the amino acid sequence of SEQ ID NO: 142; and/or the amino acid sequence of SEQ ID NO: 124, or a variant thereof having at least about 80% (such as at least about any of 80%, 85%, 90%, 95%, 96%, 97%, 98%, or 99%) sequence identity to the amino acid sequence of SEQ ID NO: 124.

In some embodiments, the bispecific antibody described in this application comprises: the amino acid sequence of SEQ ID NO: 175, or a variant thereof having at least about 80% (such as at least about any of 80%, 85%, 90%, 95%, 96%, 97%, 98%, or 99%) sequence identity to the amino acid sequence of SEQ ID NO: 175; and/or the amino acid sequence of SEQ ID NO: 173, or a variant thereof having at least about 80% (such as at least about any of 80%, 85%, 90%, 95%, 96%, 97%, 98%, or 99%) sequence identity to the amino acid sequence of SEQ ID NO: 173.

### Hetero H, CrossMab format bispecific

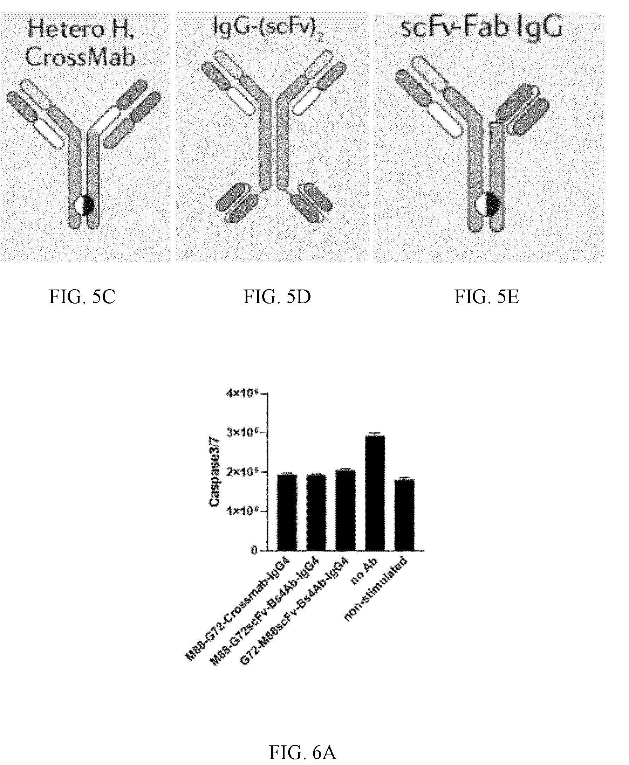

In some embodiments, any one of the bispecific antibody described herein has Hetero H, CrossMab format, which is a bivalent bispecific antibody consisting of heterodimer, comprising two antigen-binding domains Fab. In other embodiments, the bispecific antibody further comprises two Fc domains comprising C_{H}2 and C_{H}3 domains. In some embodiments, the amino acid residue in C_{H}3 domain of one of the Fc is substituted with a larger side chain volume amino acid residue to form a "knob", the amino acid residue in C_{H}3 domain of the other Fc is substituted with a smaller side chain volume amino acid residue to form a "hole", which can promote the binding of heterodimers. Wherein, in Fab, the location of the light chain constant domain (C_{L}) and the heavy chain constant domain 1 (C_{H}1) can be exchanged with each other; or the location of the heavy chain variable domain (V_{H} ) and the light chain variable domain (V_{L}) can be exchanged with each other; or the light chain constant domain (C_{L}) and the heavy chain constant domain 1 (C_{H}1) as well as the heavy chain variable domain (V_{H} ) and the light chain variable domain (V_{L}) can be exchanged with each other simultaneously. An exemplary Hetero H, CrossMab format is shown in FIG. 5C.

In some embodiments, the Fc is derived from wild-type human IgG1 Fc or IgG4 Fc. In other embodiments, the C_{H}3 domain of the IgG1 Fc or IgG4 Fc comprises, but is not limited to the amino acid substitution described below: S354C, T366W, Y349C, T366S, L368A and/or Y407V, wherein the numbering is according to EU numbering system of Kabat. In other embodiments, the IgG4 Fc further comprises the S228P amino acid substitution, wherein the numbering is according to EU numbering system of Kabat.

In other embodiments of the application, one of the antigen-binding domains specifically binds to TRAIL and the other antigen-binding domain specifically binds to FasL. In some embodiments, the bispecific antibody can bind to TRAIL and FasL simultaneously.

In some embodiments, the bispecific antibody described herein has Hetero H, CrossMab format, which is a heterodimer consisting of two different monomers, each of which comprises two polypeptide chains. Wherein the first monomer comprises the first heavy chain and the first light chain specifically binding to one of the antigens, and the second monomer comprises the second heavy chain and the second light chain specifically binding to the other antigen. In some embodiments, the first heavy chain of the bispecific antibody comprises V_{H}1-C_{H}1 structure from N- terminus to C-terminus; the first light chain of the bispecific antibody comprises V_{L}1-C_{L} structure from N- terminus to C-terminus. In some embodiments, the first heavy chain further comprises an Fc comprising C_{H}2 and C_{H}3 domains. In some embodiments, the first heavy chain of the bispecific antibody comprises V_{H}1-C_{H}1-C_{H}2-C_{H}3 structure from N- terminus to C-terminus; the first light chain of the bispecific antibody comprises V_{L}1-C_{L} structure from N-terminus to C-terminus. The V_{H}1 and V_{L}1 are the heavy chain variable domain and light chain variable domain specifically binding to one of the antigens, respectively, C_{H}1 is a heavy chain constant domain 1, C_{L} is a light chain constant domain. Wherein, the V_{H}1-C_{H}1 and V_{L}1-C_{L} form the antigen-binding domain (Fab) binding to one of the antigens. In some embodiments, the second heavy chain of the bispecific antibody comprises V_{H}2-C_{L} structure from N- terminus to C-terminus; the second light chain of the bispecific antibody comprises V_{L}2-C_{H}1 structure from N- terminus to C-terminus. In some embodiments, the second heavy chain further comprises an Fc comprising C_{H}2 and C_{H}3 domains. In some embodiments, the second heavy chain of the bispecific antibody comprises V_{H}2-C_{L}-C_{H}2-C_{H}3 structure from N- terminus to C-terminus; the second light chain of the bispecific antibody comprises V_{L}2-C_{H}1 structure from N- terminus to C-terminus. The V_{H}2 and V_{L}2 are the heavy chain variable domain and light chain variable domain specifically binding to the other antigen, respectively, C_{H}1 is a heavy chain constant domain 1, C_{L} is a light chain constant domain. Wherein, the V_{H}2-C_{L} and V_{L}2-C_{H}1 form the antigen-binding domain (Fab) binding to the other antigen. In some embodiments, the location of C_{L} and C_{H}1 of the first monomer or the second monomer of the bispecific antibody can be exchanged with each other. In some embodiments, the location of V_{H}1 and V_{L}1 can be exchanged with each other. In other embodiments, the location of V_{H}2 and V_{L}2 can be exchanged with each other. In some embodiments, the amino acid residue in C_{H}3 domain of the first heavy chain is substituted with a larger side chain volume amino acid residue to form a "knob", and the amino acid residue in C_{H}3 domain of the second heavy chain is substituted with a smaller side chain volume amino acid residue to form a "hole". In other embodiments, the amino acid residue in C_{H}3 domain of the second heavy chain is substituted with a larger side chain volume amino acid residue to form a "knob", and the amino acid residue in C_{H}3 domain of the first heavy chain is substituted with a smaller side chain volume amino acid residue to form a "hole". In some embodiments, the C_{H}3 domain comprises, but is not limited to the amino acid substitution described below: S354C, T366W, Y349C, T366S, L368A and/or Y407V, wherein the numbering is according to EU numbering system of Kabat.

In some embodiments, the V_{H}1 and V_{L}1 are the heavy chain variable domain and light chain variable domain specifically binding to FasL, respectively, the V_{H}2 and V_{L}2 are the heavy chain variable domain and light chain variable domain specifically binding to TRAIL, respectively, *i.e.,* the V_{H}1-C_{H}1 and V_{L}1-C_{L} form the antigen-binding domain (Fab) specifically binding to FasL, V_{H}2-C_{L} and V_{L}2-C_{H}1 form the antigen-binding domain (Fab) specifically binding to TRAIL. In another embodiment, the V_{H}1 and V_{L}1 are the heavy chain variable domain and light chain variable domain specifically binding to TRAIL, respectively, the V_{H}2 and V_{L}2 are the heavy chain variable domain and light chain variable domain specifically binding to FasL, respectively, *i.e.,* the V_{H}1-C_{H}1 and V_{L}1-C_{L} form the antigen-binding domain (Fab) specifically binding to TRAIL, V_{H}2-C_{L} and V_{L}2-C_{H}1 form the antigen-binding domain (Fab) specifically binding to FasL.

In some embodiments, the bispecific antibody described in this application comprises: the amino acid sequence of SEQ ID NO: 125, or a variant thereof having at least about 80% (such as at least about any of 80%, 85%, 90%, 95%, 96%, 97%, 98%, or 99%) sequence identity to the amino acid sequence of SEQ ID NO: 125.

In some embodiments, the bispecific antibody described in this application comprises: the amino acid sequence of SEQ ID NO: 126, or a variant thereof having at least about 80% (such as at least about any of 80%, 85%, 90%, 95%, 96%, 97%, 98%, or 99%) sequence identity to the amino acid sequence of SEQ ID NO: 126.

In some embodiments, the bispecific antibody described in this application comprises: the amino acid sequence of SEQ ID NO: 127, or a variant thereof having at least about 80% (such as at least about any of 80%, 85%, 90%, 95%, 96%, 97%, 98%, or 99%) sequence identity to the amino acid sequence of SEQ ID NO: 127.

In some embodiments, the bispecific antibody described in this application comprises: the amino acid sequence of SEQ ID NO: 128, or a variant thereof having at least about 80% (such as at least about any of 80%, 85%, 90%, 95%, 96%, 97%, 98%, or 99%) sequence identity to the amino acid sequence of SEQ ID NO: 128.

In some embodiments, the bispecific antibody described in this application comprises: the amino acid sequence of SEQ ID NO: 129 or a variant thereof having at least about 80% (such as at least about any of 80%, 85%, 90%, 95%, 96%, 97%, 98%, or 99%) sequence identity to the amino acid sequence of SEQ ID NO: 129.

In some embodiments, the bispecific antibody described in this application comprises: the amino acid sequence of SEQ ID NO: 130, or a variant thereof having at least about 80% (such as at least about any of 80%, 85%, 90%, 95%, 96%, 97%, 98%, or 99%) sequence identity to the amino acid sequence of SEQ ID NO: 130.

In some embodiments, the bispecific antibody described in this application comprises: the amino acid sequence of SEQ ID NO: 125, or a variant thereof having at least about 80% (such as at least about any of 80%, 85%, 90%, 95%, 96%, 97%, 98%, or 99%) sequence identity to the amino acid sequence of SEQ ID NO: 125; and/or the amino acid sequence of SEQ ID NO: 126, or a variant thereof having at least about 80% (such as at least about any of 80%, 85%, 90%, 95%, 96%, 97%, 98%, or 99%) sequence identity to the amino acid sequence of SEQ ID NO: 126; and/or the amino acid sequence of SEQ ID NO: 127, or a variant thereof having at least about 80% (such as at least about any of 80%, 85%, 90%, 95%, 96%, 97%, 98%, or 99%) sequence identity to the amino acid sequence of SEQ ID NO: 127; and/or the amino acid sequence of SEQ ID NO: 129 or a variant thereof having at least about 80% (such as at least about any of 80%, 85%, 90%, 95%, 96%, 97%, 98%, or 99%) sequence identity to the amino acid sequence of SEQ ID NO: 129.

In some embodiments, the bispecific antibody described in this application comprises: the amino acid sequence of SEQ ID NO: 125, or a variant thereof having at least about 80% (such as at least about any of 80%, 85%, 90%, 95%, 96%, 97%, 98%, or 99%) sequence identity to the amino acid sequence of SEQ ID NO: 125; and/or the amino acid sequence of SEQ ID NO: 130, or a variant thereof having at least about 80% sequence identity to the amino acid sequence of SEQ ID NO: 130; and/or the amino acid sequence of SEQ ID NO: 128, or a variant thereof having at least about 80% (such as at least about any of 80%, 85%, 90%, 95%, 96%, 97%, 98%, or 99%) sequence identity to the amino acid sequence of SEQ ID NO: 128; and/or the amino acid sequence of SEQ ID NO: 129 or a variant thereof having at least about 80% (such as at least about any of 80%, 85%, 90%, 95%, 96%, 97%, 98%, or 99%) sequence identity to the amino acid sequence of SEQ ID NO: 129.

In some embodiments, the bispecific antibody described in this application comprises: the amino acid sequence of SEQ ID NO: 143, or a variant thereof having at least about 80% (such as at least about any of 80%, 85%, 90%, 95%, 96%, 97%, 98%, or 99%) sequence identity to the amino acid sequence of SEQ ID NO: 143; and/or the amino acid sequence of SEQ ID NO: 126, or a variant thereof having at least about 80% (such as at least about any of 80%, 85%, 90%, 95%, 96%, 97%, 98%, or 99%) sequence identity to the amino acid sequence of SEQ ID NO: 126; and/or the amino acid sequence of SEQ ID NO: 144, or a variant thereof having at least about 80% (such as at least about any of 80%, 85%, 90%, 95%, 96%, 97%, 98%, or 99%) sequence identity to the amino acid sequence of SEQ ID NO: 144; and/or the amino acid sequence of SEQ ID NO: 129 or a variant thereof having at least about 80% (such as at least about any of 80%, 85%, 90%, 95%, 96%, 97%, 98%, or 99%) sequence identity to the amino acid sequence of SEQ ID NO: 129

In some embodiments, the bispecific antibody described in this application comprises: the amino acid sequence of SEQ ID NO: 145, or a variant thereof having at least about 80% (such as at least about any of 80%, 85%, 90%, 95%, 96%, 97%, 98%, or 99%) sequence identity to the amino acid sequence of SEQ ID NO: 145; and/or the amino acid sequence of SEQ ID NO: 130, or a variant thereof having at least about 80% (such as at least about any of 80%, 85%, 90%, 95%, 96%, 97%, 98%, or 99%) sequence identity to the amino acid sequence of SEQ ID NO: 130; and/or the amino acid sequence of SEQ ID NO: 146, or a variant thereof having at least about 80% (such as at least about any of 80%, 85%, 90%, 95%, 96%, 97%, 98%, or 99%) sequence identity to the amino acid sequence of SEQ ID NO: 146; and/or the amino acid sequence of SEQ ID NO: 129 or a variant thereof having at least about 80% (such as at least about any of 80%, 85%, 90%, 95%, 96%, 97%, 98%, or 99%) sequence identity to the amino acid sequence of SEQ ID NO: 129;

### IgG-(scFv)₂ format bispecific antibody

In some embodiments, the bispecific antibody described herein has IgG-(scFv)₂ format, which consists of two identical monomers, each monomer comprising two antigen-binding domains, one of which is Fab and the other of which is scFv. In some embodiments, the bispecific antibody further comprises an Fc comprising C_{H}2 and C_{H}3 domains. The scFv connects to the carboxyl terminal of the Fc through the peptide linker (L). An exemplary IgG-(scFv)₂ format is shown in FIG. 5D.

In some embodiments of the application, one of the antigen-binding domains (Fab or scFv) specifically binds to FasL and the other antigen-binding domain (Fab or scFv) specifically binds to TRAIL. In some embodiments, the bispecific antibody can bind to TRAIL and FasL simultaneously.

In some embodiments, the bispecific antibody disclosed herein consists of two identical monomers, each of which comprises a heavy chain and a light chain. In some embodiments, the heavy chain of the bispecific antibody comprises V_{H}1-C_{H}1-C_{H}2-C_{H}3 structure from N- terminus to C-terminus. In some embodiments, the light chain of the bispecific antibody comprises V_{L}1-C_{L} structure from N- terminus to C-terminus. In some embodiments, the heavy chain further comprises an Fc comprising C_{H}2 and C_{H}3 domains. In other embodiments, the heavy chain of the bispecific antibody comprises V_{H}1-C_{H}1-C_{H}2-C_{H}3-L-V_{H}2-L3-V_{L}2 structure from N- terminus to C-terminus. In other embodiments, the heavy chain of the bispecific antibody comprises V_{H}1-C_{H}1-C_{H}2-C_{H}3-L-V_{L}2-L3-V_{H}2 structure from N- terminus to C-terminus. In some embodiments, the light chain of the bispecific antibody comprises V_{L}1-C_{L} structure from N- terminus to C-terminus. Wherein, the V_{H}1 and V_{L}1 are the heavy chain variable domain and light chain variable domain specifically binding to one of the antigens, respectively; the V_{H}2 and V_{L}2 are the heavy chain variable domain and light chain variable domain specifically binding to the other antigen, respectively; C_{H}1 is a heavy chain constant domain 1; C_{L} is a light chain constant domain; L and L3 are peptide linkers. The V_{H}1-C_{H}1 and V_{L}l-C_{L} form one of the antigen-binding domains (Fab) of the bispecific antibody; the V_{H}2-L3-V_{L}2 or V_{L}2-L3-V_{H}2 form the other antigen-binding domain (scFv) of the bispecific antibody.

In some embodiments, the V_{H}1 and V_{L}1 are the heavy chain variable domain and light chain variable domain specifically binding to FasL, respectively, *i.e.,* the V_{H}1-C_{H}1 and V_{L}1-C_{L} form the antigen-binding domain (Fab) specifically binding to FasL; the V_{H}2 and V_{L}2 are the heavy chain variable domain and light chain variable domain specifically binding to TRAIL, respectively, *i.e.,* the V_{H}2-L3-V_{L}2 or V_{L}2-L3-V_{H}2 form the antigen-binding domain (scFv) specifically binding to TRAIL. In other embodiments, the V_{H}1 and V_{L}1 are the heavy chain variable domain and light chain variable domain specifically binding to TRAIL, respectively, *i.e.,* the V_{H}1-C_{H}1 and V_{L}1-C_{L} form the antigen-binding domain (Fab) specifically binding to TRAIL; the V_{H}2 and V_{L}2 are the heavy chain variable domain and light chain variable domain specifically binding to FasL, respectively, *i.e.,* the V_{H}2-L3-V_{L}2 or V_{L}2-L3-V_{H}2 form the antigen-binding domain (scFv) specifically binding to FasL. In some embodiments, the bispecific antibody comprises C_{H}1 comprising amino acid sequence SEQ ID No: 102 or 103. In some embodiments, the bispecific antibody comprises C_{H}2-C_{H}3 comprising amino acid sequence SEQ ID No: 100 or 101.

Peptide linker (or linker) may be used to join domains and/or regions of the chimeric heavy chain of the bispecific antibody into a contiguous molecule. In some embodiments, the bispecific antibody may include additional linkers, such as a flexible linker interconnecting the variable heavy and light chains of an scFv. In some embodiments, the bispecific antibody may include additional linkers, such as a flexible linker interconnecting the variable heavy and light chains of an scFv and other linkers that connect other binding units to the core structure of the bispecific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (e.g., the same or different length and/or amino acid sequence).

In some aspects, the peptide linker (linker) comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (e.g., 1, 2, 3, 4, 5, 6, 7, 8, 9 or 10). A preferred Gly-Ser linker is (Gly₄Ser)₂, *i.e.,* GGGGSGGGGS(SEQ ID NO: 147), and (Gly₄Ser)₄, *i.e.,* GGGGSGGGGSGGGGSGGGGS(SEQ ID NO: 148). Another preferred Gly-Ser linker is (Gly₄Ser)₃, *i.e.,,* GGGGSGGGGSGGGGS (SEQ ID NO: 149). In other aspects, two or more Gly-Ser linker are incorporated in series in a peptide linker. In some aspects, the peptide linker comprises at least a portion of a hinge region (e.g., derived from an IgGl, IgG2, IgG3, or IgG4 molecule) and a series of Gly- Ser amino acid residues (e.g., a Gly-Ser linker such as (Gly₄Ser)ₙ).

In some embodiments, the peptide linker comprises a Gly-Ser or all Gly linker and a portion or modified portion of a hinge domain. In some embodiments, the peptide linker (e.g., L) of the bispecific antibody connecting the antigen-binding domain to the C_{H}3 of the Fc carboxyl terminal comprises the amino acid sequence GGGGSGGGGTGGGGS (SEQ ID NO: 159).

In some embodiments, regardless of the peptide linker used to interconnect the antigen-binding domain to Fc (e.g., L), the bispecific antibody may optionally comprise additional peptide linkers. The lengths and sequence of such additional peptide linkers are independently selected. For example, the bispecific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 148).

### ScFv-Fab IgG format bispecific antibody

In some embodiments, the bispecific antibody described herein has scFv-Fab IgG format, which is in the form of heterodimer. The heterodimer comprises a first monomer and a second monomer, while the first monomer comprises the antigen-binding domain (Fab) binding to one of the antigens, the second monomer comprises the antigen-binding domain (scFv) binding to the other antigen. In some embodiments, the bispecific antibody further comprises two Fc domains comprising C_{H}2 and C_{H}3 domains. The two Fc domains further comprise amino acid substitution, which can promote the binding of heterodimers. An exemplary scFv-Fab IgG format is shown in FIG. 5E.

In some embodiments, one of the antigen-binding domains (Fab or scFv) specifically binds to TRAIL and the other antigen-binding domain (scFv or Fab) specifically binds to FasL. In some embodiments, the bispecific antibody can bind to TRAIL and FasL simultaneously.

In some embodiments, the bispecific antibody described herein has scFv-Fab IgG format, which is in the form of heterodimer. The heterodimer comprises a first monomer and a second monomer, while the first monomer comprises two polypeptide chains: the first heavy chain and light chain, wherein the first heavy chain comprises V_{H}1-C_{H}1 structure from N- terminus to C-terminus, the light chain comprises V_{L}1-C_{L} structure. In some embodiments, the first heavy chain further comprises an Fc comprising C_{H}2 and C_{H}3 domains. In some embodiments, the first heavy chain comprises V_{H}1-C_{H}1-C_{H}2-C_{H}3 structure from N- terminus to C-terminus, the light chain comprises V_{L}1-C_{L} structure. The V_{H}1 and V_{L}1 are the heavy chain variable domain and light chain variable domain specifically binding to one of the antigens, respectively; C_{H}1 is a heavy chain constant domain 1; C_{L} is a light chain constant domain. The V_{H}1-C_{H}1 and V_{L}l-C_{L} form one of the antigen-binding domains (Fab). The second monomer comprises one polypeptide chain: the second heavy chain. The second heavy chain comprises V_{H}2-L3-V_{L}2 structure or V_{L}2-L3-V_{H}2 structure from N- terminus to C-terminus. In some embodiments, the second heavy chain further comprises an Fc comprising C_{H}2 and C_{H}3 domains. In some embodiments, the second heavy chain comprises V_{H}2-L3-V_{L}2-C_{H}2-C_{H}3 structure or V_{L}2-L3-V_{H}2-C_{H}2-C_{H}3 structure from N- terminus to C-terminus. The V_{H}2 and V_{L}2 are the heavy chain variable domain and light chain variable domain specifically binding to the other antigen, respectively*;* L3 is a peptide linker. The V_{H}2-L3-V_{L}2 or V_{L}2-L3-V_{H}2 form the other antigen-binding domains (scFv).

In some embodiments, the V_{H}1 and V_{L}1 are the heavy chain variable domain and light chain variable domain specifically binding to FasL, respectively, *i.e.,* the V_{H}1-C_{H}1 and V_{L}1-C_{L} form the antigen-binding domain (Fab) specifically binding to FasL; the V_{H}2 and V_{L}2 are the heavy chain variable domain and light chain variable domain specifically binding to TRAIL, respectively, *i.e.,* the V_{H}2-L3-V_{L}2 or V_{L}2-L3-V_{H}2 form the antigen-binding domain (scFv) specifically binding to TRAIL. In other embodiments, the V_{H}1 and V_{L}1 are the heavy chain variable domain and light chain variable domain specifically binding to TRAIL, respectively, *i.e.,* the V_{H}1-C_{H}1 and V_{L}1-C_{L} form the antigen-binding domain (Fab) specifically binding to TRAIL. The V_{H}2 and V_{L}2 are the heavy chain variable domain and light chain variable domain specifically binding to FasL, respectively, *i.e.,* the V_{H}2-L3-V_{L}2 or V_{L}2-L3-V_{H}2 form the antigen-binding domain (scFv) specifically binding to FasL.

In some embodiments, the Fc is derived from human wild-type IgG1. In another embodiment, relative to human wild-type IgG1, the Fc in one monomer comprises, but is not limited to the amino acid substitution described below: E357Q and S364K; relative to human wild-type IgG1, the Fc in the other monomer comprises, but is not limited to the amino acid substitution described below: Q295E, L368D, K370S, N384D, Q418E and N421D, wherein the numbering is according to EU numbering system of Kabat. In some embodiments, exemplary peptide linker (e.g., L3) connecting V_{H}1 with V_{L}1 of scFv comprises GKPGSGKPGSGKPGSGKPGS (SEQ ID NO: 158).

Exemplary antibody sequences are shown in Tables 2-9, wherein the CDR numbering is according to the EU numbering system of Chothia. Those skilled in the art will recognize that many algorithms are known for prediction of CDR positions and for delimitation of antibody heavy chain and light chain variable regions. CDRs, V_{H} and/or V_{L} sequences from the antibodies specifically binding to TRAIL, the antibodies specifically binding to FasL or the bispecific antibody described herein, but based on prediction algorithms other than those exemplified in the tables below, are within the scope of this invention. The antibody or antigen-binding fragment specifically binding to FasL can selected from the anti- FasL antibody described in the PCT application PCT/CN2023/072293, which was incorporated here in this invention.

**Table 2: CDR sequences of exemplary antibody specifically binding to TRAIL**

| **Antibody Name** | **HC-CDR1** | **HC-CDR2** | **HC-CDR3** |
|---|---|---|---|
| G10 | GFTFSNY (SEQ ID NO: 1) | GGGS (SEQ ID NO: 3) | TAMD (SEQ ID NO: 5) |
| G10-1 | | | |
| G10-2 | | | |
| G10-3 | | | |
| G10-4 | | | |
| G10-5 | | | |
| G10-6 | | | |
| G72 | GFNIKDT (SEQ ID NO: 2) | PANG (SEQ ID NO: 4) | YYGSSGA (SEQ ID NO: 6) |
| G72-1 | | | |
| G72-2 | | | |
| G72-3 | | | |
| G72-4 | | | |
| G72-5 | | | |
| G72-6 | | | |
| G72-7 | | | |
| G72-8 | | | |
| G72-9 | | | |
| G72-10 | | | |
| G72-11 | | | |
| G72-12 | | | |
| G72-13 | | | |
| G72-14 | | | |
| G72-15 | | | |
| G45 | | | |
| G72-16 | GFNIKDT (SEQ ID NO: 2) | PANA (SEQ ID NO: 161) | YYGSSGA (SEQ ID NO: 6) |
| | | | |

| **Antibody Name** | **LC-CDR1** | **LC-CDR2** | **LC-CDR3** |
|---|---|---|---|
| G10 | SQEISGF (SEQ ID NO: 7) | AAS (SEQ ID NO: 9) | YASYPR (SEQ ID NO: 11) |
| G10-1 | | | |
| G10-2 | | | |
| G10-3 | | | |
| G10-4 | | | |
| G10-5 | | | |
| G10-6 | | | |
| G72 | SSSVSY (SEQ ID NO: 8) | DTS (SEQ ID NO: 10) | WSSYPY (SEQ ID NO: 12) |
| G72-1 | | | |
| G72-2 | | | |
| G72-3 | | | |
| G72-4 | | | |
| G72-5 | | | |
| G72-6 | | | |
| G72-7 | | | |
| G72-8 | | | |
| G72-9 | | | |
| G72-10 | | | |
| G72-11 | | | |
| G72-12 | | | |
| G72-13 | | | |
| G72-14 | | | |
| G72-15 | | | |
| G72-16 | | | |
| G45 | | | |

**Table 3: V_{H}/V_{L} sequences of exemplary antibody specifically binding to TRAIL**

| **SEQ ID NO** | **Description** | **Sequence** |
|---|---|---|
| 13 | G10 V_{H} | EVMLVESGGGLVQPGGSLKLSCAASGFTFSNYGMSWVRQTP EKRLEWVATISGGGSYTYYPDNLKGRFTISRENAKNTLYLQM SSLRSEDTAMYYCAAPTAMDYWGQGTSVTVSS |
| 14 | G10-1 V_{H} | EVQLVESGGGLVQPGGSLRLSCAASGFTFSNYGMSWVRQAP GKRLEWVATISGGGSYTYYPDNLKGRFTISRDNAKNSLYLQM NSLRAEDTAVYYCAAPTAMDYWGQGTLVTVSS |
| | G10-2 V_{H} | |
| | G10-3 V_{H} | |
| | G10-4 V_{H} | |
| | G10-5 V_{H} | |
| 15 | G72 V_{H} | EVQLQQSGAELVKPGASVKLSCTASGFNIKDTYMHWVKQRP EQGLEWIGRIRPANGNTKYDPKFQGKATVTADISSNTAYLQLS SLTSEDTAVYYCASYYYGSSGAYWGQGTLVTVSA |
| 16 | G72-1 V_{H} | EVQLVQSGAEVKKPGATVKISCKASGFNIKDTYMHWVQQAP GKGLEWIGRIRPANGNTKYDPKFQGRATVTADISTDTAYMEL SSLRSEDTAVYYCASYYYGSSGAYWGQGTLVTVSS |
| | G72-6 V_{H} | |
| | G72-11 V_{H} | |
| 17 | G72-2 V_{H} | EVQLVQSGAEVKKPGATVKISCKASGFNIKDTYMHWVQQAP GKGLEWMGRIRPANGNTKYDPKFQGRATVTADISTDTAYME LSSLRSEDTAVYYCASYYYGSSGAYWGQGTLVTVSS |
| | G72-7 V_{H} | |
| | G72-12 V_{H} | |
| 18 | G72-3 V_{H} | EVQLVQSGAEVKKPGATVKISCKASGFNIKDTYMHWVQQAP GKGLEWIGRIRPANGNTKYDPKFQGRATVTADTSTDTAYMEL SSLRSEDTAVYYCASYYYGSSGAYWGQGTLVTVSS |
| | G72-8 V_{H} | |
| | G72-13 V_{H} | |
| 19 | G72-4 V_{H} | EVQLVQSGAEVKKPGATVKISCKASGFNIKDTYMHWVQQAP GKGLEWIGRIRPANGNTKYDPKFQGRVTVTADTSTDTAYMEL SSLRSEDTAVYYCASYYYGSSGAYWGQGTLVTVSS |
| | G72-9 V_{H} | |
| | G72-14 V_{H} | |
| 20 | G72-5 V_{H} | EVQLVQSGAEVKKPGATVKISCKASGFNIKDTYMHWVQQAP GKGLEWIGRIRPANGNTKYDPKFQGRVTITADTSTDTAYMEL SSLRSEDTAVYYCASYYYGSSGAYWGQGTLVTVSS |
| | G72-10 V_{H} | |
| | G72-15 V_{H} | |
| 21 | G45 V_{H} | DVQLQESGAELVKPGASVKLSCTASGFNIKDTYMHWVKQRP EQGLEWIGRIRPANGNTKYDPKFQGRATITADTSSNTAYLQLS SLTSEDTAVYYCASYYYGSSGAYWGQGTTVTVSS |
| 164 | G10-6 V_{H} | EVQLVESGGGLVQPGGSLRLSCAASGFTFSNYGMSWVRQAP GKCLEWVATISGGGSYTYYPDNLKGRFTISRDNAKNSLYLQM NSLRAEDTAVYYCAAPTAMDYWGQGTLVTVSS |
| 166 | G72-16 V_{H} | EVQLVQSGAEVKKPGATVKISCKASGFNIKDTYMHWVQQAP GKGLEWIGRIRPANANAKYDPKFQGRATVTADISTDTAYMEL SSLRSEDTAVYYCASYYYGSSGAYWGQGTLVTVSS |
| | | |
| 22 | G10 V_{L} | DILLTQSPSSLSASLGERVSLTCRASQEISGFLSWLQQKPDGTI |
| | | KRLIYAASTLDSGVPKRFSGSRSGSDYSLTISSLESDDFADYYC LQYASYPRTFGGGTKLEIK |
| 23 | G10-1 V_{L} | DIQLTQSPSFLSASVGDRVTITCRASQEISGFLSWLQQKPGKTI KRLIYAASTLDSGVPSRFSGSRSGSEYTLTISSLQPEDFATYYC LQYASYPRTFGGGTKVEIK |
| 24 | G10-2 V_{L} | DIQLTQSPSFLSASVGDRVTITCRASQEISGFLSWLQQKPGKTI KRLIYAASTLDSGVPSRFSGSRSGTEYTLTISSLQPEDFATYYC LQYASYPRTFGGGTKVEIK |
| 25 | G10-3 V_{L} | DIQLTQSPSFLSASVGDRVTITCRASQEISGFLSWLQQKPGKTP KRLIYAASTLDSGVPSRFSGSRSGTEYTLTISSLQPEDFATYYC LQYASYPRTFGGGTKVEIK |
| 26 | G10-4 V_{L} | DIQLTQSPSFLSASVGDRVTITCRASQEISGFLSWLQQKPGKTI KRLIYAASTLDSGVPSRFSGSRSGTEFTLTISSLQPEDFATYYCL QYASYPRTFGGGTKVEIK |
| 27 | G10-5 V_{L} | DIQLTQSPSFLSASVGDRVTITCRASQEISGFLSWLQQKPGKTP KRLIYAASTLDSGVPSRFSGSRSGTEFTLTISSLQPEDFATYYCL QYASYPRTFGGGTKVEIK |
| 28 | G72 V_{L} | DIVMTQSPAIMSASLGEKVTMTCSVSSSVSYMYWYQQKPGSS PRLLIYDTSSLASGVPVRFSGSGSGTSYSLTISRMEAEDAATYY CQQWSSYPYTFGGGTKLEIK |
| 29 | G72-1 V_{L} | EIVMTQSPATLSVSPGERATLSCSVSSSVSYMYWYQQKPGQSP RLLIYDTSSLASGIPARFSGSGSGTEYTLTISSLQSEDFAVYYCQ QWSSYPYTFGGGTKVEIK |
| | G72-2 V_{L} | |
| | G72-3 V_{L} | |
| | G72-4 V_{L} | |
| | G72-5 V_{L} | |
| 30 | G72-6 V_{L} | EIVMTQSPATLSVSPGERATLSCSVSSSVSYMYWYQQKPGQSP RLLIYDTSSLASGIPARFSGSGSGTEFTLTISSLQSEDFAVYYCQ QWSSYPYTFGGGTKVEIK |
| | G72-7 V_{L} | |
| | G72-8 V_{L} | |
| | G72-9 V_{L} | |
| | G72-10 V_{L} | |
| 31 | G72-11 V_{L} | EIVMTQSPATLSVSPGERATLSCSVSSSVSYMYWYQQKPGQA PRLLIYDTSSLASGIPARFSGSGSGTEFTLTISSLQSEDFAVYYC QQWSSYPYTFGGGTKVEIK |
| | G72-12 V_{L} | |
| | G72-13 V_{L} | |
| | G72-14 V_{L} | |
| | G72-15 V_{L} | |
| 32 | G45 V_{L} | QIVLTQSPAIMSASPGEKVTMTCSASSSVSYMYWYQQKPGSS PRLLIYDTSNLASGVPVRFSGSGSGTSYSLTISRMEAEDAATY YCQQWSSYPYTFGGGTKLEIK |
| 168 | G10-6 V_{L} | DIQLTQSPSFLSASVGDRVTITCRASQEISGFLSWLQQKPGKTI KRLIYAASTLDTGVPSRFSGSRSGSEYTLTISSLQPEDFATYYC LQYASYPRTFGCGTKVEIK |
| 170 | G72-16 V_{L} | EIVMTQSPATLSVSPGERATLSCSVSSSVSYMYWYQQKPGQA PRLLIYDTSSLASGIPARFSGSGSGTEFTLTISSLQSEDFAVYYC QQWSSYPYTFGGGTKVEIK |

**Table 3-2: V_{H}/V_{L} cysteine variant sequences of exemplary antibody specifically binding to TRAIL**

| **SEQ ID NO** | **Description** | **Sequence** |
|---|---|---|
| 33 | G10 V_{H} cysteine variant | EVMLVESGGGLVQPGGSLKLSCAASGFTFSNYGMSWVRQTP EKCLEWVATISGGGSYTYYPDNLKGRFTISRENAKNTLYLQM SSLRSEDTAMYYCAAPTAMDYWGQGTSVTVSS |
| 34 | G10-1 V_{H} | EVQLVESGGGLVQPGGSLRLSCAASGFTFSNYGMSWVRQAP GKCLEWVATISGGGSYTYYPDNLKGRFTISRDNAKNSLYLQ MNSLRAEDTAVYYCAAPTAMDYWGQGTLVTVSS |
| | G10-2 V_{H} | |
| | G10-3 V_{H} | |
| | G10-4 V_{H} | |
| | G10-5 V_{H} | |
| | cysteine variant | |
| 35 | G72 V_{H} | |
| | cysteine variant | EVQLQQSGAELVKPGASVKLSCTASGFNIKDTYMHWVKQRP EQCLEWIGRIRPANGNTKYDPKFQGKATVTADISSNTAYLQL SSLTSEDTAVYYCASYYYGSSGAYWGQGTLVTVSA |
| 36 | G72-1 V_{H} | EVQLVQSGAEVKKPGATVKISCKASGFNIKDTYMHWVQQAP GKCLEWIGRIRPANGNTKYDPKFQGRATVTADISTDTAYMEL SSLRSEDTAVYYCASYYYGSSGAYWGQGTLVTVSS |
| | G72-6 V_{H} | |
| | G72-11 V_{H} | |
| | cysteine variant | |
| 37 | G72-2 V_{H} | EVQLVQSGAEVKKPGATVKISCKASGFNIKDTYMHWVQQAP GKCLEWMGRIRPANGNTKYDPKFQGRATVTADISTDTAYME LSSLRSEDTAVYYCASYYYGSSGAYWGQGTLVTVSS |
| | G72-7 V_{H} | |
| | G72-12 V_{H} | |
| | cysteine variant | |
| 38 | G72-3 V_{H} | EVQLVQSGAEVKKPGATVKISCKASGFNIKDTYMHWVQQAP GKCLEWIGRIRPANGNTKYDPKFQGRATVTADTSTDTAYME LSSLRSEDTAVYYCASYYYGSSGAYWGQGTLVTVSS |
| | G72-8 V_{H} | |
| | G72-13 V_{H} | |
| | cysteine variant | |
| 39 | G72-4 V_{H} | EVQLVQSGAEVKKPGATVKISCKASGFNIKDTYMHWVQQAP GKCLEWIGRIRPANGNTKYDPKFQGRVTVTADTSTDTAYME LSSLRSEDTAVYYCASYYYGSSGAYWGQGTLVTVSS |
| | G72-9 V_{H} | |
| | G72-14 V_{H} | |
| | cysteine variant | |
| 40 | G72-5 V_{H} | EVQLVQSGAEVKKPGATVKISCKASGFNIKDTYMHWVQQAP GKCLEWIGRIRPANGNTKYDPKFQGRVTITADTSTDTAYMEL SSLRSEDTAVYYCASYYYGSSGAYWGQGTLVTVSS |
| | G72-10 V_{H} | |
| | G72-15 V_{H} | |
| | cysteine variant | |
| | | |
| 41 | G10 V_{L} | DILLTQSPSSLSASLGERVSLTCRASQEISGFLSWLQQKPDGTI KRLIYAASTLDSGVPKRFSGSRSGSDYSLTISSLESDDFADYY CLQYASYPRTFGCGTKLEIK |
| | cysteine variant | |
| 42 | G10-1 V_{L} | DIQLTQSPSFLSASVGDRVTITCRASQEISGFLSWLQQKPGKTI KRLIYAASTLDSGVPSRFSGSRSGSEYTLTISSLQPEDFATYYC LQYASYPRTFGCGTKVEIK |
| | cysteine variant | |
| 43 | G10-2 V_{L} | DIQLTQSPSFLSASVGDRVTITCRASQEISGFLSWLQQKPGKTI KRLIYAASTLDSGVPSRFSGSRSGTEYTLTISSLQPEDFATYYC LQYASYPRTFGCGTKVEIK |
| | cysteine variant | |
| 44 | G10-3 V_{L} | DIQLTQSPSFLSASVGDRVTITCRASQEISGFLSWLQQKPGKT PKRLIYAASTLDSGVPSRFSGSRSGTEYTLTISSLQPEDFATYY CLQYASYPRTFGCGTKVEIK |
| | cysteine variant | |
| 45 | G10-4 V_{L} | DIQLTQSPSFLSASVGDRVTITCRASQEISGFLSWLQQKPGKT KRLIYAASTLDSGVPSRFSGSRSGTEFTLTISSLQPEDFATYYC LQYASYPRTFGCGTKVEIK |
| | cysteine variant | |
| 46 | G10-5 V_{L} | DIQLTQSPSFLSASVGDRVTITCRASQEISGFLSWLQQKPGKT PKRLIYAASTLDSGVPSRFSGSRSGTEFTLTISSLQPEDFATYY CLQYASYPRTFGCGTKVEIK |
| | cysteine variant | |
| 47 | G72 V_{L} | DIVMTQSPAIMSASLGEKVTMTCSVSSSVSYMYWYQQKPGS SPRLLIYDTSSLASGVPVRFSGSGSGTSYSLTISRMEAEDAAT YYCQQWSSYPYTFGCGTKLEIK |
| | cysteine variant | |
| 48 | G72-1 V_{L} | EIVMTQSPATLSVSPGERATLSCSVSSSVSYMYWYQQKPGQS PRLLIYDTSSLASGIPARFSGSGSGTEYTLTISSLQSEDFAVYY[?] QQWSSYPYTFGCGTKVEIK |
| | G72-2 V_{L} | |
| | G72-3 V_{L} | |
| | G72-4 V_{L} | |
| | G72-5 V_{L} | |
| | cysteine variant | |
| 49 | G72-6 V_{L} | EIVMTQSPATLSVSPGERATLSCSVSSSVSYMYWYQQKPGQS PRLLIYDTSSLASGIPARFSGSGSGTEFTLTISSLQSEDFAVYYC QQWSSYPYTFGCGTKVEIK |
| | G72-7 V_{L} | |
| | G72-8 V_{L} | |
| | G72-9 V_{L} | |
| | G72-10 V_{L} | |
| | cysteine variant | |
| 50 | G72-11 V_{L} | EIVMTQSPATLSVSPGERATLSCSVSSSVSYMYWYQQKPGQA PRLLIYDTSSLASGIPARFSGSGSGTEFTLTISSLQSEDFAVYYC QQWSSYPYTFGCGTKVEIK |
| | G72-12 V_{L} | |
| | G72-13 V_{L} | |
| | G72-14 V_{L} | |
| | G72-15 V_{L} | |
| | cysteine variant | |

**Table 4: CDR sequences of exemplary antibody specifically binding to FasL**

| **Antibody Name** | **HC-CDR1** | **HC-CDR2** | **HC-CDR3** |
|---|---|---|---|
| FL-M78 | | | |
| humFL-M78-1 | GFTFSSY (SEQ ID NO: 51) | SGGG (SEQ ID NO: 53) | YDNYLYAMD (SEQ ID NO: 55) |
| humFL-M78-2 | | | |
| humFL-M78-3 | | | |
| humFL-M78-4 | | | |
| humFL-M78-5 | | | |

| FL-M88 | | | |
|---|---|---|---|
| humFL-M88-1 | GYTFTDY (SEQ ID NO: 52) | TYQG (SEQ ID NO: 54) | PDWDYAMD (SEQ ID NO: 56) |
| humFL-M88-2 | | | |
| humFL-M88-3 | | | |
| humFL-M88-4 | | | |
| humFL-M88-5 | | | |
| humFL-M88-6 | | | |
| humFL-M88-7 | | | |
| humFL-M88-8 | | | |
| humFL-M88-9 | | | |
| humFL-M88-10 | | | |
| humFL-M88-11 | | | |
| humFL-M88-12 | | | |
| humFL-M88-13 | | | |
| humFL-M88-14 | | | |
| humFL-M88-15 | | | |
| humFL-M88-16 | | | |
| humFL-M88-17 | | | |
| humFL-M88-18 | | | |
| humFL-M88-19 | | | |
| | | | |

| **Antibody Name** | **LC-CDR1** | **LC-CDR2** | **LC-CDR3** |
|---|---|---|---|
| FL-M78 | | | |
| humFL-M78-1 | SQTIGTW (SEQ ID NO: 57) | AAT (SEQ ID NO: 59) | LYSTPF (SEQ ID NO: 61) |
| humFL-M78-2 | | | |
| humFL-M78-3 | | | |
| humFL-M78-4 | | | |
| humFL-M78-5 | | | |

| FL-M88 | | | |
|---|---|---|---|
| humFL-M88-1 | | | |
| humFL-M88-2 | | | |
| humFL-M88-3 | | | |
| humFL-M88-4 | | | |
| humFL-M88-5 | | | |
| humFL-M88-6 | | | |
| humFL-M88-7 | | | |
| humFL-M88-8 | SQSVDYDGDSY (SEQ ID NO: 58) | AAS (SEQ ID NO: 60) | SRELPY (SEQ ID NO: 62) |
| humFL-M88-9 | | | |
| humFL-M88-10 | | | |
| humFL-M88-11 | | | |
| humFL-M88-12 | | | |
| humFL-M88-13 | | | |
| humFL-M88-14 | | | |
| humFL-M88-15 | | | |
| humFL-M88-16 | | | |
| humFL-M88-17 | | | |
| humFL-M88-18 | | | |
| humFL-M88-19 | SQSVDYEGDSY (SEQ ID NO: 162) | AAS (SEQ ID NO: 60) | SRELPY (SEQ ID NO: 62) |

**Table 5-1: V_{H}/V_{L} sequences of exemplary antibody specifically binding to FasL**

| **SEQ ID NO** | **Description** | **Sequence** |
|---|---|---|
| 63 | FL-M78 V_{H} | DVQLQESGGGLVQPGGSRKLSCAASGFTFSSYDMSW VRQTPEKRLEWVAFISSGGGSTYYPDTVKGRFTISRDN AKNTLYLQMSSLKSEDSAMYYCGRHYDNYLYAMDY WGQGTSVTVSS |
| 64 | humFL-M78-1 V_{H} | EVQLVESGGGLVQPGGSLRLSCAASGFTFSSYDMSWV |
| | humFL-M78-3 V_{H} | RQAPGKRLEWVAFISSGGGSTYYPDTVKGRFTISRDN AKNSLYLQMNSLRAEDTAVYYCGRHYDNYLYAMDY WGQGTLVTVSS |
| 65 | humFL-M78-2 V_{H} | EVQLVESGGGLVQPGGSLRLSCAASGFTFSSYDMSWV RQAPGKGLEWVAFISSGGGSTYYPDTVKGRFTISRDN AKNSLYLQMNSLRAEDTAVYYCGRHYDNYLYAMDY WGQGTLVTVSS |
| | humFL-M78-4 V_{H} | |
| 66 | FL-M88 V_{H} | EVQLQQSGAELVRPGVSVKISCKGSGYTFTDYTIHWV KQSHAKSLEWIGVISTYQGDASYNQKFKGKATMTVD KSSSTAYMELARLTSEDSAIYYCARFPDWDYAMDYW GQGTSVTVSS |
| 67 | humFL-M88-1 V_{H} | QVQLVQSGAEVKKPGASVKVSCKGSGYTFTDYTIHW VRQAPGQGLEWIGVISTYQGDASYNQKFKGRATMTV DKSTSTAYMELSSLRSEDTAVYYCARFPDWDYAMDY WGQGTLVTVSS |
| | humFL-M88-7 V_{H} | |
| | humFL-M88-13 V_{H} | |
| 68 | humFL-M88-2 V_{H} | QVQLVQSGAEVKKPGASVKVSCKASGYTFTDYTIHW VRQAPGQGLEWIGVISTYQGDASYNQKFKGRATMTV DKSTSTAYMELSSLRSEDTAVYYCARFPDWDYAMDY WGQGTLVTVSS |
| | humFL-M88-8 V_{H} | |
| | humFL-M88-14 V_{H} | |
| 69 | humFL-M88-3 V_{H} | QVQLVQSGAEVKKPGASVKVSCKGSGYTFTDYTIHW VRQAPGQGLEWMGVISTYQGDASYNQKFKGRATMT VDKSTSTAYMELSSLRSEDTAVYYCARFPDWDYAMD YWGQGTLVTVSS |
| | humFL-M88-9 V_{H} | |
| | humFL-M88-15 V_{H} | |
| 70 | humFL-M88-4 V_{H} | QVQLVQSGAEVKKPGASVKVSCKGSGYTFTDYTIHW VRQAPGQGLEWMGVISTYQGDASYNQKFKGRVTMT VDKSTSTAYMELSSLRSEDTAVYYCARFPDWDYAMD YWGQGTLVTVSS |
| | humFL-M88-10 V_{H} | |
| | humFL-M88-16 V_{H} | |
| 71 | humFL-M88-5 V_{H} | QVQLVQSGAEVKKPGASVKVSCKGSGYTFTDYTIHW VRQAPGQGLEWMGVISTYQGDASYNQKFKGRVTMT VDKSTSTVYMELSSLRSEDTAVYYCARFPDWDYAMD YWGQGTLVTVSS |
| | humFL-M88-11 V_{H} | |
| | humFL-M88-17 V_{H} | |
| 72 | humFL-M88-6 V_{H} | QVQLVQSGAEVKKPGASVKVSCKASGYTFTDYTIHW VRQAPGQGLEWMGVISTYQGDASYNQKFKGRVTMT VDTSTSTVYMELSSLRSEDTAVYYCARFPDWDYAMD YWGQGTLVTVSS |
| | humFL-M88-12 V_{H} | |
| | humFL-M88-18 V_{H} | |
| 163 | humFL-M78-5 V_{H} | EVQLVESGGGLVQPGGSLRLSCAASGFTFSSYDMSWV RQAPGKRLEWVAFISSGGGSTYYPDTVKGRFTISRDN AKNSLYLQMNSLRAEDTAVYYCGRHYDNYLYAMDY WGQGTLVTVSS |
| 165 | humFL-M88-19 V_{H} | QVQLVQSGAEVKKPGASVKVSCKGSGYTFTDYTIHW VRQAPGQGLEWMGVISTYQGDASYNQKFKGRATMT VDKSTSTAYMELSSLRSEDTAVYYCARFPDWDYAMD YWGQGTLVTVSS |
| | | |
| 73 | FL-M78 V_{L} | DIVMTQSPASQSASLGESVTITCLASQTIGTWLAWYQ QKPGKSPQLLIYAATSLADGVPSRFSGSGSGTKFSFKIS SLQAEDFVSYYCQQLYSTPFTFGGGTKLEIK |
| 74 | humFL-M78-1 V_{L} | DIQMTQSPSSLSASVGDRVTITCLASQTIGTWLAWYQ QKPGKSPKLLIYAATSLADGVPSRFSGSGSGTDFTLTIS SLQPEDFATYYCQQLYSTPFTFGQGTKVEIK |
| | humFL-M78-2 V_{L} | |
| 75 | humFL-M78-3 V_{L} | DIQMTQSPSSLSASVGDRVTITCLASQTIGTWLAWYQ QKPGKAPKLLIYAATSLADGVPSRFSGSGSGTDFTLTIS SLQPEDFATYYCQQLYSTPFTFGQGTKVEIK |
| | humFL-M78-4 V_{L} | |
| 76 | FL-M88 V_{L} | EIVLTQSPASLAVSLGQRATISCKASQSVDYDGDSYMN WYQQKPGQPPKLLIYAASNLESGVPARFSGSGSGTDF TLNIHPVEEEDAATYYCQHSRELPYTFGGGTKLEIK |
| 77 | humFL-M88-1 V_{L} | EIVMTQSPATLSVSPGERATLSCKASQSVDYDGDSYM NWYQQKPGQPPRLLIYAASNLESGIPARFSGSGSGTEF TLTISSLQSEDFAVYYCQHSRELPYTFGQGTKLEIK |
| | humFL-M88-2 V_{L} | |
| | humFL-M88-3 V_{L} | |
| | humFL-M88-4 V_{L} | |
| | humFL-M88-5 V_{L} | |
| | humFL-M88-6 V_{L} | |
| 78 | humFL-M88-7 V_{L} | EIVMTQSPATLSVSPGERATLSCKASQSVDYDGDSYM NWYQQKPGQAPRLLIYAASNLESGIPARFSGSGSGTEF TLTISSLQSEDFAVYYCQHSRELPYTFGQGTKLEIK |
| | humFL-M88-8 V_{L} | |
| | humFL-M88-9 V_{L} | |
| | humFL-M88-10 V_{L} | |
| | humFL-M88-11 V_{L} | |
| | humFL-M88-12 V_{L} | |
| 79 | humFL-M88-13 V_{L} | EIVMTQSPATLSVSPGERATLSCKASQSVDYDGDSYM NWYQQKPGQPPRLLIYAASNLESGIPARFSGSGSGTEF TLTISSLQSEDFAVYYCQQSRELPYTFGQGTKLEIK |
| | humFL-M88-14 V_{L} | |
| | humFL-M88-15 V_{L} | |
| | humFL-M88-16 V_{L} | |
| | humFL-M88-17 V_{L} | |
| | humFL-M88-18 V_{L} | |
| 167 | humFL-M78-5 V_{L} | DIQMTQSPSSLSASVGDRVTITCLASQTIGTWLAWYQ QKPGKSPKLLIYAATSLASGVPSRFSGSGSGTDFTLTIS SLQPEDFATYYCQQLYSTPFTFGQGTKVEIK |
| 169 | humFL-M88-19 V_{L} | EIVMTQSPATLSVSPGERATLSCKASQSVDYEGDSYM NWYQQKPGQPPRLLIYAASNLESGIPARFSGSGSGTEF TLTISSLQSEDFAVYYCQQSRELPYTFGQGTKLEIK |

**Table 5-2: V_{H}/V_{L} cysteine variant sequences of exemplary antibody specifically binding to FasL**

| **SEQ ID NO** | **Description** | **Sequence** |
|---|---|---|
| 80 | FL-M78 V_{H} cysteine variant | DVQLQESGGGLVQPGGSRKLSCAASGFTFSSYDMSWV RQTPEKCLEWVAFISSGGGSTYYPDTVKGRFTISRDNA KNTLYLQMSSLKSEDSAMYYCGRHYDNYLYAMDYWG QGTSVTVSS |
| 81 | humFL-M78-1 V_{H} | EVQLVESGGGLVQPGGSLRLSCAASGFTFSSYDMSWVR QAPGKCLEWVAFISSGGGSTYYPDTVKGRFTISRDNAK NSLYLQMNSLRAEDTAVYYCGRHYDNYLYAMDYWGQ GTLVTVSS |
| | humFL-M78-2 V_{H} | |
| | humFL-M78-3 V_{H} | |
| | humFL-M78-4V_{H} cysteine variant | |
| 82 | FL-M88 V_{H} cysteine variant | EVQLQQSGAELVRPGVSVKISCKGSGYTFTDYTIHWVK QSHAKCLEWIGVISTYQGDASYNQKFKGKATMTVDKS |
| | | SSTAYMELARLTSEDSAIYYCARFPDWDYAMDYWGQG TSVTVSS |
| 83 | humFL-M88-1 V_{H} | QVQLVQSGAEVKKPGASVKVSCKGSGYTFTDYTIHWV RQAPGQCLEWIGVISTYQGDASYNQKFKGRATMTVDK STSTAYMELSSLRSEDTAVYYCARFPDWDYAMDYWGQ GTLVTVSS |
| | humFL-M88-7 V_{H} | |
| | humFL-M88-13 V_{H} cysteine variant | |
| 84 | humFL-M88-2 V_{H} | QVQLVQSGAEVKKPGASVKVSCKASGYTFTDYTIHWV RQAPGQCLEWIGVISTYQGDASYNQKFKGRATMTVDK STSTAYMELSSLRSEDTAVYYCARFPDWDYAMDYWGQ GTLVTVSS |
| | humFL-M88-8 V_{H} | |
| | humFL-M88-14 V_{H} cysteine variant | |
| 85 | humFL-M88-3 V_{H} | QVQLVQSGAEVKKPGASVKVSCKGSGYTFTDYTIHWV RQAPGQCLEWMGVISTYQGDASYNQKFKGRATMTVD KSTSTAYMELSSLRSEDTAVYYCARFPDWDYAMDYWG QGTLVTVSS |
| | humFL-M88-9 V_{H} | |
| | humFL-M88-15 V_{H} cysteine variant | |
| 86 | humFL-M88-4 V_{H} | QVQLVQSGAEVKKPGASVKVSCKGSGYTFTDYTIHWV RQAPGQCLEWMGVISTYQGDASYNQKFKGRVTMTVD KSTSTAYMELSSLRSEDTAVYYCARFPDWDYAMDYWG QGTLVTVSS |
| | humFL-M88-10 V_{H} | |
| | humFL-M88-16 V_{H} cysteine variant | |
| 87 | humFL-M88-5 V_{H} | QVQLVQSGAEVKKPGASVKVSCKGSGYTFTDYTIHWV RQAPGQCLEWMGVISTYQGDASYNQKFKGRVTMTVD KSTSTVYMELSSLRSEDTAVYYCARFPDWDYAMDYWG QGTLVTVSS |
| | humFL-M88-11 V_{H} | |
| | humFL-M88-17 V_{H} cysteine variant | |
| 88 | humFL-M88-6 V_{H} | QVQLVQSGAEVKKPGASVKVSCKASGYTFTDYTIHWV RQAPGQCLEWMGVISTYQGDASYNQKFKGRVTMTVD TSTSTVYMELSSLRSEDTAVYYCARFPDWDYAMDYWG QGTLVTVSS |
| | humFL-M88-12 V_{H} | |
| | humFL-M88-18 V_{H} cysteine variant | |
| | | |
| 89 | FL-M78 V_{L} cysteine variant | DIVMTQSPASQSASLGESVTITCLASQTIGTWLAWYQQ KPGKSPQLLIYAATSLADGVPSRFSGSGSGTKFSFKISSL QAEDFVSYYCQQLYSTPFTFGCGTKLEIK |
| 90 | humFL-M78-1 V_{L} | DIQMTQSPSSLSASVGDRVTITCLASQTIGTWLAWYQQ KPGKSPKLLIYAATSLADGVPSRFSGSGSGTDFTLTISSL |
| | humFL-M78-2 V_{L} cysteine variant | |
| | | QPEDFATYYCQQLYSTPFTFGCGTKVEIK |
| 91 | humFL-M78-3 V_{L} | DIQMTQSPSSLSASVGDRVTITCLASQTIGTWLAWYQQ KPGKAPKLLIYAATSLADGVPSRFSGSGSGTDFTLTISSL QPEDFATYYCQQLYSTPFTFGCGTKVEIK |
| | humFL-M78-4 V_{L} cysteine variant | |
| 92 | FL-M88 V_{L} cysteine variant | EIVLTQSPASLAVSLGQRATISCKASQSVDYDGDSYMN WYQQKPGQPPKLLIYAASNLESGVPARFSGSGSGTDFT LNIHPVEEEDAATYYCQHSRELPYTFGCGTKLEIK |
| 93 | humFL-M88-1 V_{L} | EIVMTQSPATLSVSPGERATLSCKASQSVDYDGDSYMN WYQQKPGQPPRLLIYAASNLESGIPARFSGSGSGTEFTL TISSLQSEDFAVYYCQHSRELPYTFGCGTKLEIK |
| | humFL-M88-2 V_{L} | |
| | humFL-M88-3 V_{L} | |
| | humFL-M88-4 V_{L} | |
| | humFL-M88-5 V_{L} | |
| | humFL-M88-6 V_{L} cysteine variant | |
| 94 | humFL-M88-7 V_{L} | EIVMTQSPATLSVSPGERATLSCKASQSVDYDGDSYMN WYQQKPGQAPRLLIYAASNLESGIPARFSGSGSGTEFTL TISSLQSEDFAVYYCQHSRELPYTFGCGTKLEIK |
| | humFL-M88-8 V_{L} | |
| | humFL-M88-9 V_{L} | |
| | humFL-M88-10 V_{L} | |
| | humFL-M88-11 V_{L} | |
| | humFL-M88-12 V_{L} cysteine variant | |
| 95 | humFL-M88-13 V_{L} | EIVMTQSPATLSVSPGERATLSCKASQSVDYDGDSYMN WYQQKPGQPPRLLIYAASNLESGIPARFSGSGSGTEFTL TISSLQSEDFAVYYCQQSRELPYTFGCGTKLEIK |
| | humFL-M88-14 V_{L} | |
| | humFL-M88-15 V_{L} | |
| | humFL-M88-16 V_{L} | |
| | humFL-M88-17 V_{L} | |
| | humFL-M88-18 V_{L} cysteine variant | |

**Table 6: Exemplary antibody constant region sequences**

| **SEQ ID NO** | **Description** | **Sequence** |
|---|---|---|
| 96 | IgG1 heavy chain constant region | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKVEPKSCDKTHTCPPCPAPELLGGPSVFLFP PKPKDTLMISRTPEVTCVVVDVSHEDPEVKFNWYVDGVEVH NAKTKPREEQYNSTYRVVSVLTVLHQDWLNGKEYKCKVSN KALPAPIEKTISKAKGQPREPQVYTLPPSREEMTKNQVSLTCL VKGFYPSDIAVEWESNGQPENNYKTTPPVLDSDGSFFLYSKL TVDKSRWQQGNVFSCSVMHEALHNHYTQKSLSLSPGK |
| 97 | IgG4 heavy chain constant region | ASTKGPSVFPLAPCSRSTSESTAALGCLVKDYFPEPVTVSWN SGALTSGVHTFPAVLQSSGLYSLSSVVTVPSSSLGTKTYTCNV DHKPSNTKVDKRVESKYGPPCPSCPAPEFLGGPSVFLFPPKPK DTLMISRTPEVTCVVVDVSQEDPEVQFNWYVDGVEVHNAK TKPREEQFNSTYRVVSVLTVLHQDWLNGKEYKCKVSNKGLP SSIEKTISKAKGQPREPQVYTLPPSQEEMTKNQVSLTCLVKGF YPSDIAVEWESNGQPENNYKTTPPVLDSDGSFFLYSRLTVDK SRWQEGNVFSCSVMHEALHNHYTQKSLSLSLGK |
| 98 | light chain constant region (kappa) | |
| 99 | light chain constant region (lambda) | GQPKAAPSVTLFPPSSEELQANKATLVCLISDFYPGAVTVAW KADSSPVKAGVETTTPSKQSNNKYAASSYLSLTPEQWKSHRS YSCQVTHEGSTVEKTVAPTECS |
| 100 | Exemplary IgG1 C_{H}2-C_{H}3 | APELLGGPSVFLFPPKPKDTLMISRTPEVTCVVVDVSHEDPE VKFNWYVDGVEVHNAKTKPREEQYNSTYRVVSVLTVLHQD WLNGKEYKCKVSNKALPAPIEKTISKAKGQPREPQVYTLPPS REEMTKNQVSLTCLVKGFYPSDIAVEWESNGQPENNYKTTPP VLDSDGSFFLYSKLTVDKSRWQQGNVFSCSVMHEALHNHYT QKSLSLSPGK |
| 101 | Exemplary IgG4 C_{H}2-C_{H}3 | APEFLGGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQ EEMTKNQVSLTCLVKGFYPSDIAVEWESNGQPENNYKTTPPV LDSDGSFFLYSRLTVDKSRWQEGNVFSCSVMHEALHNHYTQ KSLSLSLGK |
| 102 | Exemplary IgG1 C_{H}1 | ASTKGPSVFPLAPSSKSTSGGTAALGCLVKDYFPEPVTVSWN SGALTSGVHTFPAVLQSSGLYSLSSVVTVPSSSLGTQTYICNV NHKPSNTKVDKKV |
| 103 | Exemplary IgG4 C_{H}1 | ASTKGPSVFPLAPCSRSTSESTAALGCLVKDYFPEPVTVSWN SGALTSGVHTFPAVLQSSGLYSLSSVVTVPSSSLGTKTYTCNV DHKPSNTKVDKRV |

**Table 7-1: Partial heavy chain and light chain sequences of the exemplary DVD-Ig format bispecific antibodies specifically binding to TRAIL and FasL**

| **SEQ ID NO** | **Description** | **Sequence** |
|---|---|---|
| 104 | M78-1-G10-1-DVD-IgG4 partial heavy chain (humFL-M78-1V_{H}-G10-1V_{H}-IgG4C_{H}1) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSGGGGSGGGGSE VQLVESGGGLVQPGGSLRLSCAASGFTFSNYGMSWVRQAPGKRL EWVATISGGGSYTYYPDNLKGRFTISRDNAKNSLYLQMNSLRAE DTAVYYCAAPTAMDYWGQGTLVTVSSASTKGPSVFPLAPCSRST SESTAALGCLVKDYFPEPVTVSWNSGALTSGVHTFPAVLQSSGLY SLSSVVTVPSSSLGTKTYTCNVDHKPSNTKVDKRV |
| 105 | M78-1-G10-1-DVD-IgG4 light chain (humFL-M78-1V_{L}-G10-1V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST |
| | | PFTFGQGTKVEIKGGGGSGGGGSDIQLTQSPSFLSASVGDRVTITCR ASQEISGFLSWLQQKPGKTIKRLIYAASTLDSGVPSRFSGSRSGSEY TLTISSLQPEDFATYYCLQYASYPRTFGGGTKVEIKRTVAAPSVFIFP PSDEQLKSGTASVVCLLNNFYPREAKVQWKVDNALQSGNSQESV TEQDSKDSTYSLSSTLTLSKADYEKHKVYACEVTHQGLSSPVTKSF NRGEC |
| 104 | M78-1-G10-2-DVD-IgG4 partial heavy chain (humFL-M78-1V_{H}-G10-2V_{H}-IgG4C_{H}1) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSGGGGSGGGGSEV QLVESGGGLVQPGGSLRLSCAASGFTFSNYGMSWVRQAPGKRLE WVATISGGGSYTYYPDNLKGRFTISRDNAKNSLYLQMNSLRAEDT AVYYCAAPTAMDYWGQGTLVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRV |
| 106 | M78-1-G10-2-DVD-IgG4 light chain (humFL-M78-1V_{L}-G10-2V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKGGGGSGGGGSDIQLTQSPSFLSASVGDRVTITCR ASQEISGFLSWLQQKPGKTIKRLIYAASTLDSGVPSRFSGSRSGTEY TLTISSLQPEDFATYYCLQYASYPRTFGGGTKVEIKRTVAAPSVFIFP PSDEQLKSGTASVVCLLNNFYPREAKVQWKVDNALQSGNSQESV TEQDSKDSTYSLSSTLTLSKADYEKHKVYACEVTHQGLSSPVTKSF NRGEC |
| 107 | G10-1-M78-1-DVD-IgG4 partial heavy chain (G10-1V_{H}-humFL-M78-1V_{H}-IgG4C_{H}1) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSGGGGSGGGGSEVQLVES GGGLVQPGGSLRLSCAASGFTFSSYDMSWVRQAPGKRLEWVAFI SSGGGSTYYPDTVKGRFTISRDNAKNSLYLQMNSLRAEDTAVYY CGRHYDNYLYAMDYWGQGTLVTVSSASTKGPSVFPLAPCSRSTS ESTAALGCLVKDYFPEPVTVSWNSGALTSGVHTFPAVLQSSGLYS LSSVVTVPSSSLGTKTYTCNVDHKPSNTKVDKRV |
| 108 | G10-1-M78-1-DVD-IgG4 light chain (G10-1V_{L}-humFL-M78-1V_{L}-C_{L}) | DIQLTQSPSFLSASVGDRVTITCRASQEISGFLSWLQQKPGKTIKRLI YAASTLDSGVPSRFSGSRSGSEYTLTISSLQPEDFATYYCLQYASYP RTFGGGTKVEIKGGGGSGGGGSDIQMTQSPSSLSASVGDRVTITCL ASQTIGTWLAWYQQKPGKSPKLLIYAATSLADGVPSRFSGSGSGT DFTLTISSLQPEDFATYYCQQLYSTPFTFGQGTKVEIKRTVAAPSVFI FPPSDEQLKSGTASVVCLLNNFYPREAKVQWKVDNALQSGNSQE SVTEQDSKDSTYSLSSTLTLSKADYEKHKVYACEVTHQGLSSPVT KSFNRGEC |
| 107 | G10-2-M78-1-DVD-IgG4 partial heavy chain (G10-2V_{H}-humFL-M78-1VH-IgG4C_{H}1) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSGGGGSGGGGSEVQLVESG GGLVQPGGSLRLSCAASGFTFSSYDMSWVRQAPGKRLEWVAFISS GGGSTYYPDTVKGRFTISRDNAKNSLYLQMNSLRAEDTAVYYCG RHYDNYLYAMDYWGQGTLVTVSSASTKGPSVFPLAPCSRSTSEST AALGCLVKDYFPEPVTVSWNSGALTSGVHTFPAVLQSSGLYSLSSV VTVPSSSLGTKTYTCNVDHKPSNTKVDKRV |
| 109 | G10-2-M78-1-DVD-IgG4 light chain (G10-2V_{L}-humFL-M78-1V_{L}-C_{L}) | DIQLTQSPSFLSASVGDRVTITCRASQEISGFLSWLQQKPGKTIKRLI YAASTLDSGVPSRFSGSRSGTEYTLTISSLQPEDFATYYCLQYASYP RTFGGGTKVEIKGGGGSGGGGSDIQMTQSPSSLSASVGDRVTITCL ASQTIGTWLAWYQQKPGKSPKLLIYAATSLADGVPSRFSGSGSGT |
| | | DFTLTISSLQPEDFATYYCQQLYSTPFTFGQGTKVEIKRTVAAPSVFI FPPSDEQLKSGTASVVCLLNNFYPREAKVQWKVDNALQSGNSQE SVTEQDSKDSTYSLSSTLTLSKADYEKHKVYACEVTHQGLSSPVT KSFNRGEC |
| 110 | M88-15-G72-11-DVD-IgG4 partial heavy chain (humFL-M88-15V_{H}-G72-11V_{H}-IgG4C_{H}1) | QVQLVQSGAEVKKPGASVKVSCKGSGYTFTDYTIHWVRQAPGQ GLEWMGVISTYQGDASYNQKFKGRATMTVDKSTSTAYMELSSLR SEDTAVYYCARFPDWDYAMDYWGQGTLVTVSSGGGGSGGGGSE VQLVQSGAEVKKPGATVKISCKASGFNIKDTYMHWVQQAPGKG LEWIGRIRPANGNTKYDPKFQGRATVTADISTDTAYMELSSLRSE DTAVYYCASYYYGSSGAYWGQGTLVTVSSASTKGPSVFPLAPCS RSTSESTAALGCLVKDYFPEPVTVSWNSGALTSGVHTFPAVLQSS GLYSLSSVVTVPSSSLGTKTYTCNVDHKPSNTKVDKRV |
| 111 | M88-15-G72-11-DVD-IgG4 light chain (humFL-M88-15V_{L}-G72-11V_{L}-C_{L}) | EIVMTQSPATLSVSPGERATLSCKASQSVDYDGDSYMNWYQQKP GQPPRLLIYAASNLESGIPARFSGSGSGTEFTLTISSLQSEDFAVYY CQQSRELPYTFGQGTKLEIKGGGGSGGGGSEIVMTQSPATLSVSPG ERATLSCSVSSSVSYMYWYQQKPGQAPRLLIYDTSSLASGIPARFS GSGSGTEFTLTISSLQSEDFAVYYCQQWSSYPYTFGGGTKVEIKRT VAAPSVFIFPPSDEQLKSGTASVVCLLNNFYPREAKVQWKVDNAL QSGNSQESVTEQDSKDSTYSLSSTLTLSKADYEKHKVYACEVTHQ GLSSPVTKSFNRGEC |
| 172 | M88-19-G72-16-DVD-IgG4 partial heavy chain (humFL-M88-19V_{H}-G72-16V_{H}-IgG4C_{H}1) | QVQLVQSGAEVKKPGASVKVSCKGSGYTFTDYTIHWVRQAPGQ GLEWMGVISTYQGDASYNQKFKGRATMTVDKSTSTAYMELSSLR SEDTAVYYCARFPDWDYAMDYWGQGTLVTVSSGGGGSGGGGSE VQLVQSGAEVKKPGATVKISCKASGFNIKDTYMHWVQQAPGKG LEWIGRIRPANANAKYDPKFQGRATVTADISTDTAYMELSSLRSE DTAVYYCASYYYGSSGAYWGQGTLVTVSSASTKGPSVFPLAPCS RSTSESTAALGCLVKDYFPEPVTVSWNSGALTSGVHTFPAVLQSS GLYSLSSVVTVPSSSLGTKTYTCNVDHKPSNTKVDKRV |
| 174 | M88-19-G72-16-DVD-IgG4 light chain (humFL-M88-19V_{L}-G72-16V_{L}-C_{L}) | EIVMTQSPATLSVSPGERATLSCKASQSVDYEGDSYMNWYQQKP GQPPRLLIYAASNLESGIPARFSGSGSGTEFTLTISSLQSEDFAVYY CQQSRELPYTFGQGTKLEIKGGGGSGGGGSEIVMTQSPATLSVSPG ERATLSCSVSSSVSYMYWYQQKPGQAPRLLIYDTSSLASGIPARFS GSGSGTEFTLTISSLQSEDFAVYYCQQWSSYPYTFGGGTKVEIKRT VAAPSVFIFPPSDEQLKSGTASVVCLLNNFYPREAKVQWKVDNAL QSGNSQESVTEQDSKDSTYSLSSTLTLSKADYEKHKVYACEVTHQ GLSSPVTKSFNRGEC |

**Table 7-2: Partial heavy chain and light chain sequences of the exemplary Bs4Ab format bispecific antibodies specifically binding to TRAIL and FasL**

| **SEQ ID NO** | **Description** | **Sequence** |
|---|---|---|
| 112 | M88-G72scFv-Bs4Ab-IgG1 partial heavy chain (FL-M88V_{H}-C_{H}1-G72scFv) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGG SGGGGSEVQLQQSGAELVKPGASVKLSCTASGFNIKDTYMHWVK QRPEQCLEWIGRIRPANGNTKYDPKFQGKATVTADISSNTAYLQLS SLTSEDTAVYYCASYYYGSSGAYWGQGTLVTVSAGGGGSGGGGS |
| | | GGGGSGGGGSDIVMTQSPAIMSASLGEKVTMTCSVSSSVSYMYW YQQKPGSSPRLLIYDTSSLASGVPVRFSGSGSGTSYSLTISRMEAED AATYYCQQWSSYPYTFGCGTKLEIK |
| 113 | M88-G72scFv-Bs4Ab-IgG1 light chain (FL-M88V_{L}-C_{L}) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 112 | M88-G72scFv-Bs4Ab-IgG4 partial heavy chain (FL-M88V_{H}-C_{H}1-G72scFv) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCGGGG SGGGGSEVQLQQSGAELVKPGASVKLSCTASGFNIKDTYMHWVK QRPEQCLEWIGRIRPANGNTKYDPKFQGKATVTADISSNTAYLQLS SLTSEDTAVYYCASYYYGSSGAYWGQGTLVTVSAGGGGSGGGGS GGGGSGGGGSDIVMTQSPAIMSASLGEKVTMTCSVSSSVSYMYW YQQKPGSSPRLLIYDTSSLASGVPVRFSGSGSGTSYSLTISRMEAED AATYYCQQWSSYPYTFGCGTKLEIK |
| 113 | M88-G72scFv-Bs4Ab-IgG4 light chain (FL-M88V_{L}-C_{L}) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 114 | G72-M88scFv-Bs4Ab-IgG1 partial heavy chain (G72V_{H}-C_{H}1-FL-M88scFv) | EVQLQQSGAELVKPGASVKLSCTASGFNIKDTYMHWVKQRPEQG LEWIGRIRPANGNTKYDPKFQGKATVTADISSNTAYLQLSSLTSEDT AVYYCASYYYGSSGAYWGQGTLVTVSAASTKGPSVFPLAPSSKST SGGTAALGCLVKDYFPEPVTVSWNSGALTSGVHTFPAVLQSSGLYS LSSVVTVPSSSLGTQTYICNVNHKPSNTKVDKKVEPKSCGGGGSG GGGSEVQLQQSGAELVRPGVSVKISCKGSGYTFTDYTIHWVKQSH AKCLEWIGVISTYQGDASYNQKFKGKATMTVDKSSSTAYMELAR LTSEDSAIYYCARFPDWDYAMDYWGQGTSVTVSSGGGGSGGGGS GGGGSGGGGSEIVLTQSPASLAVSLGQRATISCKASQSVDYDGDSY MNWYQQKPGQPPKLLIYAASNLESGVPARFSGSGSGTDFTLNIHP VEEEDAATYYCQHSRELPYTFGCGTKLEIK |
| 115 | G72-M88scFv-Bs4Ab-IgG1 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |
| 114 | G72-M88scFv-Bs4Ab-IgG4 partial heavy chain (G72V_{H}-C_{H}1-FL-M88scFv) | EVQLQQSGAELVKPGASVKLSCTASGFNIKDTYMHWVKQRPEQG LEWIGRIRPANGNTKYDPKFQGKATVTADISSNTAYLQLSSLTSEDT AVYYCASYYYGSSGAYWGQGTLVTVSAASTKGPSVFPLAPSSKST SGGTAALGCLVKDYFPEPVTVSWNSGALTSGVHTFPAVLQSSGLYS LSSVVTVPSSSLGTQTYICNVNHKPSNTKVDKKVEPKSCGGGGSG GGGSEVQLQQSGAELVRPGVSVKISCKGSGYTFTDYTIHWVKQSH AKCLEWIGVISTYQGDASYNQKFKGKATMTVDKSSSTAYMELAR LTSEDSAIYYCARFPDWDYAMDYWGQGTSVTVSSGGGGSGGGGS GGGGSGGGGSEIVLTQSPASLAVSLGQRATISCKASQSVDYDGDSY MNWYQQKPGQPPKLLIYAASNLESGVPARFSGSGSGTDFTLNIHP |
| | | VEEEDAATYYCQHSRELPYTFGCGTKLEIK |
| 115 | G72-M88scFv-Bs4Ab-IgG4 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |
| 116 | M78-1-G10-1scFv-Bs4Ab-IgG4 partial heavy chain (humFL-M78-1V_{H}-C_{H}1-G10-1scFv) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQS SGLYSLSSVVTVPSSSLGTQTYICNVNHKPSNTKVDKKVEPKSCGG GGSGGGGSEVQLVESGGGLVQPGGSLRLSCAASGFTFSNYGMSW VRQAPGKCLEWVATISGGGSYTYYPDNLKGRFTISRDNAKNSLYL QMNSLRAEDTAVYYCAAPTAMDYWGQGTLVTVSSGGGGSGGGG SGGGGSGGGGSDIQLTQSPSFLSASVGDRVTITCRASQEISGFLSWL QQKPGKTIKRLIYAASTLDSGVPSRFSGSRSGSEYTLTISSLQPEDFA TYYCLQYASYPRTFGCGTKVEIK |
| 119 | M78-1-G10-1scFv-Bs4Ab-IgG4 light chain (humFL-M78-1V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |
| 117 | M78-1-G10-1scFv-2-Bs4Ab-IgG4 partial heavy chain (humFL-M78-1V_{H}-C_{H}1-G10-1scFv-2) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQS SGLYSLSSVVTVPSSSLGTQTYICNVNHKPSNTKVDKKVEPKSCGG GGSGGGGSDIQLTQSPSFLSASVGDRVTITCRASQEISGFLSWLQQ KPGKTIKRLIYAASTLDSGVPSRFSGSRSGSEYTLTISSLQPEDFATY YCLQYASYPRTFGCGTKVEIKGGGGSGGGGSGGGGSGGGGSEVQ LVESGGGLVQPGGSLRLSCAASGFTFSNYGMSWVRQAPGKCLEW VATISGGGSYTYYPDNLKGRFTISRDNAKNSLYLQMNSLRAEDTAV YYCAAPTAMDYWGQGTLVTVSS |
| 119 | M78-1-G10-1scFv-2-Bs4Ab-IgG4 light chain (humFL-M78-1V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |
| 118 | M78-1-G10-2scFv-Bs4Ab-IgG4 partial heavy chain (humFL-M78-1V_{H}-C_{H}1-G10-2scFv) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQS SGLYSLSSVVTVPSSSLGTQTYICNVNHKPSNTKVDKKVEPKSCGG GGSGGGGSEVQLVESGGGLVQPGGSLRLSCAASGFTFSNYGMSW VRQAPGKRLEWVATISGGGSYTYYPDNLKGRFTISRDNAKNSLYL QMNSLRAEDTAVYYCAAPTAMDYWGQGTLVTVSSGGGGSGGGG |
| | | SGGGGSGGGGSDIQLTQSPSFLSASVGDRVTITCRASQEISGFLSWL QQKPGKTIKRLIYAASTLDSGVPSRFSGSRSGTEYTLTISSLQPEDFA TYYCLQYASYPRTFGGGTKVEIK |
| 119 | M78-1-G10-2scFv-Bs4Ab-IgG4 light chain (humFL-M78-1V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |
| 120 | G10-1-M78-1scFv-Bs4Ab-IgG4 partial heavy chain (G10-1V_{H}-C_{H}1-humFL-M78-1scFv) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSASTKGPSVFPLAPSSKSTS GGTAALGCLVKDYFPEPVTVSWNSGALTSGVHTFPAVLQSSGLYSL SSVVTVPSSSLGTQTYICNVNHKPSNTKVDKKVEPKSCGGGGSGG GGSEVQLVESGGGLVQPGGSLRLSCAASGFTFSSYDMSWVRQAP GKCLEWVAFISSGGGSTYYPDTVKGRFTISRDNAKNSLYLQMNSL RAEDTAVYYCGRHYDNYLYAMDYWGQGTLVTVSSGGGGSGGGG SGGGGSGGGGSDIQMTQSPSSLSASVGDRVTITCLASQTIGTWLA WYQQKPGKSPKLLIYAATSLADGVPSRFSGSGSGTDFTLTISSLQPE DFATYYCQQLYSTPFTFGCGTKVEIK |
| 121 | G10-1-M78-1scFv-Bs4Ab-IgG4 light chain (G10-1V_{L}-C_{L}) | DIQLTQSPSFLSASVGDRVTITCRASQEISGFLSWLQQKPGKTIKRLI YAASTLDSGVPSRFSGSRSGSEYTLTISSLQPEDFATYYCLQYASYP RTFGGGTKVEIKRTVAAPSVFIFPPSDEQLKSGTASVVCLLNNFYPR EAKVQWKVDNALQSGNSQESVTEQDSKDSTYSLSSTLTLSKADY EKHKVYACEVTHQGLSSPVTKSFNRGEC |
| 120 | G10-2-M78-1scFv-Bs4Ab-IgG4 partial heavy chain (G10-2V_{H}-C_{H}1-humFL-M78-1scFv) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSASTKGPSVFPLAPSSKSTS GGTAALGCLVKDYFPEPVTVSWNSGALTSGVHTFPAVLQSSGLYSL SSVVTVPSSSLGTQTYICNVNHKPSNTKVDKKVEPKSCGGGGSGG GGSEVQLVESGGGLVQPGGSLRLSCAASGFTFSSYDMSWVRQAP GKCLEWVAFISSGGGSTYYPDTVKGRFTISRDNAKNSLYLQMNSL RAEDTAVYYCGRHYDNYLYAMDYWGQGTLVTVSSGGGGSGGGG SGGGGSGGGGSDIQMTQSPSSLSASVGDRVTITCLASQTIGTWLA WYQQKPGKSPKLLIYAATSLADGVPSRFSGSGSGTDFTLTISSLQPE DFATYYCQQLYSTPFTFGCGTKVEIK |
| 122 | G10-2-M78-1scFv-Bs4Ab-IgG4 light chain (G10-2V_{L}-C_{L}) | DIQLTQSPSFLSASVGDRVTITCRASQEISGFLSWLQQKPGKTIKRLI YAASTLDSGVPSRFSGSRSGTEYTLTISSLQPEDFATYYCLQYASYP RTFGGGTKVEIKRTVAAPSVFIFPPSDEQLKSGTASVVCLLNNFYPR EAKVQWKVDNALQSGNSQESVTEQDSKDSTYSLSSTLTLSKADY EKHKVYACEVTHQGLSSPVTKSFNRGEC |
| 123 | G72-11-M88scFv-Bs4Ab-IgG4 partial heavy chain (G72-11V_{H}-C_{H}1-FL-M88scFv) | EVQLVQSGAEVKKPGATVKISCKASGFNIKDTYMHWVQQAPGKG LEWIGRIRPANGNTKYDPKFQGRATVTADISTDTAYMELSSLRSED TAVYYCASYYYGSSGAYWGQGTLVTVSSASTKGPSVFPLAPSSKS TSGGTAALGCLVKDYFPEPVTVSWNSGALTSGVHTFPAVLQSSGLY SLSSVVTVPSSSLGTQTYICNVNHKPSNTKVDKKVEPKSCGGGGS GGGGSEVQLQQSGAELVRPGVSVKISCKGSGYTFTDYTIHWVKQS HAKCLEWIGVISTYQGDASYNQKFKGKATMTVDKSSSTAYMELA RLTSEDSAIYYCARFPDWDYAMDYWGQGTSVTVSSGGGGSGGGG SGGGGSGGGGSEIVLTQSPASLAVSLGQRATISCKASQSVDYDGDS |
| | | YMNWYQQKPGQPPKLLIYAASNLESGVPARFSGSGSGTDFTLNIH PVEEEDAATYYCQHSRELPYTFGCGTKLEIK |
| 124 | G72-11-M88scFv-Bs4Ab-IgG4 light chain (G72-11V_{L}-C_{L}) | EIVMTQSPATLSVSPGERATLSCSVSSSVSYMYWYQQKPGQAPRLL IYDTSSLASGIPARFSGSGSGTEFTLTISSLQSEDFAVYYCQQWSSYP YTFGGGTKVEIKRTVAAPSVFIFPPSDEQLKSGTASVVCLLNNFYPR EAKVQWKVDNALQSGNSQESVTEQDSKDSTYSLSSTLTLSKADY EKHKVYACEVTHQGLSSPVTKSFNRGEC |
| 171 | M78-5-G10-6scFv-Bs4Ab-IgG4 partial heavy chain (humFL-M78-5V_{H}-C_{H}1-G10-6scFv) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQS SGLYSLSSVVTVPSSSLGTQTYICNVNHKPSNTKVDKKVEPKSCGG GGSGGGGSEVQLVESGGGLVQPGGSLRLSCAASGFTFSNYGMSW VRQAPGKCLEWVATISGGGSYTYYPDNLKGRFTISRDNAKNSLYL QMNSLRAEDTAVYYCAAPTAMDYWGQGTLVTVSSGGGGSGGGG SGGGGSGGGGSDIQLTQSPSFLSASVGDRVTITCRASQEISGFLSWL QQKPGKTIKRLIYAASTLDTGVPSRFSGSRSGSEYTLTISSLQPEDFA TYYCLQYASYPRTFGCGTKVEIK |
| 173 | M78-5-G10-6scFv-Bs4Ab-IgG4 light chain (humFLM78-5V_{L}-C_{L}) | DIQMTQSPSSLSASVGDRVTITCLASQTIGTWLAWYQQKPGKSPKL LIYAATSLASGVPSRFSGSGSGTDFTLTISSLQPEDFATYYCQQLYST PFTFGQGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |

**Table 7-3: Partial heavy chain and light chain sequences of the Hetero H, CrossMab format exemplary bispecific antibodies specifically binding to TRAIL and FasL**

| **SEQ ID NO** | **Description** | **Sequence** |
|---|---|---|
| 125 | M88-G72-CrossMab-IgG1 partial heavy chain (FL-M88V_{H}-C_{L}) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASVAAPSVFIFPPSDE QLKSGTASVVCLLNNFYPREAKVQWKVDNALQSGNSQESVTEQD SKDSTYSLSSTLTLSKADYEKHKVYACEVTHQGLSSPVTKSFNRGE C |
| 126 | M88-G72-CrossMab-IgG1 light chain (FL-M88V_{L}-C_{H}1) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKSSASTKGPSVFPLAPSSKSTSGGTAALG CLVKDYFPEPVTVSWNSGALTSGVHTFPAVLQSSGLYSLSSVVTVP SSSLGTQTYICNVNHKPSNTKVDKKVEPKSC |
| 127 | M88-G72-CrossMab-IgG1 partial heavy chain (G72V_{H}-IgG1C_{H}1) | EVQLQQSGAELVKPGASVKLSCTASGFNIKDTYMHWVKQRPEQG LEWIGRIRPANGNTKYDPKFQGKATVTADISSNTAYLQLSSLTSEDT AVYYCASYYYGSSGAYWGQGTLVTVSAASTKGPSVFPLAPSSKST SGGTAALGCLVKDYFPEPVTVSWNSGALTSGVHTFPAVLQSSGLYS LSSVVTVPSSSLGTQTYICNVNHKPSNTKVDKKV |
| 129 | M88-G72-CrossMab-IgG1 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |
| 125 | M88-G72-CrossMab-IgG4 partial heavy chain (FL-M88V_{H}-C_{L}) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASVAAPSVFIFPPSDE QLKSGTASVVCLLNNFYPREAKVQWKVDNALQSGNSQESVTEQD SKDSTYSLSSTLTLSKADYEKHKVYACEVTHQGLSSPVTKSFNRGE C |
| 130 | M88-G72-CrossMab-IgG4 light chain (FL-M88V_{L}-C_{H}1) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKSSASTKGPSVFPLAPCSRSTSESTAALG CLVKDYFPEPVTVSWNSGALTSGVHTFPAVLQSSGLYSLSSVVTVP SSSLGTKTYTCNVDHKPSNTKVDKRVESKYG |
| 128 | M88-G72-CrossMab-IgG4 partial heavy chain (G72V_{H}-IgG4C_{H}1) | EVQLQQSGAELVKPGASVKLSCTASGFNIKDTYMHWVKQRPEQG LEWIGRIRPANGNTKYDPKFQGKATVTADISSNTAYLQLSSLTSEDT AVYYCASYYYGSSGAYWGQGTLVTVSAASTKGPSVFPLAPCSRST SESTAALGCLVKDYFPEPVTVSWNSGALTSGVHTFPAVLQSSGLYS LSSVVTVPSSSLGTKTYTCNVDHKPSNTKVDKRV |
| 129 | M88-G72-CrossMab-IgG4 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |

**Table 8-1: Full length heavy chain and light chain sequences of the exemplary DVD-Ig format bispecific antibodies specifically binding to TRAIL and FasL**

| **SEQ ID NO** | **Description** | **Sequence** |
|---|---|---|
| 131 | M78-1-G10-1-DVD-IgG4 heavy chain (humFL-M78-1V_{H}-G10-1V_{H}-IgG4C_{H}) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSGGGGSGGGGSEV QLVESGGGLVQPGGSLRLSCAASGFTFSNYGMSWVRQAPGKRLE WVATISGGGSYTYYPDNLKGRFTISRDNAKNSLYLQMNSLRAEDT AVYYCAAPTAMDYWGQGTLVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAP EFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 105 | M78-1-G10-1-DVD-IgG4 light chain (humFL-M78-1V_{L}-G10-1V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKGGGGSGGGGSDIQLTQSPSFLSASVGDRVTITCR ASQEISGFLSWLQQKPGKTIKRLIYAASTLDSGVPSRFSGSRSGSEY TLTISSLQPEDFATYYCLQYASYPRTFGGGTKVEIKRTVAAPSVFIFP PSDEQLKSGTASVVCLLNNFYPREAKVQWKVDNALQSGNSQESV TEQDSKDSTYSLSSTLTLSKADYEKHKVYACEVTHQGLSSPVTKSF NRGEC |
| 131 | M78-1-G10-2-DVD-IgG4 heavy chain (humFL-M78-1V_{H}-G10-2V_{H}-IgG4C_{H}) | EVQLVESGGGLVQPGGSLRLSCAASGFTFSSYDMSWVRQAPGKR LEWVAFISSGGGSTYYPDTVKGRFTISRDNAKNSLYLQMNSLRAE |
| | | DTAVYYCGRHYDNYLYAMDYWGQGTLVTVSSGGGGSGGGGSEV QLVESGGGLVQPGGSLRLSCAASGFTFSNYGMSWVRQAPGKRLE WVATISGGGSYTYYPDNLKGRFTISRDNAKNSLYLQMNSLRAEDT AVYYCAAPTAMDYWGQGTLVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAP EFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 106 | M78-1-G10-2-DVD-IgG4 light chain (humFL-M78-1V_{L}-G10-2V_{L}-C_{L}) | DIQMTQSPSSLSASVGDRVTITCLASQTIGTWLAWYQQKPGKSPKL LIYAATSLADGVPSRFSGSGSGTDFTLTISSLQPEDFATYYCQQLYST PFTFGQGTKVEIKGGGGSGGGGSDIQLTQSPSFLSASVGDRVTITCR ASQEISGFLSWLQQKPGKTIKRLIYAASTLDSGVPSRFSGSRSGTEY TLTISSLQPEDFATYYCLQYASYPRTFGGGTKVEIKRTVAAPSVFIFP PSDEQLKSGTASVVCLLNNFYPREAKVQWKVDNALQSGNSQESV TEQDSKDSTYSLSSTLTLSKADYEKHKVYACEVTHQGLSSPVTKSF NRGEC |
| 132 | G10-1-M78-1-DVD-IgG4 heavy chain (G10-1V_{H}-humFL-M78-1V_{H}-IgG4C_{H}) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSGGGGSGGGGSEVQLVESG GGLVQPGGSLRLSCAASGFTFSSYDMSWVRQAPGKRLEWVAFISS GGGSTYYPDTVKGRFTISRDNAKNSLYLQMNSLRAEDTAVYYCG RHYDNYLYAMDYWGQGTLVTVSSASTKGPSVFPLAPCSRSTSEST AALGCLVKDYFPEPVTVSWNSGALTSGVHTFPAVLQSSGLYSLSSV VTVPSSSLGTKTYTCNVDHKPSNTKVDKRVESKYGPPCPPCPAPEF LGGPSVFLFPPKPKDTLMISRTPEVTCVVVDVSQEDPEVQFNWYV DGVEVHNAKTKPREEQFNSTYRVVSVLTVLHQDWLNGKEYKCK VSNKGLPSSIEKTISKAKGQPREPQVYTLPPSQEEMTKNQVSLTCL VKGFYPSDIAVEWESNGQPENNYKTTPPVLDSDGSFFLYSRLTVDK SRWQEGNVFSCSVMHEALHNHYTQKSLSLSLGK |
| 108 | G10-1-M78-1-DVD-IgG4 light chain (G10-1V_{L}-humFL-M78-1V_{L}-C_{L}) | DIQLTQSPSFLSASVGDRVTITCRASQEISGFLSWLQQKPGKTIKRLI YAASTLDSGVPSRFSGSRSGSEYTLTISSLQPEDFATYYCLQYASYP RTFGGGTKVEIKGGGGSGGGGSDIQMTQSPSSLSASVGDRVTITCL ASQTIGTWLAWYQQKPGKSPKLLIYAATSLADGVPSRFSGSGSGT DFTLTISSLQPEDFATYYCQQLYSTPFTFGQGTKVEIKRTVAAPSVFI FPPSDEQLKSGTASVVCLLNNFYPREAKVQWKVDNALQSGNSQE SVTEQDSKDSTYSLSSTLTLSKADYEKHKVYACEVTHQGLSSPVT KSFNRGEC |
| 132 | G10-2-M78-1-DVD-IgG4 heavy chain (G10-2V_{H}-humFL-M78-1VH-IgG4C_{H}) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSGGGGSGGGGSEVQLVESG GGLVQPGGSLRLSCAASGFTFSSYDMSWVRQAPGKRLEWVAFISS GGGSTYYPDTVKGRFTISRDNAKNSLYLQMNSLRAEDTAVYYCG RHYDNYLYAMDYWGQGTLVTVSSASTKGPSVFPLAPCSRSTSEST AALGCLVKDYFPEPVTVSWNSGALTSGVHTFPAVLQSSGLYSLSSV VTVPSSSLGTKTYTCNVDHKPSNTKVDKRVESKYGPPCPPCPAPEF LGGPSVFLFPPKPKDTLMISRTPEVTCVVVDVSQEDPEVQFNWYV DGVEVHNAKTKPREEQFNSTYRVVSVLTVLHQDWLNGKEYKCK |
| | | VSNKGLPSSIEKTISKAKGQPREPQVYTLPPSQEEMTKNQVSLTCL VKGFYPSDIAVEWESNGQPENNYKTTPPVLDSDGSFFLYSRLTVDK SRWQEGNVFSCSVMHEALHNHYTQKSLSLSLGK |
| 109 | G10-2-M78-1-DVD-IgG4 light chain (G10-2V_{L}-humFL-M78-1V_{L}-C_{L}) | DIQLTQSPSFLSASVGDRVTITCRASQEISGFLSWLQQKPGKTIKRLI YAASTLDSGVPSRFSGSRSGTEYTLTISSLQPEDFATYYCLQYASYP RTFGGGTKVEIKGGGGSGGGGSDIQMTQSPSSLSASVGDRVTITCL ASQTIGTWLAWYQQKPGKSPKLLIYAATSLADGVPSRFSGSGSGT DFTLTISSLQPEDFATYYCQQLYSTPFTFGQGTKVEIKRTVAAPSVFI FPPSDEQLKSGTASVVCLLNNFYPREAKVQWKVDNALQSGNSQE SVTEQDSKDSTYSLSSTLTLSKADYEKHKVYACEVTHQGLSSPVT KSFNRGEC |
| 133 | M88-15-G72-11-DVD-IgG4 heavy chain (humFL-M88-15V_{H}-G72-11V_{H}-IgG4C_{H}) | QVQLVQSGAEVKKPGASVKVSCKGSGYTFTDYTIHWVRQAPGQG LEWMGVISTYQGDASYNQKFKGRATMTVDKSTSTAYMELSSLRS EDTAVYYCARFPDWDYAMDYWGQGTLVTVSSGGGGSGGGGSEV QLVQSGAEVKKPGATVKISCKASGFNIKDTYMHWVQQAPGKGLE WIGRIRPANGNTKYDPKFQGRATVTADISTDTAYMELSSLRSEDTA VYYCASYYYGSSGAYWGQGTLVTVSSASTKGPSVFPLAPCSRSTS ESTAALGCLVKDYFPEPVTVSWNSGALTSGVHTFPAVLQSSGLYSL SSVVTVPSSSLGTKTYTCNVDHKPSNTKVDKRVESKYGPPCPPCPA PEFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 111 | M88-15-G72-11-DVD-IgG4 light chain (humFL-M88-15V_{L}-G72-11V_{L}-C_{L}) | EIVMTQSPATLSVSPGERATLSCKASQSVDYDGDSYMNWYQQKP GQPPRLLIYAASNLESGIPARFSGSGSGTEFTLTISSLQSEDFAVYYC QQSRELPYTFGQGTKLEIKGGGGSGGGGSEIVMTQSPATLSVSPGE RATLSCSVSSSVSYMYWYQQKPGQAPRLLIYDTSSLASGIPARFSG SGSGTEFTLTISSLQSEDFAVYYCQQWSSYPYTFGGGTKVEIKRTVA APSVFIFPPSDEQLKSGTASVVCLLNNFYPREAKVQWKVDNALQS GNSQESVTEQDSKDSTYSLSSTLTLSKADYEKHKVYACEVTHQGL SSPVTKSFNRGEC |
| 176 | M88-19-G72-16-DVD-IgG4 heavy chain (humFL-M88-19V_{H}-G72-16V_{H}-IgG4C_{H}) | QVQLVQSGAEVKKPGASVKVSCKGSGYTFTDYTIHWVRQAPGQG LEWMGVISTYQGDASYNQKFKGRATMTVDKSTSTAYMELSSLRS EDTAVYYCARFPDWDYAMDYWGQGTLVTVSSGGGGSGGGGSEV QLVQSGAEVKKPGATVKISCKASGFNIKDTYMHWVQQAPGKGLE WIGRIRPANANAKYDPKFQGRATVTADISTDTAYMELSSLRSEDTA VYYCASYYYGSSGAYWGQGTLVTVSSASTKGPSVFPLAPCSRSTS ESTAALGCLVKDYFPEPVTVSWNSGALTSGVHTFPAVLQSSGLYSL SSVVTVPSSSLGTKTYTCNVDHKPSNTKVDKRVESKYGPPCPPCPA PEFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 174 | M88-19-G72-16-DVD-IgG4 light chain (humFL-M88-19V_{L}-G72-16V_{L}-C_{L}) | EIVMTQSPATLSVSPGERATLSCKASQSVDYEGDSYMNWYQQKPG QPPRLLIYAASNLESGIPARFSGSGSGTEFTLTISSLQSEDFAVYYCQ QSRELPYTFGQGTKLEIKGGGGSGGGGSEIVMTQSPATLSVSPGER ATLSCSVSSSVSYMYWYQQKPGQAPRLLIYDTSSLASGIPARFSGS GSGTEFTLTISSLQSEDFAVYYCQQWSSYPYTFGGGTKVEIKRTVA |
| | | APSVFIFPPSDEQLKSGTASVVCLLNNFYPREAKVQWKVDNALQS GNSQESVTEQDSKDSTYSLSSTLTLSKADYEKHKVYACEVTHQGL SSPVTKSFNRGEC |
| 201 | M88-20-G72-16-DVD-IgG4 heavy chain (humFL-M88-20V_{H}-G72-16V_{H}-IgG4C_{H}) | QVQLVQSGAEVKKPGASVKVSCKGSGYTFTDYTIHWVRQAPGQG LEWMGVISTYQGDASYNQKFKGRATMTVDKSTSTAYMELSSLRS EDTAVYYCARFPDWDYAMDYWGQGTLVTVSSGGGGSGGGGSEV QLVQSGAEVKKPGATVKISCKASGFNIKDTYMHWVQQAPGKGLE WIGRIRPANANAKYDPKFQGRATVTADISTDTAYMELSSLRSEDTA VYYCASYYYGSSGAYWGQGTLVTVSSASTKGPSVFPLAPCSRSTS ESTAALGCLVKDYFPEPVTVSWNSGALTSGVHTFPAVLQSSGLYSL SSVVTVPSSSLGTKTYTCNVDHKPSNTKVDKRVESKYGPPCPPCPA PEFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 185 | M88-20-G72-16-DVD-IgG4 light chain (humFL-M88-20V_{L}-G72-16V_{L}-C_{L}) | EIVMTQSPATLSVSPGERATLSCKASQSVDYEGDTYMNWYQQKPG QPPRLLIYAASNLESGIPARFSGSGSGTEFTLTISSLQSEDFAVYYCQ QSRELPYTFGQGTKLEIKGGGGSGGGGSEIVMTQSPATLSVSPGER ATLSCSVSSSVSYMYWYQQKPGQAPRLLIYDTSSLASGIPARFSGS GSGTEFTLTISSLQSEDFAVYYCQQWSSYPYTFGGGTKVEIKRTVA APSVFIFPPSDEQLKSGTASVVCLLNNFYPREAKVQWKVDNALQS GNSQESVTEQDSKDSTYSLSSTLTLSKADYEKHKVYACEVTHQGL SSPVTKSFNRGEC |
| 202 | M88-15-G72-16-DVD-IgG4 heavy chain (humFL-M88-15V_{H}-G72-16V_{H}-IgG4C_{H}) | QVQLVQSGAEVKKPGASVKVSCKGSGYTFTDYTIHWVRQAPGQG LEWMGVISTYQGDASYNQKFKGRATMTVDKSTSTAYMELSSLRS EDTAVYYCARFPDWDYAMDYWGQGTLVTVSSGGGGSGGGGSEV QLVQSGAEVKKPGATVKISCKASGFNIKDTYMHWVQQAPGKGLE WIGRIRPANANAKYDPKFQGRATVTADISTDTAYMELSSLRSEDTA VYYCASYYYGSSGAYWGQGTLVTVSSASTKGPSVFPLAPCSRSTS ESTAALGCLVKDYFPEPVTVSWNSGALTSGVHTFPAVLQSSGLYSL SSVVTVPSSSLGTKTYTCNVDHKPSNTKVDKRVESKYGPPCPPCPA PEFLGGPSVFLFPPKPKDTLMISRTPEVTCVVVDVSQEDPEVQFNW YVDGVEVHNAKTKPREEQFNSTYRVVSVLTVLHQDWLNGKEYK CKVSNKGLPSSIEKTISKAKGQPREPQVYTLPPSQEEMTKNQVSLT CLVKGFYPSDIAVEWESNGQPENNYKTTPPVLDSDGSFFLYSRLTV DKSRWQEGNVFSCSVMHEALHNHYTQKSLSLSLGK |
| 186 | M88-15-G72-16-DVD-IgG4 light chain (humFL-M88-15V_{L}-G72-16V_{L}-C_{L}) | EIVMTQSPATLSVSPGERATLSCKASQSVDYDGDSYMNWYQQKP GQPPRLLIYAASNLESGIPARFSGSGSGTEFTLTISSLQSEDFAVYYC QQSRELPYTFGQGTKLEIKGGGGSGGGGSEIVMTQSPATLSVSPGE RATLSCSVSSSVSYMYWYQQKPGQAPRLLIYDTSSLASGIPARFSG SGSGTEFTLTISSLQSEDFAVYYCQQWSSYPYTFGGGTKVEIKRTVA APSVFIFPPSDEQLKSGTASVVCLLNNFYPREAKVQWKVDNALQS GNSQESVTEQDSKDSTYSLSSTLTLSKADYEKHKVYACEVTHQGL SSPVTKSFNRGEC |

**Table 8-2: Full-length heavy chain and light chain sequences of the exemplary Bs4Ab format bispecific antibodies specifically binding to TRAIL and FasL**

| **SEQ IDNO** | **Description** | **Sequence** |
|---|---|---|
| 134 | M88-G72scFv-Bs4Ab-IgG1 heavy chain FL-M88V_{H}-C_{H}1-G72scFv-IgG1Fc) | EVQLQQSGAELVRPGVSVKISCKGSGYTFTDYTIHWVKQSHAKSL EWIGVISTYQGDASYNQKFKGKATMTVDKSSSTAYMELARLTSE DSAIYYCARFPDWDYAMDYWGQGTSVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKKVEPKSCG GGGSGGGGSEVQLQQSGAELVKPGASVKLSCTASGFNIKDTYMH WVKQRPEQCLEWIGRIRPANGNTKYDPKFQGKATVTADISSNTAY LQLSSLTSEDTAVYYCASYYYGSSGAYWGQGTLVTVSAGGGGSG GGGSGGGGSGGGGSDIVMTQSPAIMSASLGEKVTMTCSVSSSVSY MYWYQQKPGSSPRLLIYDTSSLASGVPVRFSGSGSGTSYSLTISRM EAEDAATYYCQQWSSYPYTFGCGTKLEIKGGGGSGGGGSDKTHT CPPCPAPELLGGPSVFLFPPKPKDTLMISRTPEVTCVVVDVSHEDP EVKFNWYVDGVEVHNAKTKPREEQYNSTYRVVSVLTVLHQDWL NGKEYKCKVSNKALPAPIEKTISKAKGQPREPQVYTLPPSREEMT KNQVSLTCLVKGFYPSDIAVEWESNGQPENNYKTTPPVLDSDGSF FLYSKLTVDKSRWQQGNVFSCSVMHEALHNHYTQKSLSLSPGK |
| 113 | M88-G72scFv-Bs4Ab-IgG1 light chain (FL-M88V_{L}-C_{L}) | EIVLTQSPASLAVSLGQRATISCKASQSVDYDGDSYMNWYQQKPG QPPKLLIYAASNLESGVPARFSGSGSGTDFTLNIHPVEEEDAATYY CQHSRELPYTFGGGTKLEIKRTVAAPSVFIFPPSDEQLKSGTASVV CLLNNFYPREAKVQWKVDNALQSGNSQESVTEQDSKDSTYSLSS TLTLSKADYEKHKVYACEVTHQGLSSPVTKSFNRGEC |
| 135 | M88-G72scFv-Bs4Ab-IgG4 heavy chain (FL-M88V_{H}-C_{H}1-G72scFv-IgG4Fc) | EVQLQQSGAELVRPGVSVKISCKGSGYTFTDYTIHWVKQSHAKSL EWIGVISTYQGDASYNQKFKGKATMTVDKSSSTAYMELARLTSE DSAIYYCARFPDWDYAMDYWGQGTSVTVSSASTKGPSVFPLAPS SKSTSGGTAALGCLVKDYFPEPVTVSWNSGALTSGVHTFPAVLQS SGLYSLSSVVTVPSSSLGTQTYICNVNHKPSNTKVDKKVEPKSCG GGGSGGGGSEVQLQQSGAELVKPGASVKLSCTASGFNIKDTYMH WVKQRPEQCLEWIGRIRPANGNTKYDPKFQGKATVTADISSNTAY LQLSSLTSEDTAVYYCASYYYGSSGAYWGQGTLVTVSAGGGGSG GGGSGGGGSGGGGSDIVMTQSPAIMSASLGEKVTMTCSVSSSVSY MYWYQQKPGSSPRLLIYDTSSLASGVPVRFSGSGSGTSYSLTISRM EAEDAATYYCQQWSSYPYTFGCGTKLEIKGGGGSGGGGSESKYG PPCPPCPAPEFLGGPSVFLFPPKPKDTLMISRTPEVTCVVVDVSQED PEVQFNWYVDGVEVHNAKTKPREEQFNSTYRVVSVLTVLHQDW LNGKEYKCKVSNKGLPSSIEKTISKAKGQPREPQVYTLPPSQEEM TKNQVSLTCLVKGFYPSDIAVEWESNGQPENNYKTTPPVLDSDGS FFLYSRLTVDKSRWQEGNVFSCSVMHEALHNHYTQKSLSLSLGK |
| 113 | M88-G72scFv-Bs4Ab-IgG4 light chain (FL-M88V_{L}-C_{L}) | EIVLTQSPASLAVSLGQRATISCKASQSVDYDGDSYMNWYQQKPG QPPKLLIYAASNLESGVPARFSGSGSGTDFTLNIHPVEEEDAATYY CQHSRELPYTFGGGTKLEIKRTVAAPSVFIFPPSDEQLKSGTASVV CLLNNFYPREAKVQWKVDNALQSGNSQESVTEQDSKDSTYSLSS TLTLSKADYEKHKVYACEVTHQGLSSPVTKSFNRGEC |
| 136 | G72-M88scFv-Bs4Ab-IgG1 heavy chain (G72V_{H}-C_{H}1-FL-M88scFv-IgG1Fc) | EVQLQQSGAELVKPGASVKLSCTASGFNIKDTYMHWVKQRPEQG LEWIGRIRPANGNTKYDPKFQGKATVTADISSNTAYLQLSSLTSED TAVYYCASYYYGSSGAYWGQGTLVTVSAASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCGGGG SGGGGSEVQLQQSGAELVRPGVSVKISCKGSGYTFTDYTIHWVK QSHAKCLEWIGVISTYQGDASYNQKFKGKATMTVDKSSSTAYME |
| | | LARLTSEDSAIYYCARFPDWDYAMDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SDKTHTCPPCPAPELLGGPSVFLFPPKPKDTLMISRTPEVTCVVVD VSHEDPEVKFNWYVDGVEVHNAKTKPREEQYNSTYRVVSVLTV LHQDWLNGKEYKCKVSNKALPAPIEKTISKAKGQPREPQVYTLPP SREEMTKNQVSLTCLVKGFYPSDIAVEWESNGQPENNYKTTPPVL DSDGSFFLYSKLTVDKSRWQQGNVFSCSVMHEALHNHYTQKSLS LSPGK |
| 115 | G72-M88scFv-Bs4Ab-IgG1 light chain (G72V_{L}-C_{L}) | DIVMTQSPAIMSASLGEKVTMTCSVSSSVSYMYWYQQKPGSSPR LLIYDTSSLASGVPVRFSGSGSGTSYSLTISRMEAEDAATYYCQQW SSYPYTFGGGTKLEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| 137 | G72-M88scFv-Bs4Ab-IgG4 heavy chain (G72V_{H}-C_{H}1-FL-M88scFv-IgG4Fc) | EVQLQQSGAELVKPGASVKLSCTASGFNIKDTYMHWVKQRPEQG LEWIGRIRPANGNTKYDPKFQGKATVTADISSNTAYLQLSSLTSED TAVYYCASYYYGSSGAYWGQGTLVTVSAASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCGGGG SGGGGSEVQLQQSGAELVRPGVSVKISCKGSGYTFTDYTIHWVK QSHAKCLEWIGVISTYQGDASYNQKFKGKATMTVDKSSSTAYME LARLTSEDSAIYYCARFPDWDYAMDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 115 | G72-M88scFv-Bs4Ab-IgG4 light chain (G72V_{L}-C_{L}) | DIVMTQSPAIMSASLGEKVTMTCSVSSSVSYMYWYQQKPGSSPR LLIYDTSSLASGVPVRFSGSGSGTSYSLTISRMEAEDAATYYCQQW SSYPYTFGGGTKLEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |
| 138 | M78-1-G10-1scFv-Bs4Ab-IgG4 heavy chain (humFL-M78-1V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV |
| | | QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 119 | M78-1-G10-1scFv-Bs4Ab-IgG4 light chain (humFL-M78-1V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 139 | M78-1-G10-1scFv-2-Bs4Ab-IgG4 heavy chain (humFL-M78-1V_{H}-C_{H}1-G10-1scFv-2-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSDIQLTQSPSFLSASVGDRVTITCRASQEISGFLSWL QQKPGKTIKRLIYAASTLDSGVPSRFSGSRSGSEYTLTISSLQPEDFA TYYCLQYASYPRTFGCGTKVEIKGGGGSGGGGSGGGGSGGGGSE VQLVESGGGLVQPGGSLRLSCAASGFTFSNYGMSWVRQAPGKCL EWVATISGGGSYTYYPDNLKGRFTISRDNAKNSLYLQMNSLRAED TAVYYCAAPTAMDYWGQGTLVTVSSGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 119 | M78-1-G10-1scFv-2-Bs4Ab-IgG4 light chain (humFL-M78-1V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 140 | M78-1-G10-2scFv-Bs4Ab-IgG4 heavy chain (humFL-M78-1V_{H}-C_{H}1-G10-2scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKRLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGTEYTLTISSLQPE DFATYYCLQYASYPRTFGGGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 119 | M78-1-G10-2scFv-Bs4Ab-IgG4 light chain (humFL-M78-1V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY |
| | | STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 141 | G10-1-M78-1scFv-Bs4Ab-IgG4 heavy chain (G10-1V_{H}-C_{H}1-humFL-M78-1scFv-IgG4Fc) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSASTKGPSVFPLAPSSKSTS GGTAALGCLVKDYFPEPVTVSWNSGALTSGVHTFPAVLQSSGLYS LSSVVTVPSSSLGTQTYICNVNHKPSNTKVDKKVEPKSCGGGGSG GGGSEVQLVESGGGLVQPGGSLRLSCAASGFTFSSYDMSWVRQA PGKCLEWVAFISSGGGSTYYPDTVKGRFTISRDNAKNSLYLQMNS LRAEDTAVYYCGRHYDNYLYAMDYWGQGTLVTVSSGGGGSGGG GSGGGGSGGGGSDIQMTQSPSSLSASVGDRVTITCLASQTIGTWL AWYQQKPGKSPKLLIYAATSLADGVPSRFSGSGSGTDFTLTISSLQ PEDFATYYCQQLYSTPFTFGCGTKVEIKGGGGSGGGGSESKYGPP CPPCPAPEFLGGPSVFLFPPKPKDTLMISRTPEVTCVVVDVSQEDP EVQFNWYVDGVEVHNAKTKPREEQFNSTYRVVSVLTVLHQDWL NGKEYKCKVSNKGLPSSIEKTISKAKGQPREPQVYTLPPSQEEMT KNQVSLTCLVKGFYPSDIAVEWESNGQPENNYKTTPPVLDSDGSF FLYSRLTVDKSRWQEGNVFSCSVMHEALHNHYTQKSLSLSLGK |
| 121 | G10-1-M78-1scFv-Bs4Ab-IgG4 light chain (G10-1V_{L}-C_{L}) | DIQLTQSPSFLSASVGDRVTITCRASQEISGFLSWLQQKPGKTIKRL IYAASTLDSGVPSRFSGSRSGSEYTLTISSLQPEDFATYYCLQYASY PRTFGGGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |
| 141 | G10-2-M78-1scFv-Bs4Ab-IgG4 heavy chain (G10-2V_{H}-C_{H}1-humFL-M78-1scFv-IgG4Fc) | EVQLVESGGGLVQPGGSLRLSCAASGFTFSNYGMSWVRQAPGKR LEWVATISGGGSYTYYPDNLKGRFTISRDNAKNSLYLQMNSLRAE DTAVYYCAAPTAMDYWGQGTLVTVSSASTKGPSVFPLAPSSKSTS GGTAALGCLVKDYFPEPVTVSWNSGALTSGVHTFPAVLQSSGLYS LSSVVTVPSSSLGTQTYICNVNHKPSNTKVDKKVEPKSCGGGGSG GGGSEVQLVESGGGLVQPGGSLRLSCAASGFTFSSYDMSWVRQA PGKCLEWVAFISSGGGSTYYPDTVKGRFTISRDNAKNSLYLQMNS LRAEDTAVYYCGRHYDNYLYAMDYWGQGTLVTVSSGGGGSGGG GSGGGGSGGGGSDIQMTQSPSSLSASVGDRVTITCLASQTIGTWL AWYQQKPGKSPKLLIYAATSLADGVPSRFSGSGSGTDFTLTISSLQ PEDFATYYCQQLYSTPFTFGCGTKVEIKGGGGSGGGGSESKYGPP CPPCPAPEFLGGPSVFLFPPKPKDTLMISRTPEVTCVVVDVSQEDP EVQFNWYVDGVEVHNAKTKPREEQFNSTYRVVSVLTVLHQDWL NGKEYKCKVSNKGLPSSIEKTISKAKGQPREPQVYTLPPSQEEMT KNQVSLTCLVKGFYPSDIAVEWESNGQPENNYKTTPPVLDSDGSF FLYSRLTVDKSRWQEGNVFSCSVMHEALHNHYTQKSLSLSLGK |
| 122 | G10-2-M78-1scFv-Bs4Ab-IgG4 light chain (G10-2V_{L}-C_{L}) | DIQLTQSPSFLSASVGDRVTITCRASQEISGFLSWLQQKPGKTIKRL IYAASTLDSGVPSRFSGSRSGTEYTLTISSLQPEDFATYYCLQYASY PRTFGGGTKVEIKRTVAAPSVFIFPPSDEQLKSGTASVVCLLNNFYP REAKVQWKVDNALQSGNSQESVTEQDSKDSTYSLSSTLTLSKAD YEKHKVYACEVTHQGLSSPVTKSFNRGEC |
| 142 | G72-11-M88scFv-Bs4Ab-IgG4 heavy chain (G72-11V_{H}-C_{H}1-FL-M88scFv-IgG4Fc) | EVQLVQSGAEVKKPGATVKISCKASGFNIKDTYMHWVQQAPGK GLEWIGRIRPANGNTKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG |
| | | GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAMDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 124 | G72-11-M88scFv-Bs4Ab-IgG4 light chain (G72-11V_{L}-C_{L}) | EIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 175 | M78-5-G10-6scFv-Bs4Ab-IgG4 heavy chain (humFL-M78-5V_{H}-C_{H}1-G10-6scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDTGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 173 | M78-5-G10-6scFv-Bs4Ab-IgG4 light chain (humFL-M78-5V_{L}-C_{L}) | DIQMTQSPSSLSASVGDRVTITCLASQTIGTWLAWYQQKPGKSPK LLIYAATSLASGVPSRFSGSGSGTDFTLTISSLQPEDFATYYCQQLYS TPFTFGQGTKVEIKRTVAAPSVFIFPPSDEQLKSGTASVVCLLNNFY PREAKVQWKVDNALQSGNSQESVTEQDSKDSTYSLSSTLTLSKA DYEKHKVYACEVTHQGLSSPVTKSFNRGEC |
| 193 | M78-6-G10-1scFv-Bs4Ab-IgG4 heavy chain (humFL-M78-6V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP |
| | | PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 177 | M78-6-G10-1scFv-Bs4Ab-IgG4 light chain (humFL-M78-6V_{L}-C_{L}) | DIQMTQSPSSLSASVGDRVTITCLASQTIGTWLAWYQQKPGKSPK LLIYAATSLAEGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 194 | M78-5-G10-1scFv-Bs4Ab-IgG4 heavy chain (humFL-M78-5V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 178 | M78-5-G10-1scFv-Bs4Ab-IgG4 light chain (humFL-M78-5V_{L}-C_{L}) | DIQMTQSPSSLSASVGDRVTITCLASQTIGTWLAWYQQKPGKSPK LLIYAATSLASGVPSRFSGSGSGTDFTLTISSLQPEDFATYYCQQLYS TPFTFGQGTKVEIKRTVAAPSVFIFPPSDEQLKSGTASVVCLLNNFY PREAKVQWKVDNALQSGNSQESVTEQDSKDSTYSLSSTLTLSKA DYEKHKVYACEVTHQGLSSPVTKSFNRGEC |
| 195 | M78-7-G10-1scFv-Bs4Ab-IgG4 heavy chain (humFL-M78-7V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYENYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 179 | M78-7-G10-1scFv-Bs4Ab-IgG4 light chain (humFL-M78-7V_{L}-C_{L}) | DIQMTQSPSSLSASVGDRVTITCLASQTIGTWLAWYQQKPGKSPK |
| | | LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 196 | M78-8-G10-1scFv-Bs4Ab-IgG4 heavy chain (humFL-M78-8V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDTYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 180 | M78-8-G10-1scFv-Bs4Ab-IgG4 light chain (humFL-M78-8V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 197 | M78-9-G10-1scFv-Bs4Ab-IgG4 heavy chain (humFL-M78-9V_{H}-C_{H}1-G10-1scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDAYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDSGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 181 | M78-9-G10-1scFv-Bs4Ab-IgG4 light chain (humFL-M78-9V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 198 | M78-1-G10-6scFv-Bs4Ab-IgG4 heavy chain (humFL-M78-6V_{H}-C_{H}1-G10-6scFv-IgG4Fc) | EVQLVESGGGLVQPGGSLRLSCAASGFTFSSYDMSWVRQAPGKR |
| | | LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDAGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 182 | M78-1-G10-6scFv-Bs4Ab-IgG4 light chain (humFL-M78-6V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 199 | M78-1-G10-7scFv-Bs4Ab-IgG4 heavy chain (humFL-M78-6V_{H}-C_{H}1-G10-7scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDNYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDTGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 183 | M78-1-G10-7scFv-Bs4Ab-IgG4 light chain (humFL-M78-6V_{L}-C_{L}) | DIQMTQSPSSLSASVGDRVTITCLASQTIGTWLAWYQQKPGKSPK LLIYAATSLADGVPSRFSGSGSGTDFTLTISSLQPEDFATYYCQQLY STPFTFGQGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 200 | M78-10-G10-7scFv-Bs4Ab-IgG4 heavy chain (humFL-M78-10V_{H}-C_{H}1-G10-7scFv-IgG4Fc) | EVQLVESGGGLVQPGGSLRLSCAASGFTFSSYDMSWVRQAPGKR LEWVAFISSGGGSTYYPDTVKGRFTISRDNAKNSLYLQMNSLRAE DTAVYYCGRHYDAYLYAMDYWGQGTLVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKKVEPKSC GGGGSGGGGSEVQLVESGGGLVQPGGSLRLSCAASGFTFSNYGM SWVRQAPGKCLEWVATISGGGSYTYYPDNLKGRFTISRDNAKNSL |
| | | YLQMNSLRAEDTAVYYCAAPTAMDYWGQGTLVTVSSGGGGSGG GGSGGGGSGGGGSDIQLTQSPSFLSASVGDRVTITCRASQEISGFLS WLQQKPGKTIKRLIYAASTLDTGVPSRFSGSRSGSEYTLTISSLQPE DFATYYCLQYASYPRTFGCGTKVEIKGGGGSGGGGSESKYGPPCP PCPAPEFLGGPSVFLFPPKPKDTLMISRTPEVTCVVVDVSQEDPEV QFNWYVDGVEVHNAKTKPREEQFNSTYRVVSVLTVLHQDWLNG KEYKCKVSNKGLPSSIEKTISKAKGQPREPQVYTLPPSQEEMTKN QVSLTCLVKGFYPSDIAVEWESNGQPENNYKTTPPVLDSDGSFFLY SRLTVDKSRWQEGNVFSCSVMHEALHNHYTQKSLSLSLGK |
| 184 | M78-10-G10-7scFv-Bs4Ab-IgG4 light chain (humFL-M78-10V_{L}-C_{L}) | DIQMTQSPSSLSASVGDRVTITCLASQTIGTWLAWYQQKPGKSPK LLIYAATSLASGVPSRFSGSGSGTDFTLTISSLQPEDFATYYCQQLYS TPFTFGQGTKVEIKRTVAAPSVFIFPPSDEQLKSGTASVVCLLNNFY PREAKVQWKVDNALQSGNSQESVTEQDSKDSTYSLSSTLTLSKA DYEKHKVYACEVTHQGLSSPVTKSFNRGEC |
| 203 | G72-16-M88-22scFv-Bs4Ab-IgG4 heavy chain (G72-16V_{H}-C_{H}1-FL-M88-22scFv-IgG4Fc) | EVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAMDYWGQGTSVTVSSGGGGSG GGGSGGGGSGGGGSEIVLTQSPASLAVSLGQRATISCKASQSVDYE GDTYMNWYQQKPGQPPKLLIYAASNLESGVPARFSGSGSGTDFTL NIHPVEEEDAATYYCQHSRELPYTFGCGTKLEIKGGGGSGGGGSE SKYGPPCPPCPAPEFLGGPSVFLFPPKPKDTLMISRTPEVTCVVVD VSQEDPEVQFNWYVDGVEVHNAKTKPREEQFNSTYRVVSVLTVL HQDWLNGKEYKCKVSNKGLPSSIEKTISKAKGQPREPQVYTLPPS QEEMTKNQVSLTCLVKGFYPSDIAVEWESNGQPENNYKTTPPVLD SDGSFFLYSRLTVDKSRWQEGNVFSCSVMHEALHNHYTQKSLSLS LGK |
| 187 | G72-16-M88-22scFv-Bs4Ab-IgG4 light chain (G72-16V_{L}-C_{L}) | EIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 204 | G72-16-M88-21scFv-Bs4Ab-IgG4 heavy chain (G72-16V_{H}-C_{H}1-FL-M88-21scFv-IgG4Fc) | EVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAMDYWGQGTSVTVSSGGGGSG GGGSGGGGSGGGGSEIVLTQSPASLAVSLGQRATISCKASQSVDYE GDSYMNWYQQKPGQPPKLLIYAASNLESGVPARFSGSGSGTDFTL NIHPVEEEDAATYYCQHSRELPYTFGCGTKLEIKGGGGSGGGGSE SKYGPPCPPCPAPEFLGGPSVFLFPPKPKDTLMISRTPEVTCVVVD VSQEDPEVQFNWYVDGVEVHNAKTKPREEQFNSTYRVVSVLTVL |
| | | HQDWLNGKEYKCKVSNKGLPSSIEKTISKAKGQPREPQVYTLPPS QEEMTKNQVSLTCLVKGFYPSDIAVEWESNGQPENNYKTTPPVLD SDGSFFLYSRLTVDKSRWQEGNVFSCSVMHEALHNHYTQKSLSLS LGK |
| 188 | G72-16-M88-21scFv-Bs4Ab-IgG4 light chain (G72-16V_{L}-C_{L}) | EIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 205 | G72-16-M88scFv-Bs4Ab-IgG4 heavy chain (G72-16V_{H}-C_{H}1-FL-M88scFv-IgG4Fc) | EVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAMDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 189 | G72-16-M88scFv-Bs4Ab-IgG4 light chain (G72-16V_{L}-C_{L}) | EIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 206 | G72-17-M88scFv-Bs4Ab-IgG4 heavy chain (G72-17V_{H}-C_{H}1-FL-M88scFv-IgG4Fc) | QVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAMDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 190 | G72-17-M88scFv-Bs4Ab-IgG4 light chain (G72-17V_{L}-C_{L}) | DIVMTQSPATLSVSPGERATLSCSVSSSVSYMYWYQQKPGQAPRL LIYDTSSLASGIPARFSGSGSGTEFTLTISSLQSEDFAVYYCQQWSS |
| | | YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 207 | G72-17-M88-23scFv-Bs4Ab-IgG4 heavy chain (G72-17V_{H}-C_{H}1-FL-M88-23 scFv-IgG4Fc) | QVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYALDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 191 | G72-17-M88-23scFv-Bs4Ab-IgG4 light chain (G72-17V_{L}-C_{L}) | DIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |
| 208 | G72-17-M88-24scFv-Bs4Ab-IgG4 heavy chain (G72-17V_{H}-C_{H}1-FL-M88-24scFv-IgG4Fc) | QVQLVQSGAEVKKPGATVKISCKASGFNIKDTYMHWVQQAPGK GLEWIGRIRPANANAKYDPKFQGRATVTADISTDTAYMELSSLRSE DTAVYYCASYYYGSSGAYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCGGG GSGGGGSEVQLQQSGAELVRPGVSVKISCKGSGYTFTDYTIHWV KQSHAKCLEWIGVISTYQGDASYNQKFKGKATMTVDKSSSTAYM ELARLTSEDSAIYYCARFPDWDYAVDYWGQGTSVTVSSGGGGSG GGGSGGGGSGGGGSEIVLTQSPASLAVSLGQRATISCKASQSVDY DGDSYMNWYQQKPGQPPKLLIYAASNLESGVPARFSGSGSGTDF TLNIHPVEEEDAATYYCQHSRELPYTFGCGTKLEIKGGGGSGGGG SESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTLP PSQEEMTKNQVSLTCLVKGFYPSDIAVEWESNGQPENNYKTTPPV LDSDGSFFLYSRLTVDKSRWQEGNVFSCSVMHEALHNHYTQKSL SLSLGK |
| 192 | G72-17-M88-24scFv-Bs4Ab-IgG4 light chain (G72-17V_{L}-C_{L}) | DIVMTQSPATLSVSPGERATLSCSVSSSVSYMYWYQQKPGQAPRL LIYDTSSLASGIPARFSGSGSGTEFTLTISSLQSEDFAVYYCQQWSS YPYTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHOGLSSPVTKSFNRGEC |

**Table 8-3: Full-length heavy chain and light chain sequences of the exemplary Hetero H, CrossMab format bispecific antibodies specifically binding to TRAIL and FasL**

| **SEQ ID NO** | **Description** | **Sequence** |
|---|---|---|
| 143 | M88-G72-CrossMab-IgG1 heavy chain (FL-M88V_{H}-C_{L}-IgG1Fc-hole) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASVAAPSVFIFPPSDE QLKSGTASVVCLLNNFYPREAKVQWKVDNALQSGNSQESVTEQD SKDSTYSLSSTLTLSKADYEKHKVYACEVTHQGLSSPVTKSFNRGE CDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVCTLPPSRD ELTKNQVSLSCAVKGFYPSDIAVEWESNGQPENNYKTTPPVLDSD GSFFLVSKLTVDKSRWQQGNVFSCSVMHEALHNHYTQKSLSLSPG K |
| 126 | M88-G72-CrossMab-IgG1 light chain (FL-M88V_{L}-C_{H}1) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKSSASTKGPSVFPLAPSSKSTSGGTAALG CLVKDYFPEPVTVSWNSGALTSGVHTFPAVLQSSGLYSLSSVVTVP SSSLGTQTYICNVNHKPSNTKVDKKVEPKSC |
| 144 | M88-G72-CrossMab-IgG1 heavy chain (G72V_{H}-IgG1C_{H}-knob) | EVQLQQSGAELVKPGASVKLSCTASGFNIKDTYMHWVKQRPEQG LEWIGRIRPANGNTKYDPKFQGKATVTADISSNTAYLQLSSLTSED TAVYYCASYYYGSSGAYWGQGTLVTVSAASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPCRDELTKN QVSLWCLVKGFYPSDIAVEWESNGQPENNYKTTPPVLDSDGSFFL YSKLTVDKSRWQQGNVFSCSVMHEALHNHYTQKSLSLSPGK |
| 129 | M88-G72-CrossMab-IgG1 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |
| 145 | M88-G72-CrossMab-IgG4 heavy chain (FL-M88V_{H}-C_{L}-IgG4Fc-hole) | EVQLQQSGAELVRPGVSVKISCKGSGYTFTDYTIHWVKQSHAKSL EWIGVISTYQGDASYNQKFKGKATMTVDKSSSTAYMELARLTSED SAIYYCARFPDWDYAMDYWGQGTSVTVSSASVAAPSVFIFPPSDE QLKSGTASVVCLLNNFYPREAKVQWKVDNALQSGNSQESVTEQD SKDSTYSLSSTLTLSKADYEKHKVYACEVTHQGLSSPVTKSFNRGE CESKYGPPCPPCPAPEFLGGPSVFLFPPKPKDTLMISRTPEVTCVVV DVSQEDPEVQFNWYVDGVEVHNAKTKPREEQFNSTYRVVSVLTV LHQDWLNGKEYKCKVSNKGLPSSIEKTISKAKGQPREPQVCTLPPS QEEMTKNQVSLSCAVKGFYPSDIAVEWESNGQPENNYKTTPPVLD SDGSFFLVSRLTVDKSRWQEGNVFSCSVMHEALHNHYTQKSLSLS LGK |
| 130 | M88-G72-CrossMab-IgG4 light chain (FL-M88V_{L}-C_{H}1) | EIVLTQSPASLAVSLGQRATISCKASQSVDYDGDSYMNWYQQKPG QPPKLLIYAASNLESGVPARFSGSGSGTDFTLNIHPVEEEDAATYYC QHSRELPYTFGGGTKLEIKSSASTKGPSVFPLAPCSRSTSESTAALG |
| | | CLVKDYFPEPVTVSWNSGALTSGVHTFPAVLQSSGLYSLSSVVTVP SSSLGTKTYTCNVDHKPSNTKVDKRVESKYG |
| 146 | M88-G72-CrossMab-IgG4 heavy chain (G72V_{H}-IgG4C_{H}-knob) | EVQLQQSGAELVKPGASVKLSCTASGFNIKDTYMHWVKQRPEQG LEWIGRIRPANGNTKYDPKFQGKATVTADISSNTAYLQLSSLTSED TAVYYCASYYYGSSGAYWGQGTLVTVSAASTKGPSVFPLAPCSRS TSESTAALGCLVKDYFPEPVTVSWNSGALTSGVHTFPAVLQSSGLY SLSSVVTVPSSSLGTKTYTCNVDHKPSNTKVDKRVESKYGPPCPPC PAPEFLGGPSVFLFPPKPKDTLMISRTPEVTCVVVDVSQEDPEVQFN WYVDGVEVHNAKTKPREEQFNSTYRVVSVLTVLHQDWLNGKEY KCKVSNKGLPSSIEKTISKAKGQPREPQVYTLPPCQEEMTKNQVSL WCLVKGFYPSDIAVEWESNGQPENNYKTTPPVLDSDGSFFLYSRLT VDKSRWQEGNVFSCSVMHEALHNHYTQKSLSLSLGK |
| 129 | M88-G72-CrossMab-IgG4 light chain (G72V_{L}-C_{L}) | DIVMTQSPAIMSASLGEKVTMTCSVSSSVSYMYWYQQKPGSSPRL LIYDTSSLASGVPVRFSGSGSGTSYSLTISRMEAEDAATYYCQQWS SYPYTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |

**Table 9: Exemplary peptide linker (or linker) sequences**

| **SEQ ID NO** | **Sequence** |
|---|---|
| 147 | GGGGSGGGGS |
| 148 | GGGGSGGGGSGGGGSGGGGS |
| 149 | GGGGSGGGGSGGGGS |
| 150 | ASTKGP |
| 151 | TVAAP |
| 152 | EPKSDKTGGGGSGGGGS |
| 153 | EPKSCGKTGGGGSGGGGS |
| 154 | EPKSCGGGGSGGGGS |
| 155 | GGGGSGGGGSEPKSDKTHTCPPCP |
| 156 | GGGGSGGGGSCPPCP |
| 157 | GGGGSGGGGSDKTHTCPPCP |
| 158 | GKPGSGKPGSGKPGSGKPGS |
| 159 | GGGGSGGGGTGGGGS |
| 160 | GGGGSGGGGSESKYGPPCPPCP |

### Combination of antibodies that specifically bind to TRAIL and FasL

On the one hand, this application provides a pharmaceutical composition comprising: (i) an antibody or antigen-binding fragment specifically binding to TRAIL, and (ii) an antibody or antigen-binding fragment specifically binding to FasL.

In some embodiments, there is provided a pharmaceutical composition comprising: (i) an antibody or antigen-binding fragment specifically binding to TRAIL, and (ii) an antibody or antigen-binding fragment specifically binding to FasL, wherein the antibody or antigen-binding fragment specifically binding to TRAIL comprises: (a) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or (b) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs. In some embodiments, the antibody or antigen-binding fragment specifically binding to FasL comprises: (a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or (b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, there is provided a pharmaceutical composition comprising: (i) an antibody or antigen-binding fragment specifically binding to TRAIL, and (ii) an antibody or antigen-binding fragment specifically binding to FasL, wherein the antibody or antigen-binding fragment specifically binding to FasL comprises: (a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or (b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, the antibody or antigen-binding fragment specifically binding to TRAIL in the pharmaceutical compositions comprises: (a) a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (b) a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, the antibody or antigen-binding fragment specifically binding to FasL in the pharmaceutical compositions comprises: (a) a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73; or (b) a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76.

In some embodiments, the antibody or antigen-binding fragment specifically binding to TRAIL in the pharmaceutical compositions comprises: a V_{H} comprising the amino acid sequence of any one of SEQ ID NOs: 13-21, or a variant thereof having at least about 80% (such as at least about any of 80%, 85%, 90%, 95%, 96%, 97%, 98%, or 99%) sequence identity to the amino acid sequence of any one of SEQ ID NOs: 13-21; and b) a V_{L} comprising the amino acid sequence of any one of SEQ ID NOs: 22-32, or a variant thereof having at least about 80% (such as at least about any of 80%, 85%, 90%, 95%, 96%, 97%, 98%, or 99%) sequence identity to the amino acid sequence of any one of SEQ ID NOs: 22-32.

In some embodiments, the antibody or antigen-binding fragment specifically binding to TRAIL in the pharmaceutical compositions comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27. (vii) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; (viii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29; (ix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29; (x) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29; (xi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29; (xii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29; (xiii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30; (xiv) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30; (xv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30; (xvi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30; (xvii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; (xviii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; (xix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31; (xx) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31; (xxi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31; (xxii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or (xxiii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

In some embodiments, the antibody or antigen-binding fragment specifically binding to FasL in the pharmaceutical compositions comprises: a V_{H} comprising the amino acid sequence of any one of SEQ ID NOs: 63-72, or a variant thereof having at least about 80% (such as at least about any of 80%, 85%, 90%, 95%, 96%, 97%, 98%, or 99%) sequence identity to the amino acid sequence of any one of SEQ ID NOs: 63-72; and b) a V_{L} comprising the amino acid sequence of any one of SEQ ID NOs: 73-79, or a variant thereof having at least about 80% (such as at least about any of 80%, 85%, 90%, 95%, 96%, 97%, 98%, or 99%) sequence identity to the amino acid sequence of any one of SEQ ID NOs: 73-79.

In some embodiments, the antibody or antigen-binding fragment specifically binding to FasL in the pharmaceutical compositions comprises: (i) a V_{H} comprising the amino acid sequence of SEQ ID NO: 63, or a variant thereof having at least about 80% sequence identity to the amino acid sequence of SEQ ID NO:63; and a V_{L} comprising the amino acid sequence of SEQ ID NO: 73, or a variant thereof having at least about 80% sequence identity to the amino acid sequence of SEQ ID NO: 73; (ii) a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74; (iii) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 74, or a variant thereof having at least about 80% sequence identity to the amino acid sequence of SEQ ID NO: 74; (iv) a V_{H} comprising the amino acid sequence of SEQ ID NO: 64, or a variant thereof having at least about 80% sequence identity to the amino acid sequence of SEQ ID NO: 64; and a V_{L} comprising the amino acid sequence of SEQ ID NO: 75, or a variant thereof having at least about 80% sequence identity to the amino acid sequence of SEQ ID NO: 75; (v) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 75, or a variant thereof having at least about 80% sequence identity to the amino acid sequence of SEQ ID NO: 75; (vi) a V_{H} comprising the amino acid sequence of SEQ ID NO:66, or a variant thereof having at least about 80% sequence identity to the amino acid sequence of SEQ ID NO: 66; and a V_{L} comprising the amino acid sequence of SEQ ID NO: 76, or a variant thereof having at least about 80% sequence identity to the amino acid sequence of SEQ ID NO: 76. (vii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 77, or a variant thereof having at least about 80% sequence identity to the amino acid sequence of SEQ ID NO: 77; (viii) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 77, or a variant thereof having at least about 80% sequence identity to the amino acid sequence of SEQ ID NO: 77; (ix) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 77, or a variant thereof having at least about 80% sequence identity to the amino acid sequence of SEQ ID NO: 77; (x) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 77, or a variant thereof having at least about 80% sequence identity to the amino acid sequence of SEQ ID NO: 77; (xi) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 77, or a variant thereof having at least about 80% sequence identity to the amino acid sequence of SEQ ID NO: 77; (xii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 77, or a variant thereof having at least about 80% sequence identity to the amino acid sequence of SEQ ID NO: 77; (xiii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 78, or a variant thereof having at least about 80% sequence identity to the amino acid sequence of SEQ ID NO: 78; (xiv) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 78, or a variant thereof having at least about 80% sequence identity to the amino acid sequence of SEQ ID NO: 78; (xv) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 78, or a variant thereof having at least about 80% sequence identity to the amino acid sequence of SEQ ID NO: 78; (xvi) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78; (xvii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 78, or a variant thereof having at least about 80% sequence identity to the amino acid sequence of SEQ ID NO: 78; (xviii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 78, or a variant thereof having at least about 80% sequence identity to the amino acid sequence of SEQ ID NO: 78; (xix) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 79, or a variant thereof having at least about 80% sequence identity to the amino acid sequence of SEQ ID NO: 79; (xx) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 79, or a variant thereof having at least about 80% sequence identity to the amino acid sequence of SEQ ID NO: 79; (xxi) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 79, or a variant thereof having at least about 80% sequence identity to the amino acid sequence of SEQ ID NO: 79; (xxii) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 79, or a variant thereof having at least about 80% sequence identity to the amino acid sequence of SEQ ID NO: 79; (xxiii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 79, or a variant thereof having at least about 80% sequence identity to the amino acid sequence of SEQ ID NO: 79; or (xxiv) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 79, or a variant thereof having at least about 80% sequence identity to the amino acid sequence of SEQ ID NO: 79.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78.

In some embodiments, there is provided a pharmaceutical composition comprising: an antibody or antigen-binding fragment specifically binding to TRAIL, and an antibody or antigen-binding fragment specifically binding to FasL, wherein the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 69, or a variant thereof having at least about 80% sequence identity to the amino acid sequence of SEQ ID NO:69; and a V_{L} comprising the amino acid sequence of SEQ ID NO: 79, or a variant thereof having at least about 80% sequence identity to the amino acid sequence of SEQ ID NO: 79.

In some embodiments, in the methods or uses described herein, the antibody or antigen-binding fragment specifically binding to TRAIL and the antibody or antigen-binding fragment specifically binding to FasL are administered concurrently. In some embodiments, the antibody or antigen-binding fragment specifically binding to TRAIL and the antibody or antigen-binding fragment specifically binding to FasL are administered consecutively.

In some embodiments, according to the pharmaceutical compositions described in this application, the ratio by molar mass of the antibody or antigen-binding fragment specifically binding to TRAIL and the antibody or antigen-binding fragment specifically binding to FasL is about 20:1, 10:1, 9:1, 8:1, 7:1, 6:1, 5:1, 4:1, 3:1, 2: 1, 1:1, 1:2, 1:3, 1:4, 1:5, 1:6, 1:7, 1:8, 1:9, 1:10 or 1:20. In other embodiments, the ratio by molar mass of the antibody or antigen-binding fragment specifically binding to TRAIL and the antibody or antigen-binding fragment specifically binding to FasL is about 5:1, 4:1, 3:1, 2:1, 1:1, 1:2, 1:3, 1:4 or 1:5, n other embodiments, the ratio by molar mass of the antibody or antigen-binding fragment specifically binding to TRAIL and the antibody or antigen-binding fragment specifically binding to FasL is about 2:1 or 1:1.

### Binding affinity

Binding affinity can be indicated by Kd, Koff, Kon, or Ka. The term "Koff", as used herein, is intended to refer to the off-rate constant for dissociation of an antigen-binding domain from the antigen-binding domain/antigen complex, as determined from a kinetic selection set up. The term "Kon", as used herein, is intended to refer to the on-rate constant for association of an antibody to the antigen to form the antigen-binding domain/antigen complex. The term dissociation constant "Kd", as used herein, refers to the dissociation constant of a particular antibody-antigen interaction, and describes the concentration of antigen required to occupy one half of all of the antibody-binding domains present in a solution of antibody molecules at equilibrium, and is equal to Koff/Kon. The measurement of Kd presupposes that all **binding** agents are in solution. In the case where the antibody is tethered to a cell wall, *e.g.,* in a yeast expression system, the corresponding equilibrium rate constant is expressed as EC50, which gives a good approximation of Kd. The affinity constant, Ka, is the inverse of the dissociation constant, Kd.

The dissociation constant (Kd) is used as an indicator showing affinity of antigen-binding domain moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M. An antibody specifically binding to a target may have a Kd of, for example, ≤ 10⁻⁷ M, ≤ 10⁻¹ M, ≤ 10⁻⁹ M, ≤ 10⁻¹⁰ M, ≤ 10⁻¹¹ M, ≤ 10⁻¹² M, or ≤ 10⁻¹³ M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans. In some embodiments, the antibody or antigen-binding fragment specifically binding to TRAIL specifically binds to a target TRAIL with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the antibody or antigen-binding fragment specifically binding to TRAIL and TRAIL, is about 10⁻⁷ M to about 10⁻¹³ M, about 1×10⁻⁷ M to about 5× 10⁻¹³ M, about 10⁻⁷ M to about 10⁻¹² M, about 10⁻⁷M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5× 10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1 × 10⁻¹² M, about 5×10⁻¹⁰ M to about 1×10⁻¹¹ M, about 10⁻¹⁰M to about10⁻¹³ M, about 1×10⁻¹⁰ M to about 5× 10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the antibody or antigen-binding fragment specifically binding to TRAIL and TRAIL is about 10⁻⁷ M to about 10⁻¹³ M.

In some embodiments, the Kd of the binding between the antibody or antigen-binding fragment specifically binding to TRAIL and a non-target is higher than the Kd of the binding between the antibody or antigen-binding fragment specifically binding to TRAIL and the target, and is herein referred to in some embodiments as the binding affinity of the antibody or antigen-binding fragment specifically binding to TRAIL to the target (e.g., TRAIL) is higher than that to a non-target. In some embodiments, the non-target is an antigen that is not TRAIL. In some embodiments, the Kd of the binding between the antibody or antigen-binding fragment specifically binding to TRAIL and a non- TRAIL target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody or antigen-binding fragment specifically binding to TRAIL and a target TRAIL.

In some embodiments, the antibody or antigen-binding fragment specifically binding to FasL specifically binds to a target FasL with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the antibody or antigen-binding fragment specifically binding to FasL and FasL, is about 10⁻⁷M to about 10⁻¹³ M, about 1×10⁻⁷M to about 5× 10⁻¹³ M, about 10⁻⁷M to about 10⁻¹²M, about 10⁻⁷M to about 10⁻¹¹ M, about 10⁻⁷M to about 10⁻¹⁰ M, about 10⁻⁷M to about 10⁻⁹ M, about 10⁻⁸ M to about 10⁻¹³ M, about 1×10⁻⁸ M to about 5 × 10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1 × 10⁻¹³ M, about 5×10⁻⁹ M to about 1 × 10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1 × 10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5 × 10⁻¹³ M, about 10⁻¹¹ M to about 10⁻¹² M, or about 10⁻¹² M to about 10⁻¹³ M. In some embodiments, the Kd of the binding between the antibody or antigen-binding fragment specifically binding to FasL and FasL is about 10⁻⁷ M to about 10⁻¹³ M.

In some embodiments, the Kd of the binding between the antibody or antigen-binding fragment specifically binding to FasL and a non-target is higher than the Kd of the binding between the antibody or antigen-binding fragment specifically binding to FasL and the target, and is herein referred to in some embodiments as the binding affinity of the antibody or antigen-binding fragment specifically binding to FasL to the target (*e.g.,* FasL) is higher than that to a non-target. In some embodiments, the non-target is an antigen that is not FasL. In some embodiments, the Kd of the binding between the antibody or antigen-binding fragment specifically binding to FasL and a non-FasL target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody or antigen-binding fragment specifically binding to FasL and a target FasL.

In some embodiments, any of the bispecific antibodies described in this applications comprise a first antigen-binding domain specifically binding to TRAIL, and a second antigen-binding domain specifically binding to FasL. Wherein the first antigen-binding domain specifically binding to a target TRAIL with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the first antigen-binding domain and TRAIL, is about 10⁻⁷M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1 × 10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1 × 10⁻¹³ M, about 5×10⁻¹⁰ M to about 1 × 10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1 × 10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the first antigen-binding domain and TRAIL is about 10⁻⁷ M to about 10⁻¹³ M. Wherein the second antigen-binding domain specifically binding to a target FasL with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the second antigen-binding domain and FasL, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1 × 10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1 × 10⁻¹³ M, about 5×10⁻¹⁰ M to about 1 × 10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1 × 10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the second antigen-binding domain and FasL is about 10⁻⁷ M to about 10⁻¹³ M.

In some embodiments, any of the bispecific antibodies described in this applications comprise a first antigen-binding domain specifically binding to TRAIL, and a second antigen-binding domain specifically binding to FasL. In some embodiments, the Kd of the binding between the first antigen-binding domain and a non-target is higher than the Kd of the binding between the first antigen-binding domain and the target, and is herein referred to in some embodiments as the binding affinity of the first antigen-binding domain to the target (*e.g.,* TRAIL) is higher than that to a non-target. In some embodiments, the non-target is an antigen that is not TRAIL. In some embodiments, the Kd of the binding between the first antigen-binding domain and a non-TRAIL target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the first antigen-binding domain and a target TRAIL. In some embodiments, the Kd of the binding between the second antigen-binding domain and a non-target is higher than the Kd of the binding between the second antigen-binding domain and the target, and is herein referred to in some embodiments as the binding affinity of the second antigen-binding domain to the target (*e.g.,* FasL) is higher than that to a non-target. In some embodiments, the non-target is an antigen that is not FasL. In some embodiments, the Kd of the binding between the second antigen-binding domain and a non-FasL target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the second antigen-binding domain and a target FasL.

### Nucleic Acids

Nucleic acid molecules encoding the antibody or antigen-binding fragment specifically binding to TRAIL, the antibody or antigen-binding fragment specifically binding to FasL and the bispecific antibody are also contemplated.

In some embodiments, there is provided a nucleic acid (or a set of nucleic acids) encoding an antibody or antigen-binding fragment specifically binding to TRAIL or an antibody or antigen-binding fragment specifically binding to FasL or the bispecific antibody specifically binding to TRAIL and FasL, including any of the antibody or antigen-binding fragment specifically binding to TRAIL, or the antibody or antigen-binding fragment specifically binding to FasL, or the bispecific antibody specifically binding to TRAIL and FasL described herein. In some embodiments, the nucleic acid (or a set of nucleic acids) encoding the antibody or antigen-binding fragment or bispecific antibody described herein may further comprises a nucleic acid sequence encoding a peptide tag (such as protein purification tag, *e.g.,* His-tag, HA tag).

Also provided here are isolated host cells comprising the antibody or antigen-binding fragment specifically binding to TRAIL, or the antibody or antigen-binding fragment specifically binding to FasL, or the bispecific antibody specifically binding to TRAIL and FasL; or a nucleic acid molecular coding the antibodies or antigen-binding fragments described herein; or a vector comprising a nucleic acid molecular described herein. The present application also includes variants to these nucleic acid sequences. For example, the variants include nucleotide sequences that hybridize to the nucleic acid sequences encoding the antibodies or antigen-binding fragments or bispecific antibodies of the present application under at least moderately stringent hybridization conditions.

The present application also provides vectors in which a nucleic acid of the present application is inserted.

In brief summary, the expression of an antibody or antigen-binding fragment or bispecific antibody by a natural or synthetic nucleic acid encoding the antibody or antigen-binding fragment or bispecific antibody can be achieved by inserting the nucleic acid into an appropriate expression vector, such that the nucleic acid is operably linked to 5' and 3' regulatory elements, including for example a promoter (*e.g.,* a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequence.

The nucleic acids of the present application may also be used for nucleic acid immunization and gene therapy, using standard gene delivery protocols. Methods for gene delivery are known in the art. See, *e.g.,* U.S. Pat. Nos. 5,399,346; 5,580,859; 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers (*see, e.g.,* WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the antibody or antigen-binding fragment or bispecific antibody is inducible. In some embodiments, a nucleic acid sequence encoding the antibody or antigen-binding fragment or bispecific antibody is operably linked to an inducible promoter, including any inducible promoter described herein.

### Inducible promoters

The use of an inducible promoter provides a molecular switch capable of turning on expression of the polynucleotide sequence which it is operatively linked when such expression is desired, or turning off the expression when expression is not desired. Exemplary inducible promoter systems for use in eukaryotic cells include, but are not limited to, hormone-regulated elements (e.g., *see* Mader, S. and White, J. H. (1993) Proc. Natl. Acad. Sci. USA 90:5603-5607), synthetic ligand-regulated elements (see, *e.g.,* Spencer, D. M. et al 1993) Science 262: 1019-1024) and ionizing radiation-regulated elements (*e.g., see* Manome, Y. et al. (1993) Biochemistry 32: 10607-10613; Datta, R. et al. (1992) Proc. Natl. Acad. Sci. USA 89: 1014-10153). Further exemplary inducible promoter systems for use in in vitro or in vivo mammalian systems are reviewed in Gingrich et al. (1998) Annual Rev. Neurosci 21:377-405. In some embodiments, the inducible promoter system for use to express the antibody or antigen-binding fragment or bispecific antibody is the Tet system. In some embodiments, the inducible promoter system for use to express the antibody or antigen-binding fragment or bispecific antibody is the lac repressor system from *E. coli.*

An exemplary inducible promoter system for use in the present application is the Tet system. Such systems are based on the Tet system described by Gossen et al. (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, *e.g.,* in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahydrotetracene-3-carboxamide.

In one embodiment, a TetR is codon-optimized for expression in mammalian cells, *e.g.,* murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (i.e., bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (e.g., a prokaryote) may be tailored for improved expression in a different organism (e.g., a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, *e.g.,* isopropyl-β-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, *e.g.,* enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene (*e.g.,* Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding an antibody or antigen-binding fragment or bispecific antibody according to any of the antigen-binding fragment, antigen-binding protein or bispecific molecule described herein. In some embodiments, the nucleic acid comprises one or more nucleic acid sequences encoding the heavy and light chains of the antibody or antigen-binding fragment or bispecific antibody. In some embodiments, each of the one or more nucleic acid sequences is contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, *e.g.,* human cells. Other viral vectors can be derived from lentivirus, poxviruses, herpes simplex virus 1, adenoviruses and adeno-associated viruses, and the like. *See,* for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (*e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the application.

### Preparation of antibodies or antigen-binding fragments and bispecific antibodies

In some embodiments, the antibody or antigen-binding fragment (e.g. an antibody or antigen-binding fragment specifically binding to TRAIL or FasL) is a monoclonal antibody. In some embodiments, the antibody or antigen-binding fragment or bispecific antibody is derived from a monoclonal antibody. In some embodiments, the antibody or antigen-binding fragment or bispecific antibody comprises V_{H} and V_{L} domains, or variants thereof, from the monoclonal antibody. In some embodiments, the antibody or antigen-binding fragment or bispecific antibody further comprises C_{H}1 and C_{L} domains, or variants thereof, from the monoclonal antibody. Monoclonal antibodies can be prepared, e.g., using known methods in the art, including hybridoma methods, yeast display, phage display methods, or using recombinant DNA methods. Additionally, exemplary yeast display and phage display methods are described herein and in the Examples below. The bispecific antibody can be prepared by methods known in the art, including chemical coupling method, hybridoma method, and genetic engineering method, etc..

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized in vitro. The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an in vitro binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980*).*

After the desired hybridoma cells are identified, the clones can be sub cloned by limiting dilution procedures and grown by standard methods. Goding, supra. Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the sub clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the antibodies or antigen-binding fragments or bispecific antibodies described herein, the antibody or antigen-binding fragment or bispecific antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, e.g., in Hoogenboom et al., Methods in Molecular Biology 178:1-37 (O'Brien et al., ed., Human Press, Totowa, N.J., 2001) and further described, *e.g.,* in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology 248:161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phages typically display antibody fragments, either as scFv fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The antibodies or antigen-binding fragments or bispecific antibodies can be prepared using phage display to screen libraries for antigen-binding moieties specific to the target antigen (such as TRAIL or FasL). The library can be a human scFv phage display library having a diversity of at least 1 × 10⁹ (such as at least about any of 1 × 10⁹, 2.5 × 10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naive human library constructed from DNA extracted from human PNMCs and spleens from healthy donors, encompassing all human heavy and light chain subfamilies. In some embodiments, the library is a naive human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see, e.g.,* Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target antigen (such as TRAIL or FasL) with high affinity can be selected by iterative binding of phage to the target antigen, which is bound to a solid support (such as, for example, beads for solution panning or mammalian cells for cell panning), followed by removal of non-bound phage and by elution of specifically bound phage. The bound phage clones are then eluted and used to infect an appropriate host cell, such as E. coli XL1-Blue, for expression and purification. The panning can be performed for multiple (such as about any of 2, 3, 4, 5, 6 or more) rounds with solution panning, cell panning, or a combination of both, to enrich for phage clones binding specifically to the target antigen. Enriched phage clones can be tested for specific binding to the target antigen by any methods known in the art, including for example ELISA and FACS.

Monoclonal antibodies and bispecific molecules can also be made by recombinant DNA methods, such as those described in U.S. Patent No. 4,816,567. DNA encoding the monoclonal antibodies of the application can be readily isolated and sequenced using conventional procedures (*e.g*., by using oligonucleotide probes that are capable of binding specifically to genes encoding the heavy and light chains of murine antibodies). Hybridoma cells as described above or antigen-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant regions and/or framework regions in place of the homologous non-human sequences (U.S. Patent No. 4,816,567; Morrison *et al., supra*) or by covalently joining to the immunoglobulin coding sequence all or part of the coding sequence for a non-immunoglobulin polypeptide. Such a non-immunoglobulin polypeptide can be substituted for the constant regions of an antibody of the application, or can be substituted for the variable regions of one antigen-combining site of an antibody of the application to create a chimeric bivalent antibody. In some embodiments, additional variable regions targeting a different epitope or antigen can be included to generate a chimeric bispecific antibody.

The antigen-binding proteins can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly Fab fragments, can be accomplished using any method known in the art.

Chemical coupling method is the technology that was first applied to prepare bispecific antibodies. In 1985, Brennan used the chemical binding of two monoclonal antibody G1 fragments for the first time to prepare bispecific antibodies (Brennan M, et al. Preparation of bispecific antibodies by chemical recombination of monoclonal immunoglobulin G1 fragments [J]. Science, 1985, 229(4708):81-83). The chemical coupling method mainly comprises two modes, one is to directly couple two monoclonal antibodies or their derivatives to form bispecific antibodies; the other is to dissociate two monoclonal antibodies into free light chains and heavy chains through various physical and chemical methods firstly, and then recombine these light chains and heavy chains. The advantages of chemical coupling method are rapidness, simple operation and high recovery rate, but it is also easy to disrupt the antigen-binding domain of antibodies, affect antibody activity, and easy to form polymers.

Preparation of bispecific antibody by hybridoma cell lines refers to the fusion of two different hybridoma cell lines by cell fusion techniques, followed by identification and isolation of cells that can produce antibodies with specific therapeutics (Kohler, G, et al. Continuous cultures of fused cells secreting antibody of predefined specificity [J]. J Immunol., 2005, 174(5):2453-2455). Because two kinds of hybridoma cells can produce two different light-heavy chains, and the light-heavy chains can be randomly combined, the bispecific antibody prepared by the method has larger randomness and low preparation efficiency.

Genetic engineering technologies are also currently used to prepare a variety of bispecific antibodies (Roland E K. Antibody-cytokine fusion proteins [J]. Arch Biochem Biophys., 2012, 526(2):194-205). Editing recombinant antibodies by genetic engineering can solve the problem of random combination by limiting the binding selectivity of two pairs of light-heavy chain in variety ways. KiH (Knob into hole) and CrossMab are two common technologies currently applied to improve the problem of light-heavy chain pairing. KiH technology introduces asymmetric mutation structures into C_{H}3 domain ("knob" mutation refers to substituting a smaller residue with a larger amino acid residue in C_{H}3 domain, whereas "hole" mutation refers to substituting a larger residue with a smaller amino acid residue). The Fc region of engineered bispecific antibodies is more prone to heterodimerization than homodimerization due to steric effects (Ridgway J B, et al. "Knobs-into-holes" engineering of antibody CH3 domains for heavy chain heterodimerization [J]. Protein Eng. 1996, 9(7):617-621). While introducing a Y349C mutation in the glycosylated C_{H}3 domain can lead to the formation of disulfide bonds between glycosylated heavy chains, enhancing the stability of KiH (Kuglstatter A, et al. Structural differences between glycosylated, disulfide-linked heterodimeric knob-into-hole Fc fragment and its homodimeric knob-knob and hole-hole side products [J]. Protein Eng Des Sel. ,2017, 30(9):649-656). In addition to steric effects, the charge effect of amino acid residues is also used to enhance heterodimerization between two heavy chains of a bispecific antibody. "Positive electricity" is generated in one chain and negative electricity is generated in the paired chain through structural simulation and molecular design modification, and the formation of heavy chain heterodimer is promoted by the mode that like charges repel each other and unlike charges attractive each other. The formation of heterodimer can be increased to some extent by the mode of respective mutation K409D and D399K, K409D/K392D and D399K/E356K, or E356K/E357K/D399K and K370E/K409D/K439E in two chains (IGAWA T, et al. Methods for producing polypeptides by regulating polypeptide; association: US, 20100015133A1 [P].2006). Combining the KiH steric effect with the charge effect is also one of the strategies to further enhance heterodimerization.

The CrossMab technology is a new antibody pairing technology developed by Roche based on KiH technology by exchanging the domain of light chain with the heavy chain of one Fab of bispecific antibodies, with no exchange on the other one. The exchanged light chain will contain a portion of the homologous heavy chain fragment, making them unable to pair with the non-exchanged heavy chain, thereby ensuring the correct combination between the light chains and heavy chains (Schaefer W, et al. Immunoglobulin domain crossover as a generic approach for the production of bispecific IgG antibodies [J]. Proc Natl Acad Sci USA, 2011, 108 (27):11187-11192). Includes the form of "CrossMab Fab", "CrossMab V_{H}-V_{L}" or "CrossMab C_{H}1-C_{L}" etc..

Antibody variable regions with the desired binding specificities (antibody-antigen combining sites) can be fused to immunoglobulin constant-domain sequences. The fusion preferably is with an immunoglobulin heavy-chain constant region, comprising at least part of the hinge, C_{H}2, and C_{H}3 domains. In some embodiments, the first heavy-chain constant domain (C_{H}1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism. In some embodiments, antibody variable regions targeting different epitopes or different antigens can be fused to immunoglobulin constant-domain sequences to generate a chimeric bispecific antibody.

### Human and Humanized Antibodies

The antibodies or antigen-binding fragments or bispecific antibodies can comprise humanized antibody moieties or human antibody moieties. Humanized forms of non-human (e.g., murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable regions, in which all or substantially all of the CDR regions correspond to those of a non-human immunoglobulin, and all or substantially all of the FR regions are those of a human immunoglobulin consensus sequence.

Generally, a humanized antibody or humanized antibody moiety has one or more amino acid residues introduced into it from a source that is non-human. These non-human amino acid residues are often referred to as "import" residues, which are typically taken from an "import" variable region. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable region has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals *(e.g.,* mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, e.g., mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies or human antibody moieties may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p. 77 (1985) and Boerner et al., J. Immunol., 147(1): 86-95 (1991).

### Variants of antibodies or antigen-binding fragments

In some embodiments, amino acid sequences of the antibodies or antigen-binding fragments (e.g., antibody specifically binding to TRAIL or FasL, or bispecific antibody specifically binding to TRAIL and FasL) variants provided herein are contemplated. For example, it may be desirable to improve the binding affinity and/or other biological properties of the antibody or antigen-binding fragment. Amino acid sequences of an antigen-binding entity variants may be prepared by introducing appropriate modifications into the nucleotide sequence encoding the antigen-binding entity, or by peptide synthesis. Such modifications include, for example, deletions from, and/or insertions into and/or substitutions of residues within the amino acid sequences of the antigen-binding entity. Any combination of deletion, insertion, and substitution can be made to arrive at the final construct, provided that the final construct possesses the desired characteristics, *e.g.,* antigen-binding.

In some embodiments, variants of the antibodies or antigen-binding fragments having one or more amino acid substitutions are provided. Sites of interest for substitutional mutagenesis include the HVRs and FRs. Amino acid substitutions may be introduced into an antibody of interest and the products screened for a desired activity, *e.g.,* improved bioactivity, retained/improved antigen binding, decreased immunogenicity. In some embodiments, the amino acid substitutions described herein are limited to "exemplary substitutions" shown in Table 10 of this application. In some embodiments, the amino acid substitutions are limited to "preferred substitutions" shown in Table 10 of this application.

Conservative substitutions are shown in Table 10 below.

**Table 10: CONSERVATIVE SUBSTITUTIONS**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, e.g., using phage display-based affinity maturation techniques. Briefly, one or more CDR residues are mutated and the variant antibody moieties displayed on phage and screened for a particular biological activity (e.g., bioactivity based on RBC lysis inhibition assay or binding affinity). Alterations *(e.g.,* substitutions) may be made in HVRs, *e.g.,* to improve bioactivity based on RBC lysis inhibition assay or antibody affinity. Such alterations may be made in HVR "hotspots," i.e., residues encoded by codons that undergo mutation at high frequency during the somatic maturation process *(see, e.g.,* Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).)

In some embodiments of affinity maturation, diversity is introduced into the variable genes chosen for maturation by any of a variety of methods *(e.g.,* error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues *(e.g.,* 4-6 residues at a time) are randomized. HVR residues involved in antigen binding may be specifically identified, *e.g.,* using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (*e.g.,* conservative substitutions as provided herein) that do not substantially reduce binding affinity may be made in HVRs. Such alterations may be outside of HVR "hotspots" or SDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g.,* charged residues such as Arg, Asp, His, Lys, and Glu) are identified and replaced by a neutral or negatively charged amino acid (*e.g.,* Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antigen-binding moiety with an N-terminal methionyl residue. Other insertional variants of the antigen-binding moiety include the fusion to the N- or C-terminus of the antigen-binding moiety to an enzyme (*e.g.* for ADEPT) or a polypeptide which increases the serum half-life of the antigen-binding moiety.

### Fc Variants

In some embodiments, one or more amino acid modifications may be introduced into the Fc region of an antibody or antigen-binding fragment (e.g., a full-length antibody specifically binding to TRAIL, full-length antibody specifically binding to FasL, or full-length bispecific antibody specifically binding to TRAIL and FasL, or fusion protein comprising the antibody or antigen-binding fragment or bispecific antibody) provided herein, thereby generating an Fc variant. In some embodiments, the Fc variant has enhanced ADCC effector function, often related to binding to Fc receptors (FcRs). In some embodiments, the Fc variant has decreased ADCC effector function. There are many examples of changes or mutations to Fc sequences that can alter effector function. For example, WO 00/42072 and Shields et al. J Biol. Chem. 9(2): 6591-6604 (2001) describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell (*e.g.,* an infected cell), whose membrane-surface antigens have been bound by specific antigen-binding moieties (*e.g.,* an antibody specifically binding to TRAIL, antibody specifically binding to FasL or bispecific antibody specifically binding to TRAIL and FasL). The typical ADCC involves activation of NK cells by antibodies. An NK cell expresses CD16 which is an Fc receptor. This receptor recognizes, and binds to, the Fc portion of an antibody bound to the surface of a target cell. The most common Fc receptor on the surface of an NK cell is called CD16 or FcγRIII. Binding of the Fc receptor to the Fc region of an antibody results in NK cell activation, release of cytolytic granules and consequent target cell apoptosis.

In some embodiments, the application contemplates the variant of antibody or antigen-binding fragment specifically binding to TRAIL, antibody or antigen-binding fragment specifically binding to FasL or bispecific antibody specifically binding to TRAIL and FasL (such as a variant of full-length antibody specifically binding to TRAIL, full-length antibody specifically binding to FasL or bispecific antibody specifically binding to TRAIL and FasL) comprising an Fc region that possesses some but not all effector functions, which makes it a desirable candidate for applications in which the half-life of the antibody or antigen-binding fragment specifically binding to TRAIL, antibody or antigen-binding fragment specifically binding to FasL or bispecific antibody specifically binding to TRAIL and FasL *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Pat. No. 5,500,362 (see, *e.g.* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CytoTox 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.,* Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.,* U.S. Pat. No. 6,737,056; WO 2004/056312, and Shields et al., J. Biol. Chem. 9(2): 6591-6604 (2001).)

In some embodiments, alterations are made in the Fc region that result in altered *(i.e.,* either improved or diminished) opsonization, e.g. such as described in Moore et al., MAbs. 2(2): 181-189 (2010).

In some embodiments, there is provided an antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or full-length bispecific antibody specifically binding to TRAIL and FasL) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.)*.* Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (U.S. Pat. No. 7,371,826).

*See* also Duncan & Winter, Nature 322:738-40 (1988); U.S. Pat. No. 5,648,260; U.S. Pat. No. 5,624,821; and WO 94/29351 concerning other examples of Fc variants.

Antibodies or antigen-binding fragments specifically binding to TRAIL, antibodies or antigen-binding fragments specifically binding to FasL or bispecific antibodies specifically binding to TRAIL and FasL (such as full-length antibodies specifically binding to TRAIL, full-length antibodies specifically binding to FasL or bispecific antibodies specifically binding to TRAIL and FasL) comprising any of the Fc variants described herein, or combinations thereof, are contemplated.

### Glycosylation Variants

In some embodiments, an antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) provided herein is altered to increase or decrease the glycosylation extent of the antibody or antigen-binding fragment specifically binding to TRAIL, antibody or antigen-binding fragment specifically binding to FasL or bispecific antibody specifically binding to TRAIL and FasL. Addition or deletion of glycosylation sites to an antibody or antigen-binding fragment specifically binding to TRAIL, antibody or antigen-binding fragment specifically binding to FasL or bispecific antibody specifically binding to TRAIL and FasL may be conveniently accomplished by altering the amino acid sequence of the antibody or antigen-binding fragment specifically binding to TRAIL, antibody or antigen-binding fragment specifically binding to FasL or bispecific antibody specifically binding to TRAIL and FasL or polypeptide portion thereof such that one or more glycosylation sites is created or removed.

Where the antibody or antigen-binding fragment specifically binding to TRAIL, antibody or antigen-binding fragment specifically binding to FasL or bispecific antibody specifically binding to TRAIL and FasL comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al., TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody or antigen-binding fragment specifically binding to TRAIL, antibody or antigen-binding fragment specifically binding to FasL or bispecific antibody specifically binding to TRAIL and FasL of the application may be made in order to create antibody or antigen-binding fragment specifically binding to TRAIL, antibody or antigen-binding fragment specifically binding to FasL or bispecific antibody specifically binding to TRAIL and FasL variants with certain improved properties.

The N-glycans attached to the CH2 domain of Fc is heterogeneous. Antibodies or Fc fusion proteins generated in CHO cells are fucosylated by fucosyltransferase activity. *See* Shoji-Hosaka et al., J. Biochem. 2006, 140:777- 83. Normally, a small percentage of naturally occurring afucosylated IgGs may be detected in human serum. N-glycosylation of the Fc is important for binding to Fc R; and afucosylation of the N-glycan increases Fc's binding capacity to Fc RIIIa. Increased Fc RIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) variants are provided comprising an Fc region wherein a carbohydrate structure attached to the Fc region has reduced fucose or lacks fucose, which may improve ADCC function. Specifically, the antibody or antigen-binding fragment specifically binding to TRAIL, antibody or antigen-binding fragment specifically binding to FasL or bispecific antibody specifically binding to TRAIL and FasL variants are contemplated herein that have reduced fucose relative to the amount of fucose on the same antibody or antigen-binding fragment specifically binding to TRAIL, antibody or antigen-binding fragment specifically binding to FasL or bispecific antibody specifically binding to TRAIL and FasL produced in a wild-type CHO cell. That is, they are characterized by having a lower amount of fucose than they would otherwise have if produced by native CHO cells (*e.g*., a CHO cell that produce a native glycosylation pattern, such as, a CHO cell containing a native FUT8 gene). In some embodiments, the antibody or antigen-binding fragment specifically binding to TRAIL, antibody or antigen-binding fragment specifically binding to FasL or bispecific antibody specifically binding to TRAIL and FasL is one wherein less than about 50%, 40%, 30%, 20%, 10%, or 5% of the N-linked glycans thereon comprise fucose. For example, the amount of fucose in such an antibody or antigen-binding fragment specifically binding to TRAIL, antibody or antigen-binding fragment specifically binding to FasL or bispecific antibody specifically binding to TRAIL and FasL may be from 1% to 80%, from 1% to 65%, from 5% to 65% or from 20% to 40%. In some embodiments, the antibody or antigen-binding fragment specifically binding to TRAIL, antibody or antigen-binding fragment specifically binding to FasL or bispecific antibody specifically binding to TRAIL and FasL is one wherein none of the N-linked glycans thereon comprise fucose, *i.e*., wherein the antibody or antigen-binding fragment specifically binding to TRAIL, antibody or antigen-binding fragment specifically binding to FasL or bispecific antibody specifically binding toTRAIL and FasL is completely without fucose, or has no fucose or is afucosylated. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering system of Fc region residues); however, Asn297 may also be located about ±3 amino acids upstream or downstream of position 297, *i.e*., between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.*, especially at Example 11), and knockout cell lines, such asα-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding toTRAIL and FasL) variants are further provided with bisected oligosaccharides, *e*.*g*., in which a biantennary oligosaccharide attached to the Fc region of the antibody or antigen-binding fragment specifically binding to TRAIL, antibody or antigen-binding fragment specifically binding to FasL or bispecific antibody specifically binding to TRAIL and FasL is bisected by GlcNAc. Such antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) variants may have reduced fucosylation and/or improved ADCC function. Examples of such antibody variants are described, *e.g.,* in WO 2003/011878 (Jean-Mairet et al.); U.S. Pat. No. 6,602,684 (Umana et al.); US 2005/0123546 (Umana et al.), and Ferrara et al., Biotechnology and Bioengineering, 93(5): 851-861 (2006). Antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) variants with at least one galactose residue in the oligosaccharide attached to the Fc region are also provided. Such antibody or antigen-binding fragment specifically binding to TRAIL, antibody or antigen-binding fragment specifically binding to FasL or bispecific antibody specifically binding to TRAIL and FasL variants may have improved CDC function. Such antibody variants are described, *e*.*g*., in WO 1997/30087 (Patel et al.); WO 1998/58964 (Raju, S.); and WO 1999/22764 (Raju, S.).

In some embodiments, the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) variants comprising an Fc region are capable of binding to an FcγRIII. In some embodiments, the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) variants comprising an Fc region have ADCC activity in the presence of human effector cells (*e.g*., T cell) or have increased ADCC activity in the presence of human effector cells compared to the otherwise same antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) comprising a human wild-type Fc region.

### Cysteine Engineered Variants

In some embodiments, it may be desirable to create cysteine engineered antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) in which one or more amino acid residues are substituted with cysteine residues. In some embodiments, the substituted residues occur at accessible sites of the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL). By substituting those residues with cysteine, reactive thiol groups are thereby positioned at accessible sites of the antibody or antigen-binding fragment specifically binding to TRAIL, antibody or antigen-binding fragment specifically binding to FasL or bispecific antibody specifically binding to TRAIL and FasL and may be used to conjugate the antibody or antigen-binding fragment specifically binding to TRAIL, antibody or antigen-binding fragment or antigen-binding fragment specifically binding to FasL or bispecific antibody specifically binding to TRAIL and FasL to other moieties, such as drug moieties or linker-drug moieties, to create an antibody or antigen-binding fragment specifically binding to TRAIL, antibody or antigen-binding fragment specifically binding to FasL or bispecific antibody specifically binding to TRAIL and FasL immunoconjugate, as described further herein. Cysteine engineered antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) may be generated as described, e.g., in U.S. Pat. No. 7,521,541.

### Derivatives

In some embodiments, a bispecific antibody specifically binding to TRAIL and FasL (such as a bispecific antibody specifically binding to TRAIL and FasL) provided herein may be further modified to contain additional non-proteinaceous moieties that are known in the art and readily available. The moieties suitable for derivatization of the bispecific antibodies specifically binding to TRAIL and FasL include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone) polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the bispecific antibodies specifically binding to TRAIL and FasL may vary, and if more than one polymer are attached, they can be the same or different molecules. In general, the number and/or type of polymers used for derivatization can be determined based on considerations including, but not limited to, the particular properties or functions of the bispecific antibody specifically binding to TRAIL and FasL to be improved, whether the bispecific antibody specifically binding to TRAIL and FasL derivative will be used in a therapy under defined conditions, etc.

### Pharmaceutical Compositions

Provided herein are pharmaceutical compositions comprising: (i) the antibody or antigen-binding fragment specifically binding to TRAIL and (ii)the antibody or antigen-binding fragment specifically binding to FasL. Also provided herein are pharmaceutical compositions (such as pharmaceutical compositions, also referred to herein as formulations) comprising any of the antibodies or antigen-binding fragments specifically binding to TRAIL, antibodies or antigen-binding fragments specifically binding to FasL or bispecific antibodies specifically binding to TRAIL and FasL (such as a full-length antibody specifically binding to TRAIL, full-length antibody specifically binding to FasL or bispecific antibody specifically binding to TRAIL and FasL), nucleic acids encoding the antibodies or antigen-binding fragments, vectors comprising the nucleic acids encoding the antibodies or antigen-binding fragments, or host cells comprising the nucleic acids or vectors described herein. In some embodiments, there is provided a pharmaceutical composition comprising any one of the antibodies or antigen-binding fragments specifically binding to TRAIL, antibodies or antigen-binding fragments specifically binding to FasL or bispecific antibodies specifically binding to TRAIL and FasL described herein and a pharmaceutically acceptable carrier.

Suitable formulations of the antibodies or antigen-binding fragments specifically binding to TRAIL, antibodies or antigen-binding fragments specifically binding to FasL or bispecific antibodies specifically binding to TRAIL and FasL are obtained by mixing an antibody or antigen-binding fragments specifically binding to TRAIL, antibody or antigen-binding fragments specifically binding to FasL or bispecific antibody specifically binding to TRAIL and FasL having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the antibodies or antigen-binding fragments specifically binding to TRAIL, antibodies or antigen-binding fragments specifically binding to FasL or bispecific antibodies specifically binding to TRAIL and FasL of this application into cells.

The formulation herein may also contain one or more active compounds in addition to the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) as necessary for the particular indication being treated, preferably those with complementary activities that do not adversely affect each other. For example, it may be desirable to further provide other agents with therapeutic activity, such as antibiotics agent in addition to the antibody or antigen-binding fragment specifically binding to TRAIL, antibody or antigen-binding fragment specifically binding to FasL or bispecific antibody specifically binding to TRAIL and FasL. Such molecules are suitably present in combination in amounts that are effective for the purpose intended. The effective amount of such other active agents depends on the amount of antibody or antigen-binding fragment specifically binding to TRAIL, antibody or antigen-binding fragment specifically binding to FasL or bispecific antibody specifically binding to TRAIL and FasL present in the formulation, the type of disease or disorder or treatment, and other factors discussed above. These are generally used in the same dosages and with administration routes as described herein or about from 1 to 99% of the heretofore employed dosages.

The antibody or antigen-binding fragment specifically binding to TRAIL, antibody specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the antibodies or antigen-binding fragments specifically binding to TRAIL, antibodies or antigen-binding fragments specifically binding to FasL or bispecific antibodies specifically binding to TRAIL and FasL (such as full-length antibodies specifically binding to TRAIL, full-length antibodies specifically binding to FasL or bispecific antibodies specifically binding to TRAIL and FasL) can be prepared. Suitable examples of sustained-release preparations include semipermeable matrices of solid hydrophobic polymers containing the antibody (or fragment thereof), which matrices are in the form of shaped articles, *e.g.,* films, or microcapsules. Examples of sustained-release matrices include polyesters, hydrogels (for example, poly(2-hydroxyethyl-methacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37 °C, resulting in a loss of biological activity and possible changes in immunogenicity. Rational strategies can be devised for stabilization of antibodies or antigen-binding fragments specifically binding to TRAIL, antibodies or antigen-binding fragments specifically binding to FasL or bispecific antibodies specifically binding to TRAIL and FasL depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the antibody or antigen-binding fragment specifically binding to TRAIL, antibody or antigen-binding fragment specifically binding to FasL or bispecific antibody specifically binding to TRAIL and FasL (such as a full-length antibody specifically binding to TRAIL, full-length antibody specifically binding to FasL or bispecific antibody specifically binding to TRAIL and FasL) is formulated in a buffer comprising a citrate, NaCl, acetate, succinate, glycine, polysorbate 80 (Tween 80), or any combination of the foregoing. In some embodiments, the antibody or antigen-binding fragment specifically binding to TRAIL, antibody or antigen-binding fragment specifically binding to FasL or bispecific antibody specifically binding to TRAIL and FasL is formulated in a buffer having a pH between about 4 and 9.

The formulations to be used for *in vivo* administration must be sterile. This is readily accomplished by, *e*.*g*., filtration through sterile filtration membranes.

### Methods of preventing or treatment diseases

In some embodiments, there is provided a method of preventing or treating a disease or condition in an individual in need thereof, comprising administering to the individual an effective amount of any one of the antibodies or antigen-binding fragments specifically binding to TRAIL described above or a composition containing it, and/or any one of the bispecific antibodies specifically binding to TRAIL and FasL described above or a composition containing it, and/or antibody or antigen-binding fragment specifically binding to TRAIL and antibody or antigen-binding fragment specifically binding to FasL or a compositions containing them.

In some embodiments, there is provided the use of any one of the antibodies or antigen-binding fragments specifically binding to TRAIL described above or a composition containing it, and/or any one of the bispecific antibodies specifically binding to TRAIL and FasL described above or a composition containing it, and/or antibody or antigen-binding fragment specifically binding to TRAIL and antibody or antigen-binding fragment specifically binding to FasL or a compositions containing them in the manufacture of a medicament for preventing or treating a disease or condition in an individual in need thereof.

In some embodiments, the disease or condition is associated with TRAIL and/or FasL signaling pathway, comprising inflammatory diseases, autoimmune diseases, transplant-related diseases, liver diseases, neurodegenerative disorders or cancer. In some embodiments, the disease or condition is selected from the group consisting of transplant rejection, graft-versus-host disorders, liver injury, systemic inflammatory response syndrome, sepsis, multiple organ dysfunction syndrome, trauma, multiple sclerosis, idiopathic pulmonary fibrosis, osteoarthritis, inflammatory bowel disease, Crohn's disease, ulcerative colitis, acute lung injury, acute respiratory distress syndrome, myocardial infarction, cardiomyopathy, ischemic reperfusion injury, diabetes, brain injury, spinal cord injury, acute viral hepatitis B, acute viral hepatitis C, chronic hepatitis C, chronic Hepatitis B, alcoholic hepatitis, non-alcoholic steatohepatitis, cirrhosis, drug-induced liver injury/liver failure, autoimmune hepatitis, chronic kidney disease, acute kidney disease, diabetic kidney disease, and cancer.

In some embodiments, the antibody or antigen-binding fragment specifically binding to TRAIL in the method or use described herein comprises: (i) a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (ii) a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, the antibody or antigen-binding fragment specifically binding to TRAIL in the method or use described herein comprises: (i) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or (ii) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

In some embodiments, the antibody or antigen-binding fragment specifically binding to TRAIL in the method or use described herein comprises: a V_{H} comprising the amino acid sequence of any one of SEQ ID NOs: 13-21, or a variant thereof having at least about 80% (such as at least about any of 80%, 85%, 90%, 95%, 96%, 97%, 98%, or 99%) sequence identity to the amino acid sequence of any one of SEQ ID NOs: 13-21; and a V_{L} comprising the amino acid sequence of any one of SEQ ID NOs: 22-32, or a variant thereof having at least about 80% (such as at least about any of 80%, 85%, 90%, 95%, 96%, 97%, 98%, or 99%) sequence identity to the amino acid sequence of any one of SEQ ID NOs: 22-32.

In some embodiments, the antibody or antigen-binding fragment specifically binding to TRAIL in the method or use described herein comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27. (vii) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; (viii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29; (ix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29; (x) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29; (xi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29; (xii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29; (xiii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30; (xiv) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30; (xv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30; (xvi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30; (xvii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; (xviii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; (xix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31; (xx) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31; (xxi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31; (xxii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or (xxiii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

In some embodiments, there is provided a method of preventing or treating a disease or condition in an individual in need thereof, comprising administering to the individual an effective amount bispecific antibody specifically binding to TRAIL and FasL or a composition containing it, wherein the bispecific antibody comprising a first antigen-binding domain specifically binding to TRAIL, and a second antigen-binding domain specifically binding to FasL.

In some embodiments, there is provided the use of a bispecific antibody specifically binding to TRAIL and FasL or a composition containing it in the manufacture of a medicament for preventing or treating a disease or condition in an individual in need thereof, wherein the bispecific antibody comprising a first antigen-binding domain specifically binding to TRAIL, and a second antigen-binding domain specifically binding to FasL.

In some embodiments, in the method or use described herein, the bispecific antibody comprising a first antigen-binding domain specifically binding to TRAIL, and a second antigen-binding domain specifically binding to FasL, wherein the first antigen-binding domain comprises: (i) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; or (ii) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; or (iii) a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12. In some embodiments, the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, in the method or use described herein, the bispecific antibody comprising a first antigen-binding domain specifically binding to TRAIL, and a second antigen-binding domain specifically binding to FasL, wherein the second antigen-binding domain comprises: (i) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61; or (ii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or (iii) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, the first antigen-binding domain specifically binding to TRAIL in the method or use comprises: (i)a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (ii)a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28; or (iii)a V_{H} comprising an HC-CDR1, an HC-CDR2, and an HC-CDR3 of a V_{H} comprising the amino acid sequence of SEQ ID NO: 164; and a V_{L} comprising an LC-CDR1, an LC-CDR2, and an LC-CDR3 of a V_{L} comprising the amino acid sequence of SEQ ID NO: 168; or (iv)a V_{H} comprising an HC-CDR1, an HC-CDR2, and an HC-CDR3 of a V_{H} comprising the amino acid sequence of SEQ ID NO: 166; and a V_{L} comprising an LC-CDR1, an LC-CDR2, and an LC-CDR3 of a V_{L} comprising the amino acid sequence of SEQ ID NO: 170.

In some embodiments, the first antigen-binding domain specifically binding to TRAIL in the method or use comprises: a V_{H} comprising the amino acid sequence of any one of SEQ ID NOs: 13-20, 33-40, 164 and 166, or a variant thereof having at least about 80% (such as at least about any of 80%, 85%, 90%, 95%, 96%, 97%, 98%, or 99%) sequence identity to the amino acid sequence of any one of SEQ ID NOs: 13-20, 33-40, 164 and 166; and a V_{L} comprising the amino acid sequence of any one of SEQ ID NOs: 22-31, 41-50, 168 and 170, or a variant thereof having at least about 80% (such as at least about any of 80%, 85%, 90%, 95%, 96%, 97%, 98%, or 99%) sequence identity to the amino acid sequence of any one of SEQ ID NOs: 22-31, 41-50, 168 and 170.

In some embodiments, the first antigen-binding domain specifically binding to TRAIL in the method or use comprises: (i)a V_{H} comprising the amino acid sequence of any one of SEQ ID NOs: 13 and 33, or a variant thereof having at least about 80% sequence identity to the amino acid sequence of any one of SEQ ID NOs: 13 and 33; and a V_{L} comprising the amino acid sequence of any one of SEQ ID NOs: 22 and 41, or a variant thereof having at least about 80% sequence identity to the amino acid sequence of any one of SEQ ID NOs: 22 and 41; (ii)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42; (iii)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43; (iv)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 25 and 44, or a variant thereof having at least about 80% sequence identity to the amino acid sequence of any one of SEQ ID NOs: 25 and 44; (v)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 26 and 45, or a variant thereof having at least about 80% sequence identity to the amino acid sequence of any one of SEQ ID NOs: 26 and 45; (vi)a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 27 and 46, or a variant thereof having at least about 80% sequence identity to the amino acid sequence of any one of SEQ ID NOs: 27 and 46; (vii)a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47; (viii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (ix)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (x)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 29 and 48, or a variant thereof having at least about 80% sequence identity to the amino acid sequence of any one of SEQ ID NOs: 29 and 48; (xiii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xiv)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xv)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xvi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xvii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 30 and 49, or a variant thereof having at least about 80% sequence identity to the amino acid sequence of any one of SEQ ID NOs: 30 and 49; (xviii)a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xix)a V_{H} comprising the amino acid sequence of any one of SEQ ID NOs: 17 and 37, or a variant thereof having at least about 80% sequence identity to the amino acid sequence of any one of SEQ ID NOs: 17 and 37; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xx)a V_{H} comprising the amino acid sequence of any one of SEQ ID NOs: 18 and 38, or a variant thereof having at least about 80% sequence identity to the amino acid sequence of any one of SEQ ID NOs: 18 and 38; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxi)a V_{H} comprising the amino acid sequence of any one of SEQ ID NOs: 19 and 39, or a variant thereof having at least about 80% sequence identity to the amino acid sequence of any one of SEQ ID NOs: 19 and 39; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxii)a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 31 and 50, or a variant thereof having at least about 80% sequence identity to the amino acid sequence of any one of SEQ ID NOs: 31 and 50; (xxiii) a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; or (xxiv) a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170.

In some embodiments, the second antigen-binding domain specifically binding to FasL in the method or use comprises: (i)a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73; or (ii)a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76; or (iii)a V_{H} comprising an HC-CDR1, an HC-CDR2, and an HC-CDR3 of a V_{H} comprising the amino acid sequence of SEQ ID NO: 163; and a V_{L} comprising an LC-CDR1, an LC-CDR2, and an LC-CDR3 of a V_{L} comprising the amino acid sequence of SEQ ID NO: 167; or (iv)a V_{H} comprising an HC-CDR1, an HC-CDR2, and an HC-CDR3 of a V_{H} comprising the amino acid sequence of SEQ ID NO: 165; and a V_{L} comprising an LC-CDR1, an LC-CDR2, and an LC-CDR3 of a V_{L} comprising the amino acid sequence of SEQ ID NO: 169.

In some embodiments, the second antigen-binding domain specifically binding to FasL in the method or use comprises: a V_{H} comprising the amino acid sequence of any one of SEQ ID NOs: 63-72, 80-88, 163 and 165, or a variant thereof having at least about 80% (such as at least about any of 80%, 85%, 90%, 95%, 96%, 97%, 98%, or 99%) sequence identity to the amino acid sequence of any one of SEQ ID NOs: 63-72, 80-88, 163 and 165; and a V_{L} comprising the amino acid sequence of any one of SEQ ID NOs: 73-79, 89-95, 167 and 169, or a variant thereof having at least about 80% (such as at least about any of 80%, 85%, 90%, 95%, 96%, 97%, 98%, or 99%) sequence identity to the amino acid sequence of any one of SEQ ID NOs: 73-79, 89-95, 167 and 169.

In some embodiments, the second antigen-binding domain specifically binding to FasL in the method or use comprises: (i)a V_{H} comprising the amino acid sequence of any one of SEQ ID NOs: 63 and 80, or a variant thereof having at least about 80% sequence identity to the amino acid sequence of any one of SEQ ID NOs: 63 and 80; and a V_{L} comprising the amino acid sequence of any one of SEQ ID NOs: 73 and 89, or a variant thereof having at least about 80% sequence identity to the amino acid sequence of any one of SEQ ID NOs: 73 and 89; (ii)a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90; (iii)a V_{H} comprising the amino acid sequence of any one of SEQ ID NOs: 65 and 81, or a variant thereof having at least about 80% sequence identity to the amino acid sequence of any one of SEQ ID NOs: 65 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90; (iv)a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 75 and 91, or a variant thereof having at least about 80% sequence identity to the amino acid sequence of any one of SEQ ID NOs: 75 and 91; (v)a V_{H} comprising the amino acid sequence of any one of SEQ ID NOs: 65 and 81, or a variant thereof having at least about 80% sequence identity to the amino acid sequence of any one of SEQ ID NOs: 65 and 81; and a V_{L} comprising the amino acid sequence of any one of SEQ ID NOs: 75 and 91, or a variant thereof having at least about 80% sequence identity to the amino acid sequence of any one of SEQ ID NOs: 75 and 91; (vi)a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92; (vii)a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (viii)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (ix)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (x)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xi)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xii)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 77 and 93, or a variant thereof having at least about 80% sequence identity to the amino acid sequence of any one of SEQ ID NOs: 77 and 93; (xiii) a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xiv)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xv)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xvi)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xvii)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xviii)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 78 and 94, or a variant thereof having at least about 80% sequence identity to the amino acid sequence of any one of SEQ ID NOs: 78 and 94; (xix) a V_{H} comprising the amino acid sequence of any one of SEQ ID NOs: 67 and 83, or a variant thereof having at least about 80% sequence identity to the amino acid sequence of any one of SEQ ID NOs: 67 and 83; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xx)a V_{H} comprising the amino acid sequence of any one of SEQ ID NOs: 68 and 84, or a variant thereof having at least about 80% sequence identity to the amino acid sequence of any one of SEQ ID NOs: 68 and 84,; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxi)a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxii)a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxiii)a V_{H} comprising the amino acid sequence of any one of SEQ ID NOs: 71 and 87, or a variant thereof having at least about 80% sequence identity to the amino acid sequence of any one of SEQ ID NOs: 71 and 87; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxiv)a V_{H} comprising the amino acid sequence of any one of SEQ ID NOs: 72 and 88, or a variant thereof having at least about 80% sequence identity to the amino acid sequence of any one of SEQ ID NOs: 72 and 88; and a V_{L} comprising the amino acid sequence of any one of SEQ ID NOs: 79 and 95, or a variant thereof having at least about 80% sequence identity to the amino acid sequence of any one of SEQ ID NOs: 79 and 95; (xxv) a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167; or (xxvi) a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43; and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50; and wherein the second antigen-binding domain comprises: a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; and wherein the second antigen-binding domain comprises: a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167.

In some embodiments, in the method or use described herein, the bispecific antibody comprising a first antigen-binding domain specifically binding to TRAIL, and a second antigen-binding domain specifically binding to FasL, wherein the first antigen-binding domain comprises: a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170; and wherein the second antigen-binding domain comprises: a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

In some embodiments, there is provided a method of preventing or treating a disease or condition in an individual in need thereof, comprising administering to the individual an effective amount of a bispecific antibodies specifically binding to TRAIL and FasL described above or a composition containing it, which method is more efficacious than administering a same valency amount of an antibody or antigen-binding fragment specifically binding to TRAIL or a same valency amount of an antibody or antigen-binding fragment specifically binding to FasL. In some embodiments, the method comprising administering an effective amount of a bispecific antibodies specifically binding to TRAIL and FasL described above enhances the inhibition of FasL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of a bispecific antibodies specifically binding to TRAIL and FasL described above enhances the inhibition of TRAIL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of a bispecific antibodies specifically binding to TRAIL and FasL described above enhances the inhibition of FasL and TRAIL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL.

In some embodiments, there are provided a method of preventing or treating a disease or condition in an individual in need thereof, comprising administering to the individual an effective amount of pharmaceutical compositions comprising: (i) an antibody or antigen-binding fragment specifically binding to TRAIL, and (ii) an antibody or antigen-binding fragment specifically binding to FasL.

In some embodiments, there is provided the use of a pharmaceutical composition containing (i) the antibody or antigen-binding fragment specifically binding to TRAIL and (ii) the antibody or antigen-binding fragment specifically binding to FasL in the manufacture of a medicament for treating a disease or condition.

In some embodiments, there are provided a method of preventing or treating a disease or condition in an individual in need thereof, comprising administering to the individual: (i) an antibody or antigen-binding fragment specifically binding to TRAIL, and (ii) an antibody or antigen-binding fragment specifically binding to FasL.

In some embodiments, there is provided the use of (i) an antibody or antigen-binding fragment specifically binding to TRAIL, and (ii) an antibody or antigen-binding fragment specifically binding to FasL in the manufacture of a medicament for treating a disease or condition.

In some embodiments, an antibody or antigen-binding fragment specifically binding to TRAIL is administered simultaneously with an antibody or antigen-binding fragment specifically binding to FasL. In some embodiments, an antibody or antigen-binding fragment specifically binding to TRAIL is administered consecutively with an antibody or antigen-binding fragment specifically binding to FasL.

In some embodiments, in the methods or uses described herein, the antibody or antigen-binding fragment specifically binding to TRAIL comprises: (a) a V_{H} comprising an HC-CDR1, an HC-CDR2, and an HC-CDR3 of a V_{H} comprising the amino acid sequence of SEQ ID NO: 13; and a V_{L} comprising an LC-CDR1, an LC-CDR2, and an LC-CDR3 of a V_{L} comprising the amino acid sequence of SEQ ID NO: 22; or (b) a V_{H} comprising an HC-CDR1, an HC-CDR2, and an HC-CDR3 of a V_{H} comprising the amino acid sequence of SEQ ID NO: 15; and a V_{L} comprising an LC-CDR1, an LC-CDR2, and an LC-CDR3 of a V_{L} comprising the amino acid sequence of SEQ ID NO: 28.

In some embodiments, in the methods or uses described herein, the antibody or antigen-binding fragment specifically binding to FasL comprises: (a) a V_{H} comprising an HC-CDR1, an HC-CDR2, and an HC-CDR3 of a V_{H} comprising the amino acid sequence of SEQ ID NO: 63; and a V_{L} comprising an LC-CDR1, an LC-CDR2, and an LC-CDR3 of a V_{L} comprising the amino acid sequence of SEQ ID NO: 73; or (b) a V_{H} comprising an HC-CDR1, an HC-CDR2, and an HC-CDR3 of a V_{H} comprising the amino acid sequence of SEQ ID NO: 66; and a V_{L} comprising an LC-CDR1, an LC-CDR2, and an LC-CDR3 of a V_{L} comprising the amino acid sequence of SEQ ID NO: 76.

In some embodiments, in the methods or uses described herein, the antibody or antigen-binding fragment specifically binding to TRAIL comprises: (a) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or (b) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

In some embodiments, in the methods or uses described herein, the antibody or antigen-binding fragment specifically binding to FasL comprises: (a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or (b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the methods or uses described herein, the antibody or antigen-binding fragment specifically binding to TRAIL comprises: a V_{H} comprising the amino acid sequence of any one of SEQ ID NOs: 13-21, or a variant thereof having at least about 80% (such as at least about any of 80%, 85%, 90%, 95%, 96%, 97%, 98%, or 99%) sequence identity to the amino acid sequence of any one of SEQ ID NOs: 13-21; and b) a V_{L} comprising the amino acid sequence of any one of SEQ ID NOs: 22-32, or a variant thereof having at least about 80% (such as at least about any of 80%, 85%, 90%, 95%, 96%, 97%, 98%, or 99%) sequence identity to the amino acid sequence of any one of SEQ ID NOs: 22-32.

In some embodiments, in the methods or uses described herein, the antibody or antigen-binding fragment specifically binding to TRAIL comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; (iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25; (v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27. (vii) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; (viii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29; (ix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29; (x) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29; (xi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29; (xii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29; (xiii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30; (xiv) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30; (xv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30; (xvi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30; (xvii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; (xviii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; (xix) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31; (xx) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31; (xxi) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31; (xxii) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or (xxiii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

In some embodiments, in the methods or uses described herein, the antibody or antigen-binding fragment specifically binding to FasL comprises: a V_{H} comprising the amino acid sequence of any one of SEQ ID NOs: 63-72, or a variant thereof having at least about 80% (such as at least about any of 80%, 85%, 90%, 95%, 96%, 97%, 98%, or 99%) sequence identity to the amino acid sequence of any one of SEQ ID NOs: 63-72; and b) a V_{L} comprising the amino acid sequence of any one of SEQ ID NOs: 73-79, or a variant thereof having at least about 80% (such as at least about any of 80%, 85%, 90%, 95%, 96%, 97%, 98%, or 99%) sequence identity to the amino acid sequence of any one of SEQ ID NOs: 73-79.

In some embodiments, in the methods or uses described herein, the antibody or antigen-binding fragment specifically binding to FasL comprises: (i) a V_{H} comprising the amino acid sequence of SEQ ID NO: 63, or a variant thereof having at least about 80% sequence identity to the amino acid sequence of SEQ ID NO:63; and a V_{L} comprising the amino acid sequence of SEQ ID NO: 73, or a variant thereof having at least about 80% sequence identity to the amino acid sequence of SEQ ID NO: 73; (ii) a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74; (iii) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 74, or a variant thereof having at least about 80% sequence identity to the amino acid sequence of SEQ ID NO: 74; (iv) a V_{H} comprising the amino acid sequence of SEQ ID NO: 64, or a variant thereof having at least about 80% sequence identity to the amino acid sequence of SEQ ID NO: 64; and a V_{L} comprising the amino acid sequence of SEQ ID NO: 75, or a variant thereof having at least about 80% sequence identity to the amino acid sequence of SEQ ID NO: 75; (v) a V_{H} comprising the amino acid sequence of SEQ ID NO: 65, or a variant thereof having at least about 80% sequence identity to the amino acid sequence of SEQ ID NO: 65; and a V_{L} comprising the amino acid sequence of SEQ ID NO: 75, or a variant thereof having at least about 80% sequence identity to the amino acid sequence of SEQ ID NO: 75; (vi) a V_{H} comprising the amino acid sequence of SEQ ID NO:66, or a variant thereof having at least about 80% sequence identity to the amino acid sequence of SEQ ID NO: 66; and a V_{L} comprising the amino acid sequence of SEQ ID NO: 76, or a variant thereof having at least about 80% sequence identity to the amino acid sequence of SEQ ID NO: 76. (vii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 77, or a variant thereof having at least about 80% sequence identity to the amino acid sequence of SEQ ID NO: 77; (viii) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 77, or a variant thereof having at least about 80% sequence identity to the amino acid sequence of SEQ ID NO: 77; (ix) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 77, or a variant thereof having at least about 80% sequence identity to the amino acid sequence of SEQ ID NO: 77; (x) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 77, or a variant thereof having at least about 80% sequence identity to the amino acid sequence of SEQ ID NO: 77; (xi) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 77, or a variant thereof having at least about 80% sequence identity to the amino acid sequence of SEQ ID NO: 77; (xii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 77, or a variant thereof having at least about 80% sequence identity to the amino acid sequence of SEQ ID NO: 77; (xiii) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 78, or a variant thereof having at least about 80% sequence identity to the amino acid sequence of SEQ ID NO: 78; (xiv) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 78, or a variant thereof having at least about 80% sequence identity to the amino acid sequence of SEQ ID NO: 78; (xv) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 78, or a variant thereof having at least about 80% sequence identity to the amino acid sequence of SEQ ID NO: 78; (xvi) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78; (xvii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 78, or a variant thereof having at least about 80% sequence identity to the amino acid sequence of SEQ ID NO: 78; (xviii) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 78, or a variant thereof having at least about 80% sequence identity to the amino acid sequence of SEQ ID NO: 78; (xix) a V_{H} comprising the amino acid sequence of SEQ ID NO: 67, or a variant thereof having at least about 80% sequence identity to the amino acid sequence of SEQ ID NO: 67; and a V_{L} comprising the amino acid sequence of SEQ ID NO: 79, or a variant thereof having at least about 80% sequence identity to the amino acid sequence of SEQ ID NO: 79; (xx) a V_{H} comprising the amino acid sequence of SEQ ID NO: 68, or a variant thereof having at least about 80% sequence identity to the amino acid sequence of SEQ ID NO: 68; and a V_{L} comprising the amino acid sequence of SEQ ID NO: 79, or a variant thereof having at least about 80% sequence identity to the amino acid sequence of SEQ ID NO: 79; (xxi) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 79, or a variant thereof having at least about 80% sequence identity to the amino acid sequence of SEQ ID NO: 79; (xxii) a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 79, or a variant thereof having at least about 80% sequence identity to the amino acid sequence of SEQ ID NO: 79; (xxiii) a V_{H} comprising the amino acid sequence of SEQ ID NO: 71, or a variant thereof having at least about 80% sequence identity to the amino acid sequence of SEQ ID NO: 71; and a V_{L} comprising the amino acid sequence of SEQ ID NO: 79, or a variant thereof having at least about 80% sequence identity to the amino acid sequence of SEQ ID NO: 79; or (xxiv) a V_{H} comprising the amino acid sequence of SEQ ID NO: 72, or a variant thereof having at least about 80% sequence identity to the amino acid sequence of SEQ ID NO: 72; and a V_{L} comprising the amino acid sequence of SEQ ID NO: 79, or a variant thereof having at least about 80% sequence identity to the amino acid sequence of SEQ ID NO: 79.

In some embodiments, in the methods or uses described herein, the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, in the methods or uses described herein, the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs.

In some embodiments, in the methods or uses described herein, the antibody or antigen-binding fragment specifically binding to TRAIL comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the methods or uses described herein, the antibody or antigen-binding fragment specifically binding to TRAIL comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

In some embodiments, in the methods or uses described herein, the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, in the methods or uses described herein, the antibody or antigen-binding fragment specifically binding to TRAIL comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74.

In some embodiments, in the methods or uses described herein, the antibody or antigen-binding fragment specifically binding to TRAIL comprises: a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, in the methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76.

In some embodiments, in the methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78.

In some embodiments, in the methods or uses described herein, the antibody or antigen-binding fragment specifically binding to TRAIL comprises: a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 69, or a variant thereof having at least about 80% sequence identity to the amino acid sequence of SEQ ID NO:69; and a V_{L} comprising the amino acid sequence of SEQ ID NO: 79, or a variant thereof having at least about 80% sequence identity to the amino acid sequence of SEQ ID NO: 79.

In some embodiments, the method comprises administering pharmaceutical compositions comprising an antibody or antigen-binding fragment specifically binding to TRAIL and an antibody or antigen-binding fragment specifically binding to FasL described herein, the ratio by molar mass of the antibody or antigen-binding fragment specifically binding to TRAIL and an antibody or antigen-binding fragment specifically binding to FasL is about 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10 or 1:20. In other embodiments, t the ratio by molar mass of the antibody or antigen-binding fragment specifically binding to TRAIL and an antibody or antigen-binding fragment specifically binding to FasL is about 5:1, 4:1, 3:1, 2:1, 1:1, 1:2, 1:3, 1:4 or 1:5. In other embodiments, t the ratio by molar mass of the antibody or antigen-binding fragment specifically binding to TRAIL and an antibody or antigen-binding fragment specifically binding to FasL is about 2:1 or 1:1.

In some embodiments, the method comprises administering (i) an antibody or antigen-binding fragment specifically binding to TRAIL and (ii) an antibody or antigen-binding fragment specifically binding to FasL described herein, the ratio by molar mass of the antibody or antigen-binding fragment specifically binding to TRAIL and an antibody or antigen-binding fragment specifically binding to FasL is about 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10 or 1:20. In other embodiments, t the ratio by molar mass of the antibody or antigen-binding fragment specifically binding to TRAIL and an antibody or antigen-binding fragment specifically binding to FasL is about 5:1, 4:1, 3:1, 2:1, 1:1, 1:2, 1:3, 1:4 or 1:5. In other embodiments, t the ratio by molar mass of the antibody or antigen-binding fragment specifically binding to TRAIL and an antibody or antigen-binding fragment specifically binding to FasL is about 2:1 or 1:1.

In some embodiments, there is provided a method of preventing or treating a disease or condition in an individual in need thereof, comprising administering to the individual an effective amount of pharmaceutical compositions comprising an antibody or antigen-binding fragment specifically binding to TRAIL and an antibody or antigen-binding fragment specifically binding to FasL described herein, which method is more efficacious than administering a same valency amount of an antibody or antigen-binding fragment specifically binding to TRAIL or a same valency amount of an antibody or antigen-binding fragment specifically binding to FasL. In some embodiments, the method comprising administering an effective amount of pharmaceutical compositions comprising an antibody or antigen-binding fragment specifically binding to TRAIL and an antibody or antigen-binding fragment specifically binding to FasL described herein enhances the inhibition of FasL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of pharmaceutical compositions comprising an antibody or antigen-binding fragment specifically binding to TRAIL and an antibody or antigen-binding fragment specifically binding to FasL described herein enhances the inhibition of TRAIL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of pharmaceutical compositions comprising an antibody or antigen-binding fragment specifically binding to TRAIL and an antibody or antigen-binding fragment specifically binding to FasL described herein enhances the inhibition of TRAIL and FasL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL.

In some embodiments, there is provided a method of preventing or treating a disease or condition in an individual in need thereof, comprising administering to the individual an effective amount of (i) an antibody or antigen-binding fragment specifically binding to TRAIL and (ii) an antibody or antigen-binding fragment specifically binding to FasL described herein, which method is more efficacious than administering a same valency amount of an antibody or antigen-binding fragment specifically binding to TRAIL or a same valency amount of an antibody or antigen-binding fragment specifically binding to FasL. In some embodiments, the method comprising administering an effective amount of (i) an antibody or antigen-binding fragment specifically binding to TRAIL and (ii) an antibody or antigen-binding fragment specifically binding to FasL described herein enhances the inhibition of FasL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of (i) an antibody or antigen-binding fragment specifically binding to TRAIL and (ii) an antibody or antigen-binding fragment specifically binding to FasL described herein enhances the inhibition of TRAIL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL. In some embodiments, the method comprising administering an effective amount of (i) an antibody or antigen-binding fragment specifically binding to TRAIL and (ii) an antibody or antigen-binding fragment specifically binding to FasL described herein enhances the inhibition of TRAIL and FasL induced apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to TRAIL or a same valency amount of an antibody or antigen-binding fragment specifically binding to FasL.

### Articles of Manufacture and Kits

In some embodiments of the application, there is provided an article of manufacture containing materials useful for preventing or treating a disease or condition in an individual in need thereof, or for delivering an antibody or antigen-binding fragment (such as an antibody or antigen-binding fragment specifically binding to TRAIL or an antibody or antigen-binding fragment specifically binding to FasL) or bispecific antibody (such as a bispecific antibody specifically binding to TRAIL and FasL) or a pharmaceutical composition comprising antibodies or antigen-binding fragments specifically binding to TRAIL or antibodies or antigen-binding fragments specifically binding to FasL, to a cell attached by a pathogen expressing TRAIL and FasL. The article of manufacture can comprise a container and a label or package insert on or associated with the container. Suitable containers include, for example, bottles, vials, syringes, etc. The containers may be formed from a variety of materials such as glass or plastic. Generally, the container holds a composition which is effective for treating a disease or disorder described herein, and may have a sterile access port (for example the container may be an intravenous solution bag or a vial having a stopper pierceable by a hypodermic injection needle). In some embodiments, at least one active agent in the composition is an antibody or antigen-binding fragment or bispecific antibody of the application. The label or package insert indicates that the composition is used for treating the particular condition. The label or package insert will further comprise instructions for administering the bispecific antibody or the pharmaceutical composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating bacterial infections. In some embodiments, the package insert indicates that the composition is used for treating a disease or condition in an individual in need thereof.

Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, e.g., useful for preventing or treating a disease or condition in an individual in need thereof, or for delivering an antibody or antigen-binding fragment specifically binding to TRAIL, an antibody or antigen-binding fragment specifically binding to FasL, or a bispecific antibody specifically binding to TRAIL and FasL (such as a full-length antibody specifically binding to TRAIL, full-length antibody specifically binding to FasL, or bispecific antibody specifically binding to TRAIL and FasL) to a cell attached by a pathogen expressing TRAIL or FasL, optionally in combination with the articles of manufacture. Kits of the application include one or more containers comprising antibody or antigen-binding fragment specifically binding to TRAIL, antibody or antigen-binding fragment specifically binding to FasL, or bispecific antibody specifically binding to TRAIL and FasL composition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert (e.g., a paper sheet included in the kit), but machine-readable instructions (e.g., instructions carried on a magnetic or optical storage disk) are also acceptable.

For example, in some embodiments, the kit comprises a composition comprising an antibody or antigen-binding fragment specifically binding to TRAIL, an antibody or antigen-binding fragment specifically binding to FasL, or bispecific antibody specifically binding to TRAIL and FasL (such as a full-length antibody specifically binding to TRAIL, full-length antibody specifically binding to FasL, or bispecific antibody specifically binding to TRAIL and FasL). In some embodiments, the kit comprises a) a composition comprising any one of the antibodies or antigen-binding fragments specifically binding to TRAIL, an antibodies or an antigen-binding fragments specifically binding to FasL, or bispecific antibodies specifically binding to TRAIL and FasL described herein, and b) an effective amount of at least one other agent, wherein the other agent enhances the effect (e.g., treatment effect, detecting effect) of the antibody or antigen-binding fragment specifically binding to TRAIL, antibody or antigen-binding fragment specifically binding to FasL, or bispecific antibody specifically binding to TRAIL and FasL. In some embodiments, the kit comprises a) a composition comprising any one of the antibodies or antigen-binding fragments specifically binding to TRAIL, antibodies or an antigen-binding fragments specifically binding to FasL, or bispecific antibodies specifically binding to TRAIL and FasL described herein, and b) instructions for administering the antibody or antigen-binding fragment specifically binding to TRAIL, antibody or antigen-binding fragment specifically binding to FasL, or bispecific antibody specifically binding to TRAIL and FasL composition to an individual for treating a disease or condition in an individual in need thereof. In some embodiments, the kit comprises a) a composition comprising any one of the antibodies or antigen-binding fragments specifically binding to TRAIL, antibodies or an antigen-binding fragments specifically binding to FasL, or bispecific antibodies specifically binding to TRAIL and FasL described herein, b) an effective amount of at least one other agent, wherein the other agent enhances the effect (e.g., treatment effect, detecting effect) of the antibody or antigen-binding fragment specifically binding to TRAIL, antibody or antigen-binding fragment specifically binding to FasL, or bispecific antibody specifically binding to TRAIL and FasL, and c) instructions for administering the antibody or antigen-binding fragment specifically binding to TRAIL, antibody or antigen-binding fragment specifically binding to FasL, or bispecific antibody specifically binding to TRAIL and FasL composition and the other agent(s) to an individual for useful for treating a disease or condition in an individual in need thereof. The antibody or antigen-binding fragment specifically binding to TRAIL, antibody or antigen-binding fragment specifically binding to FasL, or bispecific antibody specifically binding to TRAIL and FasL and the other agent(s) can be present in separate containers or in a single container. For example, the kit may comprise one distinct composition or two or more compositions wherein one composition comprises an antibody or antigen-binding fragment specifically binding to TRAIL, an antibody or antigen-binding fragment specifically binding to FasL, or bispecific antibody specifically binding to TRAIL and FasL and another composition comprises another agent.

In some embodiments, the kit comprises a nucleic acid (or set of nucleic acids) encoding an antibody or antigen-binding fragment specifically binding to TRAIL, an antibody or antigen-binding fragment specifically binding to FasL, or bispecific antibody specifically binding to TRAIL and FasL (such as a full-length antibody specifically binding to TRAIL, full-length antibody specifically binding to FasL, or bispecific antibody specifically binding to TRAIL and FasL). In some embodiments, the kit comprises a) a nucleic acid (or set of nucleic acids) encoding an antibody or antigen-binding fragment specifically binding to TRAIL, an antibody or antigen-binding fragment specifically binding to FasL, or bispecific antibody specifically binding to TRAIL and FasL, and b) a host cell for expressing the nucleic acid (or set of nucleic acids). In some embodiments, the kit comprises a) a nucleic acid (or set of nucleic acids) encoding an antibody or antigen-binding fragment specifically binding to TRAIL, an antibody or antigen-binding fragment specifically binding to FasL, or bispecific antibody specifically binding to TRAIL and FasL and b) instructions for i) expressing the antibody or antigen-binding fragment specifically binding to TRAIL, the antibody or antigen-binding fragment specifically binding to FasL, or bispecific antibody specifically binding to TRAIL and FasL in a host cell, ii) preparing a composition comprising the antibody or antigen-binding fragment specifically binding to TRAIL, antibody or antigen-binding fragment specifically binding to FasL, or bispecific antibody specifically binding to TRAIL and FasL, and iii) administering the composition comprising the antibody or antigen-binding fragment specifically binding to TRAIL, an antibody or antigen-binding fragment specifically binding to FasL, or bispecific antibody specifically binding to TRAIL and FasL to an individual for treating or preventing a disease or condition in an individual in need thereof. In some embodiments, the kit comprises a) a nucleic acid (or set of nucleic acids) encoding an antibody or antigen-binding fragment specifically binding to TRAIL, an antibody or antigen-binding fragment specifically binding to FasL, or bispecific antibody specifically binding to TRAIL and FasL, b) a host cell for expressing the nucleic acid (or set of nucleic acids), and c) instructions for i) expressing the antibody or antigen-binding fragment specifically binding to TRAIL, antibody or antigen-binding fragment specifically binding to FasL, or bispecific antibody specifically binding to TRAIL and FasL in the host cell, ii) preparing a composition comprising the antibody or antigen-binding fragment specifically binding to TRAIL, antibody or antigen-binding fragment specifically binding to FasL, or bispecific antibody specifically binding to TRAIL and FasL and iii) administering the composition comprising the antibody or antigen-binding fragment specifically binding to TRAIL, antibody or antigen-binding fragment specifically binding to FasL, or bispecific antibody specifically binding to TRAIL and FasL to an individual for treating or preventing a disease or condition in an individual in need thereof.

The kits of the application are in suitable packaging. Suitable packaging includes, but is not limited to, vials, bottles, jars, flexible packaging (e.g., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the compositions comprising an antibody or antigen-binding fragment specifically binding to TRAIL, an antibody or antigen-binding fragment specifically binding to FasL, or a bispecific antibody specifically binding to TRAIL and FasL, generally include information as to dosage, dosing schedule, and route of administration for the intended treatment. The containers may be unit doses, bulk packages (e.g., multi-dose packages) or sub-unit doses. For example, kits may be provided that contain sufficient dosages of an antibody or antigen-binding fragment specifically binding to TRAIL, an antibody or antigen-binding fragment specifically binding to FasL, or bispecific antibody specifically binding to TRAIL and FasL (such as a full-length antibody specifically binding to TRAIL, full-length antibody specifically binding to FasL, or bispecific antibody specifically binding to TRAIL and FasL) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the antibody or antigen-binding fragment specifically binding to TRAIL, antibody or antigen-binding fragment specifically binding to FasL, or bispecific antibody specifically binding to TRAIL and FasL and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

### Example 1: Generation of recombinant TRAIL, FasL and DR5 antigen

cDNA encoding protein extracellular region of human TRAIL (hTRAIL; GenBank ID: 8743), Cynomolgus monkey TRAIL (CynoTRAIL; GenBank ID: 101864918), mouse TRAIL (mouTRAIL; GenBank ID: 22035), human FasL (hFasL; GenBank ID: 356), Cynomolgus monkey FasL (CynoFasL; GenBank ID: 102139406), mouse FasL (mouFasL; GenBank ID: 14103) (synthesized by sino biological Inc.,) were subcloned into eukaryotic or prokaryotic expression vector respectively. His-tag and/or GST-tag and/or human Fc (hFc)-tag and/or other conventionally used tags were added to the terminal of cDNA described above, and plasmids containing human, monkey or mouse TRAIL/FasL extracellular region encoding sequences were constructed. The plasmids were transfected into 293F cells or *E. coli* to express and produce fusion proteins containing TRAIL/FasL extracellular regions, which named such as His-hTRAIL, GST-hTRAIL, GST-CynoTRAIL, GST-mouTRAIL, His-hFasL, hFc-hFasL, hFc-CynoFasL, hFc-mouFas respectively, wherein "His" stands for His tag, "GST" stands for GST tag, "hFc" stands for human Fc fragmen.

As described above, human Fc-tag was added to the terminal of human DR5 (hDR5; GenBank ID: 8795) extracellular region encoding sequence to construct eukaryotic expression vector containing encoding sequence of DR5 extracellular region-Fc fusion proteins. The expression vector was transfected into 293F cells to express and produce fusion proteins, which named "sDR5-Fc", wherein "Fc" stands for human Fc fragment.

Recombinant proteins His-hTRAIL, His-hFasL, hFc-hFasL, hFc-CynoFasL, hFc-mouFasL and sDR5-Fc were expressed and purified respectively. Briefly, 293F cells were transfected with the expression vectors containing His-hTRAIL, His-hFasL, hFc-hFasL, hFc-CynoFasL, hFc-mouFasL, sDR5-Fc encoding sequence respectively, and cultured at 37°C, 5% CO₂ and 120rpm for 5 days. The culture media was collected respectively.

Recombinant fusion proteins with His-tag were purified using Ni-HisCap Smart 6FF 5ml preloaded column (Smart-Lifesciences, SA036C15) for immobilized metal affinity chromatography (IMAC) analysis according to manufacturer's protocol. Specifically, the cartridges were first equilibrated with buffer A1 (50mM Na₃PO₄, 0.15M NaCl, pH 7.2) with a flow rate of 150cm/h. The culture supernatant, whose pH was adjusted to 7.2, flowed through the cartridges at room temperature (RT) with a flow rate of 150cm/h. Next, 6×column volumes of buffer A1 was used to equilibrate the cartridges at the flow rate of 150cm/h. 10 × column volumes of 50mM PBS solution (containing 0.15M NaCl and 0.2M Imidazole, pH 7.2) was used to wash the cartridges and the eluent was collected. The purified protein was concentrated using an ultrafiltration tube and the solvent of the purified protein was replaced with PBS.

Recombinant fusion proteins with Fc fragment were purified using protein A-MabCap At 4FF 5ml preloaded column (Smart-Lifesciences, SA023C15). Specifically, the cartridges were first equilibrated with PBS (containing 50mM PB and 0.15M NaCl, pH 7.2) with a flow rate of 5ml/min. The culture supernatant, whose pH was adjusted to 7.2, flowed through the cartridges at room temperature (RT) with a flow rate of 5ml/min. Next, 6×column volumes of PBS was used to equilibrate the cartridges at the flow rate of 5ml/min. 6-10 × column volumes of PBS solution was used to equilibrated the cartridges at the flow rate of 5ml/min. After complete equilibrium, eluent (containing 0.1M Gly and 150mM NaCl, pH3.2) was added for elution and eluent was collected. The purified protein was concentrated using an ultrafiltration tube and the solvent of the purified protein was replaced with PBS.

Recombinant proteins GST-hTRAIL, GST-CynoTRAIL and GST-mouTRAIL were expressed and purified respectively. Briefly, 2 The plasmid containing GST tag was introduced into
*E. coli* OrigamiB competent cells, and then the competent cells were added to LB liquid medium and cultured at 37°C and 220rpm until OD600 reaching 0.8-1.0. Isopropyl-β-D-thiogalactopyranoside (IPTG, (GPCSCI., AC367-100G) was added at a final concentration of 0.4mM. The expression was induced at 22°C overnight, and the bacteria were collected after centrifugation. The bacteria were lysed by ultrasonic, the supernatant was collected by centrifugation at 20000g for 30min.

Recombinant fusion proteins with GST-tag were purified with using GSTCap 4FF 5ml preloaded column (Smart-Lifesciences, SA010C15) by affinity chromatography. Specifically, the cartridges were first equilibrated with PBS (pH 7.4) with a flow rate of 5ml/min. The culture supernatant, whose pH was adjusted to 7.4, flowed through the cartridges at room temperature (RT) with a flow rate of 5ml/min. Next. 6-10 × column volumes of PBS solution was used to equilibrated the cartridges at the flow rate of 5ml/min. 10 × column volumes of PBS solution (containing 10mM reduced glutathione, pH 7.4) was used to wash the cartridges and the eluent was collected. The purified protein was concentrated using an ultrafiltration tube and the solvent of the purified protein was replaced with PBS.

### Example 2: Selection of Anti-TRAIL scFv antibodies

### 2.1 Construction of scFv Antibody phage display library:

Mice were immunized with His-hTRAIL antigen and adjuvant. After immunization, serum was collected from each immunized animal and analyzed for IgG titer by ELISA. After several rounds of immunization, spleen was used to establish a phage library. Briefly, RNAs were extracted from spleen of the immunized mice and then reverse-transcribed into cDNAs. V_{H} and V_{L} fragments were amplified using V_{H}- and V_{K}-specific primers. Upon gel extraction and purification, scFvs were generated by linking V_{H} and V_{K}, and were cloned into the phage display plasmid, which were then electroporated into *TG1* competent cells, and the scFv antibody phage display library generated by infecting *TG1* with phage.

### 2.2 Selection of anti-TRAIL scFv antibodies:

After several rounds of panning, scFvs which specifically bound to human TRAIL were isolated from the phage display library. Briefly, scFv phage library at 2x10¹¹ PFU was added to an ELISA plate coated with His-hTRAIL, which incubated at 37°C for 2 h. After washing 8-15 times (the times of washing increased with increasing number of selection rounds) with PBST solution, the phages binding with human TRAIL were captured by the TRAIL antigen on the ELISA plate and the unbound phages were washed off. The phages specifically binding to human TRAIL were eluted with Glycine-HCl solution (pH2.2). *TG1* cells in exponentially growing phase were infected with those phages. After adding ampicillin and incubating for 1 h, helper phage were added and incubated overnight at 28°C and 200rpm shaking. Next day, the culture medium was collected and centrifugated, then, the supernatant was harvested and subjected to the next round of screening until the positive scFv antibody library was obtained.

### 2.3 Selection of anti-TRAIL scFv antibodies using ELISA:

The phages enriched from the previous panning were screened by ELISA. Briefly, His-hTRAILwas dissolved in PBS and coated on 96-well plates with 0. 1µg/well followed by incubation at 4°C overnight. The 96-well plates were washed with PBST and 90µL of PBS containing 4% skim milk was added to each well. 10µL of supernatant containing phage-scFv was added to the corresponding well and incubated for 1-2h at 37°C. After washing with PBST solution, the M13-HRP antibody (Sino Biological, 11973-MM05T-H) diluted at 1:4000 was added with 100µL/well and incubated for 1h at 37°C. After washing six times with PBST, 100µL of TMB was added to each well. The plates were incubated for 10min at room temperature avoiding light contact. The development reaction was terminated using 2 M H₂SO₄. Plates were read for the absorbance at 450 nm using a microplate reader. A panel of positive scFv antibodies were obtained and sequenced at the end of screening.

### Example 3: Generation and characterization of full-length anti-TRAIL chimeric antibodies

### 3.1 Generation of full-length anti-TRAIL chimeric antibodies

The positive scFv antibodies were reformatted as chimeric antibody molecules with a human IgG1 or IgG4 heavy chain constant domain, and a human kappa (κ) light chain constant domain. V_{H} and V_{L} were amplified from the phage expression vectors and introduced into eukaryotic expression vectors pTT5-L1 (containing κ constant domain) and pTT5-H1 (containing IgG1 heavy chain constant domain), or pTT5-H4 (containing IgG4 heavy chain constant domain), respectively. Plasmid expressing the light chain and plasmid expressing the heavy chains were extracted and used to co-transfect 293F cells. After the cells were cultured at 37°C, 5% CO₂ and 120rpm for 5 days, the culture media was purified using Protein A affinity chromatography. Briefly, the antibody purification using Protein A column is the same as that used in Example 1. The concentration of antibody was determined after ultrafiltration and then subjected to further biochemical and biological analysis.

### 3.2 Determination of anti-TRAIL antibodies activity

### 3.2.1 Anti-TRAIL antibody inhibited TRAIL antigen-induced apoptosis of HepG2 cells:

When actinomycin D was added to inhibit cell division, exogenous TRAIL could activate apoptosis signaling pathway and mediate apoptosis. The amount of ATP in cells could reflect the viability of cells, which was inversely proportional to the degree of apoptosis. Based on the above principles, the amount of ATP could be detected by CellTiter-Glo^{®} luminescent cell viability assay kit, and then the activity of anti-TRAIL chimeric antibody (reformatted as human IgG4) to inhibit TRAIL antigen-induced apoptosis of HepG2 cells was determined.

HepG2 cells at logarithmic growth stage (purchased from the cell bank of Peking Union Medical College, 1101HUM-PUMC000035) were inoculated into 96-well plates with 2×10⁴ cells/wells. Each antibody sample was diluted to 108nM and subsequently diluted in a specific proportion. 50µL actinomycin D (Hanhui-pharma, H20023504), 25µL TRAIL recombinant fusion protein (Novoprotein, C022) and 25µL diluted anti-TRAIL antibody were added to a 96-well plate containing HepG2 cells. The final concentrations of actinomycin D and TRAIL were 1µg/mL and 0.5 ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each anti-TRAIL antibody was calculated.

As shown in Table 11, anti-TRAIL chimeric antibodies G10, G45, G72 all had significant inhibitory effects on TRAIL antigen-induced apoptosis of HepG2 cells. The inhibition of TRAIL antigen-induced apoptosis of HepG2 cells by the above anti-TRAIL antibodies were significantly better than or comparable to the reference MAB375-500 (commercial anti-TRAIL antibody, RD).

**Table 11: The inhibition of TRAIL antigen-induced apoptosis of HepG2 cells by the anti-TRAIL chimeric antibodies**

| **Antibody Name** | **IC₅₀(nM)** | **Antibody Name** | **IC₅₀(nM)** |
|---|---|---|---|
| **G10** | **0.008848** | **G72** | **0.02868** |
| **G45** | **0.1152** | **MAB375-500** | **0.1027** |

### 3.2.2 Anti-TRAIL antibody inhibited TRAIL antigen-induced apoptosis of Jurkat cells:

The experimental principle is the same as above. The amount of ATP in cells is used to reflect the apoptosis level, and then the activity of anti-TRAIL chimeric antibody (reformatted as human IgG4) to inhibit TRAIL antigen-induced apoptosis of Jurkat cells was determined.

Jurkat cells at logarithmic growth stage (purchased from the cell bank of Peking Union Medical College, 1101HUM-PUMC000075) were inoculated into 96-well plates with 5×10⁴ cells/wells. Each antibody sample was diluted to 86.4nM and subsequently diluted in a specific proportion. 50µL actinomycin D, 25µL TRAIL recombinant fusion protein (Novoprotein, C022) and 25µL diluted anti-TRAIL antibody were added to a 96-well plate containing HepG2 cells. The final concentrations of actinomycin D and TRAIL were 32ng/mL and 20ng/mL, respectively. The cells were cultured at 37°C, 5%CO₂ for24h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each anti-TRAIL antibody was calculated.

As shown in Table 12, anti-TRAIL chimeric antibodies G10, G45, G72 all had significant inhibitory effects on TRAIL antigen-induced apoptosis of Jurkat cells. The inhibition of TRAIL antigen-induced apoptosis of Jurkat cells by the above anti-TRAIL antibodies were significantly better than or comparable to the reference MAB375-500.

**Table 12: The inhibition of TRAIL antigen-induced apoptosis of Jurkat cells by the anti-TRAIL chimeric antibodies**

| **Antibody Name** | **IC₅₀(nM)** | **Antibody Name** | **IC₅₀(nM)** |
|---|---|---|---|
| **G10** | **0.2439** | **G72** | **0.2973** |
| **G45** | **0.4338** | **MAB375-500** | **0.4819** |

### 3.3 Determination of binding ability of anti-TRAIL antibody:

The constructed full-length anti-TRAIL chimeric antibody (reformatted as human IgG4) were subjected to binding assays with human TRAIL to reflect the binding activity of anti-TRAIL antibody and human TRAIL antigen. Briefly, the His-hTRAIL antigen was first dissolved in PBS and coated on 96-well plates with 0. 1µg/well followed by incubation at 4°C overnight. The 96-well plates were blocked with 5% milk for 1h at 37°C and washed with PBST for 6 times. Firstly, each antibody sample was diluted to 133nM, and then serially diluted at a ratio of 1:4. 100µL of serially diluted antibodies were added to each well and incubated for 1h at 37°C and washed with PBST for 6 times. 100µL of the anti-human IgG-HRP (Sigma, A8667, 1:10000 dilution) was added to each well and incubated for 1h at 37°C. After washing with PBST for 6 times, TMB (Beyotime, P0209-100mL) was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

As shown in Table 13, the selected anti-TRAIL chimeric antibodies G10, G45, G72 all had well binding ability to human TRAIL antigen.

**Table 13: The binding ability of anti-TRAIL chimeric antibody to human TRAIL**

| **Antibody Name** | **EC₅₀(nM)** |
|---|---|
| **G10** | **0.9942** |
| **G45** | **0.4694** |
| **G72** | **0.3728** |

In addition, the affinity of full-length anti-TRAIL chimeric antibodies (reformatted as human IgG4) with mouse and monkey TRAIL were also detected. Briefly, His-mouTRAIL (Sino biological, 50166-M07E) or CynoTRAIL (sino biological, 90098-CNAE) antigen was coated on ELISA plate followed by incubation at 4° C overnight. Each full-length anti-TRAIL chimeric antibodies was serially diluted and added into 96-well plates for incubation. After washing with PBST, anti-human IgG-HRP antibodies were added and incubates at room temperature. After washing with PBST for 6 times, TMB (Beyotime, P0209-100mL) was added and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

The results showed that anti-TRAIL chimeric antibody G10 had cross-reaction with monkey TRAIL antigen. G45 and G72 had cross-reacted with mouse and monkey TRAIL antigens (results not shown).

### Example 4: Humanization of Anti-TRAIL Antibodies

Based on the typical structure of V_{H}/V_{L} CDR of the mouse antibody G10 and G72 respectively, heavy and light chain variable domain sequences were aligned with the sequences in antibody Germline database to obtain human germline templates of high homology. The CDR regions of the mouse antibody were grafted into selected human germline templates to produce humanized variable regions, which were then recombined with corresponding human IgG constant regions (preferably IgG4 heavy chain and κ light chain). The embedded residues, the residues that interact directly with the CDR regions, and the residues that have an important effect on the conformation of V_{L} and V_{H} were then reverse-mutated based on the three-dimensional structure of the mouse antibody, meanwhile, the specific amino acids in the CDR region could be mutated, and different light chains and heavy chains can be designed and combined to obtain a series of humanized molecules.

Finally, humanized antibodies of G10 were obtained six, named G10-1~G10-6, respectively; humanized antibodies of G72 were obtained sixteen, named G72-1~G72-16, respectively.

### Example 5: Characterization of the humanized Anti-TRAIL Antibodies

Activity of the G10, G72 humanized anti-TRAIL antibodies were tested according to the corresponding protocol as described in example 3, including the determination of binding ability to human, mouse and monkey TRAIL antigens, and inhibition of TRAIL antigen induced apoptosis of HepG2 or Jurkat cells.

### 5.1 Humanized anti-TRAIL antibody inhibited TRAIL antigen-induced apoptosis of HepG2 or Jurkat cells:

Using the corresponding protocol described in Example 3.2, the activity of G10, G72 humanized anti-TRAIL antibodies (reformatted as human IgG4) to inhibit TRAIL antigen-induced apoptosis of HepG2 or Jurkat cells was determined.

As shown in Table 14, G10 humanized antibodies all had significant inhibitory effects on TRAIL antigen-induced apoptosis of HepG2 or Jurkat cells. Among them, the inhibition of TRAIL antigen-induced apoptosis of HepG2 or Jurkat cells by G10-1~G10-5 were better than the parental chimeric antibody G10, and better than the reference antibody MAB375-500 or comparable to sDR5-Fc (TRAIL antagonist, Shenzhen Institute of Advanced Technology).

**Table 14: The inhibition of TRAIL antigen-induced apoptosis by the G10 humanized antibodies**

| **Antibody Name** | **HepG2 IC₅₀(nM)** | **Jurkat IC₅₀(nM)** |
|---|---|---|
| **G10-1** | **0.0435** | **0.1806** |
| **G10-2** | **0.0443** | **0.2527** |
| **G10-3** | **0.06311** | **0.2068** |
| **G10-4** | **0.05707** | **0.1457** |
| **G10-5** | **0.05119** | **0.2014** |
| **G10** | **0.07263** | **0.3228** |
| **sDR5-Fc** | **0.07735** | **0.2998** |
| **MAB375-500** | **0.4025** | **0.6987** |

As shown in Table 15, G72 humanized antibodies all had significant inhibitory effects on TRAIL antigen-induced apoptosis of HepG2 or Jurkat cells. Among them, the inhibition of TRAIL antigen-induced apoptosis of HepG2 by G72-1 ~ G72-15 were comparable to the parental chimeric antibody G72, TRAIL antagonist sDR5-Fc, and better than the reference antibody MAB375-500. Except for G72-7, G72-9 and G72-13, the inhibition of TRAIL antigen-induced apoptosis of Jurkat cells by other humanized antibodies were better than or comparable to the parental chimeric antibody G72, TRAIL antagonist sDR5-Fc or the reference antibody MAB375-500, while the inhibition of TRAIL antigen-induced apoptosis of Jurkat cells by G72-7, G72-9 and G72-13 were comparable to the reference antibody MAB375-500.

**Table 15: The inhibition of TRAIL antigen-induced apoptosis by the G72 humanized antibodies**

| **Antibody Name** | **HepG2 IC₅₀(nM)** | **Jurkat IC₅₀(nM)** |
|---|---|---|
| **G72-1** | **0.09044** | **0.192** |
| **G72-2** | **0.1121** | **0.2583** |
| **G72-3** | **0.07541** | **0.3543** |
| **G72-4** | **0.08602** | **0.5642** |
| **G72-5** | **0.1112** | **0.05223** |
| **G72-6** | **0.08978** | **0.5938** |
| **G72-7** | **0.1157** | **1.195** |
| **G72-8** | **0.1097** | **0.3934** |
| **G72-9** | **0.1733** | **1.258** |
| **G72-10** | **0.09007** | **0.3166** |
| **G72-11** | **0.08714** | **0.4088** |
| **G72-12** | **0.08374** | **0.3417** |
| **G72-13** | **0.07597** | **1.016** |
| **G72-14** | **0.07018** | **/** |
| **G72-15** | **0.0806** | **/** |
| **G72** | **0.0986** | **0.4425** |
| **sDR5-Fc** | **0.0627** | **0.3556** |
| **MAB375-500** | **0.3533** | **0.7123** |

| | | |
|---|---|---|
| **Note:** "/" **indicates that the relevant data is not detected (the same below).** | | |

### 5.2 Determination of binding ability of humanized anti-TRAIL antibody:

### 5.2.1 ELISA binding assay:

Using the corresponding protocol described in Example 3.3, the binding activity of G10, G72 humanized anti-TRAIL antibodies (reformatted as human IgG4) to mouse or monkey TRAIL antigen was determined.

As shown in Table 16-1 and 16-2, G10, G72 humanized antibodies all had well binding ability to human TRAIL antigen. Among them, the binding ability of G10-1~G10-4 to human TRAIL antigen were comparable to the parental chimeric antibody G10, but better than TRAIL antagonist sDR5-Fc and the reference antibody MAB375-500. Among the G72 humanized antibodies, the binding ability of G72-1 to human TRAIL antigen was comparable to the parental chimeric antibody G72, TRAIL antagonist sDR5-Fc and reference antibody MAB375-500, the binding ability of other G72 humanized antibodies to human TRAIL antigen were better than the parental chimeric antibody G72, TRAIL antagonist sDR5-Fc and reference antibody MAB375-500.

**Table 16-1: The binding ability of G10 humanized antibodies to human TRAIL**

| **Antibody Name** | **EC₅₀(nM)** | **Antibody Name** | **EC₅₀(nM)** |
|---|---|---|---|
| **G10-1** | **0.6787** | **G10** | **0.5662** |
| **G10-2** | **0.4705** | **sDR5-Fc** | **3.441** |
| **G10-3** | **0.4813** | **MAB375-500** | **2.878** |
| **G10-4** | **0.6405** | | |

**Table 16-2: The binding ability of G72 humanized antibodies to human TRAIL**

| **Antibody Name** | **EC₅₀(nM)** | **Antibody Name** | **EC₅₀(nM)** |
|---|---|---|---|
| **G72-1** | **2.696** | **G72-14** | **0.8991** |
| **G72-2** | **1.572** | **G72-15** | **0.8224** |
| **G72-3** | **1.629** | **G72** | **2.336** |
| **G72-8** | **1.452** | **sDR5-Fc** | **3.017** |
| **G72-10** | **0.8877** | **MAB375-500** | **2.916** |
| **G72-11** | **0.9128** | | |
| **G72-12** | **0.8088** | | |

In addition, the affinity of G10, G72 humanized antibodies (reformatted as human IgG4) with mouse and monkey TRAIL were also detected. The results showed that G10 humanized antibodies had cross-reaction with monkey TRAIL antigen. G72 humanized antibodies had cross-reacted with mouse and monkey TRAIL antigens. Among them, the binding ability of G10 humanized antibodies to monkey TRAIL antigen were comparable to the parental chimeric antibody G10, the binding ability of some G72 humanized antibodies to mouse or monkey TRAIL antigen were increased, which were better than the parental chimeric antibody G72 (results not shown).

### 5.2.2 Characterization of the binding affinity and dissociation constant (Kd) of the humanized antibodies:

The binding affinity of the full length anti-TRAIL chimeric antibodies (reformatted as human IgG4) with human, mouse and monkey were characterized using Biacore T200 (GE). Anti-human Fc antibodies were stabilized on sensor chip CM5. G10, G72 and their humanized antibodies were diluted to 2 µg/ml and added into the channel to flow through and captured by the chip, with the capture time of 40 s and the flow rate of 30µL/min. His-hTRAIL prepared in example 1 and CynoTRAIL (sino biological, 90098-CNAE) antigen were diluted with 1×HBS-EP buffer (pH 7.4) and each antigen concentration range included 0M, 1.343E-10M, 2.685E-10M, 5.37E-10M, 1.074E-9M, 2.148E-9M, 4.296E-9M, 8.592E-9M, 1.718E-8M. The binding time, the dissociation time and the flow rate were set to 120s, 1200s, 30µL/min, respectively. Using the SPR technology, the association and dissociation rates were measured, and binding affinity was determined. Table 17 lists the Kon, Koff, and Kd values of G10, G72, and their exemplary humanized antibodies with human, mouse, and monkey TRAIL antigens.

As shown in Table 17, G10 and its exemplary humanized antibodies G10-1 and G10-2 could bind to human and monkey TRAIL antigens, and G72 and its exemplary humanized antibodies G72-10 and G72-11 could bind to human, mouse and monkey TRAIL antigens. This result was consistent with the conclusion of ELISA binding assay in 3.3 and 5.2.1. Moreover, the binding ability of G10-1, G10-2 to human, monkey TRAIL antigen were comparable to the parental chimeric antibody G10. The binding ability of G72-10, G72-11 to human, monkey TRAIL antigen was comparable to the parental chimeric antibody G72, while the binding ability of G72-10, G72-11 to mouse TRAIL antigen were better than the parental chimeric antibody G72, and the binding ability of G10, G72 and their humanized antibodies to TRAIL were better than the reference antibody MAB375-500.

**Table17: Characterization of antibodies Kon, Koff, and Kd values**

| **Antibody** | **Antigen** | **Kon (1/Ms)** | **Koff (1/s)** | **Kd (M)** |
|---|---|---|---|---|
| G10-1 | His-hTRAIL | 4.338E+05 | 1.690E-04 | 3.896E-10 |
| G10-2 | | 3.857E+05 | 1.822E-04 | 4.725E-10 |
| G10 | | 6.691E+05 | 1.651E-04 | 2.467E-10 |
| G72-10 | | 1.153E+06 | 1.809E-04 | 1.569E-10 |
| G72-11 | | 1.292E+06 | 1.805E-04 | 1.396E-10 |
| G72 | | 1.043E+06 | 1.805E-04 | 1.730E-10 |
| MAB375-500 | | 5.204E+04 | 1.152E-04 | 4.135E-09 |
| | | | | |

| **Antibody** | **Antigen** | **Kon (1/Ms)** | **Koff (1/s)** | **Kd (M)** |
|---|---|---|---|---|
| G10-1 | CynoTRAIL | 6.855E+06 | 8.385E-04 | 1.223E-11 |
| G10-2 | | 8.662E+06 | 1.252E-04 | 1.445E-11 |
| G10 | | 8.435E+06 | 1.021E-04 | 1.120E-11 |
| G72-10 | | 7.029E+06 | 1.296E-04 | 1.843E-11 |
| G72-11 | | 8.658E+06 | 1.624E-04 | 1.876E-11 |
| G72 | | 4.947E+06 | 1.226E-04 | 2.478E-11 |
| MAB375-500 | | 1.668E+06 | 3.006E-04 | 1.806E-10 |
| | | | | |

| **Antibody** | **Antigen** | **Kon (1/Ms)** | **Koff (1/s)** | **Kd (M)** |
|---|---|---|---|---|
| G10-1 | His-mouTRATT | / | / | / |
| G10-2 | | / | / | / |
| G10 | | / | / | / |
| G72-10 | | 4.374E+05 | 3.738E-04 | 8.544E-10 |
| G72-11 | | 1.112E+06 | 5.766E-04 | 5.187E-10 |
| G72 | | 9.143E+04 | 2.859E-03 | 3.127E-09 |
| MAB375-500 | | / | / | / |

### 5.3 Anti-trail antibody inhibited killing of HepG2 cells by PBMC:

PBMC cells contain killer T cells and killer NK cells. Therefore, PBMC cells could be used to mimic the natural blood immune environment in vivo. Plant hemagglutinin (PHA-P) could activate PBMC cells. In the presence of PHA-P, PBMC co-cultured with HepG2 cells could activate TRAIL-DR4/5 mediated apoptosis pathway in HepG2 cells and killed it. Based on the above experimental principles, the degree of apoptosis of HepG2 cells could be determined by detecting the amount of activated Caspase3/7, and then the inhibiting activity of humanized antibodies of G10 and G72 (reformatted as human IgG4) on killing of HepG2 cells by PBMC were determined.

HepG2 cells (2×10⁴ cells/well) and PBMC cells (7.5×10⁴ cells/well) isolated from human fresh blood (donated by volunteers) were seeded into 96-well plates, and PHA-P (Sigma, L8754) and IL2 (SL pharma, S19991009) were added simultaneously. The final concentrations of PHA-P and IL2 were 10 µg/ml and 100 U/ml, respectively. Subsequently, anti-TRAIL humanized antibody with a final concentration of 333nM was added to the wells of the experimental group, while the control group was set up. The anti-TRAIL antibody was not added into the non-antibody group, and it was used as the negative control group. sDR5-Fc had the function of antagonizing TRAIL signaling pathway, and the group supplemented with sDR5-Fc recombinant protein was used as positive control group. Cultured at 37°C, 5%CO₂ for 4.5 h. Caspase-Glo^{®} 3/7 reagent was added into the 96-well plate with 50µL/well, and incubated for 10min. The fluorescence values were read using a microplate reader.

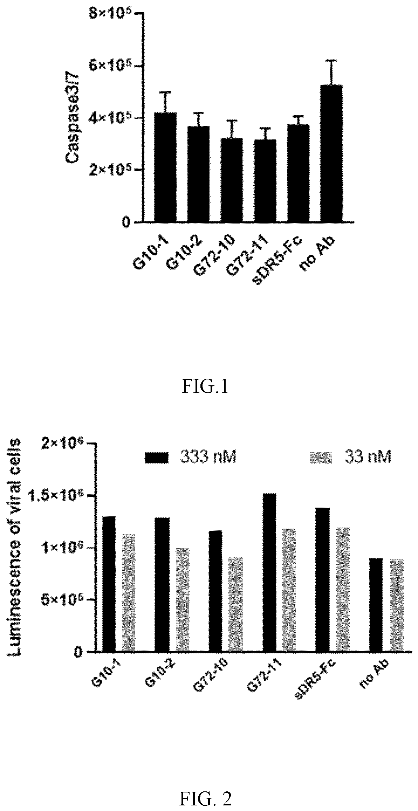

The results of the exemplary humanized antibody were shown in Figure 1, G10-1, G10-2, G72-10 and G72-11 could effectively inhibit killing of HepG2 cells by PBMC, and the inhibiting activity of G10-1, G10-2, G72-10 and G72-11 on killing of HepG2 cells by PBMC was comparable to the reference sDR5-Fc.

### 5.4 Anti-trail antibody inhibited killing of HepG2 cells by granulocytes:

Acetaminophen (APAP) could upregulate the expression of DR5 in hepatocytes, thereby increasing their sensitivity to TRAIL induced cell apoptosis signals. When co-culturing TRAIL expressing granulocytes with HepG2 cells in the presence of APAP, granulocytes killed HepG2 cells by activating the TRAIL-DR4/5-mediated apoptosis pathway in HepG2 cells. The amount of ATP in cells could reflect the viability of cells, which was inversely proportional to the degree of apoptosis. Based on the above principles, the amount of ATP could be detected by Cell Titer-Glo^{®} luminescent cell viability assay kit, and then the inhibiting activity of humanized antibodies of G10 and G72 (reformatted as human IgG4) on killing of HepG2 cells by granulocytes were determined.

HepG2 cells (2×10⁴ cells/well) were seeded into 96-well plates, and APAP (Aladdin, A105810) and actinomycin D were added simultaneously. The final concentrations of APAP and actinomycin D were 10mM and 1µg/ml, respectively. Cultured at 37°C, 5%CO₂ for 24 h. Granulocytes (1.5×10⁵ cells/well) isolated from human fresh blood (donated by volunteers) were seeded into 96-well plates. Subsequently, anti-TRAIL humanized antibody was added into the wells of the experimental group (two concentration gradients were set up to make the final concentration of 33nm and 333nm respectively). While the control group was set up. The anti-TRAIL antibody was not added into the non-antibody group, and it was used as the negative control group and the group supplemented with sDR5-Fc recombinant protein was used as positive control group. Cultured at 37°C, 5%CO₂ for 24h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader.

The results of the exemplary antibody were shown in Figure 2, G10-1, G10-2, G72-10 and G72-11 could effectively inhibit killing of HepG2 cells by granulocytes, and the inhibiting activity of G10-1, G10-2, G72-10 and G72-11 on killing of HepG2 cells by granulocytes was comparable to the reference sDR5-Fc.

### Example 6: Role of anti-TRAIL antibody in a mouse model of APAP-induced liver injury

Acetaminophen (APAP) was metabolized through the liver, and its metabolite could form protein adducts with human proteins in hepatocytes; It could also consume glutathione in the body, causing oxidative stress and cytotoxicity. It was a common drug that causes liver damage. The liver cell death directly caused by APAP could lead to innate immune activation or simultaneously trigger adaptive immune activation. Large numbers of immune cells expressed FASL/TRAIL, which in turn promoted more liver cell death. When liver cells died, they released Alanine transaminase (ALT) into the blood, which raised the level of ALT in the blood. Therefore, ALT was an important indicator reflecting liver injury.

Twenty female BALB/C mice (Beijing Vital River Laboratory Animal Technology Co., Ltd., aged 6-8 weeks) were randomly divided into different groups, the experimental group consisted of model control group (no antibody injection group/no antibody group), low-dose group and high-dose group, with 6 mice in each group, and simultaneously set up a blank control group, with 2 mice in each group. Firstly, all the mice were fasted, but water was allowed. After fasting for about 16h, mice in each experimental group were intraperitoneally injected with 200 mg kg APAP (Aladdin, A105808). When injecting APAP for 30 minutes, anti-TRAIL antibody (using G72 as an example) was injected into each experimental group of mice through the tail vein, with a dosage of 10mg/kg in the low-dose group and 20mg/kg in the high-dose group, meanwhile, the mice of model control group were only given an equal amount of normal saline. When injecting APAP for 24 hours, 200µL of blood was taken from the orbital venous plexus of mice and centrifuged at 1500g at room temperature for 10 min. The serum was collected and ALT level was detected with ALT Detection Kit (Nanjing Jiancheng, C009-2-1).

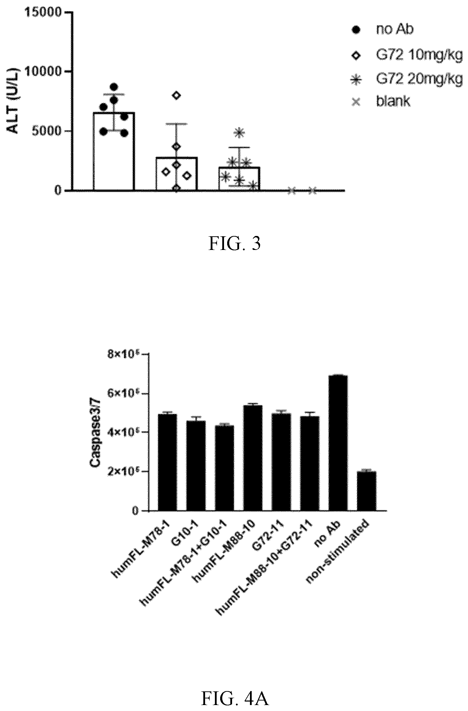

As shown in Figure 3, the exemplary anti-TRAIL antibody G72 significantly inhibited APAP-induced ALT elevation in a dose-dependent manner. It was suggested that anti-TRAIL antibody of this application could play a role in the treatment of liver injury.

### Example 7: Combination of antibody specifically binding to TRAIL and antibody specifically binding to FasL

### 7.1 Combination of antibody specifically binding to TRAIL and antibody specifically binding to FasL in inhibiting the killing of HepG2 cells by PBMC:

PBMC cells contain killer T cells and killer NK cells. Therefore, PBMC cells could be used to mimic the natural blood immune environment in vivo. Plant hemagglutinin (PHA-P) could activate PBMC cells. In the presence of PHA-P, PBMC co-cultured with HepG2 cells could activate TRAIL-DR4/5 mediated apoptosis pathway in HepG2 cells and killed it. Based on the above experimental principles, the degree of apoptosis of HepG2 cells could be determined by detecting the amount of activated Caspase3/7, and then the inhibitory effect of combination of antibody specifically binding to TRAIL and antibody specifically binding to FasL on killing of HepG2 cells by PBMC were determined.

HepG2 cells (2×10⁴ cells/well) and PBMC cells (3.75×10⁴ cells/well) isolated from human fresh blood (donated by volunteers) were seeded into 96-well plates, and PHA-P and IL2 were added simultaneously. The final concentrations of PHA-P and IL2 were 10 µg/ml and 100 U/ml, respectively. Subsequently, anti-TRAIL antibody and/or anti-FasL antibody with a final concentration of 333nM were added to the wells of the experimental group, while the control group was set up, wherein only antibody was not added into the non-antibody group, and PHA-P and antibody was not added into the non-stimulation group. Cultured at 37°C, 5%CO₂ for 18h. Caspase-Glo^{®} 3/7 reagent was added into the 96-well plate with 50µL/well, and incubated for 10min. The fluorescence values were read using a microplate reader.

The results of the exemplary antibody were shown in Figure 4A, anti-TRAIL antibody G10-1, G72-11 or anti-FasL antibody humFL-M78-1, humFL-M88-10 could effectively inhibit killing of HepG2 cells by PBMC. When combined with anti-TRAIL antibody and anti-FasL antibody, the effect of inhibiting the killing of HepG2 cells by PBMC was enhanced, these results suggested that antibody specifically binding to TRAIL and antibody specifically binding to FasL had synergistic effect in inhibiting the killing of HepG2 cells by PBMC.

### 7.2 Combination of antibody specifically binding to TRAIL and antibody specifically binding to FasL in inhibiting the killing of HepG2 cells by Jurkat cells:

The experimental principle was the same as above, the degree of apoptosis of Jurkat cells could be determined by detecting the amount of activated Caspase3/7, and then the inhibitory effect of combination of antibody specifically binding to TRAIL and antibody specifically binding to FasL on killing of Jurkat cells by PBMC were determined.

Jurkat cells (2.5×10⁴ cells/well) and PBMC cells (3.75×10⁴ cells/well) isolated from human fresh blood (donated by volunteers) were seeded into 96-well plates, and PHA-P and IL2 were added simultaneously. The final concentrations of PHA-P and IL2 were 10 µg/ml and 100 U/ml, respectively. Subsequently, anti-TRAIL antibody and/or anti-FasL antibody with a final concentration of 333nM were added to the wells of the experimental group, while the control group was set up, wherein only antibody was not added into the non-antibody group, and PHA-P and antibody was not added into the non-stimulation group. Cultured at 37°C, 5%CO₂ for 18h. Caspase-Glo^{®} 3/7 reagent was added into the 96-well plate with 50µL/well, and incubated for 10min. The fluorescence values were read using a microplate reader.

The results of the exemplary antibody were shown in Figure 4B, anti-TRAIL antibody G10-1, G72-11 or anti-FasL antibody humFL-M78-1, humFL-M88-10 could effectively inhibit killing of Jurkat cells by PBMC. When combined with anti-TRAIL antibody and anti-FasL antibody, the effect of inhibiting the killing of Jurkat cells by PBMC was enhanced, these results suggested that antibody specifically binding to TRAIL and antibody specifically binding to FasL had synergistic effect in inhibiting the killing of Jurkat cells by PBMC.

### Example 8: Preparation of bispecific antibodies specifically binding to TRAIL and FasL with different formats

In the following sequence design: V_{H} and V_{L} represented the heavy chain variable domain and the light chain variable domain of an antibody, respectively; C_{H} represented the antibody heavy chain constant domain, including C_{H}1, C_{H}2 and C_{H}3 domain; scFv was an antibody formed by linking V_{H} and V_{L} of the antibody through a peptide linker (linker); IgG1Fc represented the Fc region of an IgG1 subclass antibody, comprising C_{H}2 and C_{H}3 domain; C_{L} represented the light chain constant domain.

### 8.1 Construction of DVD-Ig format bispecific antibodies:

1) Sequence design: DVD-Ig (Dual-variable domain-Ig) bispecific antibody, the structure of which is connecting the V_{L} and V_{H} domains of another antibody respectively to the N-terminus of the V_{L} and V_{H} of a normal IgG antibody, forming an antigen-binding domain by the interaction between V_{H} and V_{L} of two antibodies, which can simultaneously bind to the corresponding antigen to achieve bispecific. DVD-Ig format bispecific antibodies were described in literature Wu C, et al. Molecular construction and optimization of anti-human IL-1alpha/beta dual variable domain immunoglobulin (DVD-Ig) molecules. MAbs. 2009 Jul-Aug;1(4):339-47. The schematic diagram was shown in FIG. 5A. Table 18-1 showed the composition design of exemplary DVD-Ig format bispecific antibodies used in the embodiments, and the specific amino acid sequences of the heavy chains and light chains thereof were shown in Table 8-1. Genscript Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: after cleavage by restriction endonuclease, the genes encoding light chains and heavy chains of bispecific antibodies were subcloned into pTTα1 vector, respectively. Recombinant plasmids expressing light chains and heavy chains of the antibodies were extracted and co-transfected into 293F cells. Cultured at 37 °C, 5% CO₂, 120rpm for 7 days, culture solution was purified by protein A affinity chromatography column. Briefly, the protein A column was first equilibrated with 6× column volumes of 50mM PBS buffer (containing 0.15M NaCl, pH 7.2) with a flow rate of 150 cm/h. The culture supernatant, whose pH was adjusted to 7.2, flowed through the cartridges with a flow rate of 150cm/h. After loading, the column was equilibrated again and finally eluted with 50mM citrate-sodium citrate buffer (pH 3.5) and the eluent was collected. The protein was concentrated with 30kDa ultrafiltration tube and the buffer was replaced with PBS. After endotoxin removal by endotoxin removal kit (SA 031K, Changzhou Smart Lifesciences), protein concentration and endotoxin content were measured, respectively. The endotoxin-removed bispecific antibody (DVD-Ig format) samples specifically binding to TRAIL and FasL were subjected to reduced and non-reduced SDS-PAGE electrophoresis detection, respectively (results not shown), to ensure correct formation of structures.

**Table 18-1: Composition design of heavy chains and light chains of DVD-Ig format bispecific antibodies specifically binding to TRAIL and FasL**

| **The form of bispecific antibody** | **Antibody name** | **Heavy chain** | **Light chain** |
|---|---|---|---|
| **DVD-Ig** | M78-1-G10-1-DVD-IgG4 | humFL-M78-1V_{H}-G10-1V_{H}-IgG4 C_{H} | humFL-M78-1V_{L}- G10-1V_{L}-C_{L} |
| | M78-1-G10-2-DVD-IgG4 | humFL-M78-1V_{H}-G10-2V_{H}-IgG4C_{H} | humFL-M78-1V_{L}- G10-2V_{L}-C_{L} |
| | G10-1-M78-1-DVD-IgG4 | G10-1V_{H}-humFL-M78-1V_{H}-IgG4C_{H} | G10-1V_{L}-humFL-M78-1V_{L}-C_{L} |
| | G10-2-M78-1-DVD-IgG4 | G10-2V_{H}-humFL-M78-1V_{H}-IgG4C_{H} | G10-2V_{L}-humFL-M78-1V_{L}-C_{L} |
| | M88-15-G72-11-DVD-IgG4 | humFL-M88-15V_{H}-G72-11V_{H}- IgG4C_{H} | humFL-M88-15V_{L}-G72-11V_{L}-C_{L} |
| | M88-19-G72-16-DVD-IgG4 | humFL-M88-19V_{H}-G72-16V_{H}- IgG4C_{H} | humFL-M88-19V_{L}-G72-16V_{L}-C_{L} |
| | M88-20-G72-16-DVD-IgG4 | humFL-M88-20V_{H}-G72-16V_{H}- IgG4C_{H} | humFL-M88-20V_{L}-G72-16V_{L}-C_{L} |
| | M88-15-G72-16-DVD-IgG4 | humFL-M88-15V_{H}-G72-16V_{H}- IgG4C_{H} | humFL-M88-15V_{L}-G72-16V_{L}-C_{L} |

### 8.2 Construction of Bs4Ab format bispecific antibodies:

1) Sequence design: Bs4Ab format bispecific antibody contain a full-length IgG1 structure, the structure of which achieves bispecific by insertion of another binding unit scFv in its hinge region. Bs4Ab format bispecific antibodies were described in literature Bezabeh B, et al. Insertion of scFv into the hinge domain of full-length IgG1 monoclonal antibody results in tetravalent bispecific molecule with robust properties. MAbs. 2017 Feb/Mar;9(2):240-256. The schematic diagram was shown in FIG. 5B. Table 18-2 showed the composition design of exemplary Bs4Ab format bispecific antibodies used in the embodiments, and the specific amino acid sequences of the heavy chains and light chains thereof were shown in Table 8-2. Genscript Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: the specific operation was as described in 8.1.

**Table 18-2: Composition design of heavy chains and light chains of Bs4Ab format bispecific antibodies specifically binding to TRAIL and FasL**

| **The form of bispecific antibody** | **Antibody name** | **Heavy chain** | **Light chain** |
|---|---|---|---|
| **Bs4Ab** | M88-G72scFv-Bs4Ab-IgG1 | FL-M88V_{H}-C_{H}1-G72scFv-IgG1Fc | FL-M88V_{L}-C_{L} |
| | M88-G72scFv-Bs4Ab-IgG4 | FL-M88V_{H}-C_{H}1-G72scFv-IgG4Fc | FL-M88V_{L}-C_{L} |
| | G72-M88scFv-Bs4Ab-IgG1 | G72V_{H}-C_{H}1- FL-M88scFv-IgG1Fc | G72V_{L}-C_{L} |
| | G72-M88scFv-Bs4Ab-IgG4 | G72V_{H}-C_{H}1- FL-M88scFv-IgG4Fc | G72V_{L}-C_{L} |
| | M78-1-G10-1scFv-Bs4Ab-IgG4 | humFL-M78-1V_{H}-C_{H}1-G10-1scFv-IgG4Fc | humFL-M78-1V_{L}-C_{L} |
| | M78-1-G10-1scFv-2-Bs4Ab-IgG4 | humFL-M78-1V_{H}-C_{H}1-G10-1scFv-2-IgG4Fc | humFL-M78-1V_{L}-C_{L} |
| | M78-1-G10-2scFv-Bs4Ab-IgG4 | humFL-M78-1V_{H}-C_{H}1-G10-2scFv-IgG4Fc | humFL-M78-1V_{L}-C_{L} |
| | G10-1-M78-1scFv-Bs4Ab-IgG4 | G10-1V_{H}-C_{H}1-humFL-M78-1scFv-IgG4Fc | G10-1V_{L}-C_{L} |
| | G10-2-M78-1scFv-Bs4Ab-IgG4 | G10-2V_{H}-C_{H}1-humFL-M78-1scFv-IgG4Fc | G10-2V_{L}-C_{L} |
| | G72-11-M88scFv-Bs4Ab-IgG4 | G72-11V_{H}-C_{H}1-FL-M88scFv-IgG4Fc | G72-11V_{L}-C_{L} |
| | M78-6-G10-1scFv-Bs4Ab-IgG4 | humFL-M78-6V_{H}-C_{H}1-G10-1scFv-IgG4Fc | humFL-M78-6V_{L}-C_{L} |
| | M78-5-G10-1scFv-Bs4Ab-IgG4 | humFL-M78-5V_{H}-C_{H}1-G10-1scFv-IgG4Fc | humFL-M78-5V_{L}-C_{L} |
| | M78-7-G10-1scFv-Bs4Ab-IgG4 | humFL-M78-7V_{H}-C_{H}1-G10-1scFv-IgG4Fc | humFL-M78-7V_{L}-C_{L} |
| | M78-8-G10-1scFv-Bs4Ab-IgG4 | humFL-M78-8V_{H}-C_{H}1-G10-1scFv-IgG4Fc | humFL-M78-8V_{L}-C_{L} |
| | M78-9-G10-1scFv-Bs4Ab-IgG4 | humFL-M78-9V_{H}-C_{H}1-G10-1scFv-IgG4Fc | humFL-M78-9V_{L}-C_{L} |
| | M78-1-G10-6scFv-Bs4Ab-IgG4 | humFL-M78-1V_{H}-C_{H}1-G10-6scFv-IgG4Fc | humFL-M78-1V_{L}-C_{L} |
| | M78-1-G10-7scFv-Bs4Ab-IgG4 | humFL-M78-1V_{H}-C_{H}1-G10-7scFv-IgG4Fc | humFL-M78-1V_{L}-C_{L} |
| | M78-5-G10-6scFv-Bs4Ab-IgG4 | humFL-M78-5V_{H}-C_{H}1-G10-6scFv-IgG4Fc | humFL-M78-5V_{L}-C_{L} |
| | M78-10-G10-7scFv-Bs4Ab-IgG4 | humFL-M78-10V_{H}-C_{H}1-G10-7scFv-IgG4Fc | humFL-M78-10V_{L}-C_{L} |
| | G72-16-M88-22scFv-Bs4Ab-IgG1 | G72-16V_{H}-C_{H}1- FL-M88-22scFv-IgG1Fc | G72-16V_{L}-C_{L} |
| | G72-16-M88-21scFv-Bs4Ab-IgG1 | G72-16V_{H}-C_{H}1- FL-M88-21scFv-IgG1Fc | G72-16V_{L}-C_{L} |
| | G72-16-M88scFv-Bs4Ab-IgG1 | G72-16V_{H}-C_{H}1- FL-M88scFv-IgG1Fc | G72-16V_{L}-C_{L} |
| | G72-17-M88scFv-Bs4Ab-IgG1 | G72-17V_{H}-C_{H}1- FL-M88scFv-IgG1Fc | G72-17V_{L}-C_{L} |
| | G72-17-M88-23scFv-Bs4Ab-IgG1 | G72-17V_{H}-C_{H}1- FL-M88-23scFv-IgG1Fc | G72-17V_{L}-C_{L} |
| | G72-17-M88-24scFv-Bs4Ab-IgG1 | G72-17V_{H}-C_{H}1- FL-M88-24scFv-IgG1Fc | G72-17V_{L}-C_{L} |

### 8.3 Construction of Hetero H, CrossMab format bispecific antibodies:

1) Sequence design: Hetero H, CrossMab format bispecific antibody, that is, the knob-in-hole structure (KIH) is designed in the Fc region, while introducing two Cys residue mutations that form stable disulfide bridges (S354C on the "knob" side and Y349C on the "hole" side). By KIH method, *i.e.*, the threonine (T) at position 366 in the CH3 region of an antibody is replaced with tryptophan (W) to form a "Knobs" structure, and substitute threonine (T) at position 366 with serine (S), leucine (L) at position 368 with alanine (A), tyrosine (Y) at position 407 with valine (V) in the C_{H}3 region of the paired another antibody to form "holes" structure, which promotes dimerization of heterologous heavy chains by the decrease of steric hindrance effect after mutation and the formation of covalent disulfide bonds in the hinge region, wherein the numbering is according to EU numbering system of Kabat; the CrossMab technology is also applied to ensure proper pairing between the light chains and heavy chains of antibody. Cross MAb technology is based on the exchange of domains in one Fab arm of bispecific IgG antibodies, either as an exchange of intact Fab domains (CrossMAb Fab), or as an exchange of only variable regions in the Fab (CrossMAb V_{H}-V_{L}) or an exchange of only constant region (CrossMAb C_{H}1-C_{L}). bispecific antibodies with Hetero H, CrossMab format were described in literature Klein C, et al. The use of CrossMAb technology for the generation of bi- and multispecific antibodies. MAbs. 2016 Aug-Sep;8(6):1010-20. This application was explained in detail using CrossMab C_{H}1-C_{L} structure, the schematic diagram was shown in FIG. 5C. Table 18-3 showed the composition design of exemplary bispecific antibodies with Hetero H, CrossMab format s used in the embodiments, and the specific amino acid sequences of the heavy chains and light chains thereof were shown in Table 8-3. Genscript Biotechnology was entrusted to synthesize the genes encoding the light chain (L chain) and heavy chain (H chain) of bispecific antibodies, and coding genes were optimized to be suitable for expression in 293F cells.
2) Expression and purification of bispecific antibodies: the specific operation was as described in 8.1.

**Table 18-3: Composition design of heavy chains and light chains of Hetero H, CrossMab format bispecific antibodies specifically binding to TRAIL and FasL**

| **The form of bispecific antibody** | **Antibody name** | **Heavy chain** | **Light chain** |
|---|---|---|---|
| **Hetero H, CrossMab** | M88-G72-CrossMab-IgG1 | FL-M88V_{H}-C_{L}-IgG1Fc-hole | FL-M88V_{L}-C_{H}1 |
| | | G72V_{H}-IgG1C_{H}-knob | G72V_{L}-C_{L} |
| | M88-G72-CrossMab-IgG4 | FL-M88V_{H}-C_{L}-IgG4Fc-hole | FL-M88V_{L}-C_{H}1 |
| | | G72V_{H}- IgG4C_{H}-knob | G72V_{L}-C_{L} |

### Example 9: Determination of binding ability of bispecific antibodies:

The antigens used in this embodiment, of which His-hFasL, hFc-hFasL, hFc-cynoFasL, hFc-mouFasL, GST-hTRAIL, His-hTRAIL, GST-mouTRAIL were all prepared using the method described in Example 1; CynoTRAIL and His-mouTRAIL were purchased from sino biological Inc.

The antibodies G10-1, G72, G72-11 specifically binding to TRAIL, antibodies M88, M88-10, M78-1 specifically binding to FasL, 119-4A (anti-FasL antibody, Apogenix) or SDR5- Fc fusion protein were used as control.

### 9.1 The binding activity of bispecific antibodies to human TRAIL antigen and/or human FasL antigen

The binding of bispecific antibodies to human TRAIL antigen or human FasL antigen were detected by ELISA. Briefly, the GST-hTRAIL or His-hFasL antigen was dissolved in PBS, where His-hFasL antigen was coated on 96-well plates with 0. 1µg/well, GST-hTRAIL antigen was coated on 96-well plates with 0.2µg/well, followed by incubation at 4°C overnight. The 96-well plates were blocked with 5% milk for 1h at 37°C and washed with PBST for 6 times. When detecting the binding of antibodies to TRAIL antigen, each antibody sample was diluted to 2128nM, and then serially diluted at a ratio of 1:4, while when detecting the binding of antibodies to FasL antigen, each antibody sample was diluted to 523nM, and then serially diluted at a ratio of 1:4. 100µL of serially diluted antibodies were added to each well, incubated for 1h at 37°C and washed with PBST for 6 times. 100µL of the anti-human kappa-HRP (Southern Biotech, E1920-MJ11B, 1:4000 dilution) was added to each well and incubated for 1h at 37°C. After washing with PBST for 6 times, TMB was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

The binding of bispecific antibodies to human TRAIL antigen and human FasL antigen were detected by ELISA. Briefly, the hFc-hFasL antigen was dissolved in PBS and coated on 96-well plates with 0.2µg/well, followed by incubation at 4°C overnight. The 96-well plates were blocked with 5% milk for 1h at 37°C and washed with PBST for 6 times. Each antibody sample was diluted to 2128nM, and then serially diluted at a ratio of 1:4. 50µL of serially diluted antibodies were added to each well, incubated for 1h at 37°C and washed with PBST for 6 times. The second His-hTRAIL antigen was added into 96-well plates with 0.1µg/well and incubated at 37°C for 1h, followed by washing with PBST for 6 times. 100µL of the anti His-HRP (Beijing cwbiotech Co., Ltd., CW0285M, 1:4000 dilution) was added to each well and incubated for 1h at 37°C. After washing with PBST for 6 times, TMB was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

As shown in Table 19, the selected exemplary bispecific antibodies had good binding activity to human TRAIL antigen, human FasL antigen, and both human TRAIL and human FasL antigen simultaneously.

The binding activity of bispecific antibodies M88-G72-Crossmab-IgG1, M88-G72-Crossmab-IgG4, M88-G72scFv-Bs4Ab-IgG1, M88-G72scFv-Bs4Ab-IgG4, G72- M88scFv-Bs4Ab-IgG1, G72- M88scFv-Bs4Ab-IgG4 to human FasL antigen were better than the monoclonal antibodies FL-M88 and reference antibody 119-4A; The binding activity of the above-mentioned bispecific antibodies to human TRAIL antigen were better than the reference fusion protein sDR5 Fc, and better than or comparable to monoclonal antibody G72.

The binding activity of bispecific antibodies M78-1-G10-1scFv-Bs4Ab-IgG4, M78-1-G10-1scFv-2-Bs4Ab-IgG4, M78-1-G10-2scFv-Bs4Ab-IgG4, M78-1-G10-1-DVD-IgG4, M78-1-G10-2-DVD-IgG4, G10-1-M78-1-DVD-IgG4, G10-2-M78-1-DVD-IgG4, M88-15-G72-11-DVD-IgG4, G72-11-M88scFv-Bs4Ab-IgG4 to human FasL antigen were comparable to monoclonal antibody humFL-M78-1.

The binding activity of bispecific antibodies M78-1-G10-1scFv-Bs4Ab-IgG4, M78-1-G10-1scFv-2-Bs4Ab-IgG4, M78-1-G10-2scFv-Bs4Ab-IgG4, M78-1-G10-1-DVD-IgG4, M78-1-G10-2-DVD-IgG4 to human TRAIL antigen were better than or comparable to monoclonal antibody G10-1; the binding activity of bispecific antibodies M88-15-G72-11-DVD-IgG4, G72-11-M88scFv-Bs4Ab-IgG44 to human TRAIL antigen were better than monoclonal antibody G72-11.

**Table 19: The binding of bispecific antibodies to human TRAIL antigen and/or human FasL antigen**

| **Antibody Name** | **EC₅₀ (nM)** | | |
|---|---|---|---|
| | **His-hFasL** | **GST-hTRAIL** | **hFc-hFasL+His-hTRAIL** |
| **M88-G72-Crossmab-IgG1** | **0.1793** | **1.949** | **1.165** |
| **M88-G72-Crossmab-IgG4** | **0.3297** | **2.747** | **2.352** |
| **M88-G72scFv-Bs4Ab-IgG1** | **0.2171** | **1.471** | **1.334** |
| **M88-G72scFv-Bs4Ab-IgG4** | **0.3504** | **1.401** | **1.651** |
| **G72-M88scFv-Bs4Ab-IgG1** | **0.1659** | **0.324** | **2.375** |
| **G72-M88scFv-Bs4Ab-IgG4** | **0.2571** | **0.7195** | **1.19255** |
| **FL-M88** | **0.7049** | **/** | **/** |
| **119-4A** | **0.7355** | **/** | **/** |
| **G72** | **/** | **1.937** | **/** |
| **sDR5-Fc** | **/** | **3.02** | **/** |
| **M78-1-G10-1scFv-Bs4Ab-IgG4** | **0.1429** | **0.2539** | **0.2828** |
| **M78-1-G10-1scFv-2-Bs4Ab-IgG4** | **0.4089** | **0.4272** | **1.439** |
| **M78-1-G10-2scFv-Bs4Ab-IgG4** | **0.2596** | **0.1975** | **0.2924** |
| **G10-1-M78-1scFv-Bs4Ab-1gG4** | **1.022** | **/** | **0.261** |
| **G10-2-M78-1scFv-Bs4Ab-1gG4** | **0.4582** | ***0.1555*** | **0.2598** |
| **M78-1-G10-1-DVD-IgG4** | **0.2615** | **6.4** | **1.915** |
| **M78-1-G10-2-DVD-IgG4** | **0.2991** | **7.11** | **2.854** |
| **G10-1-M78-1-DVD-IgG4** | **4.618** | **0.1752** | **1.525** |
| **G10-2-M78-1-DVD-IgG4** | **4.231** | **0.1504** | **1.383** |
| **M88-15-G72-11-DVD-IgG4** | **0.2365** | **0.6535** | **1.686** |
| **G72-11-M88scFv-Bs4Ab-IgG4** | **0.3744** | **0.2402** | **0.2852** |
| **M88-19-G72-16-DVD-IgG4** | **1.127** | **0.4442** | **0.8047** |
| **M88-20-G72-16-DVD-IgG4** | **1.226** | **0.4471** | **0.7738** |
| **G72-16-M88-22scFv-Bs4Ab-IgG4** | **1.568** | **0.4038** | **0.5662** |
| **G72-16-M88-21scFv-Bs4Ab-IgG4** | **1.583** | **0.5983** | **0.6103** |
| **M88-15-G72-16-DVD-IgG4** | **1.643** | **0.7292** | **1.248** |
| **G72-16-M88scFv-Bs4Ab-IgG4** | **2.361** | **0.6998** | **1.224** |
| **G72-17-M88scFv-Bs4Ab-IgG4** | **2.422** | **0.7014** | **1.228** |
| **G72-17-M88-23scFv-Bs4Ab-IgG4** | **2.429** | **0.7014** | **1.234** |
| **G72-17-M88-24scFv-Bs4Ab-IgG4** | **2.441** | **0.7004** | **1.233** |
| **humFL-M78-1** | **0.143905** | **/** | **/** |
| **G10-1** | **/** | **0.4018** | **/** |
| **G72-11** | **/** | **1.612** | **/** |

### 9.2 The binding activity of bispecific antibodies to monkey TRAIL antigen and/or monkey FasL antigen

The binding of bispecific antibodies to monkey TRAIL antigen or monkey FasL antigen were detected by ELISA. Briefly, the cynoTRAIL or hFc-cynoFasL antigen was dissolved in PBS, where cynoTRAIL antigen was coated on 96-well plates with 0.1µg/well, hFc-cynoFasL antigen was coated on 96-well plates with 0.2µg/well, followed by incubation at 4°C overnight. The 96-well plates were blocked with 5% milk for 1h at 37°C and washed with PBST for 6 times. When detecting the binding of antibodies to monkey TRAIL antigen, each antibody sample was diluted to 2128nM, and then serially diluted at a ratio of 1:4, while when detecting the binding of antibodies to monkey FasL antigen, each antibody sample was diluted to 523nM, and then serially diluted at a ratio of 1:4. 100µL of serially diluted antibodies were added to each well, incubated for 1h at 37°C and washed with PBST for 6 times. 100µL of the anti-human kappa-HRP (Southern Biotech, E1920-MJ11B, 1:4000 dilution) was added to each well and incubated for 1h at 37°C. After washing with PBST for 6 times, TMB was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

The binding of bispecific antibodies to monkey TRAIL antigen and monkey FasL antigen were detected by ELISA. Briefly, the cynoTRAIL antigen was dissolved in PBS and coated on 96-well plates with 0.1µg/well, followed by incubation at 4°C overnight. The 96-well plates were blocked with 5% milk for 1h at 37°C and washed with PBST for 6 times. Each antibody sample was diluted to 2128nM, and then serially diluted at a ratio of 1:4. 50µL of serially diluted antibodies were added to each well, incubated for 1h at 37°C and washed with PBST for 6 times. The second hFc-cynoFasL antigen was added into 96-well plates with 0.2µg/well and incubated at 37°C for 1h, followed by washing with PBST for 6 times. 100µL of the anti-human IgG1 Fc-AP (Southernbiotech, 9054-04, 1:4000dilution) was added to each well and incubated for 1h at 37°C. After washing with PBST for 6 times, absorbance at 450nm was directly read using a microplate reader. The binding curves were generated by Graphpad Prism and the EC₅₀ of each anti-TRAIL antibody was calculated.

As shown in Table 20, the selected exemplary bispecific antibodies had good binding activity to monkey TRAIL antigen, monkey FasL antigen, and both monkey TRAIL and monkey FasL antigen simultaneously.

The binding activity of bispecific antibodies M78-1-G10-1scFv-Bs4Ab-IgG4, M78-1-G10-1scFv-2-Bs4Ab-IgG4 to monkey FasL antigen were comparable to the monoclonal antibodies humFL-M78-1, the binding activity of M88-15-G72-11-DVD-IgG4, G72-11-M88scFv-Bs4Ab-IgG4, G72- M88scFv-Bs4Ab-IgG4 to monkey FasL antigen were comparable to the monoclonal antibodies humFL-M88-10.

The binding activity of bispecific antibodiesM78-1-G10-1scFv-Bs4Ab-IgG4, M78-1-G10-1scFv-2-Bs4Ab-IgG4, G10-2-M78-1scFv-Bs4Ab-IgG to monkey TRAIL antigen were comparable to monoclonal antibody G10-1, the binding activity of G72-11-M88scFv-Bs4Ab-IgG4, G72- M88scFv-Bs4Ab-IgG4 to monkey TRAIL antigen were comparable to monoclonal antibody G72-11.

**Table 20: The binding of bispecific antibodies to monkey TRAIL antigen and/or monkey FasL antigen**

| **Antibody Name** | **EC₅₀ (nM)** | | |
|---|---|---|---|
| | **hFc-cynoFasL** | **cynoTRAI L** | **cynoTRAIL+hFc-cynoFasL** |
| **M78-1-G10-1scFv-Bs4Ab-IgG4** | **0.1164** | **0.269** | **1.174** |
| **M78-1-G10-1scFv-2-Bs4Ab-IgG4** | **0.08943** | **0.3704** | **/** |
| **G10-2-M78-1scFv-Bs4Ab-IgG4** | **0.5516** | **0.6466** | **1.013** |
| **M88-15-G72-11-DVD-IgG4** | **0.18** | **2.346** | **10.29** |
| **G72-11-M88scFv-Bs4Ab-IgG4** | **0.2287** | **0.3814** | **1.101** |
| **G72-M88scFv-Bs4Ab-IgG4** | **0.274** | **0.4295** | **1.039** |
| **M88-19-G72-16-DVD-IgG4** | **0.7462** | **3.498** | **32.99** |
| **M88-20-G72-16-DVD-IgG4** | **0.7214** | **5.122** | **34.14** |
| **G72-16-M88-22scFv-Bs4Ab-IgG4** | **0.7992** | **0.8626** | **0.6628** |
| **G72-16-M88-21scFv-Bs4Ab-IgG4** | **0.7618** | **0.6867** | **0.5583** |
| **humFL-M78-1** | **0.0907** | **/** | **/** |
| **humFL-M88-10** | **0.2517** | **/** | **/** |
| **G10-1** | **/** | **0.2598** | **/** |
| **G72-11** | **/** | **0.3349** | **/** |

### 9.3 The binding activity of bispecific antibodies to mouse TRAIL antigen and/or mouse FasL antigen

The binding of bispecific antibodies to mouse TRAIL antigen or mouse FasL antigen were detected by ELISA. Briefly, the hFc-mouFasL or GST-mouTRAIL antigen was dissolved in PBS and coated on 96-well plates with 0.2µg/well, followed by incubation at 4°C overnight. The 96-well plates were blocked with 5% milk for 1h at 37°C and washed with PBST for 6 times. When detecting the binding of antibodies to mouse FasL or TRAIL antigen, each antibody sample was diluted to 2128nM, and then serially diluted at a ratio of 1:4. 100µL of serially diluted antibodies were added to each well, incubated for 1h at 37°C and washed with PBST for 6 times. 100µL of the anti-human kappa-HRP (Southern Biotech, E1920-MJ11B, 1:4000 dilution) was added to each well and incubated for 1h at 37°C. After washing with PBST for 6 times, TMB was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

The binding of bispecific antibodies to mouse TRAIL antigen and mouse FasL antigen were detected by ELISA. Briefly, the hFc-mouFasL antigen was dissolved in PBS and coated on 96-well plates with 0.2µg/well, followed by incubation at 4°C overnight. The 96-well plates were blocked with 5% milk for 1h at 37°C and washed with PBST for 6 times. Each antibody sample was diluted to 2128nM, and then serially diluted at a ratio of 1:4. 50µL of serially diluted antibodies were added to each well, incubated for 1h at 37°C and washed with PBST for 6 times. The second His-mouTRAIL antigen was added into 96-well plates with 0.1µg/well and incubated at 37°C for 1h, followed by washing with PBST for 6 times. 100µL of the anti His-HRP (Beijing cwbiotech Co., Ltd., CW0285M, 1:4000 dilution) was added to each well and incubated for 1h at 37°C. After washing with PBST for 6 times, TMB was added with 100 µL/well and incubated for 10-20 minutes at 37°C. 2M H₂SO₄ was used to stop the reaction. Absorbance at 450nm was read using a microplate reader. The binding curves were generated by Graphpad Prism and the EC₅₀ of each anti-TRAIL antibody was calculated.

As shown in Table 21, the selected exemplary bispecific antibodies M88-15-G72-11-DVD-IgG4, G72-11-M88scFv-Bs4Ab-IgG4, G72- M88scFv-Bs4Ab-IgG4 had good binding activity to mouse TRAIL antigen, mouse FasL antigen, and both mouse TRAIL and mouse FasL antigen simultaneously.

**Table 21: The binding of bispecific antibodies to mouse TRAIL antigen and/or mouse FasL antigen**

| **Antibody Name** | **EC₅₀ (nM)** | | |
|---|---|---|---|
| | hFc-mouFasL | GST-mouTRAIL | hFc-mouFasL+ His-mouTRAIL |
| **M88-15-G72-11-DVD-IgG4** | 13.74 | 119.7 | 249.4 |
| **G72-11-M88scFv-Bs4Ab-IgG4** | 175.5 | 392.5 | 18.86 |
| **G72-M88scFv-Bs4Ab-IgG4** | 0.8699 | 2.155 | 5.622 |

### Example 10: Bispecific antibodies inhibited FasL and/or TRAIL antigen-induced apoptosis of HepG2 cells:

When actinomycin D was added to inhibit cell division, exogenous FasL or TRAIL could activate apoptosis signaling pathway and mediate apoptosis. The amount of ATP in cells could reflect the viability of cells, which was inversely proportional to the degree of apoptosis. Based on the above principles, the amount of ATP could be detected by CellTiter-Glo^{®} luminescent cell viability assay kit, and then the activity of bispecific antibodies to inhibit FasL and/or TRAIL antigen-induced apoptosis of HepG2 cells was determined. The antibodies G10-1, G72, G72-11 specifically binding to TRAIL, antibodies M88, M88-10, M78-1 specifically binding to FasL, anti-FasL antibody 119-4A or SDR5- Fc fusion protein were used as control.

### 10.1 Bispecific antibodies inhibited FasL antigen-induced apoptosis of HepG2 cells:

HepG2 cells at logarithmic growth stage were inoculated into 96-well plates with 2×10⁴ cells/wells. Each antibody sample was diluted to 662.5nM and then serially diluted at a ratio of 1:4. 50µL actinomycin D, 25µL hFc-hFasL prepared in example 1 and 25µL diluted bispecific antibody were added to a 96-well plate containing HepG2 cells. The final concentrations of actinomycin D and hFc-hFasL were 1µg/mL and 5 ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

### 10.2 Bispecific antibodies inhibited TRAIL antigen-induced apoptosis of HepG2 cells:

HepG2 cells at logarithmic growth stage were inoculated into 96-well plates with 2×10⁴ cells/wells. Each antibody sample was diluted to 10450nM and then serially diluted at a ratio of 1:4. 50µL actinomycin D, 25µL TRAIL recombinant fusion protein (Novoprotein, C022) and 25µL diluted bispecific antibody were added to a 96-well plate containing HepG2 cells. The final concentrations of actinomycin D and TRAIL recombinant fusion protein were 1µg/mL and 0.5 ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

### 10.3 Bispecific antibodies inhibited FasL antigen and TRAIL antigen induced apoptosis of HepG2 cells:

HepG2 cells at logarithmic growth stage were inoculated into 96-well plates with 2×10⁴ cells/wells. Each antibody sample was diluted to 10450nM and then serially diluted at a ratio of 1:4. 50µL actinomycin D, 25µL hFc-hFasL prepared in example 1, 25µL TRAIL recombinant fusion protein (Novoprotein, C022) and 25µL diluted bispecific antibody were added to a 96-well plate containing HepG2 cells. The final concentrations of actinomycin D, hFc-hFasL and TRAIL recombinant fusion protein were 1µg/Ml, 5 ng/mL and 0.5 ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

As shown in Table 22, the selected exemplary bispecific antibodies could inhibit both FasL antigen induced cell apoptosis and TRAIL induced cell apoptosis, and even inhibit cell apoptosis induced by both FasL antigen and TRAIL antigen. Among them, although the activity of these bispecific antibodies in inhibiting apoptosis of HepG2 cells induced by FasL or TRAIL antigens were equivalent or slightly weaker than corresponding monoclonal antibodies or controls, but the activity of inhibiting apoptosis of HepG2 cells induced by both FasL and TRAIL antigen were much better than corresponding monoclonal antibodies.

**Table 22: The inhibition of FasL and/or TRAIL antigen induced apoptosis of HepG2 cells by the bispecific antibodies**

| **Antibody Name** | **IC₅₀ (nM)** | | |
|---|---|---|---|
| | **hFc-hFasL** | **TRAIL** | **hFc-hFasL + TRAIL** |
| **M88-G72-Crossmab-IgG1** | 0.1065 | 3.222 | / |
| **M88-G72-Crossmab-IgG4** | 0.1133 | 3.067 | / |
| **M88-G72scFv-Bs4Ab-IgG1** | 0.02186 | 16.66 | / |
| **M88-G72scFv-Bs4Ab-IgG4** | 0.0228 | 1.711 | / |
| **G72-M88scFv-Bs4Ab-IgG1** | 0.02661 | 1.284 | / |
| **G72-M88scFv-Bs4Ab-IgG4** | 0.06083 | 0.073925 | 0.45745 |
| **FL-M88** | 0.01592 | / | / |
| **119-4A** | 0.03543 | / | / |
| **G72** | / | 1.735 | / |
| **sDR5-Fc** | / | 0.4984 | / |
| **M78-1-G10-1scFv-Bs4Ab-IgG4** | 0.06297 | 0.06074 | 0.7047 |
| **M78-1-G10-1scFv-2-Bs4Ab-IgG4** | 0.05388 | 0.05817 | 0.5205 |
| **M78-1-G10-2scFv-Bs4Ab-IgG4** | 0.0544 | 0.05627 | 0.5384 |
| **G10-1-M78-1scFv-Bs4Ab-IgG4** | 0.04984 | 0.05252 | 0.185 |
| **G10-2-M78-1scFv-Bs4Ab-IgG4** | 0.04728 | 0.05703 | 0.1639 |
| **M78-1-G10-1-DVD-IgG4** | 0.04167 | 0.04184 | 4.884 |
| **M78-1-G10-2-DVD-IgG4** | 0.03726 | 0.0417 | 6.324 |
| **G10-1-M78-1-DVD-IgG4** | 0.4353 | 0.4869 | 0.8287 |
| **G10-2-M78-1-DVD-IgG4** | 0.4313 | 0.5362 | 0.6017 |
| **M88-15-G72-11-DVD-IgG4** | 0.03719 | 0.03966 | 6.814 |
| **G72-11-M88scFv-Bs4Ab-IgG4** | 0.04067 | 0.04516 | 0.3124 |
| **M88-19-G72-16-DVD-IgG4** | 0.02309 | 0.544 | 0.2448 |
| **M78-5-G10-6scFv-Bs4Ab-IgG4** | / | / | 0.8662 |
| **M88-20-G72-16-DVD-IgG4** | 0.02275 | 0.5896 | 0.3889 |
| **G72-16-M88-22scFv-Bs4Ab-IgG4** | 0.02568 | 0.1609 | 0.6404 |
| **G72-16-M88-21scFv-Bs4Ab-IgG4** | 0.02649 | 0.1578 | 0.6157 |
| **M88-15-G72-16-DVD-IgG4** | / | / | 0.5343 |
| **G72-16-M88scFv-Bs4Ab-IgG4** | / | / | 0.5124 |
| **G72-17-M88scFv-Bs4Ab-IgG4** | / | / | 0.5388 |
| **G72-17-M88-23scFv-Bs4Ab-IgG4** | | | 0.5705 |
| **G72-17-M88-24scFv-Bs4Ab-IgG4** | / | / | 0.5616 |
| **M78-6-G10-1scFv-Bs4Ab-IgG4** | / | / | 1.052 |
| **M78-5-G10-1scFv-Bs4Ab-IgG4** | / | / | 1.07 |
| **M78-7-G10-1scFv-Bs4Ab-IgG4** | / | / | 1.155 |
| **M78-8-G10-1scFv-Bs4Ab-IgG4** | / | / | 0.8809 |
| **M78-9-G10-1scFv-Bs4Ab-IgG4** | / | / | 0.9266 |
| **M78-1-G10-6scFv-Bs4Ab-IgG4** | / | / | 0.7465 |
| **M78-1-G10-7scFv-Bs4Ab-IgG4** | / | / | 0.867 |
| **M78-10-G10-7scFv-Bs4Ab-IgG4** | / | / | 0.8147 |
| **humFL-M78-1** | **0.018225** | / | 145.7 |
| **G10-1** | / | 0.01837 | / |
| **G72-11** | / | 0.0186 | / |

### Example 11: Bispecific antibodies inhibited FasL and/or TRAIL antigen-induced apoptosis of Jurkat cells:

The experimental principle is the same as above. The amount of ATP in cells is used to reflect the apoptosis level, and then the activity of bispecific antibodies to inhibit FasL and/or TRAIL antigen induced apoptosis of Jurkat cells was determined. The antibodies G10-1, G72, G72-11 specifically binding to TRAIL, antibodies M88, M88-10, M78-1 specifically binding to FasL, anti-FasL antibody 119-4A or SDR5- Fc fusion protein were used as control.

### 11.1 Bispecific antibodies inhibited FasL antigen induced apoptosis of Jurkat cells:

Jurkat cells at logarithmic growth stage were inoculated into 96-well plates with 5×10⁴ cells/wells. Each antibody sample was diluted to 662.5nM and subsequently diluted in a specific proportion. 50µL actinomycin D, 25µL hFc-hFasL prepared in example 1, and 25µL diluted bispecific antibody were added to a 96-well plate containing Jurkat cells. The final concentrations of actinomycin D and hFc-hFasL were 32ng/mL and 5ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

### 11.2 Bispecific antibodies inhibited TRAIL antigen induced apoptosis of Jurkat cells:

Jurkat cells at logarithmic growth stage were inoculated into 96-well plates with 5×10⁴ cells/wells. Each antibody sample was diluted to 10450nM and subsequently diluted in a specific proportion. 50µL actinomycin D, 25µL TRAIL recombinant fusion protein (Novoprotein, C022), and 25µL diluted bispecific antibody were added to a 96-well plate containing Jurkat cells. The final concentrations of actinomycin D and TRAIL recombinant fusion protein were 32ng/mL and 20ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

### 11.3 Bispecific antibodies inhibited FasL antigen and TRAIL antigen induced apoptosis of Jurkat cells:

Jurkat cells at logarithmic growth stage were inoculated into 96-well plates with 5×10⁴ cells/wells. Each antibody sample was diluted to 10450nM and subsequently diluted in a specific proportion. 50µL actinomycin D, 25µL hFc-hFasL prepared in example 1, 25µL TRAIL recombinant fusion protein (Novoprotein, C022), and 25µL diluted bispecific antibody were added to a 96-well plate containing Jurkat cells. The final concentrations of actinomycin D, hFc-hFasL and TRAIL recombinant fusion protein were 32ng/mL, 5ng/mL and 20ng/mL, respectively. The cells were cultured at 37°C, 5%CO₂ for 48h. The amount of ATP was measured using the CellTiter-Glo^{®} luminescent cell viability assay kit (Promega, G7572), which was proportional to the generated luminescence signal. That is, CellTiter-Glo^{®} reagent was added to 96-well cell culture plates with 50µL/well, which incubated at room temperature for 10min after mixing. The fluorescence values were read using a microplate reader. The curve was generated by GraphPad Prism software, and the IC₅₀ value of each antibody was calculated.

As shown in Table 23, the selected exemplary bispecific antibodies could inhibit both FasL antigen induced cell apoptosis and TRAIL induced cell apoptosis, and even inhibit cell apoptosis induced by both FasL antigen and TRAIL antigen. Among them, although the activity of these bispecific antibodies in inhibiting apoptosis of Jurkat cells induced by FasL or TRAIL antigens were equivalent or slightly weaker than corresponding monoclonal antibodies or controls, but the activity of inhibiting apoptosis of Jurkat cells induced by both FasL and TRAIL antigen were much better than corresponding monoclonal antibodies.

**Table 23: The inhibition of FasL and/or TRAIL antigen induced apoptosis of Jurkat cells by the bispecific antibodies**

| **Antibody Name** | **IC₅₀ (nM)** | | |
|---|---|---|---|
| | **hFc-hFasL** | **TRAIL** | **hFc-hFasL + TRAIL** |
| **M88-G72-Crossmab-IgG1** | 0.06256 | 1.428 | / |
| **M88-G72-Crossmab-IgG4** | 0.09817 | 2.202 | / |
| **M88-G72scFv-Bs4Ab-IgG1** | 0.03489 | / | / |
| **M88-G72scFv-Bs4Ab-IgG4** | 0.0275 | 13.54 | / |
| **G72-M88scFv-Bs4Ab-IgG1** | 0.08558 | 1.872 | / |
| **G72-M88scFv-Bs4Ab-IgG4** | 0.042785 | 4.091 | 0.14417 |
| **FL-M88** | 0.01697 | / | / |
| **119-4A** | 0.05655 | / | / |
| **G72** | / | / | / |
| **sDR5-Fc** | / | / | / |
| **M78-1-G10-1scFv-Bs4Ab-IgG4** | 0.03132 | 0.6918 | 0.1348 |
| **M78-1-G10-1scFv-2-Bs4Ab-IgG4** | 0.02373 | 0.2084 | 0.07131 |
| **M78-1-G10-2scFv-Bs4Ab-IgG4** | 0.02794 | 0.2895 | 0.06003 |
| **G10-1-M78-1scFv-Bs4Ab-IgG4** | 0.02715 | 0.2366 | 0.06534 |
| **G10-2-M78-1scFv-Bs4Ab-IgG4** | 0.04055 | 0.2275 | 0.06069 |
| **M78-1-G10-1-DVD-IgG4** | 0.01823 | 1.192 | 0.05473 |
| **M78-1-G10-2-DVD-IgG4** | 0.01606 | 0.6643 | 0.04572 |
| **G10-1-M78-1-DVD-IgG4** | 0.6039 | 0.1926 | 0.1871 |
| **G10-2-M78-1-DVD-IgG4** | 0.5018 | 0.1879 | 0.204 |
| **M88-15-G72-11-DVD-IgG4** | 0.02597 | 312.5 | 0.06197 |
| **G72-11-M88scFv-Bs4Ab-IgG4** | 0.02965 | 18.09 | 0.1197 |
| **M88-19-G72-16-DVD-IgG4** | 0.02747 | 0.08368 | 12.43 |
| **M78-5-G10-6scFv-Bs4Ab-IgG4** | / | / | 0.08917 |
| **humFL-M78-1** | 0.02043 | / | 705 |
| **G10-1** | / | 0.1415 | / |
| **G72-11** | / | 9.332 | // |

### Example 12: The binding affinity of the bispecific antibodies:

The binding affinity of the bispecific antibodies with human, monkey FasL and TRAIL were characterized using Biacore T200 (GE). M78-1-G10-1scFv-Bs4Ab-IgG4, G10-2-M78-1scFv-Bs4Ab-IgG4 and G72-11-M88scFv-Bs4Ab-IgG4 were used as examples. The antigens used in this embodiment, in which His-hFasL, hFc-cynoFasL, His-hTRAIL were all prepared using the method described in Example 1. CynoTRAIL with the product number 90098-CNAE was purchased from Beijing sino biological Inc.

When the affinity of the bispecific antibody to His-hTRAIL or CynoTRAIL was detected, anti-human Fc antibodies were stabilized on sensor chip CM5. Bispecific antibody (M78-1-G10-1scFv-Bs4Ab-IgG4 or G10-2-M78-1scFv-Bs4Ab-IgG4) was diluted to 2 µg/ml and added into the channel to flow through and captured by the chip, with the capture time of 40 s and the flow rate of 30µL/min. His-hTRAIL and CynoTRAIL antigen were diluted with 1×HBS-EP buffer (pH 7.4), wherein His-hTRAIL antigen was diluted to 0M, 1.074E-8M, 2.148E-8M, 4.296E-8M, 8.591E-8M, 1.718E-7M and CynoTRAIL antigen was diluted to 0M, 6.712E-10M, 1.342E-9M, 5.369E-9M, 1.074E-8M, 2.148E-8M. The binding time, the dissociation time and the flow rate were set to 120s, 1200s, 30µL/min, respectively. The affinity of antibody to human or monkey TRAIL antigen was determined at different concentrations.

When the affinity of the bispecific antibody to His-hFasL or hFc-cynoFasL was detected, the bispecific antibody (M78-1-G10-1scFv-Bs4Ab-IgG4 or G10-2-M78-1scFv-Bs4Ab-IgG4) was diluted to 20µg/ml and CM5 chip Fc channels were activated by EDC/NHS, added diluted bispecific antibody for coupling and then blocked by ethanolamine. The final coupling amounts of the antibody on the chip reached 262.9RU (M78-1-G10-1scFv-Bs4Ab-IgG) and 327.7RU (G10-2-M78-1scFv-Bs4Ab-IgG4), respectively. His-hFasL or hFc-cynoFasL antigen were diluted with 1×HBS-EP buffer (pH 7.4), wherein hFc-cynoFasL antigen was diluted to 0 M, 7.267E-9M, 1.453E-8M, 2.907E-8M, 5.814E-8M, 1.163E-7M and His-hFasL antigen was diluted to 0 M, 1.423E-9 M, 2.846E-9 M, 5.692E-9 M, 1.138E-8 M, 2.277E-8 M. The binding time, the dissociation time and the flow rate were set to 120s, 1500s, 30µL/min, respectively. The affinity of antibody to human or monkey FasL antigen was determined at different concentrations.

As shown in Table 24, the exemplary antibody M78-1-G10-1scFv-Bs4Ab-IgG4 or G10-2-M78-1scFv-Bs4Ab-IgG4 exhibited well binding ability to both FasL and TRAIL antigens of human and monkey.

**Table 24: Characterization of the bispecific antibodies Kon, Koff, and Kd values**

| **Antibody** | **Antigen** | **Kon (1/Ms)** | **Koff (1/s)** | **Kd (M)** |
|---|---|---|---|---|
| M78-1-G10-1scFv-Bs4Ab-IgG4 | His-hTRAIL | 8.806E+04 | 1.273E-04 | 1.446E-9 |
| G10-2-M78-1scFv-Bs4Ab-IgG4 | | 8.579E+04 | 9.499E-05 | 1.107E-9 |
| G72-11-M88scFv-Bs4Ab-IgG4 | | 4.481E+05 | 1.594E-04 | 3.557E-10 |
| | | | | |

| **Antibody** | **Antigen** | **Kon (1/Ms)** | **Koff (1/s)** | **Kd (M)** |
|---|---|---|---|---|
| M78-1-G10-1scFv-Bs4Ab-IgG4 | CynoTRAIL | 1.185E+06 | 7.148E-05 | 6.032E-11 |
| G10-2-M78-1scFv-Bs4Ab-IgG4 | | 7.401E+06 | 3.376E-04 | 4.561E-11 |
| G72-11-M88scFv-Bs4Ab-IgG4 | | 4.054E+06 | 1.114E-04 | 2.747E-11 |
| | | | | |

| **Antibody** | **Antigen** | **Kon (1/Ms)** | **Koff (1/s)** | **Kd (M)** |
|---|---|---|---|---|
| M78-1-G10-1scFv-Bs4Ab-IgG4 | His-hFasL | 7.839E+05 | 8.912E-04 | 1.137E-10 |
| G10-2-M78-1scFv-Bs4Ab-IgG4 | | 3.822E+05 | 4.204E-05 | 1.100E-10 |
| G72-11-M88scFv-Bs4Ab-IgG4 | | 3.777E+04 | 6.692E-05 | 1.772E-09 |
| | | | | |

| **Antibody** | **Antigen** | **Kon (1/Ms)** | **Koff (1/s)** | **Kd (M)** |
|---|---|---|---|---|
| M78-1-G10-1scFv-Bs4Ab-IgG4 | hFc-cynoFasL | 1.320E+05 | 6.557E-05 | 4.965E-10 |
| G10-2-M78-1scFv-Bs4Ab-IgG4 | | 6.862E+05 | 4.495E-05 | 6.650E-10 |
| G72-11-M88scFv-Bs4Ab-IgG4 | | 1.235E+04 | 2.765-04 | 2.238E-08 |

### Example 13: Bispecific antibody inhibited killing of HepG2 cells by PBMC:

PBMC cells contain killer T cells and killer NK cells. Therefore, PBMC cells could be used to mimic the natural blood immune environment in vivo. Plant hemagglutinin (PHA-P) could activate PBMC cells. In the presence of PHA-P, PBMC co-cultured with HepG2 cells could activate TRAIL-DR4/5 mediated apoptosis pathway in HepG2 cells and killed it. Based on the above experimental principles, the degree of apoptosis of HepG2 cells could be determined by detecting the amount of activated Caspase3/7, and then the inhibiting activity of bispecific antibodies on killing of HepG2 cells by PBMC were determined.

HepG2 cells (2×10⁴ cells/well) and PBMC cells (3.75×10⁴ cells/well) isolated from human fresh blood (donated by volunteers) were seeded into 96-well plates, and PHA-P and IL2 were added simultaneously. The final concentrations of PHA-P and IL2 were 10 µg/ml and 100 U/ml, respectively. Subsequently, bispecific antibody with a final concentration of 333nM was added to the wells of the experimental group, while the control group was set up, wherein only antibody was not added into the non-antibody group, and PHA-P and antibody was not added into the non-stimulation group. Cultured at 37°C, 5%CO₂ for 18 h. Caspase-Glo^{®} 3/7 reagent was added into the 96-well plate with 50µL/well, and incubated for 10min. The fluorescence values were read using a microplate reader.

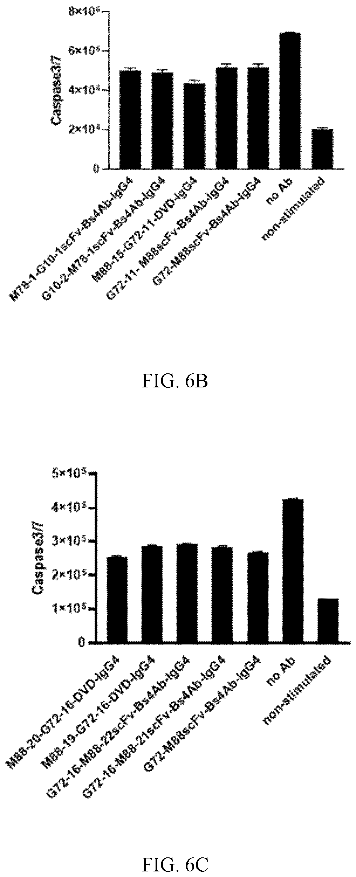

The results of the exemplary antibody were shown in Figure 6A-6C, the selected bispecific antibodies M88-G72-Crossmab-IgG4, M88-G72scFv-Bs4Ab-IgG4, G72-M88scFv-Bs4Ab-IgG4, M78-1-G10-1scFv-Bs4Ab-IgG4, G10-2-M78-1scFv-Bs4Ab-IgG4, M88-15-G72-11-DVD-IgG4, G72-11-M88scFv-Bs4Ab-IgG4, M88-19-G72-16-DVD-IgG4, M88-20-G72-16-DVD-IgG4, G72-16-M88-22scFv-Bs4Ab-IgG4, G72-16-M88-21scFv-Bs4Ab-IgG4 could effectively inhibit killing of HepG2 cells by PBMC.

### Example 14: Bispecific antibody inhibited killing of Jurkat cells by PBMC:

The experimental principle was the same as above, the degree of apoptosis of Jurkat cells could be determined by detecting the amount of activated Caspase3/7, and then the inhibiting activity of bispecific antibodies on killing of Jurkat cells by PBMC were determined.

Jurkat cells (2.5×10⁴ cells/well) and PBMC cells (3.75×10⁴ cells/well) isolated from human fresh blood (donated by volunteers) were seeded into 96-well plates, and PHA-P and IL2 were added simultaneously. The final concentrations of PHA-P and IL2 were 10 µg/ml and 100 U/ml, respectively. Subsequently, bispecific antibody with a final concentration of 333nM was added to the wells of the experimental group, while the control group was set up, wherein only antibody was not added into the non-antibody group, and PHA-P and antibody was not added into the non-stimulation group. Cultured at 37°C, 5%CO₂ for 18h. Caspase-Glo^{®} 3/7 reagent was added into the 96-well plate with 50µL/well, and incubated for 10min. The fluorescence values were read using a microplate reader.

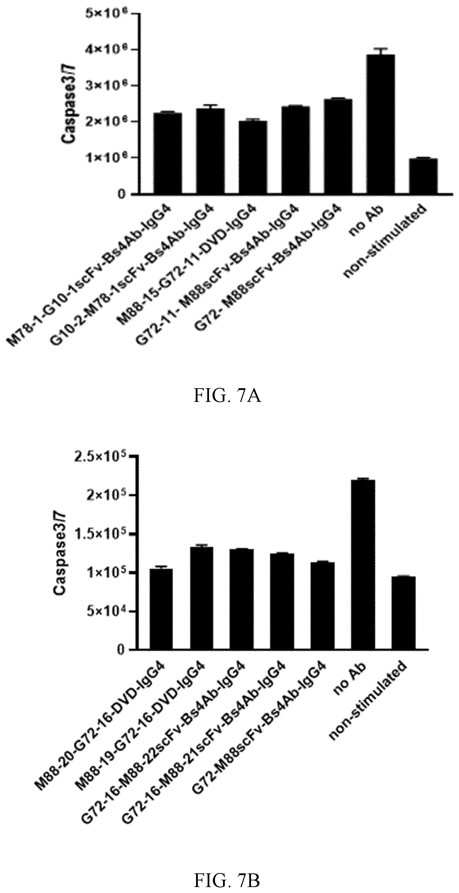

The results of the exemplary antibody were shown in Figures 7A-7B, the selected bispecific antibodies M78-1-G10-1scFv-Bs4Ab-IgG4, G10-2-M78-1scFv-Bs4Ab-IgG4, M88-15-G72-11-DVD-IgG4, G72-11-M88scFv-Bs4Ab-IgG4, G72-M88scFv-Bs4Ab-IgG4, M88-19-G72-16-DVD-IgG4, M88-20-G72-16-DVD-IgG4, G72-16-M88-22scFv-Bs4Ab-IgG4, G72-16-M88-21scFv-Bs4Ab-IgG4 could effectively inhibit killing of Jurkat cells by PBMC.

### Example 15: TRAIL/FasL bispecific antibodies, or a combination of anti-TRAIL antibody and anti-Fasl antibody in a mouse model of APAP-induced liver injury

A mouse model of liver injury was constructed using the method described in embodiment 6. Briefly, female BALB/C mice (Beijing Vital River Laboratory Animal Technology Co., Ltd., aged 6-8 weeks) were randomly divided into different groups, the experimental group consisted of model control group (no antibody injection group/no antibody group), low-dose group (administering monoclonal antibody, bispecific antibody, or a combination of two monoclonal antibody respectively) and high-dose group (administering monoclonal antibody, bispecific antibody, or a combination of two monoclonal antibody respectively), with 6 mice in each group, and simultaneously set up a blank control group, with 2 mice in each group. Firstly, all the mice were fasted, but water was allowed. After fasting for about 16h, mice in each experimental group were intraperitoneally injected with 200 mg kg APAP (Aladdin, A105808). When injecting APAP for 30 minutes, monoclonal antibody (using anti-TRAIL antibody G72 or anti-FasL antibody FL-M8 as the example), bispecific antibody (using M88-G72scFv-Bs4Ab-IgG4 or G72-M88scFv-Bs4Ab-IgG4 as the example) or both anti-TRAIL antibody (G72 as the example) and anti-FasL antibody (FL-M88 as the example) were injected into each experimental group of mice through the tail vein. In the low-dose group, both monoclonal antibody and bispecific antibody were injected at a dose of 66.67µM, while the injection doses of the combined anti-TRAIL antibody and anti-FasL antibody were 66.67µM, respectively. In the high-dose group, both monoclonal antibody and bispecific antibody were injected at a dose of 133.3µM, while the injection doses of the combined anti-TRAIL antibody and anti-FasL antibody were 133.3µM, respectively. Meanwhile, the mice of model control group were only given an equal amount of normal saline. When injecting APAP for 24 hours, 200µL of blood was taken from the orbital venous plexus of mice and centrifuged at 1500g at room temperature for 10 min. The serum was collected and ALT level was detected with ALT Detection Kit (Nanjing Jiancheng, C009-2-1).

Using the same method, the effects of several other exemplary antibodies (G72-11-M88scFv-Bs4Ab-IgG4, M88-15-G72-11-DVD-IgG4 as the example) on the mouse model of APAP-induced liver injury were also verified at a dose of 100µm.

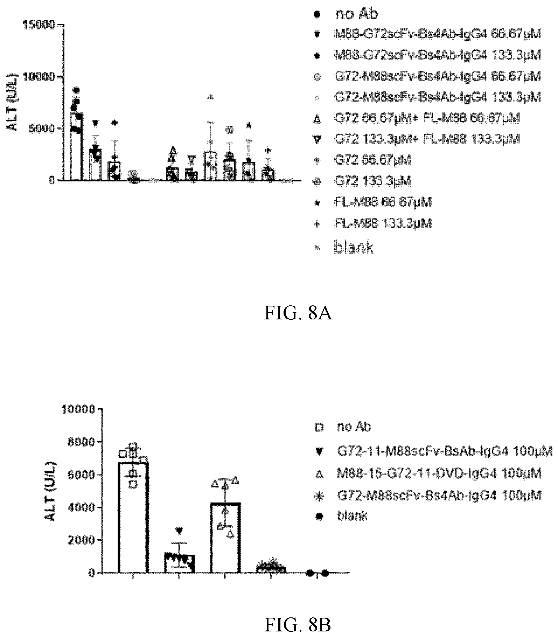

As shown in Figure 8A, the exemplary anti-TRAIL antibody G72, anti-FasL antibody FL-M88, bispecific antibody M88-G72scFv-Bs4Ab-IgG4 and G72-M88scFv-Bs4Ab-IgG4, or the combination of anti-TRAIL antibody G72 and anti-Fasl antibody FL-M88 could significantly inhibited APAP-induced ALT elevation in a dose-dependent manner.

As shown in Figure 8B, other exemplary antibody G72-11-M88scFv-Bs4Ab-IgG4, M88-15-G72-11-DVD-IgG4 also could significantly inhibited APAP-induced ALT elevation. These results indicated that anti-TRAIL/FasL bispecific antibody or the combination of anti-TRAIL antibody and anti-Fasl antibody that described in this application could play a role in the treatment of liver injury.

### Example 16: The effect of TRAIL/FasL bispecific antibodies on a cynomolgus monkey model of APAP-induced liver injury

A cynomolgus monkey model of liver injury was constructed using the method described in embodiment 6. Briefly, three cynomolgus monkey (aged 5-6 years) were randomly divided into different groups, one monkey in the model control group (no antibody injection group/no antibody group), two monkey in the experimental group (injecting anti-TRAIL/FasL bispecific antibody). Firstly, all the monkeys were fasted, but water was allowed until 7 hours after modeling to resume eating. After fasting for about 24h, cynomolgus monkeys were intravenously injected with 60 mg/kg of BSO (Sigma, B2640-5G). After 1 hour, APAP was administered by gavage to the aforementioned cynomolgus monkey at a dose of 1500mg/kg with a dosage of 5mL/kg. Meanwhile, the model control group were only given an equal amount of normal saline. When injecting APAP for 24 hours, 1mL of blood was taken from the veins of cynomolgus monkeys and centrifuged at 1500g at room temperature for 10 min. The serum was collected and alanine aminotransferase (ALT) and aspartate aminotransferase (AST) level was detected with an automated biochemical analyzer (Shenzhen mindray Biomedical Electronics Co. ,Ltd. , BS-420).

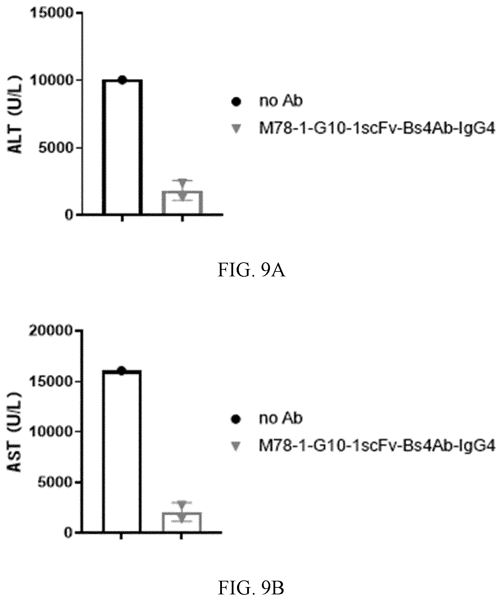

As shown in Figures 9A-9B, the exemplary bispecific antibody M78-1-G10-1scFv-Bs4Ab-IgG4 could significantly inhibit the elevation of ALT and AST induced by APAP in cynomolgus monkey. This indicated that the anti-TRAIL/FasL bispecific antibody in this application could play a role in the treatment of liver injury.

## Claims

1. An isolated antibody or antigen-binding fragment specifically binding to TRAIL, comprising:
a V_{H}, wherein the V_{H} comprises
an HC-CDR1 comprising the amino acid sequence of SEQ ID NO: 1,
an HC-CDR2 comprising the amino acid sequence of SEQ ID NO: 3, and
an HC-CDR3 comprising the amino acid sequence of SEQ ID NO: 5, or a variant thereof comprising up to about 5 amino acid substitutions in the HC-CDRs; and
a V_{L}, wherein the V_{L} comprises
an LC-CDR1 comprising the amino acid sequence of SEQ ID NO: 7,
an LC-CDR2 comprising the amino acid sequence of SEQ ID NO: 9, and
an LC-CDR3 comprising the amino acid sequence of SEQ ID NO: 11, or a variant thereof comprising up to about 5 amino acid substitutions in the LC-CDRs.

2. An isolated antibody or antigen-binding fragment specifically binding to TRAIL, comprising:
a V_{H} comprising an HC-CDR1, an HC-CDR2, and an HC-CDR3 of a V_{H} comprising the amino acid sequence of SEQ ID NO: 13; and
a V_{L} comprising an LC-CDR1, an LC-CDR2, and an LC-CDR3 of a V_{L} comprising the amino acid sequence of SEQ ID NO: 22.

3. The isolated antibody or antigen-binding fragment specifically binding to TRAIL of claim 1 or 2, comprising:
(i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22;
(ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23;
(iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24;
(iv) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 25, or a variant thereof having at least about 80% sequence identity to the amino acid sequence of SEQ ID NO: 25;
(v) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 26, or a variant thereof having at least about 80% sequence identity to the amino acid sequence of SEQ ID NO: 26; or
(vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 27, or a variant thereof having at least about 80% sequence identity to the amino acid sequence of SEQ ID NO: 27.

4. An isolated antibody or antigen-binding fragment specifically binding to TRAIL, comprising:
a V_{H}, wherein the V_{H} comprises
an HC-CDR1 comprising the amino acid sequence of SEQ ID NO: 2,
an HC-CDR2 comprising the amino acid sequence of SEQ ID NO: 4, and
an HC-CDR3 comprising the amino acid sequence of SEQ ID NO: 6, or a variant thereof comprising up to about 5 amino acid substitutions in the HC-CDRs; and
a V_{L}, wherein the V_{L} comprises
an LC-CDR1 comprising the amino acid sequence of SEQ ID NO: 8,
an LC-CDR2 comprising the amino acid sequence of SEQ ID NO: 10, and
an LC-CDR3 comprising the amino acid sequence of SEQ ID NO: 12, or a variant thereof comprising up to about 5 amino acid substitutions in the LC-CDRs.

5. An isolated antibody or antigen-binding fragment specifically binding to TRAIL, comprising:
a V_{H} comprising an HC-CDR1, an HC-CDR2, and an HC-CDR3 of a V_{H} comprising the amino acid sequence of SEQ ID NO: 15; and
a V_{L} comprising an LC-CDR1, an LC-CDR2, and an LC-CDR3 of a V_{L} comprising the amino acid sequence of SEQ ID NO: 28.

6. The isolated antibody or antigen-binding fragment specifically binding to TRAIL of claim 4 or 5, comprising:
(i) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28;
(ii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 29, or a variant thereof having at least about 80% sequence identity to the amino acid sequence of SEQ ID NO: 29;
(iii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 29, or a variant thereof having at least about 80% sequence identity to the amino acid sequence of SEQ ID NO: 29;
(iv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 29, or a variant thereof having at least about 80% sequence identity to the amino acid sequence of SEQ ID NO: 29;
(v) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 29, or a variant thereof having at least about 80% sequence identity to the amino acid sequence of SEQ ID NO: 29;
(vi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 29, or a variant thereof having at least about 80% sequence identity to the amino acid sequence of SEQ ID NO: 29;
(vii)a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 30, or a variant thereof having at least about 80% sequence identity to the amino acid sequence of SEQ ID NO: 30;
(viii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 30, or a variant thereof having at least about 80% sequence identity to the amino acid sequence of SEQ ID NO: 30;
(ix) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 30, or a variant thereof having at least about 80% sequence identity to the amino acid sequence of SEQ ID NO: 30;
(x) a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 30, or a variant thereof having at least about 80% sequence identity to the amino acid sequence of SEQ ID NO: 30;
(xi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30;
(xii) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31;
(xiii) a V_{H} comprising the amino acid sequence of SEQ ID NO: 17, or a variant thereof having at least about 80% sequence identity to the amino acid sequence of SEQ ID NO: 17; and a V_{L} comprising the amino acid sequence of SEQ ID NO: 31, or a variant thereof having at least about 80% sequence identity to the amino acid sequence of SEQ ID NO: 31;
(xiv) a V_{H} comprising the amino acid sequence of SEQ ID NO: 18, or a variant thereof having at least about 80% sequence identity to the amino acid sequence of SEQ ID NO: 18; and a V_{L} comprising the amino acid sequence of SEQ ID NO: 31, or a variant thereof having at least about 80% sequence identity to the amino acid sequence of SEQ ID NO: 31;
(xv)a V_{H} comprising the amino acid sequence of SEQ ID NO: 19, or a variant thereof having at least about 80% sequence identity to the amino acid sequence of SEQ ID NO: 19; and a V_{L} comprising the amino acid sequence of SEQ ID NO: 31, or a variant thereof having at least about 80% sequence identity to the amino acid sequence of SEQ ID NO: 31;
(xvi) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 31, or a variant thereof having at least about 80% sequence identity to the amino acid sequence of SEQ ID NO: 31; or
(xvii) a V_{H} comprising the amino acid sequence of SEQ ID NO: 21, or a variant thereof having at least about 80% sequence identity to the amino acid sequence of SEQ ID NO: 21; and a V_{L} comprising the amino acid sequence of SEQ ID NO: 32, or a variant thereof having at least about 80% sequence identity to the amino acid sequence of SEQ ID NO: 32.

7. The isolated antibody or antigen-binding fragment specifically binding to TRAIL of any one of claims 1-6, wherein the antibody or antigen-binding fragment comprises an Fc fragment.

8. The isolated antibody or antigen-binding fragment specifically binding to TRAIL of claim 7, wherein the antibody or antigen-binding fragment is a full-length IgG antibody.

9. The isolated antibody or antigen-binding fragment specifically binding to TRAIL of claim 8, wherein the antibody or antigen-binding fragment is a full-length IgG1, IgG2, IgG3, or IgG4 antibody.

10. The isolated antibody or antigen-binding fragment specifically binding to TRAIL of any one of claims 1-9, wherein the antibody or antigen-binding fragment is a chimeric, humanized or human antibody.

11. The isolated antibody or antigen-binding fragment specifically binding to TRAIL of any one of claims 1-6, wherein the antigen-binding fragment is selected from the group consisting of a Fab, a Fab', a F(ab)'2, a Fab'-SH, a single-chain Fv (scFv), an Fv fragment, a dAb, an Fd, a nanobody, a diabody, and a linear antibody.

12. A bispecific antibody, comprising a first antigen-binding domain specifically binding to TRAIL, and a second antigen-binding domain specifically binding to FasL.

13. The bispecific antibody of claim 12, wherein the first antigen-binding domain comprises:
(i) a V_{H}, wherein the V_{H} comprises
an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and
a V_{L}, wherein the V_{L} comprises
an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11; or
(ii) a V_{H}, wherein the V_{H} comprises
an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and
a V_{L}, wherein the V_{L} comprises
an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12; or
(iii) a V_{H}, wherein the V_{H} comprises
an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and
a V_{L}, wherein the V_{L} comprises
an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12.

14. The bispecific antibody of claim 12 or 13, wherein the first antigen-binding domain comprises:
(i) a V_{H} comprising the amino acid sequence of any one of SEQ ID NOs: 13 and 33, or a variant thereof having at least about 80% sequence identity to the amino acid sequence of any one of SEQ ID NOs: 13 and 33; and a V_{L} comprising the amino acid sequence of any one of SEQ ID NOs: 22 and 41, or a variant thereof having at least about 80% sequence identity to the amino acid sequence of any one of SEQ ID NOs: 22 and 41;
(ii) a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42;
(iii) a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43;
(iv) a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47;
(v) a V_{H} comprising the amino acid sequence of any one of SEQ ID NOs: 20 and 40, or a variant thereof having at least about 80% sequence identity to the amino acid sequence of any one of SEQ ID NOs: 20 and 40; and a V_{L} comprising the amino acid sequence of any one of SEQ ID NOs: 30 and 49, or a variant thereof having at least about 80% sequence identity to the amino acid sequence of any one of SEQ ID NOs: 30 and 49;
(vi) a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50;
(vii) a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168; or
(viii) a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170.

15. The bispecific antibody of any one of claims 12-14, wherein the second antigen-binding domain comprises:
(i) a V_{H}, wherein the V_{H} comprises
an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and
a V_{L}, wherein the V_{L} comprises
an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61;
(ii) a V_{H}, wherein the V_{H} comprises
an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and
a V_{L}, wherein the V_{L} comprises
an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or
(iii) a V_{H}, wherein the V_{H} comprises
an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and
a V_{L}, wherein the V_{L} comprises
an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

16. The bispecific antibody of any one of claims 12-15, wherein the second antigen-binding domain comprises:
(i) a V_{H} comprising the amino acid sequence of any one of SEQ ID NOs: 63 and 80, or a variant thereof having at least about 80% sequence identity to the amino acid sequence of any one of SEQ ID NOs: 63 and 80; and a V_{L} comprising the amino acid sequence of any one of SEQ ID NOs: 73 and 89, or a variant thereof having at least about 80% sequence identity to the amino acid sequence of any one of SEQ ID NOs: 73 and 89;
(ii) a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90;
(iii) a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92;
(iv) a V_{H} comprising the amino acid sequence of any one of SEQ ID NOs: 70 and 86, or a variant thereof having at least about 80% sequence identity to the amino acid sequence of any one of SEQ ID NOs: 70 and 86; and a V_{L} comprising the amino acid sequence of any one of SEQ ID NOs: 78 and 94, or a variant thereof having at least about 80% sequence identity to the amino acid sequence of any one of SEQ ID NOs: 78 and 94;
(v) a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95;
(vi) a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167; or
(vii)a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

17. The bispecific antibody of any one of claims 12-13 and 15,
a) wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11;
and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61;
b) wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11;
and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62;
c) wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12;
and wherein the second antigen-binding domain comprises: a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61;
d) wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12;
and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62; or
e) wherein the first antigen-binding domain comprises: a V_{H} comprising an HC-CDR1 comprising the amino acid sequence of SEQ ID NO: 2, an HC-CDR2 comprising the amino acid sequence of SEQ ID NO: 161, and an HC-CDR3 comprising the amino acid sequence of SEQ ID NO: 6; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12;
and wherein the second antigen-binding domain comprises: a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56; and a V_{L} comprising an LC-CDR1 comprising the amino acid sequence of SEQ ID NO: 162, an LC-CDR2 comprising the amino acid sequence of SEQ ID NO: 60, and an LC-CDR3 comprising the amino acid sequence of SEQ ID NO: 62.

18. The bispecific antibody of any one of claims 12-17,
a) wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 23 and 42, or a variant thereof having at least about 80% sequence identity to the amino acid sequence of any one of SEQ ID NOs: 23 and 42;
and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90;
b) wherein the first antigen-binding domain comprises: a V_{H} comprising the amino acid sequence of any one of SEQ ID NOs: 14 and 34, or a variant thereof having at least about 80% sequence identity to the amino acid sequence of any one of SEQ ID NOs: 14 and 34; and a V_{L} comprising the amino acid sequence of any one of SEQ ID NOs: 24 and 43, or a variant thereof having at least about 80% sequence identity to the amino acid sequence of any one of SEQ ID NOs: 24 and 43;
and wherein the second antigen-binding domain comprises: a V_{H} comprising the amino acid sequence of any one of SEQ ID NOs: 64 and 81, or a variant thereof having at least about 80% sequence identity to the amino acid sequence of any one of SEQ ID NOs: 64 and 81; and a V_{L} comprising the amino acid sequence of any one of SEQ ID NOs: 74 and 90, or a variant thereof having at least about 80% sequence identity to the amino acid sequence of any one of SEQ ID NOs: 74 and 90;
c) wherein the first antigen-binding domain comprises: a V_{H} comprising the amino acid sequence of any one of SEQ ID NOs: 15 and 35, or a variant thereof having at least about 80% sequence identity to the amino acid sequence of any one of SEQ ID NOs: 15 and 35; and a V_{L} comprising the amino acid sequence of any one of SEQ ID NOs: 28 and 47, or a variant thereof having at least about 80% sequence identity to the amino acid sequence of any one of SEQ ID NOs: 28 and 47;
and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92;
d) wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50;
and wherein the second antigen-binding domain comprises: a V_{H} comprising the amino acid sequence of any one of SEQ ID NOs: 66 and 82, or a variant thereof having at least about 80% sequence identity to the amino acid sequence of any one of SEQ ID NOs: 66 and 82; and a V_{L} comprising the amino acid sequence of any one of SEQ ID NOs: 76 and 92, or a variant thereof having at least about 80% sequence identity to the amino acid sequence of any one of SEQ ID NOs: 76 and 92;
e) wherein the first antigen-binding domain comprises: a V_{H} comprising the amino acid sequence of any one of SEQ ID NOs: 16 and 36, or a variant thereof having at least about 80% sequence identity to the amino acid sequence of any one of SEQ ID NOs: 16 and 36; and a V_{L} comprising the amino acid sequence of any one of SEQ ID NOs: 31 and 50, or a variant thereof having at least about 80% sequence identity to the amino acid sequence of any one of SEQ ID NOs: 31 and 50;
and wherein the second antigen-binding domain comprises: a V_{H} comprising the amino acid sequence of any one of SEQ ID NOs: 69 and 85, or a variant thereof having at least about 80% sequence identity to the amino acid sequence of any one of SEQ ID NOs: 69 and 85; and a V_{L} comprising the amino acid sequence of any one of SEQ ID NOs: 79 and 95, or a variant thereof having at least about 80% sequence identity to the amino acid sequence of any one of SEQ ID NOs: 79 and 95;
f) wherein the first antigen-binding domain comprises: a V_{H} comprising the amino acid sequence of SEQ ID NO: 164, or a variant thereof having at least about 80% sequence identity to the amino acid sequence of SEQ ID NO: 164; and a V_{L} comprising the amino acid sequence of SEQ ID NO: 168, or a variant thereof having at least about 80% sequence identity to the amino acid sequence of SEQ ID NO: 168;
and wherein the second antigen-binding domain comprises: a V_{H} comprising the amino acid sequence of SEQ ID NO: 163, or a variant thereof having at least about 80% sequence identity to the amino acid sequence of SEQ ID NO: 163; and a V_{L} comprising the amino acid sequence of SEQ ID NO: 167, or a variant thereof having at least about 80% sequence identity to the amino acid sequence of SEQ ID NO: 167; or
g) wherein the first antigen-binding domain comprises: a V_{H} comprising the amino acid sequence of SEQ ID NO: 166, or a variant thereof having at least about 80% sequence identity to the amino acid sequence of SEQ ID NO: 166; and a V_{L} comprising the amino acid sequence of SEQ ID NO: 170, or a variant thereof having at least about 80% sequence identity to the amino acid sequence of SEQ ID NO: 170;
and wherein the second antigen-binding domain comprises: a V_{H} comprising the amino acid sequence of SEQ ID NO: 165, or a variant thereof having at least about 80% sequence identity to the amino acid sequence of SEQ ID NO: 165; and a V_{L} comprising the amino acid sequence of SEQ ID NO: 169, or a variant thereof having at least about 80% sequence identity to the amino acid sequence of SEQ ID NO: 169.

19. The bispecific antibody of any one of claims 12-18, which comprises an Fc region, wherein the Fc region is selected from the group consisting of an Fc region of an IgGl, IgG2, IgG3, IgG4, IgA, IgM, IgE, and IgD.

20. The bispecific antibody of claim 19, wherein the Fc region comprises a variant Fc region.

21. The bispecific antibody of claim 20, wherein the variant Fc region comprises an substitution at one or more of positions 228, 239, 282, 289, 297, 312, 324, 330, 335, 337, 339, 356, 359, 361, 383, 384, 398, 400, 440, 422, and 442, as numbered by the EU index.

22. The bispecific antibody of any one of claims 19-21, wherein the Fc region is aglycosylated.

23. The bispecific antibody of any one of claims 19-21, wherein the Fc region is deglycosylated.

24. The bispecific antibody of any one of claims 19-23, wherein the Fc region has reduced fucosylation or is afucosylated.

25. The bispecific antibody of any one of claims 12-24, wherein the bispecific antibody has a format selected from the group consisting of DVD-IgG, Bs4Ab, Hetero H, CrossMab, IgG-(scFv)2, and scFv-Fab IgG.

26. The bispecific antibody of any one of claims 12-25, comprising four polypeptide chains:
wherein two of the polypeptide chains comprise V_{H}1-L-V_{H}2-C_{H}1 structure from the N-terminal to the C-terminal, wherein V_{H}1 is a heavy chain variable domain specifically binding to TRAIL, V_{H}2 is a heavy chain variable domain specifically binding to FasL; L is a linker; C_{H}1 is a heavy chain constant domain 1; wherein the polypeptide chain further comprises Fc that comprises C_{H}2 and C_{H}3 domains; and
the other two of the polypeptide chains comprise the structure of V_{L}1-L-V_{L}2-CL, wherein V_{L}1 is a light chain variable domain specifically binding to TRAIL; V_{L}2 is a light chain variable domain specifically binding to FasL; L is a linker; C_{L} is a light chain constant domain.

27. The bispecific antibody of any one of claims 12-25, comprising four polypeptide chains:
wherein two of the polypeptide chains comprise V_{H}1-L-V_{H}2-C_{H}1 structure from the N-terminal to the C-terminal, wherein V_{H}1 is a heavy chain variable domain specifically binding to FasL, V_{H}2 is a heavy chain variable domain specifically binding to TRAIL; L is a linker; C_{H}1 is a heavy chain constant domain 1; wherein the polypeptide chain further comprises Fc that comprises C_{H}2 and C_{H}3 domains; and
the other two of the polypeptide chains comprise the structure of V_{L}1-L-V_{L}2-C_{L}, wherein V_{L}1 is a light chain variable domain specifically binding to FasL; V_{L}2 is a light chain variable domain specifically binding to TRAIL; L is a linker; C_{L} is a light chain constant domain.

28. The bispecific antibody of any one of claims 26-27, wherein the bispecific antibody comprises:
a) the amino acid sequence of SEQ ID NO: 104, or a variant thereof having at least about 80% sequence identity to the amino acid sequence of SEQ ID NO: 104; and/or the amino acid sequence of SEQ ID NO: 105, or a variant thereof having at least about 80% sequence identity to the amino acid sequence of SEQ ID NO: 105;
b) the amino acid sequence of SEQ ID NO: 104, or a variant thereof having at least about 80% sequence identity to the amino acid sequence of SEQ ID NO: 104; and/or the amino acid sequence of SEQ ID NO: 106, or a variant thereof having at least about 80% sequence identity to the amino acid sequence of SEQ ID NO: 106;
c) the amino acid sequence of SEQ ID NO: 107, or a variant thereof having at least about 80% sequence identity to the amino acid sequence of SEQ ID NO: 107; and/or the amino acid sequence of SEQ ID NO: 108, or a variant thereof having at least about 80% sequence identity to the amino acid sequence of SEQ ID NO: 108;
d) the amino acid sequence of SEQ ID NO: 107, or a variant thereof having at least about 80% sequence identity to the amino acid sequence of SEQ ID NO: 107; and/or the amino acid sequence of SEQ ID NO: 109, or a variant thereof having at least about 80% sequence identity to the amino acid sequence of SEQ ID NO: 109;
e) the amino acid sequence of SEQ ID NO: 110, or a variant thereof having at least about 80% sequence identity to the amino acid sequence of SEQ ID NO: 110; and/or the amino acid sequence of SEQ ID NO: 111, or a variant thereof having at least about 80% sequence identity to the amino acid sequence of SEQ ID NO: 111; or
f) the amino acid sequence of SEQ ID NO: 172, or a variant thereof having at least about 80% sequence identity to the amino acid sequence of SEQ ID NO: 172; and/or the amino acid sequence of SEQ ID NO: 174, or a variant thereof having at least about 80% sequence identity to the amino acid sequence of SEQ ID NO: 174.

29. The bispecific antibody of any one of claims 26-27, wherein the bispecific antibody comprises:
a) the amino acid sequence of SEQ ID NO: 131, or a variant thereof having at least about 80% sequence identity to the amino acid sequence of SEQ ID NO: 131; and/or
the amino acid sequence of SEQ ID NO: 105, or a variant thereof having at least about 80% sequence identity to the amino acid sequence of SEQ ID NO: 105;
b) the amino acid sequence of SEQ ID NO: 131, or a variant thereof having at least about 80% sequence identity to the amino acid sequence of SEQ ID NO: 131; and/or
the amino acid sequence of SEQ ID NO: 106, or a variant thereof having at least about 80% sequence identity to the amino acid sequence of SEQ ID NO: 106;
c) the amino acid sequence of SEQ ID NO: 132, or a variant thereof having at least about 80% sequence identity to the amino acid sequence of SEQ ID NO: 132; and/or
the amino acid sequence of SEQ ID NO: 108, or a variant thereof having at least about 80% sequence identity to the amino acid sequence of SEQ ID NO: 108;
d) the amino acid sequence of SEQ ID NO: 132, or a variant thereof having at least about 80% sequence identity to the amino acid sequence of SEQ ID NO: 132; and/or
the amino acid sequence of SEQ ID NO: 109, or a variant thereof having at least about 80% sequence identity to the amino acid sequence of SEQ ID NO: 109;
e) the amino acid sequence of SEQ ID NO: 133, or a variant thereof having at least about 80% sequence identity to the amino acid sequence of SEQ ID NO: 133; and/or
the amino acid sequence of SEQ ID NO: 111, or a variant thereof having at least about 80% sequence identity to the amino acid sequence of SEQ ID NO: 111; or
f) the amino acid sequence of SEQ ID NO: 176, or a variant thereof having at least about 80% sequence identity to the amino acid sequence of SEQ ID NO: 176; and/or
the amino acid sequence of SEQ ID NO: 174, or a variant thereof having at least about 80% sequence identity to the amino acid sequence of SEQ ID NO: 174.

30. The bispecific antibody of any one of claims 12-25, comprising four polypeptide chains:
wherein two of the polypeptide chains comprise V_{H}1-C_{H}1-L1-V_{H}2-L3-V_{L}2 or V_{H}1-C_{H}1-L1-V_{L}2-L3-V_{H}2 structure from the N-terminal to the C-terminal, wherein V_{H}1 is a heavy chain variable domain specifically binding to TRAIL; V_{H}2 is a heavy chain variable domain specifically binding to FasL, V_{L}2 is a light chain variable domain specifically binding to FasL; L1 and L3 are the linker; C_{H}1 is a heavy chain constant domain 1; wherein the polypeptide chain further comprises Fc that comprises C_{H}2 and C_{H}3 domains; and
the other two of the polypeptide chains comprise V_{L}1-C_{L} structure from the N-terminal to the C-terminal, wherein V_{L}1 is a light chain variable domain specifically binding to TRAIL, C_{L} is a light chain constant domain.

31. The bispecific antibody of any one of claims 12-25, comprising four polypeptide chains:
wherein two of the polypeptide chains comprise V_{H}1-C_{H}1-L1-V_{H}2-L3-V_{L}2 or V_{H}1-C_{H}1-L1-V_{L}2-L3-V_{H}2 structure from the N-terminal to the C-terminal, wherein V_{H}1 is a heavy chain variable domain specifically binding to FasL; V_{H}2 is a heavy chain variable domain specifically binding to TRAIL, V_{L}2 is a light chain variable domain specifically binding to TRAIL; L1 and L3 are a linker; C_{H}1 is a heavy chain constant domain 1; wherein the polypeptide chain further comprises Fc that comprises C_{H}2 and C_{H}3 domains; and
the other two of the polypeptide chains comprise V_{L}1-C_{L} structure from the N-terminal to the C-terminal, wherein V_{L}1 is a light chain variable domain specifically binding to FasL, C_{L} is a light chain constant domain.

32. The bispecific antibody of any one of claims 30-31, wherein the bispecific antibody comprises:
a) the amino acid sequence of SEQ ID NO: 112, or a variant thereof having at least about 80% sequence identity to the amino acid sequence of SEQ ID NO: 112; and/or the amino acid sequence of SEQ ID NO: 113, or a variant thereof having at least about 80% sequence identity to the amino acid sequence of SEQ ID NO: 113;
b) the amino acid sequence of SEQ ID NO: 114, or a variant thereof having at least about 80% sequence identity to the amino acid sequence of SEQ ID NO: 114; and/or the amino acid sequence of SEQ ID NO: 115, or a variant thereof having at least about 80% sequence identity to the amino acid sequence of SEQ ID NO: 115;
c) the amino acid sequence of SEQ ID NO: 116, or a variant thereof having at least about 80% sequence identity to the amino acid sequence of SEQ ID NO: 116; and/or the amino acid sequence of SEQ ID NO: 119, or a variant thereof having at least about 80% sequence identity to the amino acid sequence of SEQ ID NO: 119;
d) the amino acid sequence of SEQ ID NO: 117, or a variant thereof having at least about 80% sequence identity to the amino acid sequence of SEQ ID NO: 117; and/or the amino acid sequence of SEQ ID NO: 119, or a variant thereof having at least about 80% sequence identity to the amino acid sequence of SEQ ID NO: 119;
e) the amino acid sequence of SEQ ID NO: 118, or a variant thereof having at least about 80% sequence identity to the amino acid sequence of SEQ ID NO: 118; and/or the amino acid sequence of SEQ ID NO: 119, or a variant thereof having at least about 80% sequence identity to the amino acid sequence of SEQ ID NO: 119;
f) the amino acid sequence of SEQ ID NO: 120, or a variant thereof having at least about 80% sequence identity to the amino acid sequence of SEQ ID NO: 120; and/or the amino acid sequence of SEQ ID NO: 121, or a variant thereof having at least about 80% sequence identity to the amino acid sequence of SEQ ID NO: 121;
g) the amino acid sequence of SEQ ID NO: 120, or a variant thereof having at least about 80% sequence identity to the amino acid sequence of SEQ ID NO: 120; and/or the amino acid sequence of SEQ ID NO: 122, or a variant thereof having at least about 80% sequence identity to the amino acid sequence of SEQ ID NO: 122;
h) the amino acid sequence of SEQ ID NO: 123, or a variant thereof having at least about 80% sequence identity to the amino acid sequence of SEQ ID NO: 123; and/or the amino acid sequence of SEQ ID NO: 124, or a variant thereof having at least about 80% sequence identity to the amino acid sequence of SEQ ID NO: 124; or
i) the amino acid sequence of SEQ ID NO: 171, or a variant thereof having at least about 80% sequence identity to the amino acid sequence of SEQ ID NO: 171; and/or the amino acid sequence of SEQ ID NO: 173, or a variant thereof having at least about 80% sequence identity to the amino acid sequence of SEQ ID NO: 173.

33. The bispecific antibody of any one of claims 30-31, wherein the bispecific antibody comprises:
a) the amino acid sequence of SEQ ID NO: 134, or a variant thereof having at least about 80% sequence identity to the amino acid sequence of SEQ ID NO: 134; and/or the amino acid sequence of SEQ ID NO: 113, or a variant thereof having at least about 80% sequence identity to the amino acid sequence of SEQ ID NO: 113;
b) the amino acid sequence of SEQ ID NO: 135, or a variant thereof having at least about 80% sequence identity to the amino acid sequence of SEQ ID NO: 135; and/or the amino acid sequence of SEQ ID NO: 113, or a variant thereof having at least about 80% sequence identity to the amino acid sequence of SEQ ID NO: 113;
c) the amino acid sequence of SEQ ID NO: 136, or a variant thereof having at least about 80% sequence identity to the amino acid sequence of SEQ ID NO: 136; and/or the amino acid sequence of SEQ ID NO: 115, or a variant thereof having at least about 80% sequence identity to the amino acid sequence of SEQ ID NO: 115;
d) the amino acid sequence of SEQ ID NO: 137, or a variant thereof having at least about 80% sequence identity to the amino acid sequence of SEQ ID NO: 137; and/or the amino acid sequence of SEQ ID NO: 115, or a variant thereof having at least about 80% sequence identity to the amino acid sequence of SEQ ID NO: 115;
e) the amino acid sequence of SEQ ID NO: 138, or a variant thereof having at least about 80% sequence identity to the amino acid sequence of SEQ ID NO: 138; and/or the amino acid sequence of SEQ ID NO: 119, or a variant thereof having at least about 80% sequence identity to the amino acid sequence of SEQ ID NO: 119;
f) the amino acid sequence of SEQ ID NO: 139, or a variant thereof having at least about 80% sequence identity to the amino acid sequence of SEQ ID NO: 139; and/or the amino acid sequence of SEQ ID NO: 119, or a variant thereof having at least about 80% sequence identity to the amino acid sequence of SEQ ID NO: 119;
g) the amino acid sequence of SEQ ID NO: 140, or a variant thereof having at least about 80% sequence identity to the amino acid sequence of SEQ ID NO: 140; and/or the amino acid sequence of SEQ ID NO: 119, or a variant thereof having at least about 80% sequence identity to the amino acid sequence of SEQ ID NO: 119;
h) the amino acid sequence of SEQ ID NO: 141, or a variant thereof having at least about 80% sequence identity to the amino acid sequence of SEQ ID NO: 141; and/or the amino acid sequence of SEQ ID NO: 121, or a variant thereof having at least about 80% sequence identity to the amino acid sequence of SEQ ID NO: 121;
i) the amino acid sequence of SEQ ID NO: 141, or a variant thereof having at least about 80% sequence identity to the amino acid sequence of SEQ ID NO: 141; and/or the amino acid sequence of SEQ ID NO: 122, or a variant thereof having at least about 80% sequence identity to the amino acid sequence of SEQ ID NO: 122;
j) the amino acid sequence of SEQ ID NO: 142, or a variant thereof having at least about 80% sequence identity to the amino acid sequence of SEQ ID NO: 142; and/or the amino acid sequence of SEQ ID NO: 124, or a variant thereof having at least about 80% sequence identity to the amino acid sequence of SEQ ID NO: 124; or
k) the amino acid sequence of SEQ ID NO: 175, or a variant thereof having at least about 80% sequence identity to the amino acid sequence of SEQ ID NO: 175; and/or the amino acid sequence of SEQ ID NO: 173, or a variant thereof having at least about 80% sequence identity to the amino acid sequence of SEQ ID NO: 173.

34. The bispecific antibody of any one of claims 12-25, comprising four polypeptide chains:
wherein one of the polypeptide chains contains V_{H}1-C_{H}1 structure from the N-terminal to the C-terminal, wherein V_{H}1 is a heavy chain variable domain specifically binding to TRAIL, C_{H}1 is a heavy chain constant domain 1; wherein the polypeptide chain further comprises Fc that comprises C_{H}2 and C_{H}3 domains; and
one of the polypeptide chains contains V_{L}1-C_{L} structure from the N-terminal to the C-terminal, wherein V_{L}1 is a light chain variable domain specifically binding to TRAIL, C_{L} is a light chain constant domain; and
one of the polypeptide chains contains V_{H}2-C_{L} structure from the N-terminal to the C-terminal, wherein V_{H}2 is a heavy chain variable domain specifically binding to FasL, C_{L} is a light chain constant domain; wherein the polypeptide chain further comprises Fc that comprises C_{H}2 and C_{H}3 domains; and
one of the polypeptide chains contains V_{L}2-C_{H}1 structure from the N-terminal to the C-terminal, wherein V_{L}2 is a light chain variable domain specifically binding to FasL, C_{H}1 is a heavy chain constant domain 1.

35. The bispecific antibody of any one of claims 12-25, comprising four polypeptide chains:
wherein one of the polypeptide chains contains V_{H}1-C_{H}1 structure from the N-terminal to the C-terminal, wherein V_{H}1 is a heavy chain variable domain specifically binding to FasL, C_{H}1 is a heavy chain constant domain 1; wherein the polypeptide chain further comprises Fc that comprises C_{H}2 and C_{H}3 domains; and
one of the polypeptide chains contains V_{L}1-C_{L} structure from the N-terminal to the C-terminal, wherein V_{L}1 is a light chain variable domain specifically binding to FasL, C_{L} is a light chain constant domain; and
one of the polypeptide chains contains V_{H}2-C_{L} structure from the N-terminal to the C-terminal, wherein V_{H}2 is a heavy chain variable domain specifically binding to TRAIL, C_{L} is a light chain constant domain; wherein the polypeptide chain further comprises Fc that comprises C_{H}2 and C_{H}3 domains; and
one of the polypeptide chains contains V_{L}2-C_{H}1 structure from the N-terminal to the C-terminal, wherein V_{L}2 is a light chain variable domain specifically binding to TRAIL, C_{H}1 is a heavy chain constant domain 1.

36. The bispecific antibody of any one of claims 34-35, wherein the bispecific antibody comprises:
a) the amino acid sequence of SEQ ID NO: 125, or a variant thereof having at least about 80% sequence identity to the amino acid sequence of SEQ ID NO: 125; and/or
the amino acid sequence of SEQ ID NO: 126, or a variant thereof having at least about 80% sequence identity to the amino acid sequence of SEQ ID NO: 126; and/or
the amino acid sequence of SEQ ID NO: 127, or a variant thereof having at least about 80% sequence identity to the amino acid sequence of SEQ ID NO: 127; and/or
the amino acid sequence of SEQ ID NO: 129 or a variant thereof having at least about 80% sequence identity to the amino acid sequence of SEQ ID NO: 129; or
b) the amino acid sequence of SEQ ID NO: 125, or a variant thereof having at least about 80% sequence identity to the amino acid sequence of SEQ ID NO: 125; and/or
the amino acid sequence of SEQ ID NO: 130, or a variant thereof having at least about 80% sequence identity to the amino acid sequence of SEQ ID NO: 130; and/or
the amino acid sequence of SEQ ID NO: 128, or a variant thereof having at least about 80% sequence identity to the amino acid sequence of SEQ ID NO: 128; and/or
the amino acid sequence of SEQ ID NO: 129 or a variant thereof having at least about 80% sequence identity to the amino acid sequence of SEQ ID NO: 129.

37. The bispecific antibody of any one of claims 34-35, wherein the bispecific antibody comprises:
a) the amino acid sequence of SEQ ID NO: 143, or a variant thereof having at least about 80% sequence identity to the amino acid sequence of SEQ ID NO: 143; and/or
the amino acid sequence of SEQ ID NO: 126, or a variant thereof having at least about 80% sequence identity to the amino acid sequence of SEQ ID NO: 126; and/or
the amino acid sequence of SEQ ID NO: 144, or a variant thereof having at least about 80% sequence identity to the amino acid sequence of SEQ ID NO: 144; and/or
the amino acid sequence of SEQ ID NO: 129 or a variant thereof having at least about 80% sequence identity to the amino acid sequence of SEQ ID NO: 129; or
b) the amino acid sequence of SEQ ID NO: 145, or a variant thereof having at least about 80% sequence identity to the amino acid sequence of SEQ ID NO: 145; and/or
the amino acid sequence of SEQ ID NO: 130, or a variant thereof having at least about 80% sequence identity to the amino acid sequence of SEQ ID NO: 130; and/or
the amino acid sequence of SEQ ID NO: 146, or a variant thereof having at least about 80% sequence identity to the amino acid sequence of SEQ ID NO: 146; and/or
the amino acid sequence of SEQ ID NO: 129 or a variant thereof having at least about 80% sequence identity to the amino acid sequence of SEQ ID NO: 129.

38. A nucleic acid molecule that encodes the antibody or antigen-binding fragment specifically binding to TRAIL of any one of claims 1-11 or the bispecific antibody of any one of claims 12-37.

39. A vector comprising the nucleic acid molecule of claim 38.

40. An isolated host cell comprising the antibody or antigen-binding fragment specifically binding to TRAIL of any one of claims 1-11 or the bispecific antibody of any one of claims 12-37, the nucleic acid molecule of claim 38, or the vector of claim 39.

41. A method of producing the antibody or antigen-binding fragment specifically binding to TRAIL of any one of claims 1-11 or the bispecific antibody of any one of claims 12-37, comprising:
a) culturing the host cell of claim 40 under conditions effective to express antibody; and
b) obtaining the expressed antibody from the host cell.

42. A pharmaceutical composition comprising the antibody or antigen-binding fragment specifically binding to TRAIL of any one of claims 1-11, the bispecific antibody of any one of claims 12-37, the nucleic acid molecule of claim 38, the vector of claim 39, the isolated host cell of claim 40 or the antibody produced according to the method of claim 41, and a pharmaceutically acceptable carrier or excipient.

43. A method of treating a disease or condition in an individual in need thereof, comprising administering to the individual an effective amount of the antibody or antigen-binding fragment specifically binding to TRAIL of any one of claims 1-11, the bispecific antibody of any one of claims 12-37, the nucleic acid molecule of claim 38, the vector of claim 39, the isolated host cell of claim 40, the antibody produced according to the method of claim 41 or the pharmaceutical composition of claim 42.

44. A pharmaceutical composition, comprising an antibody or antigen-binding fragment specifically binding to TRAIL and an antibody or antigen-binding fragment specifically binding to FasL, wherein the antibody or antigen-binding fragment specifically binding to TRAIL comprises:
a) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or
b) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

45. A method of treating a disease or condition in an individual in need thereof, comprising administering to the individual an effective amount of an antibody or antigen-binding fragment specifically binding to TRAIL and an antibody or antigen-binding fragment specifically binding to FasL, or a pharmaceutical composition comprising an antibody or antigen-binding fragment specifically binding to TRAIL and an antibody or antigen-binding fragment specifically binding to FasL, wherein the antibody or antigen-binding fragment specifically binding to TRAIL comprises:
a) a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs; or
b) a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs.

46. The pharmaceutical composition of claim 44, or the method of claim 45, wherein the antibody or antigen-binding fragment specifically binding to TRAIL comprises:
(i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22;
(ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23;
(iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24;
(iv) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28;
(v) a V_{H} comprising the amino acid sequence of SEQ ID NO: 20, or a variant thereof having at least about 80% sequence identity to the amino acid sequence of SEQ ID NO: 20; and a V_{L} comprising the amino acid sequence of SEQ ID NO: 30, or a variant thereof having at least about 80% sequence identity to the amino acid sequence of SEQ ID NO: 30; or
(vi) a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31.

47. The pharmaceutical composition of claim 44 or 46, or the method of claim 45 or 46, wherein the antibody or antigen-binding fragment specifically binding to FasL comprises:
a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or
b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

48. The pharmaceutical composition of any one of claims 44 or 46-47, or the method of any one of claims 45-47, wherein the antibody or antigen-binding fragment specifically binding to FasL comprises:
(i) a V_{H} comprising the amino acid sequence of SEQ ID NO: 63, or a variant thereof having at least about 80% sequence identity to the amino acid sequence of SEQ ID NO:63; and a V_{L} comprising the amino acid sequence of SEQ ID NO: 73, or a variant thereof having at least about 80% sequence identity to the amino acid sequence of SEQ ID NO: 73;
(ii) a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74;
(iii) a V_{H} comprising the amino acid sequence of SEQ ID NO:66, or a variant thereof having at least about 80% sequence identity to the amino acid sequence of SEQ ID NO: 66; and a V_{L} comprising the amino acid sequence of SEQ ID NO: 76, or a variant thereof having at least about 80% sequence identity to the amino acid sequence of SEQ ID NO: 76;
(iv) V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78; or
(v) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 79, or a variant thereof having at least about 80% sequence identity to the amino acid sequence of SEQ ID NO: 79.

49. A pharmaceutical composition, comprising an antibody or antigen-binding fragment specifically binding to TRAIL and an antibody or antigen-binding fragment specifically binding to FasL, wherein the antibody or antigen-binding fragment specifically binding to FasL comprises:
a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or
b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

50. A method of treating a disease or condition in an individual in need thereof, comprising administering to the individual an effective amount of an antibody or antigen-binding fragment specifically binding to TRAIL and an antibody or antigen-binding fragment specifically binding to FasL, or a pharmaceutical composition comprising an antibody or antigen-binding fragment specifically binding to TRAIL and an antibody or antigen-binding fragment specifically binding to FasL, wherein the antibody or antigen-binding fragment specifically binding to FasL comprises:
a) a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs; or
b) a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

51. The pharmaceutical composition of claim 49, or the method of claim 50, wherein the antibody or antigen-binding fragment specifically binding to FasL comprises:
(i) a V_{H} comprising the amino acid sequence of SEQ ID NO: 63, or a variant thereof having at least about 80% sequence identity to the amino acid sequence of SEQ ID NO:63; and a V_{L} comprising the amino acid sequence of SEQ ID NO: 73, or a variant thereof having at least about 80% sequence identity to the amino acid sequence of SEQ ID NO: 73;
(ii) a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74;
(iii) a V_{H} comprising the amino acid sequence of SEQ ID NO:66, or a variant thereof having at least about 80% sequence identity to the amino acid sequence of SEQ ID NO: 66; and a V_{L} comprising the amino acid sequence of SEQ ID NO: 76, or a variant thereof having at least about 80% sequence identity to the amino acid sequence of SEQ ID NO: 76;
(iv) V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78;
(v) a V_{H} comprising the amino acid sequence of SEQ ID NO: 69, or a variant thereof having at least about 80% sequence identity to the amino acid sequence of SEQ ID NO: 69; and a V_{L} comprising the amino acid sequence of SEQ ID NO: 79, or a variant thereof having at least about 80% sequence identity to the amino acid sequence of SEQ ID NO: 79.

52. The pharmaceutical composition of any one of claims 44, 47 and 49, the method of any one of claims 45, 47 and 50, or the use of any one of claims 46-47, 49-50, 56-57,
a) wherein the antibody or antigen-binding fragment specifically binding to TRAIL comprises a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs;
and wherein the antibody or antigen-binding fragment specifically binding to FasL comprises a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs;
b) wherein the antibody or antigen-binding fragment specifically binding to TRAIL comprises a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises a V_{H} comprising an HC-CDR1 comprising the amino acid sequence of SEQ ID NO: 51, an HC-CDR2 comprising the amino acid sequence of SEQ ID NO: 53, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 57, an LC-CDR2 comprising the amino acid sequence of SEQ ID NO: 59, and an LC-CDR3 comprising the amino acid sequence of SEQ ID NO: 61, or a variant thereof comprising up to about 5 amino acid substitutions in the LC-CDRs;
c) wherein the antibody or antigen-binding fragment specifically binding to TRAIL comprises a V_{H} comprising an HC-CDR1 comprising the amino acid sequence of SEQ ID NO: 1, an HC-CDR2 comprising the amino acid sequence of SEQ ID NO: 3, and an HC-CDR3 comprising the amino acid sequence of SEQ ID NO: 5, or a variant thereof comprising up to about 5 amino acid substitutions in the HC-CDRs; and a V_{L} comprising an LC-CDR1 comprising the amino acid sequence of SEQ ID NO: 7, an LC-CDR2 comprising the amino acid sequence of SEQ ID NO: 9, and an LC-CDR3 comprising the amino acid sequence of SEQ ID NO: 11, or a variant thereof comprising up to about 5 amino acid substitutions in the LC-CDRs;
and wherein the antibody or antigen-binding fragment specifically binding to FasL comprises a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs; or
d) wherein the antibody or antigen-binding fragment specifically binding to TRAIL comprises a V_{H} comprising an HC-CDR1 comprising the amino acid sequence of SEQ ID NO: 2, an HC-CDR2 comprising the amino acid sequence of SEQ ID NO: 4, and an HC-CDR3 comprising the amino acid sequence of SEQ ID NO: 6, or a variant thereof comprising up to about 5 amino acid substitutions in the HC-CDRs; and a V_{L} comprising an LC-CDR1 comprising the amino acid sequence of SEQ ID NO: 8, an LC-CDR2 comprising the amino acid sequence of SEQ ID NO: 10, and an LC-CDR3 comprising the amino acid sequence of SEQ ID NO: 12, or a variant thereof comprising up to about 5 amino acid substitutions in the LC-CDRs; and wherein the antibody or antigen-binding fragment specifically binding to FasL comprises a V_{H} comprising an HC-CDR1 comprising the amino acid sequence of SEQ ID NO: 52, an HC-CDR2 comprising the amino acid sequence of SEQ ID NO: 54, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 60, and an LC-CDR3 comprising the amino acid sequence of SEQ ID NO: 62, or a variant thereof comprising up to about 5 amino acid substitutions in the LC-CDRs.

53. The pharmaceutical composition of any one of claims 44,46-49 and 51, the method of any one of claims 45-48 and 50-51,
a) wherein the antibody or antigen-binding fragment specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 23, or a variant thereof having at least about 80% sequence identity to the amino acid sequence of SEQ ID NO: 23;
and wherein the antibody or antigen-binding fragment specifically binding to FasL comprises a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74;
b) wherein the antibody or antigen-binding fragment specifically binding to TRAIL comprises a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 24, or a variant thereof having at least about 80% sequence identity to the amino acid sequence of SEQ ID NO: 24; and wherein the antibody or antigen-binding fragment specifically binding to FasL comprises: a V_{H} comprising the amino acid sequence of SEQ ID NO: 64, or a variant thereof having at least about 80% sequence identity to the amino acid sequence of SEQ ID NO:64; and a V_{L} comprising the amino acid sequence of SEQ ID NO: 74, or a variant thereof having at least about 80% sequence identity to the amino acid sequence of SEQ ID NO: 74;
c) wherein the antibody or antigen-binding fragment specifically binding to TRAIL comprises a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 28, or a variant thereof having at least about 80% sequence identity to the amino acid sequence of SEQ ID NO: 28; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76;
d) wherein the antibody or antigen-binding fragment specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 66, or a variant thereof having at least about 80% sequence identity to the amino acid sequence of SEQ ID NO:66; and a V_{L} comprising the amino acid sequence of SEQ ID NO: 76, or a variant thereof having at least about 80% sequence identity to the amino acid sequence of SEQ ID NO: 76;
e) wherein the antibody or antigen-binding fragment specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 70, or a variant thereof having at least about 80% sequence identity to the amino acid sequence of SEQ ID NO: 70; and a V_{L} comprising the amino acid sequence of SEQ ID NO: 78, or a variant thereof having at least about 80% sequence identity to the amino acid sequence of SEQ ID NO: 78;
f) wherein the antibody or antigen-binding fragment specifically binding to TRAIL comprises a V_{H} comprising the amino acid sequence of SEQ ID NO: 16, or a variant thereof having at least about 80% sequence identity to the amino acid sequence of SEQ ID NO: 16; and a V_{L} comprising the amino acid sequence of SEQ ID NO: 31, or a variant thereof having at least about 80% sequence identity to the amino acid sequence of SEQ ID NO: 31; and wherein the antibody or antigen-binding fragment specifically binding to FasL comprises: a V_{H} comprising the amino acid sequence of SEQ ID NO: 69, or a variant thereof having at least about 80% sequence identity to the amino acid sequence of SEQ ID NO:69; and a V_{L} comprising the amino acid sequence of SEQ ID NO: 79, or a variant thereof having at least about 80% sequence identity to the amino acid sequence of SEQ ID NO: 79.

54. The method of any one of claims 45-48 and 50-53, wherein the antibody or antigen-binding fragment specifically binding to TRAIL and the antibody or antigen-binding fragment specifically binding to FasL are administered concurrently or consecutively to an individual.

55. The method of any one of claims 43, 45-48 and 50-53, wherein the disease or condition is inflammatory diseases, autoimmune diseases, transplant-related diseases, liver diseases, neurodegenerative disorders or cancer associated with TRAIL and/or FasL signaling pathway.

56. The method or the use of claim 55, wherein the disease or condition is selected from the group consisting of transplant rejection, graft-versus-host disorders, liver injury, pulmonary arterial hypertension, Alzheimer's disease, acute lung injury, acute respiratory distress syndrome, myocardial infarction, cardiomyopathy, ischemic reperfusion injury, diabetes, brain injury, spinal cord injury, acute viral hepatitis B, acute viral hepatitis C, chronic hepatitis C, chronic Hepatitis B, alcoholic hepatitis, non-alcoholic steatohepatitis, cirrhosis, drug-induced liver injury/liver failure, autoimmune hepatitis, chronic kidney disease, acute kidney disease, diabetic kidney disease, and cancer.
